(19) Europäisches Patentamt
European Patent Office
Office européen des brevets

(11) EP 2 668 287 B1

(12) **EUROPEAN PATENT SPECIFICATION**

(45) Date of publication and mention of the grant of the patent:
**06.09.2017 Bulletin 2017/36**

(21) Application number: **12700864.7**

(22) Date of filing: **25.01.2012**

(51) Int Cl.:
***C12Q 1/68*** *(2006.01)*

(86) International application number:
**PCT/EP2012/051164**

(87) International publication number:
**WO 2012/101183 (02.08.2012 Gazette 2012/31)**

(54) **GENES AND GENES COMBINATIONS BASED ON GENE MKNK1 PREDICTIVE OF EARLY RESPONSE OR NON RESPONSE OF SUBJECTS SUFFERING FROM RHEUMATOID ARTHRITIS TO TNF-ALPHA BLOCKING MONOCLONAL ANTIBODY**

GENE UND GENKOMBINATIONEN AUF DER BASIS DES GENS MKNK1 ZUR VORHERSAGE DES FRÜHEN ANSPRECHENS ODER NICHTANSPRECHENS VON PERSONEN MIT RHEUMATOIDER ARTHRITIS AUF TNF-ALPHA BLOCKIERENDE MONOKLONALE ANTIKÖRPER

GÈNES ET COMBINAISONS DE GÈNES BASÉS SUR LE GÈNE MKNK1 PRÉDICTIFS DE RÉPONSE PRÉCOCE OU DE NON-RÉPONSE DE SUJETS SOUFFRANT DE POLYARTHRITE RHUMATOÏDE AU ANTICORPS MONOCLONAL BLOQUANT LE TNF-ALPHA

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.01.2011 US 201161457191 P**
**25.05.2011 US 201161457743 P**
**19.01.2012 US 201261588390 P**

(43) Date of publication of application:
**04.12.2013 Bulletin 2013/49**

(73) Proprietor: **TC LAND Expression**
**44200 Nantes (FR)**

(72) Inventors:
- **CERVINO, Alessandra**
  **F-44100 Nantes (FR)**
- **POPA-NITA, Oana**
  **F-44200 Nantes (FR)**
- **PLASSAIS, Jonathan**
  **F-44100 Nantes (FR)**

(74) Representative: **Regimbeau**
**20, rue de Chazelles**
**75847 Paris Cedex 17 (FR)**

(56) References cited:
**WO-A1-2010/034864 WO-A2-2008/132176**
**WO-A2-2008/150491**

- **AGILENT TECHNOLOGIES: "Agilent SurePrint G3 Human Catalog CGH Microarrays", INTERNET CITATION, 8 January 2009 (2009-01-08), pages 1-8, XP002660065, Retrieved from the Internet: URL:http://www.chem.agilent.com/Library/brochures/5990-3368en_lo.pdf [retrieved on 2011-09-26]**
- **"Affymetrix Human Genome U133 Plus 2.0 Array", GENE EXPRESSION OMNIBUS, 2003, XP002627319,**
- **ANTONIO JULIÀ ET AL: "An Eight-Gene Blood Expression Profile Predicts the Response to Infliximab in Rheumatoid Arthritis", PLOS ONE, vol. 4, no. 10, 1 January 2009 (2009-01-01), page E7556, XP055015104, ISSN: 1932-6203, DOI: 10.1371/journal.pone.0007556 cited in the application**
- **BIENKOWSKA J R ET AL: "Convergent random forest predictor: Methodology for predicting drug response from genome-scale data applied to anti-TNF response", GENOMICS, ACADEMIC PRESS, SAN DIEGO, US, vol. 94, no. 6, 1 December 2009 (2009-12-01), pages 423-432, XP026754299, ISSN: 0888-7543, DOI: 10.1016/J.YGENO.2009.08.008 [retrieved on 2009-08-20] cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

- LEQUERRÉ THIERRY ET AL: "Gene profiling in white blood cells predicts infliximab responsiveness in rheumatoid arthritis", ARTHRITIS RESEARCH AND THERAPY, BIOMED CENTRAL, LONDON, GB, vol. 8, no. 4, 3 July 2006 (2006-07-03), page R105, XP021020585, ISSN: 1478-6354, DOI: 10.1186/AR1924 cited in the application
- SEKIGUCHI N ET AL: "Messenger ribonucleic acid expression profile in peripheral blood cells from RA patients following treatment with an anti-TNF-alpha monoclonal antibody, infliximab", RHEUMATOLOGY, OXFORD UNIVERSITY PRESS, LONDON, GB, vol. 47, no. 6, 1 June 2008 (2008-06-01), pages 780-788, XP002562468, ISSN: 1462-0324, DOI: 10.1093/RHEUMATOLOGY/KEN083 cited in the application
- TANINO M ET AL: "Prediction of efficacy of anti-TNF biologic agent, infliximab, for rheumatoid arthritis patients using a comprehensive transcriptome analysis of white blood cells", BIOCHEMICAL AND BIOPHYSICAL RESEARCH COMMUNICATIONS, ACADEMIC PRESS INC. ORLANDO, FL, US, vol. 387, no. 2, 18 September 2009 (2009-09-18), pages 261-265, XP026434615, ISSN: 0006-291X, DOI: 10.1016/J.BBRC.2009.06.149 [retrieved on 2009-07-03] cited in the application
- VAN BAARSEN LISA GM ET AL: "Regulation of IFN response gene activity during infliximab treatment in rheumatoid arthritis is associated with clinical response to treatment", ARTHRITIS RESEARCH AND THERAPY, BIOMED CENTRAL, LONDON, GB, vol. 12, no. 1, 22 January 2010 (2010-01-22), page R11, XP021070731, ISSN: 1478-6354 cited in the application
- SPENTZOS D ET AL: "Unique Gene Expression Profile Based On Pathologic Response In Epithelial Ovarian Cancer", JOURNAL OF CLINICAL ONCOLOGY, AMERICAN SOCIETY OF CLINICAL ONCOLOGY, US, vol. 23, no. 31, 1 November 2005 (2005-11-01), pages 7911-7918, XP008064557, ISSN: 0732-183X, DOI: 10.1200/JCO.2005.02.9363
- Lin Feng ET AL: "Power of Deep Sequencing and Agilent Microarray for Gene Expression Profiling Study", MOLECULAR BIOTECHNOLOGY, vol. 45, no. 2, 1 June 2010 (2010-06-01), pages 101-110, XP055264123, US ISSN: 1073-6085, DOI: 10.1007/s12033-010-9249-6
- V Gelsi-Boyer ET AL: "Gene expression profiling separates chronic myelomonocytic leukemia in two molecular subtypes", LEUKEMIA., vol. 21, no. 11, 14 November 2007 (2007-11-14), pages 2359-2362, XP055264130, US ISSN: 0887-6924, DOI: 10.1038/sj.leu.2404805

SEQUENCE MISTING

<110> TC LAND EXPRESSION

<120> Genes and genes combinations based on gene MKNK1 predictive of early response or non response of subjects suffering from inflammatory disease to cytokine targeting drugs (CyTD) or anti-inflammatory biological drugs

<130> 360323D29364

<150> US 61/457,191
<151> 2011-01-25

<150> US 61/588,390
<151> 2012-01-19

<150> US 61/457,743
<151> 2011-05-25

<160> 45

<170> PatentIn version 3.5

<210> 1
<211> 2693
<212> DNA
<213> Homo sapiens

<220>
<221> misc_feature
<223> MKNK1

<400> 1

```
tctctgatcg ggcccacccc ctggatctag caccgcctct tccgcgttct cggaggagcg      60
atctgcaggt aggggtgcgc gcgaccgctc cccggcggga gccagcgaag gtttccatgt     120
cagaggccga tggagaactg aagattgcca cctacgcaca aaggccattg agacacttcg     180
tgtagctgga agacaccaac ttcctgacag gagctttatt tcatttggga tttcaagttt     240
acagatggta tcttctcaaa agttggaaaa acctatagag atgggcagta gcgaacccct     300
tcccatcgca gatggtgaca ggaggaggaa gaagaagcgg aggggccggg ccactgactc     360
cttgccagga aagtttgaag atatgtacaa gctgacctct gaattgcttg gagagggagc     420
ctatgccaaa gttcaaggtg ccgtgagcct acagaatggc aaagagtatg ccgtcaaaat     480
catcgagaaa caagcagggc acagtcggag tagggtgttt cgagaggtgg agacgctgta     540
tcagtgtcag ggaaacaaga acattttgga gctgattgag ttctttgaag atgacacaag     600
gttttacttg gtctttgaga aattgcaagg aggttccatc ttagcccaca tccagaagca     660
aaagcacttc aatgagcgag aagccagccg agtggtgcgg gacgttgctg ctgcccttga     720
cttcctgcat accaaaggca ttgctcatcg tgatctgaaa ccagaaaata tattgtgtga     780
atctccagaa aaggtgtctc cagtgaaaat ctgtgacttt gacttgggca gtgggatgaa     840
```

actgaacaac tcctgtaccc ccataaccac accagagctg accaccccat gtggctctgc 900
agaatacatg gcccctgagg tagtggaggt cttcacggac caggccacat tctacgacaa 960
gcgctgtgac ctgtggagcc tgggcgtggt cctctacatc atgctgagtg gctacccacc 1020
cttcgtgggt cactgcgggg ccgactgtgg ctgggaccgg ggcgaggtct gcagggtgtg 1080
ccagaacaag ctgtttgaaa gcatccagga aggcaagtat gagtttcctg acaaggactg 1140
ggcacacatc tccagtgaag ccaaagacct catctccaag ctcctggtgc gagatgcaaa 1200
gcagagactt agcgccgccc aagttctgca gcacccatgg gtgcaggggc aagctccaga 1260
aaagggactc cccacgccgc aagtcctcca gaggaacagc agcacaatgg acctgacgct 1320
cttcgcagct gaggccatcg cccttaaccg ccagctatct cagcacgaag agaacgaact 1380
agcagaggag ccagaggcac tagctgatgg cctctgctcc atgaagcttt cccctccctg 1440
caagtcacgc ctggcccgga gacgggccct ggcccaggca ggccgtggtg aagacaggag 1500
cccgcccaca gcactctgaa atgctccagt cacaccttat aggccctagg cctggccagg 1560
cattgtcccc tggaaacctg tgtggctaaa gtctgctgag caggcagcag cctctgctct 1620
gtggctccat tcaggctttt tcatctacga aggccctgag gttcccatca acccccattt 1680
ccctagggtc ctggaggaaa aagctttttc caaaggggtt gtctttgaaa aggaaagcaa 1740
tcacttctca ctttgcataa ttgcctgcag caggaacatc tcttcactgg gctccacctg 1800
ctcacccgcc tgcagatctg ggatccagcc tgctctcacc gctgtagctg tggcggctgg 1860
ggctgcagcc tgcagggaga agcaagaagc atcagttgac agaggctgcc gacacgtgcc 1920
tcttccctct cttctctgtc accctcctct ggcggtcctt ccaccttcct ctgtcctccg 1980
gatgtcctct ttgcccgtct tctcccttgg ctgagcaaag ccatcccctc aattcaggga 2040
agggcaagga gccttcctca ttcaggaaat caaatcagtc ttccggtctg cagcacggaa 2100
aagcacataa tctttctttg ctgtgactga aatgtatccc tcgtttatca tcccctttgt 2160
ttgtgattgc tgctaaagtc agtagtatcg tttttttaaa aaaaaagttt ggtgttttta 2220
accatgctgt tccagcaaag atgatacctt aaactcccac tgcaagccca tgaacttccc 2280
agagagtgga acggcttgct cttctttcta gaatgtccat gcacttgggt tttaatcagc 2340
agttccctat tattctgatt ttaagctgtt cctgtgatga acttagagac agcatcggtg 2400
tctgctgctg tgtccccagg tcttgtgtgg gtggcacaga tctgggcagt tagatagtgc 2460
tctgtgccta aggtgaagcc acactagggt gaagcctcac ttccctgttt gagcaatgca 2520
gtgcctgctg cccgtgtgca tgaaggtaca gccattcaga taagtggaac tattgagtta 2580
cataaagaaa atagatttgc atttgtcagg cagacgttta tacaacacca cggtgctttt 2640
atacattgtg cttattttaa taaaactgaa attctaaaaa aaaaaaaaaa aaa 2693

<210> 2
<211> 2816
<212> DNA
<213> Homo sapiens

<220>
<221> misc_feature
<223> MKNK1

<400> 2
tctctgatcg ggcccacccc ctggatctag caccgcctct tccgcgttct cggaggagcg 60

atctgcaggt aggggtgcgc gcgaccgctc cccggcggga gccagcgaag gtttccatgt 120

cagaggccga tggagaactg aagattgcca cctacgcaca aaggccattg agacacttcg 180

tgtagctgga agacaccaac ttcctgacag gagctttatt tcatttggga tttcaagttt 240

acagatggta tcttctcaaa agttggaaaa acctatagag atgggcagta gcgaacccct 300

tcccatcgca gatggtgaca ggaggaggaa gaagaagcgg aggggccggg ccactgactc 360

cttgccagga aagtttgaag atatgtacaa gctgacctct gaattgcttg gagagggagc 420

ctatgccaaa gttcaaggtg ccgtgagcct acagaatggc aaagagtatg ccgtcaaaat 480

catcgagaaa caagcagggc acagtcggag tagggtgttt cgagaggtgg agacgctgta 540

tcagtgtcag ggaaacaaga acattttgga gctgattgag ttctttgaag atgacacaag 600

gttttacttg gtctttgaga aattgcaagg aggttccatc ttagcccaca tccagaagca 660

aaagcacttc aatgagcgag aagccagccg agtggtgcgg gacgttgctg ctgcccttga 720

cttcctgcat accaaagaca aagtctctct ctgtcaccta ggctggagtg ctatggcgcc 780

atcagggctc actgcagccc caacctccct gggctccagt gatcctccca cctcagcctc 840

ccaagtagct gggactacag gcattgctca tcgtgatctg aaaccagaaa atatattgtg 900

tgaatctcca gaaaaggtgt ctccagtgaa aatctgtgac tttgacttgg gcagtgggat 960

gaaactgaac aactcctgta cccccataac cacaccagag ctgaccaccc catgtggctc 1020

tgcagaatac atggcccctg aggtagtgga ggtcttcacg gaccaggcca cattctacga 1080

caagcgctgt gacctgtgga gcctgggcgt ggtcctctac atcatgctga gtggctaccc 1140

acccttcgtg ggtcactgcg gggccgactg tggctgggac cggggcgagg tctgcagggt 1200

gtgccagaac aagctgtttg aaagcatcca ggaaggcaag tatgagtttc ctgacaagga 1260

ctgggcacac atctccagtg aagccaaaga cctcatctcc aagctcctgg tgcgagatgc 1320

aaagcagaga cttagcgccg cccaagttct gcagcaccca tgggtgcagg ggcaagctcc 1380

agaaaaggga ctccccacgc cgcaagtcct ccagaggaac agcagcacaa tggacctgac 1440

gctcttcgca gctgaggcca tcgcccttaa ccgccagcta tctcagcacg aagagaacga 1500

actagcagag gagccagagg cactagctga tggcctctgc tccatgaagc tttcccctcc 1560

```
ctgcaagtca cgcctggccc ggagacgggc cctggcccag gcaggccgtg gtgaagacag     1620
gagcccgccc acagcactct gaaatgctcc agtcacacct tataggccct aggcctggcc     1680
aggcattgtc ccctggaaac ctgtgtggct aaagtctgct gagcaggcag cagcctctgc     1740
tctgtggctc cattcaggct ttttcatcta cgaaggccct gaggttccca tcaacccccca    1800
tttccctagg gtcctggagg aaaaagcttt ttccaaaggg gttgtctttg aaaaggaaag     1860
caatcacttc tcactttgca taattgcctg cagcaggaac atctcttcac tgggctccac     1920
ctgctcaccc gcctgcagat ctgggatcca gcctgctctc accgctgtag ctgtggcggc     1980
tggggctgca gcctgcaggg agaagcaaga agcatcagtt gacagaggct gccgacacgt     2040
gcctcttccc tctcttctct gtcaccctcc tctggcggtc cttccacctt cctctgtcct     2100
ccggatgtcc tctttgcccg tcttctccct tggctgagca aagccatccc ctcaattcag     2160
ggaagggcaa ggagccttcc tcattcagga aatcaaatca gtcttccggt ctgcagcacg     2220
gaaaagcaca taatctttct ttgctgtgac tgaaatgtat ccctcgttta tcatcccctt     2280
tgtttgtgat tgctgctaaa gtcagtagta tcgttttttt aaaaaaaaag tttggtgttt     2340
ttaaccatgc tgttccagca aagatgatac cttaaactcc cactgcaagc ccatgaactt     2400
cccagagagt ggaacggctt gctcttcttt ctagaatgtc catgcacttg ggttttaatc     2460
agcagttccc tattattctg attttaagct gttcctgtga tgaacttaga gacagcatcg     2520
gtgtctgctg ctgtgtcccc aggtcttgtg tgggtggcac agatctgggc agttagatag     2580
tgctctgtgc ctaaggtgaa gccacactag ggtgaagcct cacttccctg tttgagcaat     2640
gcagtgcctg ctgcccgtgt gcatgaaggt acagccattc agataagtgg aactattgag     2700
ttacataaag aaaatagatt tgcatttgtc aggcagacgt ttatacaaca ccacggtgct     2760
tttatacatt gtgcttattt taataaaact gaaattctaa aaaaaaaaaa aaaaaa         2816
```

<210> 3
<211> 2607
<212> DNA
<213> Homo sapiens

<220>
<221> misc_feature
<223> MKNK1

<400> 3

```
tctctgatcg ggcccacccc ctggatctag caccgcctct tccgcgttct cggaggagcg       60
atctgcaggt ttccatgtca gaggccgatg gagaactgaa gattgccacc tacgcacaaa      120
ggccattgag acacttcgtg tagctggaag acaccaactt cctgacagga gctttatttc      180
atttgggatt tcaagtttac agatggtatc ttctcaaaag ttggaaaaac ctatagagat      240
gggcagtagc gaacccccttc ccatcgcaga tggtgacagg aggaggaaga agaagcggag     300
```

gggccgggcc actgactcct tgccaggaaa gtttgaagat atgtacaagc tgacctctga 360
attgcttgga gagggagcct atgccaaagt tcaaggtgcc gtgagcctac agaatggcaa 420
agagtatgcc gtcaaaatca tcgagaaaca agcagggcac agtcggagta gggtgtttcg 480
agaggtggag acgctgtatc agtgtcaggg aaacaagaac attttggagc tgattgagtt 540
ctttgaagat gacacaaggt tttacttggt ctttgagaaa ttgcaaggag gttccatctt 600
agcccacatc cagaagcaaa agcacttcaa tgagcgagaa gccagccgag tggtgcggga 660
cgttgctgct gcccttgact tcctgcatac caaaggcatt gctcatcgtg atctgaaacc 720
agaaaatata ttgtgtgaat ctccagaaaa ggtgtctcca gtgaaaatct gtgactttga 780
cttgggcagt gggatgaaac tgaacaactc ctgtaccccc ataaccacac cagagctgac 840
caccccatgt ggctctgcag aatacatggc ccctgaggta gtggaggtct tcacggacca 900
ggccacattc tacgacaagc gctgtgacct gtggagcctg ggcgtggtcc tctacatcat 960
gctgagtggc tacccaccct tcgtgggtca ctgcggggcc gactgtggct gggaccgggg 1020
cgaggtctgc agggtgtgcc agaacaagct gtttgaaagc atccaggaag gcaagtatga 1080
gtttcctgac aaggactggg cacacatctc cagtgaagcc aaagacctca tctccaagct 1140
cctggtgcga gatgcaaagc agagacttag cgccgcccaa gttctgcagc acccatgggt 1200
gcagggggaa cagcagcaca atggacctga cgctcttcgc agctgaggcc atcgccctta 1260
accgccagct atctcagcac gaagagaacg aactagcaga ggagccagag gcactagctg 1320
atggcctctg ctccatgaag ctttcccctc cctgcaagtc acgcctggcc cggagacggg 1380
ccctggccca ggcaggccgt ggtgaagaca ggagcccgcc cacagcactc tgaaatgctc 1440
cagtcacacc ttataggccc taggcctggc caggcattgt cccctggaaa cctgtgtggc 1500
taaagtctgc tgagcaggca gcagcctctg ctctgtggct ccattcaggc tttttcatct 1560
acgaaggccc tgaggttccc atcaaccccc atttccctag ggtcctggag gaaaaagctt 1620
tttccaaagg ggttgtcttt gaaaaggaaa gcaatcactt ctcactttgc ataattgcct 1680
gcagcaggaa catctcttca ctgggctcca cctgctcacc cgcctgcaga tctgggatcc 1740
agcctgctct caccgctgta gctgtggcgg ctggggctgc agcctgcagg gagaagcaag 1800
aagcatcagt tgacagaggc tgccgacacg tgcctcttcc ctctcttctc tgtcaccctc 1860
ctctggcggt ccttccacct tcctctgtcc tccggatgtc ctctttgccc gtcttctccc 1920
ttggctgagc aaagccatcc cctcaattca gggaagggca aggagccttc ctcattcagg 1980
aaatcaaatc agtcttccgg tctgcagcac ggaaaagcac ataatctttc tttgctgtga 2040
ctgaaatgta tccctcgttt atcatcccct ttgtttgtga ttgctgctaa agtcagtagt 2100
atcgtttttt taaaaaaaaa gtttggtgtt tttaaccatg ctgttccagc aaagatgata 2160

ccttaaactc ccactgcaag cccatgaact tcccagagag tggaacggct tgctcttctt 2220

tctagaatgt ccatgcactt gggttttaat cagcagttcc ctattattct gattttaagc 2280

tgttcctgtg atgaacttag agacagcatc ggtgtctgct gctgtgtccc caggtcttgt 2340

gtgggtggca cagatctggg cagttagata gtgctctgtg cctaaggtga agccacacta 2400

gggtgaagcc tcacttccct gtttgagcaa tgcagtgcct gctgcccgtg tgcatgaagg 2460

tacagccatt cagataagtg gaactattga gttacataaa gaaaatagat ttgcatttgt 2520

caggcagacg tttatacaac accacggtgc ttttatacat tgtgcttatt ttaataaaac 2580

tgaaattcta aaaaaaaaaa aaaaaaa 2607

<210> 4
<211> 2555
<212> DNA
<213> Homo sapiens

<220>
<221> misc_feature
<223> PRF1

<400> 4
gaagtgatgt gagtggtggc tggtgcaagg agccacagtg ggctgcctgg ggggctgatg 60

ccaccattcc aggagcctcg gtgaagagag gatatccatc tgtgtagccg cttctctata 120

cgggattcca gagcccaagt gccccctgtc tctgcagctc catggcagcc cgtctgctcc 180

tcctgggcat ccttctcctg ctgctgcccc tgcccgtccc tgccccgtgc cacacagccg 240

cacgctcaga gtgcaagcgc agccacaagt tcgtgcctgg tgcatggctg gccggggagg 300

gtgtggacgt gaccagcctc cgccgctcgg gctccttccc agtggacaca caaaggttcc 360

tgcggcccga cggcacctgc accctctgtg aaaatgccct acaggagggc accctccagc 420

gcctgcctct ggcgctcacc aactggcggg cccagggctc tggctgccag cgccatgtaa 480

ccagggccaa agtcagctcc actgaagctg tggcccggga tgcggctcgt agcatccgca 540

acgactggaa ggtcgggctg gacgtgactc ctaagcccac cagcaatgtg catgtgtctg 600

tggccggctc acactcacag gcagccaact ttgcagccca gaagacccac caggaccagt 660

acagcttcag cactgacacg gtggagtgcc gcttctacag tttccatgtg gtacacactc 720

cccgctgca ccctgacttc aagagggccc tcggggacct gccccaccac ttcaacgcct 780

ccacccagcc cgcctacctc aggcttatct ccaactacgg cacccacttc atccgggctg 840

tggagctggg tggccgcata tcggccctca ctgccctgcg cacctgcgag ctggccctgg 900

aagggctcac ggacaacgag gtggaggact gcctgactgt cgaggcccag gtcaacatag 960

gcatccacgg cagcatctct gccgaagcca aggcctgtga ggagaagaag aagaagcaca 1020

agatgacggc ctccttccac caaacctacc gggagcgcca ctcggaagtg gttggcggcc 1080

atcacacctc cattaacgac ctgctgttcg ggatccaggc cgggcccgag cagtactcag 1140

cctgggtaaa ctcgctgccc ggcagccctg gcctggtgga ctacaccctg gaacccctgc 1200

acgtgctgct ggacagccag gacccgcggc gggaggcact gaggagggcc ctgagtcagt 1260

acctgacgga cagggctcgc tggagggact gcagccggcc gtgcccacca gggcggcaga 1320

agagcccccg agacccatgc cagtgtgtgt gccatggctc agcggtcacc acccaggact 1380

gctgccctcg gcagaggggc ctggcccagc tggaggtgac cttcatccaa gcatggggcc 1440

tgtgggggga ctggttcact gccacggatg cctatgtgaa gctcttcttt ggtggccagg 1500

agctgaggac gagcaccgtg tgggacaata acaaccccat ctggtcagtg cggctggatt 1560

ttggggatgt gctcctggcc acagggggc ccctgaggtt gcaggtctgg gatcaggact 1620

ctggcaggga cgatgacctc cttggcacct gtgatcaggc tcccaagtct ggttcccatg 1680

aggtgagatg caacctgaat catggccacc taaaattccg ctatcatgcc aggtgcttgc 1740

cccacctggg aggaggcacc tgcctggact atgtccccca aatgcttctg ggggagcctc 1800

caggaaaccg gagtggggcc gtgtggtgag aacagtgagc ttggaaagga ccagtatgct 1860

tggactgaag ggttctcac agtgggagcc agggctgtct tcgtattccc attagaccaa 1920

gcttgtccaa cccgaggccc gcatgcggcc caggatggct ttgaatgcgg cccaacgcaa 1980

attcgcaaac tttcttaaaa cattatgagt ttctttttgc tattttttt tttttttag 2040

ctcatcggct atcgttagtg ctagtggatt ttacatgtgg cccaacacaa ttcttcttcc 2100

aacgtggccc agagaagcca aaagattgga tacgcatcag acagatggaa aagggagatt 2160

cagactgttt ttcagggagg tggctgggtt tacacgctaa tcccgattca ccctgtccaa 2220

actgcctaag ccctccgcca ttctcaagcc ctgcagtcac agctacacag atcacagctt 2280

cagccaggag ctgggcagaa ggccaagagg ctgttcccac caggctgctc agggctggtc 2340

ttttaggacc cttcccttga gccctctatg gtgtggcaaa gccttcattg ccttaactgg 2400

agccccatca gctccagctg ctctgtcttc tttgcccaca atgctttgcc cctgagacaa 2460

atggaggcct gtcctgacct gtctcaccat gtacatagct tgataaaggg ccaataaata 2520

tgatgttatg gtgaaaaaaa aaaaaaaaaa aaaaa 2555

<210> 5
<211> 2529
<212> DNA
<213> Homo sapiens

<220>
<221> misc_feature
<223> PRF1

<400> 5

gaagtgatgt gagtggtggc tggtgcaagg agccacagtg ggctgcctgg ggggctgatg 60
ccaccattcc aggagcctcg gtgaagagag gatatccatc tgtgtagccg cttctctata 120
cgggattcca gctccatggc agcccgtctg ctcctcctgg gcatccttct cctgctgctg 180
cccctgcccg tccctgcccc gtgccacaca gccgcacgct cagagtgcaa gcgcagccac 240
aagttcgtgc ctggtgcatg gctggccggg gagggtgtgg acgtgaccag cctccgccgc 300
tcgggctcct tcccagtgga cacacaaagg ttcctgcggc ccgacggcac ctgcaccctc 360
tgtgaaaatg ccctacagga gggcaccctc cagcgcctgc ctctggcgct caccaactgg 420
cgggcccagg gctctggctg ccagcgccat gtaaccaggg ccaaagtcag ctccactgaa 480
gctgtggccc gggatgcggc tcgtagcatc cgcaacgact ggaaggtcgg gctggacgtg 540
actcctaagc ccaccagcaa tgtgcatgtg tctgtggccg gctcacactc acaggcagcc 600
aactttgcag cccagaagac ccaccaggac cagtacagct tcagcactga cacggtggag 660
tgccgcttct acagtttcca tgtggtacac actcccccgc tgcaccctga cttcaagagg 720
gccctcgggg acctgcccca ccacttcaac gcctccaccc agcccgccta cctcaggctt 780
atctccaact acggcaccca cttcatccgg gctgtggagc tgggtggccg catatcggcc 840
ctcactgccc tgcgcacctg cgagctggcc ctggaagggc tcacggacaa cgaggtggag 900
gactgcctga ctgtcgaggc ccaggtcaac ataggcatcc acggcagcat ctctgccgaa 960
gccaaggcct gtgaggagaa gaagaagaag cacaagatga cggcctcctt ccaccaaacc 1020
taccgggagc gccactcgga agtggttggc ggccatcaca cctccattaa cgacctgctg 1080
ttcgggatcc aggccgggcc cgagcagtac tcagcctggg taaactcgct gcccggcagc 1140
cctggcctgg tggactacac cctggaaccc ctgcacgtgc tgctggacag ccaggacccg 1200
cggcgggagg cactgaggag ggccctgagt cagtacctga cggacagggc tcgctggagg 1260
gactgcagcc ggccgtgccc accagggcgg cagaagagcc cccgagaccc atgccagtgt 1320
gtgtgccatg gctcagcggt caccacccag gactgctgcc ctcggcagag gggcctggcc 1380
cagctggagg tgaccttcat ccaagcatgg ggcctgtggg gggactggtt cactgccacg 1440
gatgcctatg tgaagctctt ctttggtggc caggagctga ggacgagcac cgtgtgggac 1500
aataacaacc ccatctggtc agtgcggctg gattttgggg atgtgctcct ggccacaggg 1560
gggcccctga ggttgcaggt ctgggatcag gactctggca gggacgatga cctccttggc 1620
acctgtgatc aggctcccaa gtctggttcc catgaggtga gatgcaacct gaatcatggc 1680
cacctaaaat tccgctatca tgccaggtgc ttgccccacc tgggaggagg cacctgcctg 1740
gactatgtcc cccaaatgct tctgggggag cctccaggaa accggagtgg ggccgtgtgg 1800
tgagaacagt gagcttggaa aggaccagta tgcttggact gaaggggttc tcacagtggg 1860
agccagggct gtcttcgtat tcccattaga ccaagcttgt ccaacccgag gcccgcatgc 1920

ggcccaggat ggctttgaat gcggcccaac gcaaattcgc aaactttctt aaaacattat 1980

gagtttcttt ttgctatttt tttttttttt ttagctcatc ggctatcgtt agtgctagtg 2040

gattttacat gtggcccaac acaattcttc ttccaacgtg gcccagagaa gccaaaagat 2100

tggatacgca tcagacagat ggaaaaggga gattcagact gtttttcagg gaggtggctg 2160

ggtttacacg ctaatcccga ttcaccctgt ccaaactgcc taagccctcc gccattctca 2220

agccctgcag tcacagctac acagatcaca gcttcagcca ggagctgggc agaaggccaa 2280

gaggctgttc ccaccaggct gctcagggct ggtcttttag gacccttccc ttgagccctc 2340

tatggtgtgg caaagccttc attgccttaa ctggagcccc atcagctcca gctgctctgt 2400

cttctttgcc cacaatgctt tgcccctgag acaaatggag gcctgtcctg acctgtctca 2460

ccatgtacat agcttgataa agggccaata aatatgatgt tatggtgaaa aaaaaaaaaa 2520

aaaaaaaaa 2529

<210> 6
<211> 2589
<212> DNA
<213> Homo sapiens

<220>
<221> misc_feature
<223> TBX21

<400> 6
cggcccgctg gagaggaagc ccgagagctg ccgcgcgcct gccggacgag ggcgtagaag 60

ccaggcgtca gagcccgggc tccggtgggg tcccccaccc ggccctcggg tcccccgccc 120

cctgctccct gcccatccca gcccacgcga ccctctcgcg cgcggagggg cgggtcctcg 180

acggctacgg gaaggtgcca gcccgccccg gatgggcatc gtggagccgg gttgcggaga 240

catgctgacg ggcaccgagc cgatgccggg gagcgacgag ggccgggcgc ctggcgccga 300

cccgcagcac cgctacttct acccggagcc gggcgcgcag gacgcggacg agcgtcgcgg 360

gggcggcagc ctggggtctc cctacccggg gggcgccttg gtgcccgccc cgccgagccg 420

cttccttgga gcctacgcct acccgccgcg accccaggcg gccggcttcc ccggcgcggg 480

cgagtccttc ccgccgcccg cggacgccga gggctaccag ccgggcgagg gctacgccgc 540

cccggacccg cgcgccgggc tctacccggg gccgcgtgag gactacgcgc tacccgcggg 600

actggaggtg tcggggaaac tgagggtcgc gctcaacaac cacctgttgt ggtccaagtt 660

taatcagcac cagacagaga tgatcatcac caagcaggga cggcggatgt tcccattcct 720

gtcatttact gtggccgggc tggagcccac cagccactac aggatgtttg tggacgtggt 780

cttggtggac cagcaccact ggcggtacca gagcggcaag tgggtgcagt gtggaaaggc 840

cgagggcagc atgccaggaa accgcctgta cgtccacccg gactccccca acacaggagc 900
gcactggatg cgccaggaag tttcatttgg gaaactaaag ctcacaaaca acaagggggc 960
gtccaacaat gtgacccaga tgattgtgct ccagtccctc cataagtacc agccccggct 1020
gcatatcgtt gaggtgaacg acggagagcc agaggcagcc tgcaacgctt ccaacacgca 1080
tatctttact ttccaagaaa cccagttcat tgccgtgact gcctaccaga atgccgagat 1140
tactcagctg aaaattgata ataacccctt tgccaaagga ttccgggaga actttgagtc 1200
catgtacaca tctgttgaca ccagcatccc ctccccgcct ggacccaact gtcaattcct 1260
tggggggagat cactactctc ctctcctacc caaccagtat cctgttccca gccgcttcta 1320
ccccgacctt cctggccagg cgaaggatgt ggttccccag gcttactggc tggggggcccc 1380
ccgggaccac agctatgagg ctgagtttcg agcagtcagc atgaagcctg cattcttgcc 1440
ctctgcccct gggcccacca tgtcctacta ccgaggccag gaggtcctgg cacctggagc 1500
tggctggcct gtggcacccc agtaccctcc caagatgggc ccggccagct ggttccgccc 1560
tatgcggact ctgcccatgg aacccggccc tggaggctca gagggacggg gaccagagga 1620
ccagggtccc cccttggtgt ggactgagat tgcccccatc cggccggaat ccagtgattc 1680
aggactgggc gaaggagact ctaagaggag gcgcgtgtcc ccctatcctt ccagtggtga 1740
cagctcctcc cctgctgggg ccccttctcc ttttgataag gaagctgaag gacagtttta 1800
taactatttt cccaactgag cagatgacat gatgaaagga acagaaacag tgttattagg 1860
ttggaggaca ccgactaatt tgggaaacgg atgaaggact gagaaggccc ccgctccctc 1920
tggcccttct ctgtttagta gttggttggg gaagtggggc tcaagaagga ttttggggtt 1980
caccagatgc ttcctggccc acgatgaaac ctgagagggg tgtccccttg ccccatcctc 2040
tgccctaact acagtcgttt acctggtgct gcgtcttgct tttggtttcc agctggagaa 2100
aagaagacaa gaaagtcttg ggcatgaagg agctttttgc atctagtggg tgggaggggt 2160
caggtgtggg acatgggagc aggagactcc actttcttcc tttgtacagt aactttcaac 2220
cttttcgttg gcatgtgtgt taatccctga tccaaaaaga acaaatacac gtatgttata 2280
accatcagcc cgccagggtc agggaaagga ctcacctgac tttggacagc tggcctgggc 2340
tccccctgct caaacacagt ggggatcaga gaaaaggggc tggaaagggg ggaatggccc 2400
acatctcaag aagcaagata ttgtttgtgg tggttgtgtg tgggtgtgtg ttttttcttt 2460
ttctttcttt ttattttttt tgaatggggg aggctattta ttgtactgag agtggtgtct 2520
ggatatattc cttttgtctt catcactttc tgaaaataaa cataaaactg ttaaaaaaaa 2580
aaaaaaaaa 2589

<210> 7
<211> 6472

<212> DNA
<213> Homo sapiens

<220>
<221> misc_feature
<223> TGFBR3

<400> 7
gagagccccc ggtggggccg gcggaggagc gcccttcccc ccgcgcgctg attgctgtgg 60

cgtcctgccc gtccccgccc gcgtgtgtgc gagggagggc gagtgcgccg ggtcggcctg 120

atgggggtaa tcgagggttt cggggacgcc gagcggcact ttcctcttcc cagcgagtga 180

aggagggcag tcggcggctc tcgcgccccg gccactttcc ctgcgcgatt cccggagctc 240

cctgcaggag gtgagagtcc ccagcgggtc cggatggcgt agttttgccg cggcgcagca 300

gctgccggag ctcgccgccg ccgagcgctg ggcggggaaa cttgccgccg ctttcctcca 360

acttgctgcg ggtggatctc cgctggacac accgcctccg aggcagtttg aaaattgcaa 420

ggagggactt taagactact tctgatttgc aaagattgtc tgtgctctga gcaggctgaa 480

gtgactggac gagacgcact gttggagaaa taaaaatgac ttcccattat gtgattgcca 540

tctttgccct gatgagctcc tgtttagcca ctgcaggtcc agagcctggt gcactgtgtg 600

aactgtcacc tgtcagtgcc tcccatcctg tccaggcctt gatggagagc ttcactgttt 660

tgtcaggctg tgccagcaga ggcacaactg ggctgccaca ggaggtgcat gtcctgaatc 720

tccgcactgc aggccagggg cctggccagc tacagagaga ggtcacactt cacctgaatc 780

ccatctcctc agtccacatc caccacaagt ctgttgtgtt cctgctcaac tccccacacc 840

ccctggtgtg gcatctgaag acagagagac ttgccactgg ggtctccaga ctgtttttgg 900

tgtctgaggg ttctgtggtc cagttttcat cagcaaactt ctccttgaca gcagaaacag 960

aagaaaggaa cttcccccat ggaaatgaac atctgttaaa ttgggcccga aaagagtatg 1020

gagcagttac ttcattcacc gaactcaaga tagcaagaaa catttatatt aaagtggggg 1080

aagatcaagt gttccctcca aagtgcaaca tagggaagaa ttttctctca ctcaattacc 1140

ttgctgagta ccttcaaccc aaagcagcag aagggtgtgt gatgtccagc cagccccaga 1200

atgaggaagt acacatcatc gagctaatca cccccaactc taacccctac agtgctttcc 1260

aggtggatat aacaattgat ataagacctt ctcaagagga tcttgaagtg gtcaaaaatc 1320

tcatcctgat cttgaagtgc aaaaagtctg tcaactgggt gatcaaatct tttgatgtta 1380

agggaagcct gaaaattatt gctcctaaca gtattggctt tggaaaagag agtgaaagat 1440

ctatgacaat gaccaaatca ataagagatg acattccttc aacccaaggg aatctggtga 1500

agtgggcttt ggacaatggc tatagtccaa taacttcata cacaatggct cctgtggcta 1560

atagatttca tcttcggctt gaaaataatg aggagatggg agatgaggaa gtccacacta 1620

ttcctcctga gctacggatc ctgctggacc ctggtgccct gcctgccctg cagaacccgc 1680
ccatccgggg aggggaaggc caaaatggag gccttccgtt tcctttccca gatatttcca 1740
ggagagtctg gaatgaagag ggagaagatg ggctccctcg gccaaaggac cctgtcattc 1800
ccagcataca actgtttcct ggtctcagag agccagaaga ggtgcaaggg agcgtggata 1860
ttgccctgtc tgtcaaatgt gacaatgaga agatgatcgt ggctgtagaa aaagattctt 1920
ttcaggccag tggctactcg ggatggacg tcaccctgtt ggatcctacc tgcaaggcca 1980
agatgaatgg cacacacttt gttttggagt ctcctctgaa tggctgcggt actcggcccc 2040
ggtggtcagc ccttgatggt gtggtctact ataactccat tgtgatacag gttccagccc 2100
ttggggacag tagtggttgg ccagatggtt atgaagatct ggagtcaggt gataatggat 2160
ttccgggaga tatggatgaa ggagatgctt ccctgttcac ccgacctgaa atcgtggtgt 2220
ttaattgcag ccttcagcag gtgaggaacc ccagcagctt ccaggaacag ccccacggaa 2280
acatcacctt caacatggag ctatacaaca ctgacctctt tttggtgccc tcccagggcg 2340
tcttctctgt gccagagaat ggacacgttt atgttgaggt atctgttact aaggctgaac 2400
aagaactggg atttgccatc caaacgtgct ttatctctcc atattcgaac cctgatagga 2460
tgtctcatta caccattatt gagaatattt gtcctaaaga tgaatctgtg aaattctaca 2520
gtcccaagag agtgcacttt cctatcccgc aagctgacat ggataagaag cgattcagct 2580
ttgtcttcaa gcctgtcttc aacacctcac tgctctttct acagtgtgag ctgacgctgt 2640
gtacgaagat ggagaagcac ccccagaagt tgcctaagtg tgtgcctcct gacgaagcct 2700
gcacctcgct ggacgcctcg ataatctggg ccatgatgca gaataagaag acgttcacta 2760
agccccttgc tgtgatccac catgaagcag aatctaaaga aaaaggtcca agcatgaagg 2820
aaccaaatcc aatttctcca ccaattttcc atggtctgga caccctaacc gtgatgggca 2880
ttgcgtttgc agcctttgtg atcggagcac tcctgacggg ggccttgtgg tacatctatt 2940
ctcacacagg ggagacagca ggaaggcagc aagtccccac ctccccgcca gcctcggaaa 3000
acagcagtgc tgcccacagc atcggcagca cgcagagcac gccttgctcc agcagcagca 3060
cggcctagcc caacccagcc caacccggcc caacccagcc cagcccagct cagctcagct 3120
actgccaagg gcaggaccaa tggctgagcc tcgtgtccag actcagaggg ctggattttg 3180
gttcccttgt aaagacagag tgaatttcag tataaagatc acccgttgta ttcaccccac 3240
acccagggct agtataaaca tgaccctggg cttctgtacc acactagaat tcatgtgaga 3300
aagctaaaat ggtggtcttc tccaccagcc cctcacaggc ttgggggttt tcaatgtgaa 3360
acacatgcca gtttttaaaa tgctgctttg tccaggtgag aacatccata atttggggcc 3420
ctgagtttta cccagactca aggagttggt aaagggttaa tagccagata gtagaaccag 3480
tgaggagatg cggccaaaga ttctttatat ctgaaccaag atgtaaaaca agaaatgctt 3540

tgaggctttc taagcgatcc tcctgtctaa tttgcacctt tgtctggatg cacacttctg 3600
accttgctgc cacaacctgt ggggtctgat gtgtcccttg atgggtgctg ccctcaggga 3660
ctgcaccctg acaagtgtta aggcaacatt cctttcttgt gccctgggcc aaaaccaatg 3720
ctgatgacct tatcagcttc ctgtttcttc ccatacttgc atacaccact gcaaaatgtc 3780
ttaatgcaaa ttttgtattt cttacaggcc tacagaaatt gaaaatgacc aaaatcagga 3840
accacagatt tgtgcccatt cctaatattt tgttctgcaa attaatgtat aatttgaggt 3900
gaaattcagt tataaagtca aggacgaatt tgcacagtga tatatttcta tgtgtatgca 3960
agtacaagta tataatatgt cacctggcac attcattttc tcagttgaag aagagaaaat 4020
ttgaaaatgt ccttatgctt ttagagttgc aacttaagta tatttggtag ggtgagtgtt 4080
tccactcaaa atatgtcaac ttaaaaaaaa ataggccctt tcataaaaac caaactgtag 4140
caagatgcaa atgcatggca aatcctgtcg gtctccagtt ggttatctga atagtgtcac 4200
caattccacc aagacagtgc tgagattgga aaagggcact catttggatt gccttacttc 4260
tcttgcctta aatatatccc atatatttaa tatgtcaaaa agggcttgag gtgaatttca 4320
ttaaatggaa taatatgatg ccactttgca gctaaaataa gctcagtgat acctccttgt 4380
taaaaaaaaa aaaaaaaaaa aagaatgcca gggggaatt atcatgctaa attattttac 4440
tgctgaagat agcttcattg caaattattc tttttccaga atttccccca aacaacttag 4500
aattccataa gtagaacaag atagattcta cccccaaaat attcataagc aaatcccact 4560
gcccttaaaa taaggaagtt atgcctggtt gtctgtgaat ggaacctcag taggtaatcc 4620
tgatgaagct taaaacctta gggcagtgtc tacatgggaa acaagtatgt ggaaattgac 4680
aatcattttt accaaatcgt tgagtaataa tatgacttac ctgatagata tacacattca 4740
ataaggaaca tttttttttt tcaataagga acattttaac taaaacatta ctttagggct 4800
gagatttcca ggctatctac attgatctac tgaaaaattt ctagatttcc atatacaaac 4860
gaagaagaca tgagtttaga gaattttttt cctttaattt tttttaaca gaagggtatc 4920
agagtggagg acctagatta cgctacctgt gtttgaaaat attgcgcttt gcctaacctt 4980
cggtggaatg tattgtttca gcatattcta tgtataaaaa agtttaaaga tgtcttcaaa 5040
gaagactaga gtctttaagt cacttatagc tatattttac tacaaaattt gtgtataaag 5100
atatatttaa gtgttttaga taaatttaag ttactccata tgaaatgttt aatttcagct 5160
actgtgaatt ctttcgatca gtcctttgct tttttaaaaa atcttttcca tcgtgttaaa 5220
aagaaaatta gctattagga gatattaaaa gattgatgtc ttagaaggac agggttaaaa 5280
ccttctgtaa tatatttaaa tatatatttt taagagagat gctacattgc caatgatttt 5340
ataaataatt taaaattaat ttacaaattt atagatgcaa accaaaagat ctttttaaaa 5400

gagagagaga ggaaaaaaat caccttttat ttgtttgggt ctcattgtca gaattattat 5460

tcagatcaag aaatctgtgt cgataaaacc taatctcata ttggagagtg aatgtcgtta 5520

agtatttctg aaatgtttct aaaaaatgaa catgaaaaaa ataaggtatt ttctccctag 5580

cttctcatta cagtatttct aattgcttca cactttacag ttttaataca aagtacaagc 5640

tctactttta taacgtgtac tacaagtttc tttaaaagta ttgttgtatt tcagctgaac 5700

tcttgtatct gcagtctttt gttatttctg caggctacac ccttaatttg cagaggccat 5760

tttgttgttt gtttgatgct gaagctgttg ttaaaatgcc ccaaactcca aggcagattg 5820

tctgtataat acgctatagc ttttcaccat gtggtgttta ccagctgtta ctctgtattt 5880

taaaaagcca gagatttctt ctgtctaaac tgtttctata gcattttcta aacaaaaaaa 5940

atggtttttt tctttgcctt gtaataagat tgtaaatcca tttcctgctt gtgttcttag 6000

tgggcatgat tgtgagtgtg tgtggggtgc tcacacgcgt gccagtcttt ttgtactgta 6060

actacctcat ggtttgaatg atgattgtac tgctggttgt gttaacagag cacattttgt 6120

tgggtgagtc ctatgtgatt tttttcatgt ttgttttatc tggggggttgt tcatgaaact 6180

gacagatgtt tttctaaaaa ggactattat cagtttccaa atacaatact tctctcttct 6240

ggtttttcct gaatgagcct gattttgttg ctggttttca tcatctatga gggtaaaacg 6300

agtaccctta aaatccctgt ggccagtttg aacacccctg ttgtattctt ttctctgttc 6360

agtgtgtcag ctattttgtg aagatgctta gatgtatagt tttgataacc acagttttaa 6420

atcttttatc tgtgcataat aaaaagatat atatcagtta ttaaaaaaaa aa 6472

<210> 8
<211> 6308
<212> DNA
<213> Homo sapiens

<220>
<221> misc_feature
<223> TGFBR3

<400> 8
tctttaagat ttgtagctac taagaaagaa aggagctttt tttccttggg ccttcaaact 60

gaaagaaccg catgagcctg aagctgcgca tggtcttaac atcaggctgt gcaggaagaa 120

gctatctgca gatggatgcc agcacacaca aggaagcaga gctctggcaa cattgagtca 180

aagcaaggac acaacatcag agggacggca gagaatcctt gtgtgtagat ctttggtggc 240

agtttgaaaa ttgcaaggag ggactttaag actacttctg atttgcaaag attgtctgtg 300

ctctgagcag gctgaagtga ctggacgaga cgcactgttg gagaaataaa aatgacttcc 360

cattatgtga ttgccatctt tgccctgatg agctcctgtt tagccactgc aggtccagag 420

cctggtgcac tgtgtgaact gtcacctgtc agtgcctccc atcctgtcca ggccttgatg 480

```
gagagcttca ctgttttgtc aggctgtgcc agcagaggca caactgggct gccacaggag     540
gtgcatgtcc tgaatctccg cactgcaggc caggggcctg gccagctaca gagagaggtc     600
acacttcacc tgaatcccat ctcctcagtc cacatccacc acaagtctgt tgtgttcctg     660
ctcaactccc cacacccccct ggtgtggcat ctgaagacag agagacttgc cactggggtc    720
tccagactgt ttttggtgtc tgagggttct gtggtccagt tttcatcagc aaacttctcc     780
ttgacagcag aaacagaaga aaggaacttc ccccatggaa atgaacatct gttaaattgg     840
gcccgaaaag agtatggagc agttacttca ttcaccgaac tcaagatagc aagaaacatt     900
tatattaaag tgggggaaga tcaagtgttc cctccaaagt gcaacatagg gaagaatttt     960
ctctcactca attaccttgc tgagtacctt caacccaaag cagcagaagg gtgtgtgatg    1020
tccagccagc cccagaatga ggaagtacac atcatcgagc taatcacccc caactctaac    1080
ccctacagtg ctttccaggt ggatataaca attgatataa gaccttctca agaggatctt    1140
gaagtggtca aaaatctcat cctgatcttg aagtgcaaaa agtctgtcaa ctgggtgatc    1200
aaatcttttg atgttaaggg aagcctgaaa attattgctc ctaacagtat tggctttgga    1260
aaagagagtg aaagatctat gacaatgacc aaatcaataa gagatgacat tccttcaacc    1320
caagggaatc tggtgaagtg ggctttggac aatggctata gtccaataac ttcatacaca    1380
atggctcctg tggctaatag atttcatctt cggcttgaaa ataatgagga gatgggagat    1440
gaggaagtcc acactattcc tcctgagcta cggatcctgc tggaccctgg tgccctgcct    1500
gccctgcaga acccgcccat ccggggaggg gaaggccaaa atggaggcct tccgtttcct    1560
ttcccagata tttccaggag agtctggaat gaagagggag aagatgggct ccctcggcca    1620
aaggaccctg tcattcccag catacaactg tttcctggtc tcagagagcc agaagaggtg    1680
caagggagcg tggatattgc cctgtctgtc aaatgtgaca atgagaagat gatcgtggct    1740
gtagaaaaag attcttttca ggccagtggc tactcgggga tggacgtcac cctgttggat    1800
cctacctgca aggccaagat gaatggcaca cactttgttt tggagtctcc tctgaatggc    1860
tgcggtactc ggccccggtg gtcagccctt gatggtgtgg tctactataa ctccattgtg    1920
atacaggttc cagcccttgg ggacagtagt ggttggccag atggttatga agatctggag    1980
tcaggtgata atggatttcc gggagatatg gatgaaggag atgcttccct gttcacccga    2040
cctgaaatcg tggtgtttaa ttgcagcctt cagcaggtga ggaaccccag cagcttccag    2100
gaacagcccc acggaaacat caccttcaac atggagctat acaacactga cctcttttg     2160
gtgccctccc agggcgtctt ctctgtgcca gagaatggac acgtttatgt tgaggtatct    2220
gttactaagg ctgaacaaga actgggattt gccatccaaa cgtgctttat ctctccatat    2280
tcgaaccctg ataggatgtc tcattacacc attattgaga atatttgtcc taaagatgaa    2340
```

tctgtgaaat tctacagtcc caagagagtg cactttccta tcccgcaagc tgacatggat 2400
aagaagcgat tcagctttgt cttcaagcct gtcttcaaca cctcactgct ctttctacag 2460
tgtgagctga cgctgtgtac gaagatggag aagcaccccc agaagttgcc taagtgtgtg 2520
cctcctgacg aagcctgcac ctcgctggac gcctcgataa tctgggccat gatgcagaat 2580
aagaagacgt tcactaagcc ccttgctgtg atccaccatg aagcagaatc taaagaaaaa 2640
ggtccaagca tgaaggaacc aaatccaatt tctccaccaa ttttccatgg tctggacacc 2700
ctaaccgtga tgggcattgc gtttgcagcc tttgtgatcg gagcactcct gacgggggcc 2760
ttgtggtaca tctattctca cacaggggag acagcaggaa ggcagcaagt ccccacctcc 2820
ccgccagcct cggaaaacag cagtgctgcc cacagcatcg gcagcacgca gagcacgcct 2880
tgctccagca gcagcacggc ctagcccaac ccagcccaac ccggcccaac ccagcccagc 2940
ccagctcagc tcagctactg ccaagggcag gaccaatggc tgagcctcgt gtccagactc 3000
agagggctgg attttggttc ccttgtaaag acagagtgaa tttcagtata aagatcaccc 3060
gttgtattca ccccacaccc agggctagta taaacatgac cctgggcttc tgtaccacac 3120
tagaattcat gtgagaaagc taaaatggtg gtcttctcca ccagcccctc acaggcttgg 3180
gggttttcaa tgtgaaacac atgccagttt ttaaaatgct gctttgtcca ggtgagaaca 3240
tccataattt ggggccctga gttttaccca gactcaagga gttggtaaag ggttaatagc 3300
cagatagtag aaccagtgag gagatgcggc caaagattct ttatatctga accaagatgt 3360
aaaacaagaa atgctttgag gctttctaag cgatcctcct gtctaatttg cacctttgtc 3420
tggatgcaca cttctgacct tgctgccaca acctgtgggg tctgatgtgt cccttgatgg 3480
gtgctgccct cagggactgc accctgacaa gtgttaaggc aacattcctt tcttgtgccc 3540
tgggccaaaa ccaatgctga tgaccttatc agcttcctgt ttcttcccat acttgcatac 3600
accactgcaa aatgtcttaa tgcaaatttt gtatttctta caggcctaca gaaattgaaa 3660
atgaccaaaa tcaggaacca cagatttgtg cccattccta atattttgtt ctgcaaatta 3720
atgtataatt tgaggtgaaa ttcagttata aagtcaagga cgaatttgca cagtgatata 3780
tttctatgtg tatgcaagta caagtatata atatgtcacc tggcacattc attttctcag 3840
ttgaagaaga gaaaatttga aaatgtcctt atgcttttag agttgcaact taagtatatt 3900
tggtagggtg agtgtttcca ctcaaaatat gtcaacttaa aaaaaaatag gccctttcat 3960
aaaaaccaaa ctgtagcaag atgcaaatgc atggcaaatc ctgtcggtct ccagttggtt 4020
atctgaatag tgtcaccaat tccaccaaga cagtgctgag attggaaaag ggcactcatt 4080
tggattgcct tacttctctt gccttaaata tatcccatat atttaatatg tcaaaaaggg 4140
cttgaggtga atttcattaa atggaataat atgatgccac tttgcagcta aaataagctc 4200
agtgatacct ccttgttaaa aaaaaaaaaa aaaaaaaaga atgccagggg ggaattatca 4260

tgctaaatta ttttactgct gaagatagct tcattgcaaa ttattctttt tccagaattt 4320

cccccaaaca acttagaatt ccataagtag aacaagatag attctacccc caaaatattc 4380

ataagcaaat cccactgccc ttaaaataag gaagttatgc ctggttgtct gtgaatggaa 4440

cctcagtagg taatcctgat gaagcttaaa accttagggc agtgtctaca tgggaaacaa 4500

gtatgtggaa attgacaatc atttttacca aatcgttgag taataatatg acttacctga 4560

tagatataca cattcaataa ggaacatttt tttttttcaa taaggaacat tttaactaaa 4620

acattacttt agggctgaga tttccaggct atctacattg atctactgaa aaatttctag 4680

atttccatat acaaacgaag aagacatgag tttagagaat ttttttcctt taattttttt 4740

ttaacagaag ggtatcagag tggaggacct agattacgct acctgtgttt gaaaatattg 4800

cgctttgcct aaccttcggt ggaatgtatt gtttcagcat attctatgta taaaaaagtt 4860

taaagatgtc ttcaaagaag actagagtct ttaagtcact tatagctata ttttactaca 4920

aaatttgtgt ataaagatat atttaagtgt tttagataaa tttaagttac tccatatgaa 4980

atgtttaatt tcagctactg tgaattcttt cgatcagtcc tttgcttttt taaaaaatct 5040

tttccatcgt gttaaaaaga aaattagcta ttaggagata ttaaaagatt gatgtcttag 5100

aaggacaggg ttaaaacctt ctgtaatata tttaaatata tatttttaag agagatgcta 5160

cattgccaat gattttataa ataatttaaa attaatttac aaatttatag atgcaaacca 5220

aaagatcttt ttaaaagaga gagagaggaa aaaaatcacc ttttatttgt ttgggtctca 5280

ttgtcagaat tattattcag atcaagaaat ctgtgtcgat aaaacctaat ctcatattgg 5340

agagtgaatg tcgttaagta tttctgaaat gtttctaaaa aatgaacatg aaaaaaataa 5400

ggtattttct ccctagcttc tcattacagt atttctaatt gcttcacact ttacagtttt 5460

aatacaaagt acaagctcta cttttataac gtgtactaca agtttcttta aaagtattgt 5520

tgtatttcag ctgaactctt gtatctgcag tcttttgtta tttctgcagg ctacaccctt 5580

aatttgcaga ggccattttg ttgtttgttt gatgctgaag ctgttgttaa aatgccccaa 5640

actccaaggc agattgtctg tataatacgc tatagctttt caccatgtgg tgtttaccag 5700

ctgttactct gtattttaaa aagccagaga tttcttctgt ctaaactgtt tctatagcat 5760

tttctaaaca aaaaaaatgg tttttttctt tgccttgtaa taagattgta aatccatttc 5820

ctgcttgtgt tcttagtggg catgattgtg agtgtgtgtg gggtgctcac acgcgtgcca 5880

gtctttttgt actgtaacta cctcatggtt tgaatgatga ttgtactgct ggttgtgtta 5940

acagagcaca ttttgttggg tgagtcctat gtgatttttt tcatgtttgt tttatctggg 6000

ggttgttcat gaaactgaca gatgtttttc taaaaaggac tattatcagt ttccaaatac 6060

aatacttctc tcttctggtt tttcctgaat gagcctgatt ttgttgctgg ttttcatcat 6120

ctatgagggt aaaacgagta cccttaaaat ccctgtggcc agtttgaaca cccctgttgt 6180

attcttttct ctgttcagtg tgtcagctat tttgtgaaga tgcttagatg tatagttttg 6240

ataaccacag ttttaaatct tttatctgtg cataataaaa agatatatat cagttattaa 6300

aaaaaaaa 6308

<210> 9
<211> 6475
<212> DNA
<213> Homo sapiens

<220>
<221> misc_feature
<223> TGFBR3

<400> 9
gagagccccc ggtggggccg gcggaggagc gcccttcccc ccgcgcgctg attgctgtgg 60

cgtcctgccc gtccccgccc gcgtgtgtgc gagggagggc gagtgcgccg ggtcggcctg 120

atgggggtaa tcgagggttt cggggacgcc gagcggcact ttcctcttcc cagcgagtga 180

aggagggcag tcggcggctc tcgcgccccg gccactttcc ctgcgcgatt cccggagctc 240

cctgcaggag gtgagagtcc ccagcgggtc cggatggcgt agttttgccg cggcgcagca 300

gctgccggag ctcgccgccg ccgagcgctg ggcggggaaa cttgccgccg ctttcctcca 360

acttgctgcg ggtggatctc cgctggacac accgcctccg aggcagtttg aaaattgcaa 420

ggagggactt taagactact tctgatttgc aaagattgtc tgtgctctga gcaggctgaa 480

gtgactggac gagacgcact gttggagaaa taaaaatgac ttcccattat gtgattgcca 540

tctttgccct gatgagctcc tgtttagcca ctgcaggtcc agagcctggt gcactgtgtg 600

aactgtcacc tgtcagtgcc tcccatcctg tccaggcctt gatggagagc ttcactgttt 660

tgtcaggctg tgccagcaga ggcacaactg ggctgccaca ggaggtgcat gtcctgaatc 720

tccgcactgc aggccagggg cctggccagc tacagagaga ggtcacactt cacctgaatc 780

ccatctcctc agtccacatc caccacaagt ctgttgtgtt cctgctcaac tccccacacc 840

ccctggtgtg gcatctgaag acagagagac ttgccactgg ggtctccaga ctgtttttgg 900

tgtctgaggg ttctgtggtc cagttttcat cagcaaactt ctccttgaca gcagaaacag 960

aagaaaggaa cttcccccat ggaaatgaac atctgttaaa ttgggcccga aaagagtatg 1020

gagcagttac ttcattcacc gaactcaaga tagcaagaaa catttatatt aaagtggggg 1080

aagatcaagt gttccctcca aagtgcaaca tagggaagaa ttttctctca ctcaattacc 1140

ttgctgagta ccttcaaccc aaagcagcag aagggtgtgt gatgtccagc cagccccaga 1200

atgaggaagt acacatcatc gagctaatca cccccaactc taacccctac agtgctttcc 1260

aggtggatat aacaattgat ataagacctt ctcaagagga tcttgaagtg gtcaaaaatc 1320

tcatcctgat cttgaagtgc aaaaagtctg tcaactgggt gatcaaatct tttgatgtta 1380
agggaagcct gaaaattatt gctcctaaca gtattggctt tggaaaagag agtgaaagat 1440
ctatgacaat gaccaaatca ataagagatg acattccttc aacccaaggg aatctggtga 1500
agtgggcttt ggacaatggc tatagtccaa taacttcata cacaatggct cctgtggcta 1560
atagatttca tcttcggctt gaaaataatg cagaggagat gggagatgag gaagtccaca 1620
ctattcctcc tgagctacgg atcctgctgg accctggtgc cctgcctgcc ctgcagaacc 1680
cgcccatccg gggaggggaa ggccaaaatg gaggccttcc gtttcctttc ccagatattt 1740
ccaggagagt ctggaatgaa gagggagaag atgggctccc tcggccaaag gaccctgtca 1800
ttcccagcat acaactgttt cctggtctca gagagccaga agaggtgcaa gggagcgtgg 1860
atattgccct gtctgtcaaa tgtgacaatg agaagatgat cgtggctgta gaaaaagatt 1920
cttttcaggc cagtggctac tcggggatgg acgtcaccct gttggatcct acctgcaagg 1980
ccaagatgaa tggcacacac tttgttttgg agtctcctct gaatggctgc ggtactcggc 2040
cccggtggtc agcccttgat ggtgtggtct actataactc cattgtgata caggttccag 2100
cccttgggga cagtagtggt tggccagatg gttatgaaga tctggagtca ggtgataatg 2160
gatttccggg agatatggat gaaggagatg cttccctgtt cacccgacct gaaatcgtgg 2220
tgtttaattg cagccttcag caggtgagga accccagcag cttccaggaa cagccccacg 2280
gaaacatcac cttcaacatg gagctataca acactgacct ctttttggtg ccctcccagg 2340
gcgtcttctc tgtgccagag aatggacacg tttatgttga ggtatctgtt actaaggctg 2400
aacaagaact gggatttgcc atccaaacgt gctttatctc tccatattcg aaccctgata 2460
ggatgtctca ttacaccatt attgagaata tttgtcctaa agatgaatct gtgaaattct 2520
acagtcccaa gagagtgcac tttcctatcc cgcaagctga catggataag aagcgattca 2580
gctttgtctt caagcctgtc ttcaacacct cactgctctt tctacagtgt gagctgacgc 2640
tgtgtacgaa gatggagaag caccccccaga agttgcctaa gtgtgtgcct cctgacgaag 2700
cctgcacctc gctggacgcc tcgataatct gggccatgat gcagaataag aagacgttca 2760
ctaagcccct tgctgtgatc caccatgaag cagaatctaa agaaaaaggt ccaagcatga 2820
aggaaccaaa tccaatttct ccaccaattt tccatggtct ggacacccta accgtgatgg 2880
gcattgcgtt tgcagccttt gtgatcggag cactcctgac gggggccttg tggtacatct 2940
attctcacac aggggagaca gcaggaaggc agcaagtccc cacctccccg ccagcctcgg 3000
aaaacagcag tgctgcccac agcatcggca gcacgcagag cacgccttgc tccagcagca 3060
gcacggccta gcccaaccca gcccaacccg gcccaaccca gcccagccca gctcagctca 3120
gctactgcca agggcaggac caatggctga gcctcgtgtc cagactcaga gggctggatt 3180

ttggttccct tgtaaagaca gagtgaattt cagtataaag atcacccgtt gtattcaccc 3240
cacacccagg gctagtataa acatgaccct gggcttctgt accacactag aattcatgtg 3300
agaaagctaa aatggtggtc ttctccacca gcccctcaca ggcttggggg ttttcaatgt 3360
gaaacacatg ccagttttta aaatgctgct ttgtccaggt gagaacatcc ataatttggg 3420
gccctgagtt ttacccagac tcaaggagtt ggtaaagggt taatagccag atagtagaac 3480
cagtgaggag atgcggccaa agattcttta tatctgaacc aagatgtaaa acaagaaatg 3540
ctttgaggct ttctaagcga tcctcctgtc taatttgcac ctttgtctgg atgcacactt 3600
ctgaccttgc tgccacaacc tgtggggtct gatgtgtccc ttgatgggtg ctgccctcag 3660
ggactgcacc ctgacaagtg ttaaggcaac attcctttct tgtgccctgg gccaaaacca 3720
atgctgatga ccttatcagc ttcctgtttc ttcccatact tgcatacacc actgcaaaat 3780
gtcttaatgc aaattttgta tttcttacag gcctacagaa attgaaaatg accaaaatca 3840
ggaaccacag atttgtgccc attcctaata ttttgttctg caaattaatg tataatttga 3900
ggtgaaattc agttataaag tcaaggacga atttgcacag tgatatattt ctatgtgtat 3960
gcaagtacaa gtatataata tgtcacctgg cacattcatt ttctcagttg aagaagagaa 4020
aatttgaaaa tgtccttatg cttttagagt tgcaacttaa gtatatttgg tagggtgagt 4080
gtttccactc aaaatatgtc aacttaaaaa aaaataggcc ctttcataaa aaccaaactg 4140
tagcaagatg caaatgcatg gcaaatcctg tcggtctcca gttggttatc tgaatagtgt 4200
caccaattcc accaagacag tgctgagatt ggaaaagggc actcatttgg attgccttac 4260
ttctcttgcc ttaaatatat cccatatatt taatatgtca aaaagggctt gaggtgaatt 4320
tcattaaatg gaataatatg atgccacttt gcagctaaaa taagctcagt gatacctcct 4380
tgttaaaaaa aaaaaaaaaa aaaaagaatg ccaggggggga attatcatgc taaattattt 4440
tactgctgaa gatagcttca ttgcaaatta ttctttttcc agaatttccc ccaaacaact 4500
tagaattcca taagtagaac aagatagatt ctacccccaa aatattcata agcaaatccc 4560
actgccctta aaataaggaa gttatgcctg gttgtctgtg aatggaacct cagtaggtaa 4620
tcctgatgaa gcttaaaacc ttagggcagt gtctacatgg gaaacaagta tgtggaaatt 4680
gacaatcatt tttaccaaat cgttgagtaa taatatgact tacctgatag atatacacat 4740
tcaataagga acattttttt ttttcaataa ggaacatttt aactaaaaca ttactttagg 4800
gctgagattt ccaggctatc tacattgatc tactgaaaaa tttctagatt tccatataca 4860
aacgaagaag acatgagttt agagaatttt tttcctttaa tttttttta acagaagggt 4920
atcagagtgg aggacctaga ttacgctacc tgtgtttgaa aatattgcgc tttgcctaac 4980
cttcggtgga atgtattgtt tcagcatatt ctatgtataa aaaagtttaa agatgtcttc 5040
aaagaagact agagtcttta agtcacttat agctatattt tactacaaaa tttgtgtata 5100

aagatatatt taagtgtttt agataaattt aagttactcc atatgaaatg tttaatttca 5160

gctactgtga attctttcga tcagtccttt gctttttttaa aaaatctttt ccatcgtgtt 5220

aaaaagaaaa ttagctatta ggagatatta aaagattgat gtcttagaag gacagggtta 5280

aaaccttctg taatatattt aaatatatat ttttaagaga gatgctacat tgccaatgat 5340

tttataaata atttaaaatt aatttacaaa tttatagatg caaaccaaaa gatcttttta 5400

aaagagagag agaggaaaaa aatcaccttt tatttgtttg ggtctcattg tcagaattat 5460

tattcagatc aagaaatctg tgtcgataaa acctaatctc atattggaga gtgaatgtcg 5520

ttaagtattt ctgaaatgtt tctaaaaaat gaacatgaaa aaaataaggt attttctccc 5580

tagcttctca ttacagtatt tctaattgct tcacacttta cagttttaat acaaagtaca 5640

agctctactt ttataacgtg tactacaagt ttctttaaaa gtattgttgt atttcagctg 5700

aactcttgta tctgcagtct tttgttattt ctgcaggcta cacccttaat ttgcagaggc 5760

cattttgttg tttgtttgat gctgaagctg ttgttaaaat gccccaaact ccaaggcaga 5820

ttgtctgtat aatacgctat agcttttcac catgtggtgt ttaccagctg ttactctgta 5880

ttttaaaaag ccagagattt cttctgtcta aactgtttct atagcatttt ctaaacaaaa 5940

aaaatggttt ttttctttgc cttgtaataa gattgtaaat ccatttcctg cttgtgttct 6000

tagtgggcat gattgtgagt gtgtgtgggg tgctcacacg cgtgccagtc tttttgtact 6060

gtaactacct catggtttga atgatgattg tactgctggt tgtgttaaca gagcacattt 6120

tgttgggtga gtcctatgtg atttttttca tgtttgtttt atctgggggt tgttcatgaa 6180

actgacagat gtttttctaa aaaggactat tatcagtttc caaatacaat acttctctct 6240

tctggttttt cctgaatgag cctgattttg ttgctggttt tcatcatcta tgagggtaaa 6300

acgagtaccc ttaaaatccc tgtggccagt ttgaacaccc ctgttgtatt cttttctctg 6360

ttcagtgtgt cagctatttt gtgaagatgc ttagatgtat agttttgata accacagttt 6420

taaatctttt atctgtgcat aataaaaaga tatatatcag ttattaaaaa aaaaa 6475

<210> 10
<211> 2234
<212> DNA
<213> Homo sapiens

<220>
<221> misc_feature
<223> IFNGR2

<400> 10
gttgactgga ggcggaggtt gcagtgagcc gagatcgccc cactgcactc cagcctggtg 60

actccgtctc aaaaaaaagg ggaggggggc gggggagagt tgaaagctta atatgtactt 120

```
tgggggctat taaagcaaac atttcgacta aaggggcgaa tcctcgaatt gtgcgatcaa      180
gcacccgaga ggagagttgg ggggggtcag gaggggtggg ggctccaggg aaagcccggg      240
ggtctgggcc ggggtctcgc ggggcccttc cggaaggatc gcggcccccg aaggtgggcg      300
tcccgcgggg ctccagtctc caggacgttc cgggaggctc cgcgctctgg gaggccggct      360
gcgtggggtc cccgcgctgc agccgcagag gccccccagg gccgcggttc ccggagcggg      420
aaagtcccgc gcgggggcgg tggcctcggg ggcgggacgg ggcgggggcg ggggcgcggg      480
cggccgagcc gaatcccctc caccgggacg ccccgctgct gctcgggaag aggcgggccc      540
tgcgcgccct gcgctcgcca tggcggtttg ggcggcgacg tgagcggctc cgcggacccc      600
gagcggggcc ccggccgcga cctgagccgc cgccgagcgc ccggggccat gcgaccgacg      660
ctgctgtggt cgctgctgct gctgctcgga gtcttcgccg ccgccgccgc ggccccgcca      720
gaccctcttt cccagctgcc cgctcctcag cacccgaaga ttcgcctgta caacgcagag      780
caggtcctga gttgggagcc agtggccctg agcaatagca cgaggcctgt tgtctaccaa      840
gtgcagttta aatacaccga cagtaaatgg ttcacggccg acatcatgtc catagggggtg      900
aattgtacac agatcacagc aacagagtgt gacttcactg ccgccagtcc ctcagcaggc      960
ttcccaatgg atttcaatgt cactctacgc cttcgagctg agctgggagc actccattct     1020
gcctgggtga caatgccttg gtttcaacac tatcggaatg tgactgtcgg gcctccagaa     1080
aacattgagg tgaccccagg agaaggctcc ctcatcatca ggttctcctc tccctttgac     1140
atcgctgata cctccacggc ctttttttgt tattatgtcc attactggga aaaaggagga     1200
atccaacagg tcaaaggccc tttcagaagc aactccattt cattggataa cttaaaaccc     1260
tccagagtgt actgtttaca agtccaggca caactgcttt ggaacaaaag taacatcttt     1320
agagtcgggc atttaagcaa catatcttgc tacgaaacaa tggcagatgc ctccactgag     1380
cttcagcaag tcatcctgat ctccgtggga acattttcgt tgctgtcggt gctggcagga     1440
gcctgtttct tcctggtcct gaaatataga ggcctgatta aatactggtt tcacactcca     1500
ccaagcatcc cattacagat agaagagtat ttaaaagacc caactcagcc catcttagag     1560
gccttggaca aggacagctc accaaaggat gacgtctggg actctgtgtc cattatctcg     1620
tttccggaaa aggagcaaga agatgttctc caaacgcttt gaaccaaagc atgggcctag     1680
cccactggct ccctggaaga gatcaagcca tcggagctgc tagagttctg tctggacttt     1740
ccagagacca gtattccctt ttgctgcctc taaaaggcct gtccctgcag acatgagaga     1800
cagcaggtct catgggggtg acaagctttt tttttttttc ttaaagaatt ttcaaaatca     1860
aattccagaa tgattttacg gagatatccc aggaaaatta aggcttctct taaacactaa     1920
aaaggcatgt aattgcttgt tagcaaaatg gatatgacac atctctgata ctttttttcat     1980
tattggttgg gctgagcagt cagaagacct ggtcgtcgtc ttgactttgg caaatgagcc     2040
```

ggagcccctt gggcaggtca cacaacctgt cccagcgagg gacaccgagt ggcccttcat 2100

gtacatccat ggtgtgctgg cttaaaatgt aattaatctt gtaaatatac tcctagtaat 2160

ttaagatttt gtttttaaac tggaaataaa agattgtata gtgcatgttt tttaaagtct 2220

aaaaaaaaaa aaaa 2234

<210> 11
<211> 2650
<212> DNA
<213> Homo sapiens

<220>
<221> misc_feature
<223> FYN

<400> 11
gccgcgctgg tggcggcggc gcgtcgttgc agttgcgcca tctgtcagga gcggagccgg 60

cgaggagggg gctgccgcgg gcgaggagga ggggtcgccg cgagccgaag gccttcgaga 120

cccgcccgcc gcccggcggc gagagtagag gcgaggttgt tgtgcgagcg gcgcgtcctc 180

tcccgcccgg gcgcgccgcg cttctcccag cgcaccgagg accgcccggg cgcacacaaa 240

gccgccgccc gcgccgcacc gcccggcggc cgccgcccgc gccagggagg gattcggccg 300

ccgggccggg gacaccccgg cgccgccccc tcggtgctct cggaaggccc accggctccc 360

gggcccgccg gggacccccc ggagccgcct cggccgcgcc ggaggagggc ggggagagga 420

ccatgtgagt gggctccgga gcctcagcgc cgcgcagttt ttttgaagaa gcaggatgct 480

gatctaaacg tggaaaaaga ccagtcctgc ctctgttgta gaagacatgt ggtgtatata 540

aagtttgtga tcgttggcgg acattttgga atttagataa tgggctgtgt gcaatgtaag 600

gataaagaag caacaaaact gacggaggag agggacggca gcctgaacca gagctctggg 660

taccgctatg gcacagaccc cacccctcag cactacccca gcttcggtgt gacctccatc 720

cccaactaca acaacttcca cgcagccggg ggccaaggac tcaccgtctt tggaggtgtg 780

aactcttcgt ctcatacggg gaccttgcgt acgagaggag gaacaggagt gacactcttt 840

gtggcccttt atgactatga agcacggaca gaagatgacc tgagttttca caaaggagaa 900

aaatttcaaa tattgaacag ctcggaagga gattggtggg aagcccgctc cttgacaact 960

ggagagacag gttacattcc cagcaattat gtggctccag ttgactctat ccaggcagaa 1020

gagtggtact ttggaaaact tggccgaaaa gatgctgagc gacagctatt gtcctttgga 1080

aacccaagag gtacctttct tatccgcgag agtgaaacca ccaaaggtgc ctattcactt 1140

tctatccgtg attgggatga tatgaaagga gaccatgtca aacattataa aattcgcaaa 1200

cttgacaatg gtggatacta cattaccacc cgggcccagt ttgaaacact tcagcagctt 1260

gtacaacatt actcagagag agctgcaggt ctctgctgcc gcctagtagt tccctgtcac 1320

aaagggatgc caaggcttac cgatctgtct gtcaaaacca aagatgtctg ggaaatccct 1380

cgagaatccc tgcagttgat caagagactg ggaaatgggc agtttgggga agtatggatg 1440

ggtacctgga atggaaacac aaaagtagcc ataaagactc ttaaaccagg cacaatgtcc 1500

cccgaatcat tccttgagga agcgcagatc atgaagaagc tgaagcacga caagctggtc 1560

cagctctatg cagtggtgtc tgaggagccc atctacatcg tcaccgagta tatgaacaaa 1620

ggaagtttac tggatttctt aaaagatgga gaaggaagag ctctgaaatt accaaatctt 1680

gtggacatgg cagcacaggt ggctgcagga atggcttaca tcgagcgcat gaattatatc 1740

catagagatc tgcgatcagc aaacattcta gtggggaatg gactcatatg caagattgct 1800

gacttcggat tggcccgatt gatagaagac aatgagtaca cagcaagaca aggtgcaaag 1860

ttccccatca agtggacggc ccccgaggca gccctgtacg ggaggttcac aatcaagtct 1920

gacgtgtggt cttttggaat cttactcaca gagctggtca ccaaaggaag agtgccatac 1980

ccaggcatga acaaccggga ggtgctggag caggtggagc gaggctacag gatgccctgc 2040

ccgcaggact gccccatctc tctgcatgag ctcatgatcc actgctggaa aaaggaccct 2100

gaagaacgcc ccacttttga gtacttgcag agcttcctgg aagactactt taccgcgaca 2160

gagccccagt accaacctgg tgaaaacctg taaggcccgg gtctgcggag agaggccttg 2220

tcccagaggc tgccccaccc ctccccatta gctttcaatt ccgtagccag ctgctcccca 2280

gcagcggaac cgcccaggat cagattgcat gtgactctga agctgacgaa cttccatggc 2340

cctcattaat gacacttgtc cccaaatccg aacctcctct gtgaagcatt cgagacagaa 2400

ccttgttatt tctcagactt tggaaaatgc attgtatcga tgttatgtaa aaggccaaac 2460

ctctgttcag tgtaaatagt tactccagtg ccaacaatcc tagtgctttc cttttttaaa 2520

aatgcaaatc ctatgtgatt ttaactctgt cttcacctga ttcaactaaa aaaaaaaaag 2580

tattattttc caaaagtggc ctctttgtct aaaacaataa aattttttt catgttttaa 2640

caaaaaccaa 2650

<210> 12
<211> 2073
<212> DNA
<213> Homo sapiens

<220>
<221> misc_feature
<223> FYN

<400> 12
gaatttagat aatgggctgt gtgcaatgta aggataaaga agcaacaaaa ctgacggagg 60

agagggacgg cagcctgaac cagagctctg ggtaccgcta tggcacagac cccacccctc 120

agcactaccc cagcttcggt gtgacctcca tccccaacta caacaacttc cacgcagccg 180
ggggccaagg actcaccgtc tttggaggtg tgaactcttc gtctcatacg gggaccttgc 240
gtacgagagg aggaacagga gtgacactct ttgtggccct ttatgactat gaagcacgga 300
cagaagatga cctgagtttt cacaaaggag aaaaatttca aatattgaac agctcggaag 360
gagattggtg ggaagcccgc tccttgacaa ctggagagac aggttacatt cccagcaatt 420
atgtggctcc agttgactct atccaggcag aagagtggta ctttggaaaa cttggccgaa 480
aagatgctga gcgacagcta ttgtcctttg gaaacccaag aggtaccttt cttatccgcg 540
agagtgaaac caccaaaggt gcctattcac tttctatccg tgattgggat gatatgaaag 600
gagaccatgt caaacattat aaaattcgca aacttgacaa tggtggatac tacattacca 660
cccgggccca gtttgaaaca cttcagcagc ttgtacaaca ttactcagag aaagctgatg 720
gtttgtgttt taacttaact gtgattgcat cgagttgtac ccccacaaact tctggattgg 780
ctaaagatgc ttgggaagtt gcacgtcgtt cgttgtgtct ggagaagaag ctgggtcagg 840
ggtgtttcgc tgaagtgtgg cttggtacct ggaatggaaa cacaaaagta gccataaaga 900
ctcttaaacc aggcacaatg tcccccgaat cattccttga ggaagcgcag atcatgaaga 960
agctgaagca cgacaagctg gtccagctct atgcagtggt gtctgaggag cccatctaca 1020
tcgtcaccga gtatatgaac aaaggaagtt tactggattt cttaaaagat ggagaaggaa 1080
gagctctgaa attaccaaat cttgtggaca tggcagcaca ggtggctgca ggaatggctt 1140
acatcgagcg catgaattat atccatagag atctgcgatc agcaaacatt ctagtgggga 1200
atggactcat atgcaagatt gctgacttcg gattggcccg attgatagaa gacaatgagt 1260
acacagcaag acaaggtgca aagttcccca tcaagtggac ggcccccgag gcagccctgt 1320
acgggaggtt cacaatcaag tctgacgtgt ggtcttttgg aatcttactc acagagctgg 1380
tcaccaaagg aagagtgcca tacccaggca tgaacaaccg ggaggtgctg gagcaggtgg 1440
agcgaggcta caggatgccc tgcccgcagg actgccccat ctctctgcat gagctcatga 1500
tccactgctg gaaaaaggac cctgaagaac gccccacttt tgagtacttg cagagcttcc 1560
tggaagacta ctttaccgcg acagagcccc agtaccaacc tggtgaaaac ctgtaaggcc 1620
cgggtctgcg gagagaggcc ttgtcccaga ggctgcccca cccctcccca ttagctttca 1680
attccgtagc cagctgctcc ccagcagcgg aaccgcccag gatcagattg catgtgactc 1740
tgaagctgac gaacttccat ggccctcatt aatgacactt gtccccaaat ccgaacctcc 1800
tctgtgaagc attcgagaca gaaccttgtt atttctcaga ctttggaaaa tgcattgtat 1860
cgatgttatg taaaaggcca aacctctgtt cagtgtaaat agttactcca gtgccaacaa 1920
tcctagtgct ttcctttttt aaaaatgcaa atcctatgtg attttaactc tgtcttcacc 1980

tgattcaact aaaaaaaaaa aagtattatt ttccaaaagt ggcctctttg tctaaaacaa 2040

taaaattttt tttcatgttt taacaaaaac caa 2073

<210> 13
<211> 2000
<212> DNA
<213> Homo sapiens

<220>
<221> misc_feature
<223> FYN

<400> 13
cagcgcaggt ctgaggagct gagaagggag gcttacgtga agggaattta gataatgggc 60

tgtgtgcaat gtaaggataa agaagcaaca aaactgacgg aggagaggga cggcagcctg 120

aaccagagct ctgggtaccg ctatggcaca gaccccaccc ctcagcacta ccccagcttc 180

ggtgtgacct ccatccccaa ctacaacaac ttccacgcag ccgggggcca aggactcacc 240

gtctttggag gtgtgaactc ttcgtctcat acggggacct tgcgtacgag aggaggaaca 300

ggagtgacac tctttgtggc cctttatgac tatgaagcac ggacagaaga tgacctgagt 360

tttcacaaag gagaaaaatt tcaaatattg aacagctcgg aaggagattg gtgggaagcc 420

cgctccttga caactggaga gacaggttac attcccagca attatgtggc tccagttgac 480

tctatccagg cagaagagtg gtactttgga aaacttggcc gaaaagatgc tgagcgacag 540

ctattgtcct ttggaaaccc aagaggtacc tttcttatcc gcgagagtga aaccaccaaa 600

ggtgcctatt cactttctat ccgtgattgg gatgatatga aaggagacca tgtcaaacat 660

tataaaattc gcaaacttga caatggtgga tactacatta ccacccgggc ccagtttgaa 720

acacttcagc agcttgtaca acattactca ggtacctgga atggaaacac aaaagtagcc 780

ataaagactc ttaaaccagg cacaatgtcc cccgaatcat tccttgagga agcgcagatc 840

atgaagaagc tgaagcacga caagctggtc cagctctatg cagtggtgtc tgaggagccc 900

atctacatcg tcaccgagta tatgaacaaa ggaagtttac tggatttctt aaaagatgga 960

gaaggaagag ctctgaaatt accaaatctt gtggacatgg cagcacaggt ggctgcagga 1020

atggcttaca tcgagcgcat gaattatatc catagagatc tgcgatcagc aaacattcta 1080

gtggggaatg gactcatatg caagattgct gacttcggat tggcccgatt gatagaagac 1140

aatgagtaca cagcaagaca aggtgcaaag ttccccatca agtggacggc ccccgaggca 1200

gccctgtacg ggaggttcac aatcaagtct gacgtgtggt cttttggaat cttactcaca 1260

gagctggtca ccaaaggaag agtgccatac ccaggcatga acaaccggga ggtgctggag 1320

caggtggagc gaggctacag gatgccctgc ccgcaggact gccccatctc tctgcatgag 1380

ctcatgatcc actgctggaa aaaggaccct gaagaacgcc ccacttttga gtacttgcag 1440

agcttcctgg aagactactt taccgcgaca gagccccagt accaacctgg tgaaaacctg 1500

taaggcccgg gtctgcggag agaggccttg tcccagaggc tgccccaccc ctccccatta 1560

gctttcaatt ccgtagccag ctgctcccca gcagcggaac cgcccaggat cagattgcat 1620

gtgactctga agctgacgaa cttccatggc cctcattaat gacacttgtc cccaaatccg 1680

aacctcctct gtgaagcatt cgagacagaa ccttgttatt tctcagactt tggaaaatgc 1740

attgtatcga tgttatgtaa aaggccaaac ctctgttcag tgtaaatagt tactccagtg 1800

ccaacaatcc tagtgctttc ctttttttaaa aatgcaaatc ctatgtgatt ttaactctgt 1860

cttcacctga ttcaactaaa aaaaaaaaag tattattttc caaaagtggc ctctttgtct 1920

aaaacaataa aattttttttt catgttttaa caaaaaccaa aaaaaaaaaa aaaaaaaaaa 1980

aaaaaaaaaa aaaaaaaaaa 2000

<210> 14
<211> 1498
<212> DNA
<213> Homo sapiens

<220>
<221> misc_feature
<223> IL1B

<400> 14
accaaacctc ttcgaggcac aaggcacaac aggctgctct gggattctct tcagccaatc 60

ttcattgctc aagtgtctga agcagccatg gcagaagtac ctgagctcgc cagtgaaatg 120

atggcttatt acagtggcaa tgaggatgac ttgttctttg aagctgatgg ccctaaacag 180

atgaagtgct ccttccagga cctggacctc tgccctctgg atggcggcat ccagctacga 240

atctccgacc accactacag caagggcttc aggcaggccg cgtcagttgt tgtggccatg 300

gacaagctga ggaagatgct ggttccctgc ccacagacct tccaggagaa tgacctgagc 360

accttctttc ccttcatctt tgaagaagaa cctatcttct tcgacacatg ggataacgag 420

gcttatgtgc acgatgcacc tgtacgatca ctgaactgca cgctccggga ctcacagcaa 480

aaaagcttgg tgatgtctgg tccatatgaa ctgaaagctc tccacctcca gggacaggat 540

atggagcaac aagtggtgtt ctccatgtcc tttgtacaag gagaagaaag taatgacaaa 600

atacctgtgg ccttgggcct caaggaaaag aatctgtacc tgtcctgcgt gttgaaagat 660

gataagccca ctctacagct ggagagtgta gatcccaaaa attacccaaa gaagaagatg 720

gaaaagcgat ttgtcttcaa caagatagaa atcaataaca agctggaatt tgagtctgcc 780

cagttcccca actggtacat cagcacctct caagcagaaa acatgcccgt cttcctggga 840

gggaccaaag gcggccagga tataactgac ttcaccatgc aatttgtgtc ttcctaaaga 900

gagctgtacc cagagagtcc tgtgctgaat gtggactcaa tccctagggc tggcagaaag 960

ggaacagaaa ggtttttgag tacggctata gcctggactt tcctgttgtc tacaccaatg 1020

cccaactgcc tgccttaggg tagtgctaag aggatctcct gtccatcagc caggacagtc 1080

agctctctcc tttcagggcc aatccccagc ccttttgttg agccaggcct ctctcacctc 1140

tcctactcac ttaaagcccg cctgacagaa accacggcca catttggttc taagaaaccc 1200

tctgtcattc gctcccacat tctgatgagc aaccgcttcc ctatttattt atttatttgt 1260

ttgtttgttt tattcattgg tctaatttat tcaaaggggg caagaagtag cagtgtctgt 1320

aaaagagcct agtttttaat agctatggaa tcaattcaat ttggactggt gtgctctctt 1380

taaatcaagt cctttaatta agactgaaaa tatataagct cagattattt aaatgggaat 1440

atttataaat gagcaaatat catactgttc aatggttctg aaataaactt cactgaag 1498

<210> 15
<211> 10668
<212> DNA
<213> Homo sapiens

<220>
<221> misc_feature
<223> CFLAR

<400> 15
gaccacgcat ggagaatttt accacccaga gacacgcgag tggccctgtg caagtttcaa 60

ctgcgcgggg ggcggggaat tccgcagaca ggattctaga atttatgtct tgtgtggtaa 120

catttcagcc ggtgggtggc ggggattagg cgtgaagcgg ttcagcaggc agaggttctc 180

ggacgccctc cggcgaagcc acctgttgat gcttttgact ttctgtcctt gttcctcgtc 240

ccatctggag catttccaat tctggttttg cggagcagca ggtctgagct tgtccggcga 300

gggtgggagt tggtcccggc ggagatccag tgggaagagc cggcggctgc ccgggcaact 360

cccccactgg aaaggattct gaaagaaatg aagtcagccc tcagaaatga agttgactgc 420

ctgctggctt tctgttgact ggcccggagc tgtactgcaa gacccttgtg agcttcccta 480

gtctaagagt aggatgtctg ctgaagtcat ccatcaggtt gaagaagcac ttgatacaga 540

tgagaaggag atgctgctct tttgtgccgg ggatgttgct atagatgtgg ttccacctaa 600

tgtcagggac cttctggata ttttacggga aagaggtaag ctgtctgtcg ggacttggc 660

tgaactgctc tacagagtga ggcgatttga cctgctcaaa cgtatcttga agatggacag 720

aaaagctgtg gagacccacc tgctcaggaa ccctcacctt gtttcggact atagagtgct 780

gatggcagag attggtgagg atttggataa atctgatgtg tcctcattaa ttttcctcat 840

gaaggattac atgggccgag gcaagataag caaggagaag agtttcttgg accttgtggt 900

tgagttggag aaactaaatc tggttgcccc agatcaactg gatttattag aaaaatgcct 960

aaagaacatc cacagaatag acctgaagac aaaaatccag aagtacaagc agtctgttca 1020
aggagcaggg acaagttaca ggaatgttct ccaagcagca atccaaaaga gtctcaagga 1080
tccttcaaat aacttcaggc tccataatgg gagaagtaaa gaacaaagac ttaaggaaca 1140
gcttggcgct caacaagaac cagtgaagaa atccattcag gaatcagaag ctttttttgcc 1200
tcagagcata cctgaagaga gatacaagat gaagagcaag cccctaggaa tctgcctgat 1260
aatcgattgc attggcaatg agacagagct tcttcgagac accttcactt ccctgggcta 1320
tgaagtccag aaattcttgc atctcagtat gcatggtata tcccagattc ttggccaatt 1380
tgcctgtatg cccgagcacc gagactacga cagctttgtg tgtgtcctgg tgagccgagg 1440
aggctcccag agtgtgtatg gtgtggatca gactcactca gggctccccc tgcatcacat 1500
caggaggatg ttcatgggag attcatgccc ttatctagca gggaagccaa agatgttttt 1560
tattcagaac tatgtggtgt cagagggcca gctggaggac agcagcctct tggaggtgga 1620
tgggccagcg atgaagaatg tggaattcaa ggctcagaag cgagggctgt gcacagttca 1680
ccgagaagct gacttcttct ggagcctgtg tactgcggac atgtccctgc tggagcagtc 1740
tcacagctca ccatccctgt acctgcagtg cctctcccag aaactgagac aagaaagaaa 1800
acgcccactc ctggatcttc acattgaact caatggctac atgtatgatt ggaacagcag 1860
agtttctgcc aaggagaaat attatgtctg gctgcagcac actctgagaa agaaacttat 1920
cctctcctac acataagaaa ccaaaaggct gggcgtagtg gctcacacct gtaatcccag 1980
cactttggga ggccaaggag ggcagatcac ttcaggtcag gagttcgaga ccagcctggc 2040
caacatggta aacgctgtcc ctagtaaaaa tacaaaaatt agctgggtgt gggtgtgggt 2100
acctgtattc ccagttactt gggaggctga ggtgggagga tcttttgaac ccaggagttc 2160
agggtcatag catgctgtga ttgtgcctac gaatagccac tgcataccaa cctgggcaat 2220
atagcaagat cccatctctt taaaaaaaaa aaaaaaggac aggaactatc ttactcaatg 2280
tattagtcat gtttctctag agggacagaa ctaataggat acatgtatat aaaaagggga 2340
gtttattaag gagtattgac tcacatgatc acagggttag gtcccacaat aggtcatctg 2400
caagcaagga agccaattca agtcccaaag ctgaagaact tggagtccaa tgtttgaggg 2460
caggaagcat tcagcatgag agaaagatgg aggccagaag actacaccag tctagtcttt 2520
ccatgttttg cctgctttta ttctggcagt gctggcagct gattagatgg tgcccaccca 2580
gattgaggat ggtctgcctt tcccagtcca ctgactcaaa tgttaaatct cctttggcag 2640
caccctcaca gatgtacccg ggaacacttt gcatccttct attcaatcaa gttgatactc 2700
agtattaacc atcacagtcc atttgggcaa ctataccaaa ttaccataga ccaggtgact 2760
taaacagcag ttatttctca cagttccgga ggctgggaaa tccaacatct aagtggtagc 2820

atatctggtg tctggtaagg catgcttcca gatcttacca gatgtcagtc ttttgatgtt 2880
ctcacatggc agaaaaagag gatgcaaact ctcaagtata tctttaaggg cacaaattcc 2940
attcatgagg gctctaccct catcacctaa ttacctccca aaggccccac cttctgatac 3000
tgtcactttg gggatactgt ctcccctttg aattctgggg ggaatacaaa cattcagttt 3060
gtaacaatag ccttatgatt tagaggttac ttgttcattc acctagacct caaattgcat 3120
tttacagcta gtcaagtata tctttctctg atttgatagt gtgacctaaa aggggaccat 3180
tgtttgaaat atcattagag ttgcttatta ttattattat tattattatt attattatta 3240
ttattattat tgagacagag tttcattctg ctgcccaggc tggagtgcag tggcatcatc 3300
ttggctcatt gcaacctctg ccttctgggt tcaagcgatt ctcctgcctc agcctcccga 3360
gtagctggga ttacaggctc ctgccaccac acccggctaa tttttgtatt tttagtggag 3420
acagggtttc caccatgttg gccagcgtgg tcttgaactc ctgacctcag gtgattcacc 3480
agcctcggcc tcccaaagtg ctgggattac aggtgtgagc cactgcacct ggcctattat 3540
tatttttaaa tttttttttt ttaattgatc attcttgggt gtttctcaca gagggtgatt 3600
tggcagggtc acaggacaat agtggaggga aggtcagcag ataaacaagt gaacaaaggt 3660
ctctggtttt cctaggcaga ggaccctgcg gccttccgca gtgtttgtgt ccctgggtac 3720
ttgagattag ggagtggtga tgactcttaa ggagcatgct gccttcaagc atctgtttaa 3780
caaagcacat cttgcactgc ccttaatcca tttaaccctg agtggacaca gcacatgttt 3840
cagagagcac agggttgggg gtaaggtcat agatcaacag catcctaagg cagaagaatt 3900
tttcttagta cagaacaaaa tgaagtctcc catgtctact tctttctaca cagacacagc 3960
aacaatctga tttctctatc ttttccccac ctttccccct tttctattcc acaaaaccgc 4020
catcgtcatc atggcctgtt ctcaatgagc tgttgggtac acctcccaga cggggtggcg 4080
gctgggcaga ggggctcctc acttcccaga tggggcggcc aggcggacgc gcccccccacc 4140
tccctcccgg acgggatagc tggccgggcg gggctgacc ccccacctcc ctccccgacg 4200
gggcggctgg ccgggcgggg gctgaccccc acgcctccct cccggacggg gcggctgcca 4260
ggcggagggg ctcctcactt ctcagacggg gtggctgctg ggcggagacg ctcctcactt 4320
cccagacagg gtggctgtcg ggcggagggg ctcctcactt ctcagacggg gcagctgcgg 4380
gcggaggggc tcctcacttc tcagacgggg tggccgggca gagaagctcc tcacatccca 4440
gacggggggg cggggcagag gcgctcccca catctcagac gatgggcggc cgggcagaga 4500
cgctcctcac ttcatcccag acggggtggc ggccgggcag aagctgtaat ctcggcaccc 4560
tgggggggcca aggcaggcgg ctgggaggcg gaggccgtag ccagctgaga tcacaccact 4620
gcactccagc ctgggcaaca ttgagcactg agtggacgag actctgcccg caatcccggc 4680
acctcgggag gccgaggctg gcagatcact cgcagtcagg agctggagac cagcccggcc 4740

aacacagtga aaccctgtct ccaccaaaaa aatacgaaaa ccagtcaggc gtggcggcgc 4800
ccgcaatggc aggcacgcgg caggccgagg cgggagaatc aggcagggag gctgcagtga 4860
gccgagatgg cagcagtaca gtccagcttc ggctcggcat cagagggaga ccgtggggag 4920
agggagaaga gagggagggg gagagggcta tttttaaaat tttttaaaat tgctgaacag 4980
gggtacctct gggcagtgtg tcagaatacc actttttaaa tattttatga tttatttatt 5040
tttctatttc ttgaggtttt aactgatgtg tatctgtatg tctatttgtg tatattttgt 5100
catgatcatg taacagagtc tgaaaagtgt cgaagagaca gttttcagga acaacaagca 5160
attattccta ctttccaagt tattttgatg ccatggtggc tcatacctat aatctgagta 5220
ctttgggagg ctgaggtgga ctgatcactt gagcccagga gtttgagacc agcctgggca 5280
acatagcaag actccatctc tacaaaaaaa gacaaaattt agctgagcgt ggtggcgtgt 5340
tcctgtagtc ccagctactt gggaggctga agtgagtgga tcccctgagc ccagagaggt 5400
caaggttgtg atgagctgtg atcacaccac tgcacttcag catgggagac agagtgagac 5460
cctgtttcag aaaaaataaa taaataaaac caccagcacc acaaacaaca acaaaaagtt 5520
attttgtact tgttttgagc acaggactcc tgagggtatc tttgcattta atattacata 5580
ggggtgccag tgggaagtaa tgtgtatgct tggcctcatg agctaaaacc ctgtgttaat 5640
tatgacagaa ggaaagtgtg tgagagagat cttaactacc tagcagctct agctgccatc 5700
ttgaaccatg aagatacggg ccacacgtag ggtagctgg gtagtgagca gcaagaagcc 5760
ttgttggatg agggcacgaa ggagcagaat cactggaatc actgtgtcag ccctaattac 5820
ctacctctgg acttttatgt gaggggaaaa aaaattgaca gtttatattt atctcaacct 5880
agttaaccca agtgatgcat tgttatgaga ttaaaatgtt tggaggccgg gtgcggtggc 5940
tcacgcctat aatcccagcc ctttgggagg ccaaggcggg cggatcacga ggtcaggaga 6000
tcaagaccat cctggctaac atgtaaaacc ccgtctctac taaaaataca aaaaattagc 6060
caggcgttgt ggcggtcgcc tgtagtccct gctatttggg aggccgaggc aagagaacgg 6120
catgaacctg ggaggtggag cttgcagcga gctgagatct tgccactgca ctccagcctg 6180
ggcgacagtg cgagactctg tctcaaaaat aaataaataa ataaataata aataaaatgt 6240
ttggaatgtt ggcttcatcc ctgggatgca aggctggttc aacatacgca aatcaagaaa 6300
cataattcat cacataaaca gaactaaaga caaaaaccac atgattatct caatagatac 6360
agaaaaggcc ttcaataaaa ttcaacgttg cttcatgtta aaaactctca ataaactagg 6420
tattgatgga aaatatctca aaataataac catttatgac aaacccacag ccattatcat 6480
actgaatggg caaaagctgg aagcattccc cttgaaaact ggcacaagac agggatgccg 6540
tctcaccact cctatttaac atagtattgg aagttctggc caagaaaatc aggcaagaga 6600

aacaaataag gggtattcaa ataggaaaag aggaagtaaa actgtgtttg cagatgacat 6660
gatactatat ctagaaaacc ccattatctc cacccaaaag ttccttaagc tgataagcaa 6720
cttcagcaaa gtctcaggat acaaaatcaa tgtgcagaaa tcacaagcat tctatacacc 6780
aacaatacac aagcagagag ccaaatcatg aatgaactcc cattcacagt tgctagaaag 6840
agaataaaat acctaggaat acagctaata agatgtgaag gatctcttca aggagaacta 6900
caaaccactg ctcaaggaaa taagagagga cacaaatgaa aaaacattcc attctcgtgg 6960
ataggaagaa tcaatatcat gaaaatggcc atactaccca aagtaattta taggttcatt 7020
gctattccca ttaaactact attgacattc ttcacagaat tagaaaaaaa ctactttaaa 7080
attcaaatgg aaccaaaaaa gagcccgtat aaccaagaca acaataagca aaaagaacaa 7140
agctggaagc atcacactac ccaacttcaa agtatactgc aaggctacag tagccaaaat 7200
ggcatggtac tggtacaaaa acagacacat agaccaatgg aacagaatag agaccagaga 7260
aagaagacca cacatctaca gccatctgat catcgacaaa cctgacaaaa acaagcaatg 7320
gggaaaagat tccctattta ataaatggtg ctgggaaaac tggctagcca tatgcagaaa 7380
attgaaactg accccttcct tacaccttat acaaaaatta actcaagatt aaagacttaa 7440
tgtaaaacct aaaactataa aaaccctaga agaaaatcta tttaatacca ttcaagacat 7500
aggcacaagc aaaggtttca tgacaaaaac atcaaaagca attgcaacaa aagcaaaaat 7560
tacaaatggg atctaattaa actaaagagc tcctgcacag caaaagaaac tatcattaga 7620
gtgaacaggc aacctacaga atgggagaac atttttgcaa tctatccatc tgacaaaggt 7680
ctaatatcca gaacctacaa ggaacttaaa acaaatttac aaggaaaaaa acaaccccat 7740
caaaaagtgg acaaaggaca tgaacagaca cttctcaaaa gaagacattt atgtggccaa 7800
caaacatata aaaaaagct caaccttact gatcattaga gaaatgcaaa ggagaaccac 7860
aatgagatac catctcatgc cggtcagaat ggtgattatt aaaaagtcaa aaaacaacag 7920
atgctggcga ggctgtggag aagtaggaac acttttacat tgttggtggg aatgtaaatt 7980
agttcaaccg ttgtggaagt gtgtgtggct attcctcaaa gatctagaac tagaaatact 8040
atttgtccca gcaatcccat tactgggtat atacccaaag gaatataaac cattttatta 8100
taaagataca tgcacatttt tgttcattgc agcactcttc acaatagcaa agacacaata 8160
gcaaatgccc atcaaagata gactggataa agaaaatgtg gtacatatac accatggaat 8220
actgtgcagt gcagccatta cagcttttgg tgatacagtg aatcagattt ttcattaatt 8280
cttttaattg gttattactg aacgtgaaaa agtaatgttt gtattgaaat cttgagtctg 8340
gccatgtttc tattttaaat tcataaagaa ttctaacaag aggaattcca agaatgtcat 8400
aaatggatgt ttctccatgg atgaaggaac tgttttattc acttgctgat aattcagcct 8460
aatccagttt gacatcatat agataagtag ttgaattatg gatttaaaat acatatcatt 8520

ttctaactcc aaaggtaata cttatttaaa tggttttgaa aatatagaaa ggcacaattt 8580
ctttttaaat ctgttattct ccaccaccac tcaatctgtc tatcatctat ctctccattc 8640
attcttccat ttgtttatat ctgttaatct ttgtatgtgt tcatgtatag cttttacatg 8700
attggaatca taatgcatat tccattttga agtctgcttt tttttacaca aaaatatgtt 8760
gtgaatattt tcctatatta tgaaatatca ttagctgagc ttttagaatt gactgcatgt 8820
tttggtacca tttagatata gtttaagata cttagaagtt atgtggcttt gccactatgg 8880
atgaatctta tttactcaat attaattact tacaaataac ctcacctaaa cactactcag 8940
ccataaaaag gaatgaatta atgacattca cagcaacctg gagactatta ctctaaagga 9000
agtaactgag gaatggaaaa ccaaacattg tatgttctca ctcataagtg ggagataagc 9060
tatgaggatg caaaggcata agaaggatac aatggacttt ggggacttag gggaaagggt 9120
gggagggggg tgaaggataa aagaatacaa attgggttca gtgtatactg ctcaggtgat 9180
gggtgcacca gaatctcaca agtaaccact taattactta cgcatgtaac cagataccac 9240
ctgttcccca aacacctatg gaaataattt tgtttttttt tttaaaaaag gaatgagatc 9300
atgtcctttg cagggacatg gatgaagctg gaagccatta tcctcagcaa actaacagag 9360
gagcaggaaa ccaaacacca catgttctca cttgtaagcg gaagctgaac aatgagaaca 9420
cacggacaca gggatgagat caacacacac tggggcctga tgcaggggcc gtagcgggga 9480
gagcatcagg ataactagct aatgcatgtg gggcttaata cctaggtgat aggttgatag 9540
gtgcagcaaa ccaccatggg acacgtttac ctatgtaaca aacccgcaca tcctgcactt 9600
gtatccagaa cttaaaatat tttaaaaatc tttagagaat acaaaaaaaa aaaaaaagat 9660
tcttcaatgc atacacaata aaattgcagt tcagtcaaac attggaagtc tttctctgac 9720
tgtctagttg gtatcttcat tttcagcttc ttcaagatcc cactccaaac actgttagct 9780
cagccaaatt gaacagctca tatctcctac ctctggatct ttggttctgg tgattgtata 9840
tttctggacc atctggaacc ccagcatatc accctacccc acatctccac atccccaaaa 9900
tataaccata cttcaagggc agttcaaata ccatctcctt ctatcctcca tgaagtcagt 9960
tatctcttcc attggaatta tcgcccccctc tcctgaacag tactatttcg tgtgaatctc 10020
ctccaagcct tcttttcatt ttatatctca tgctgtaatt cttggaaagt atgctgtagc 10080
tcaagtgcag aattctcatc agttttatct ttatatctct cctaaacact ttacctgatg 10140
aagagcctgg catacacata aatatatatt gaatgaatca gtgatggatt gaaaagagaa 10200
atgatggatc tcctaaattt taacttttat aaaatatttt gatacattca tgaccttact 10260
ttagcaagca atgaacgtga tgtaaactat tgttgatata gtttttatat tggaagtgta 10320
agtagtttgt ggcatgggat tgtgacatat cctaggtttc ctcatcttct tttattgaa 10380

atgtaattca caagccataa aatttgcccc tttaaagtaa atgatgcagt ggattttagt 10440

atatttacag agttgtgcaa tcatcaccac tatctaattc cagaacattt ccatctacct 10500

agaaactcca taccagtgag ctgccactct aatcctcctc ttcccccagc ctctagaaac 10560

aataatccat tttctgtctc tatgatttgc ctgttctaga tattttataa aaataaacat 10620

gtggcctttc gtgtctgact tccttcactt aaaaaaaaaa aaaaaaa 10668

<210> 16
<211> 1299
<212> DNA
<213> Homo sapiens

<220>
<221> misc_feature
<223> CFLAR

<400> 16
atactcagtc acacaagcca tagcaggaaa cagcgagctt gcagcctcac cgacgagtct 60

caactaaaag ggactcccgg agctaggggt ggggactcgg cctcacacag tgagtgccgg 120

ctattggact tttgtccagt gacagctgag acaacaagga ccacgggagg aggtgtagga 180

gagaagcgcc gcgaacagcg atcgcccagc accaagtccg cttccaggct ttcggtttct 240

ttgcctccat cttgggtgcg ccttcccggc gtctagggga gcgaaggctg aggtggcagc 300

ggcaggagag tccggccgcg acaggacgaa ctcccccact ggaaaggatt ctgaaagaaa 360

tgaagtcagc cctcagaaat gaagttgact gcctgctggc tttctgttga ctggcccgga 420

gctgtactgc aagacccttg tgagcttccc tagtctaaga gtaggatgtc tgctgaagtc 480

atccatcagg ttgaagaagc acttgataca gatgagaagg agatgctgct ctttttgtgc 540

cgggatgttg ctatagatgt ggttccacct aatgtcaggg accttctgga tattttacgg 600

gaaagaggta agctgtctgt cggggacttg gctgaactgc tctacagagt gaggcgattt 660

gacctgctca aacgtatctt gaagatggac agaaaagctg tggagaccca cctgctcagg 720

aaccctcacc ttgtttcgga ctatagagtg ctgatggcag agattggtga ggatttggat 780

aaatctgatg tgtcctcatt aattttcctc atgaaggatt acatgggccg aggcaagata 840

agcaaggaga agagtttctt ggaccttgtg gttgagttgg agaaactaaa tctggttgcc 900

ccagatcaac tggatttatt agaaaaatgc ctaaagaaca tccacagaat agacctgaag 960

acaaaaatcc agaagtacaa gcagtctgtt caaggagcag ggacaagtta caggaatgtt 1020

ctccaagcag caatccaaaa gagtctcaag gatccttcaa ataacttcag gatgataaca 1080

ccctatgccc attgtcctga tctgaaaatt cttggaaatt gttccatgtg attaacatgg 1140

aactgcctct acttaatcat tctgaatgat taaatcgttt cattttctaa atgtgttata 1200

atgtgtttag ccctttcttg ttgctgtatg tttagatgct ttccaatctt ttgttactac 1260

taataatgct ataaaataaa tatccttgta cttctttga 1299

<210> 17
<211> 9734
<212> DNA
<213> Homo sapiens

<220>
<221> misc_feature
<223> CFLAR

<400> 17
accttggact gaaccactat tgaagattta tttgataata atttgtaagt tttaactcag 60

tacctccctt ctgcttttat agttcaagga gcagggacaa gttacaggaa tgttctccaa 120

gcagcaatcc aaaagagtct caaggatcct tcaaataact tcaggctcca taatgggaga 180

agtaaagaac aaagacttaa ggaacagctt ggcgctcaac aagaaccagt gaagaaatcc 240

attcaggaat cagaagcttt tttgcctcag agcatacctg aagagagata caagatgaag 300

agcaagcccc taggaatctg cctgataatc gattgcattg gcaatgagac agagcttctt 360

cgagacacct tcacttccct gggctatgaa gtccagaaat tcttgcatct cagtatgcat 420

ggtatatccc agattcttgg ccaatttgcc tgtatgcccg agcaccgaga ctacgacagc 480

tttgtgtgtg tcctggtgag ccgaggaggc tcccagagtg tgtatggtgt ggatcagact 540

cactcagggc tccccctgca tcacatcagg aggatgttca tgggagattc atgcccttat 600

ctagcaggga agccaaagat gtttttttatt cagaactatg tggtgtcaga gggccagctg 660

gaggacagca gcctcttgga ggtggatggg ccagcgatga agaatgtgga attcaaggct 720

cagaagcgag ggctgtgcac agttcaccga gaagctgact tcttctggag cctgtgtact 780

gcggacatgt ccctgctgga gcagtctcac agctcaccat ccctgtacct gcagtgcctc 840

tcccagaaac tgagacaaga aagaaaacgc ccactcctgg atcttcacat tgaactcaat 900

ggctacatgt atgattggaa cagcagagtt tctgccaagg agaaatatta tgtctggctg 960

cagcacactc tgagaaagaa acttatcctc tcctacacat aagaaaccaa aaggctgggc 1020

gtagtggctc acacctgtaa tcccagcact ttgggaggcc aaggagggca gatcacttca 1080

ggtcaggagt tcgagaccag cctggccaac atggtaaacg ctgtccctag taaaaataca 1140

aaaattagct gggtgtgggt gtgggtacct gtattcccag ttacttggga ggctgaggtg 1200

ggaggatctt ttgaacccag gagttcaggg tcatagcatg ctgtgattgt gcctacgaat 1260

agccactgca taccaacctg ggcaatatag caagatccca tctctttaaa aaaaaaaaaa 1320

aaggacagga actatcttac tcaatgtatt agtcatgttt ctctagaggg acagaactaa 1380

taggatacat gtatataaaa aggggagttt attaaggagt attgactcac atgatcacag 1440

ggttaggtcc cacaataggt catctgcaag caaggaagcc aattcaagtc ccaaagctga 1500
agaacttgga gtccaatgtt tgagggcagg aagcattcag catgagagaa agatggaggc 1560
cagaagacta caccagtcta gtctttccat gttttgcctg cttttattct ggcagtgctg 1620
gcagctgatt agatggtgcc cacccagatt gaggatggtc tgcctttccc agtccactga 1680
ctcaaatgtt aaatctcctt tggcagcacc ctcacagatg tacccgggaa cactttgcat 1740
ccttctattc aatcaagttg atactcagta ttaaccatca cagtccattt gggcaactat 1800
accaaattac catagaccag gtgacttaaa cagcagttat ttctcacagt tccggaggct 1860
gggaaatcca acatctaagt ggtagcatat ctggtgtctg gtaaggcatg cttccagatc 1920
ttaccagatg tcagtctttt gatgttctca catggcagaa aaagaggatg caaactctca 1980
agtatatctt taagggcaca aattccattc atgagggctc taccctcatc acctaattac 2040
ctcccaaagg ccccaccttc tgatactgtc actttgggga tactgtctcc cctttgaatt 2100
ctgggggaa tacaaacatt cagtttgtaa caatagcctt atgatttaga ggttacttgt 2160
tcattcacct agacctcaaa ttgcatttta cagctagtca agtatatctt tctctgattt 2220
gatagtgtga cctaaaaggg gaccattgtt tgaaatatca ttagagttgc ttattattat 2280
tattattatt attattatta ttattattat tattattgag acagagtttc attctgctgc 2340
ccaggctgga gtgcagtggc atcatcttgg ctcattgcaa cctctgcctt ctgggttcaa 2400
gcgattctcc tgcctcagcc tcccgagtag ctgggattac aggctcctgc caccacaccc 2460
ggctaatttt tgtattttta gtggagacag ggtttccacc atgttggcca gcgtggtctt 2520
gaactcctga cctcaggtga ttcaccagcc tcggcctccc aaagtgctgg gattacaggt 2580
gtgagccact gcacctggcc tattattatt tttaaatttt tttttttaa ttgatcattc 2640
ttgggtgttt ctcacagagg gtgatttggc agggtcacag gacaatagtg gagggaaggt 2700
cagcagataa acaagtgaac aaaggtctct ggttttccta ggcagaggac cctgcggcct 2760
tccgcagtgt ttgtgtccct gggtacttga gattagggag tggtgatgac tcttaaggag 2820
catgctgcct tcaagcatct gtttaacaaa gcacatcttg cactgccctt aatccattta 2880
accctgagtg gacacagcac atgtttcaga gagcacaggg ttgggggtaa ggtcatagat 2940
caacagcatc ctaaggcaga agaatttttc ttagtacaga acaaaatgaa gtctcccatg 3000
tctacttctt tctacacaga cacagcaaca atctgatttc tctatctttt ccccaccttt 3060
ccccctttc tattccacaa aaccgccatc gtcatcatgg cctgttctca atgagctgtt 3120
gggtacacct cccagacggg gtggcggctg ggcagagggg ctcctcactt cccagatggg 3180
gcggccaggc ggacgcgccc cccacctccc tcccggacgg gatagctggc cgggcggggg 3240
ctgaccccc acctccctcc ccgacggggc ggctggccgg gcgggggctg acccccacgc 3300
ctccctcccg gacggggcgg ctgccaggcg gaggggctcc tcacttctca gacggggtgg 3360

ctgctgggcg gagacgctcc tcacttccca gacagggtgg ctgtcgggcg gaggggctcc 3420
tcacttctca gacggggcag ctgcgggcgg aggggctcct cacttctcag acggggtggc 3480
cgggcagaga agctcctcac atcccagacg ggggggcggg gcagaggcgc tccccacatc 3540
tcagacgatg ggcggccggg cagagacgct cctcacttca tcccagacgg ggtggcggcc 3600
gggcagaagc tgtaatctcg gcaccctggg gggccaaggc aggcggctgg gaggcggagg 3660
ccgtagccag ctgagatcac accactgcac tccagcctgg gcaacattga gcactgagtg 3720
gacgagactc tgcccgcaat cccggcacct cgggaggccg aggctggcag atcactcgca 3780
gtcaggagct ggagaccagc ccggccaaca cagtgaaacc ctgtctccac caaaaaaata 3840
cgaaaaccag tcaggcgtgg cggcgcccgc aatggcaggc acgcggcagg ccgaggcggg 3900
agaatcaggc agggaggctg cagtgagccg agatggcagc agtacagtcc agcttcggct 3960
cggcatcaga gggagaccgt ggggagaggg agaagagagg gagggggaga gggctatttt 4020
taaaattttt taaaattgct gaacaggggt acctctgggc agtgtgtcag aataccactt 4080
tttaaatatt ttatgattta tttatttttc tatttcttga ggttttaact gatgtgtatc 4140
tgtatgtcta tttgtgtata ttttgtcatg atcatgtaac agagtctgaa aagtgtcgaa 4200
gagacagttt tcaggaacaa caagcaatta ttcctacttt ccaagttatt ttgatgccat 4260
ggtggctcat acctataatc tgagtacttt gggaggctga ggtggactga tcacttgagc 4320
ccaggagttt gagaccagcc tgggcaacat agcaagactc catctctaca aaaaaagaca 4380
aaatttagct gagcgtggtg gcgtgttcct gtagtcccag ctacttggga ggctgaagtg 4440
agtggatccc ctgagcccag agaggtcaag gttgtgatga gctgtgatca caccactgca 4500
cttcagcatg ggagacagag tgagaccctg tttcagaaaa aataaataaa taaaaccacc 4560
agcaccacaa acaacaacaa aaagttattt tgtacttgtt ttgagcacag gactcctgag 4620
ggtatctttg catttaatat tacatagggg tgccagtggg aagtaatgtg tatgcttggc 4680
ctcatgagct aaaaccctgt gttaattatg acagaaggaa agtgtgtgag agagatctta 4740
actacctagc agctctagct gccatcttga accatgaaga tacgggccac acgtaggggt 4800
agctgggtag tgagcagcaa gaagccttgt tggatgaggg cacgaaggag cagaatcact 4860
ggaatcactg tgtcagccct aattacctac ctctggactt ttatgtgagg ggaaaaaaaa 4920
ttgacagttt atatttatct caacctagtt aacccaagtg atgcattgtt atgagattaa 4980
aatgtttgga ggccgggtgc ggtggctcac gcctataatc ccagcccttt gggaggccaa 5040
ggcgggcgga tcacgaggtc aggagatcaa gaccatcctg gctaacatgt aaaaccccgt 5100
ctctactaaa aatacaaaaa attagccagg cgttgtggcg gtcgcctgta gtccctgcta 5160
tttgggaggc cgaggcaaga gaacggcatg aacctgggag gtggagcttg cagcgagctg 5220

agatcttgcc actgcactcc agcctgggcg acagtgcgag actctgtctc aaaaataaat 5280
aaataaataa ataataaata aaatgtttgg aatgttggct tcatccctgg gatgcaaggc 5340
tggttcaaca tacgcaaatc aagaaacata attcatcaca taaacagaac taaagacaaa 5400
aaccacatga ttatctcaat agatacagaa aaggccttca ataaaattca acgttgcttc 5460
atgttaaaaa ctctcaataa actaggtatt gatggaaaat atctcaaaat aataaccatt 5520
tatgacaaac ccacagccat tatcatactg aatgggcaaa agctggaagc attccccttg 5580
aaaactggca caagacaggg atgccgtctc accactccta tttaacatag tattggaagt 5640
tctggccaag aaaatcaggc aagagaaaca aataaggggt attcaaatag gaaaagagga 5700
agtaaaactg tgtttgcaga tgacatgata ctatatctag aaaaccccat tatctccacc 5760
caaaagttcc ttaagctgat aagcaacttc agcaaagtct caggatacaa aatcaatgtg 5820
cagaaatcac aagcattcta tacaccaaca atacacaagc agagagccaa atcatgaatg 5880
aactcccatt cacagttgct agaaagagaa taaaatacct aggaatacag ctaataagat 5940
gtgaaggatc tcttcaagga gaactacaaa ccactgctca aggaaataag agaggacaca 6000
aatgaaaaaa cattccattc tcgtggatag gaagaatcaa tatcatgaaa atggccatac 6060
tacccaaagt aatttatagg ttcattgcta ttcccattaa actactattg acattcttca 6120
cagaattaga aaaaaactac tttaaaattc aaatggaacc aaaaaagagc ccgtataacc 6180
aagacaacaa taagcaaaaa gaacaaagct ggaagcatca cactacccaa cttcaaagta 6240
tactgcaagg ctacagtagc caaaatggca tggtactggt acaaaaacag acacatagac 6300
caatggaaca gaatagagac cagagaaaga agaccacaca tctacagcca tctgatcatc 6360
gacaaacctg acaaaaacaa gcaatgggga aaagattccc tatttaataa atggtgctgg 6420
gaaaactggc tagccatatg cagaaaattg aaactgaccc cttccttaca ccttatacaa 6480
aaattaactc aagattaaag acttaatgta aaacctaaaa ctataaaaac cctagaagaa 6540
aatctattta ataccattca agacataggc acaagcaaag gtttcatgac aaaaacatca 6600
aaagcaattg caacaaaagc aaaaattaca aatgggatct aattaaacta aagagctcct 6660
gcacagcaaa agaaactatc attagagtga acaggcaacc tacagaatgg gagaacattt 6720
ttgcaatcta tccatctgac aaaggtctaa tatccagaac ctacaaggaa cttaaaacaa 6780
atttacaagg aaaaaaacaa ccccatcaaa aagtggacaa aggacatgaa cagacacttc 6840
tcaaaagaag acatttatgt ggccaacaaa catataaaaa aaagctcaac cttactgatc 6900
attagagaaa tgcaaaggag aaccacaatg agataccatc tcatgccggt cagaatggtg 6960
attattaaaa agtcaaaaaa caacagatgc tggcgaggct gtggagaagt aggaacactt 7020
ttacattgtt ggtgggaatg taaattagtt caaccgttgt ggaagtgtgt gtggctattc 7080
ctcaaagatc tagaactaga aatactattt gtcccagcaa tcccattact gggtatatac 7140

ccaaaggaat ataaaccatt ttattataaa gatacatgca catttttgtt cattgcagca 7200
ctcttcacaa tagcaaagac acaatagcaa atgcccatca aagatagact ggataaagaa 7260
aatgtggtac atatacacca tggaatactg tgcagtgcag ccattacagc ttttggtgat 7320
acagtgaatc agatttttca ttaattcttt taattggtta ttactgaacg tgaaaaagta 7380
atgtttgtat tgaaatcttg agtctggcca tgtttctatt ttaaattcat aaagaattct 7440
aacaagagga attccaagaa tgtcataaat ggatgtttct ccatggatga aggaactgtt 7500
ttattcactt gctgataatt cagcctaatc cagtttgaca tcatatagat aagtagttga 7560
attatggatt taaaatacat atcattttct aactccaaag gtaatactta tttaaatggt 7620
tttgaaaata tagaaaggca caatttcttt ttaaatctgt tattctccac caccactcaa 7680
tctgtctatc atctatctct ccattcattc ttccatttgt ttatatctgt taatctttgt 7740
atgtgttcat gtatagcttt tacatgattg gaatcataat gcatattcca ttttgaagtc 7800
tgcttttttt tacacaaaaa tatgttgtga atattttcct atattatgaa atatcattag 7860
ctgagctttt agaattgact gcatgttttg gtaccattta gatatagttt aagatactta 7920
gaagttatgt ggctttgcca ctatggatga atcttattta ctcaatatta attacttaca 7980
aataacctca cctaaacact actcagccat aaaaaggaat gaattaatga cattcacagc 8040
aacctggaga ctattactct aaaggaagta actgaggaat ggaaaaccaa acattgtatg 8100
ttctcactca taagtgggag ataagctatg aggatgcaaa ggcataagaa ggatacaatg 8160
gactttgggg acttagggga aagggtggga ggggggtgaa ggataaaaga atacaaattg 8220
ggttcagtgt atactgctca ggtgatgggt gcaccagaat ctcacaagta accacttaat 8280
tacttacgca tgtaaccaga taccacctgt tccccaaaca cctatggaaa taattttgtt 8340
ttttttttta aaaaaggaat gagatcatgt cctttgcagg gacatggatg aagctggaag 8400
ccattatcct cagcaaacta acagaggagc aggaaaccaa acaccacatg ttctcacttg 8460
taagcggaag ctgaacaatg agaacacacg gacacaggga tgagatcaac acacactggg 8520
gcctgatgca ggggccgtag cggggagagc atcaggataa ctagctaatg catgtggggc 8580
ttaataccta ggtgataggt tgataggtgc agcaaaccac catgggacac gtttacctat 8640
gtaacaaacc cgcacatcct gcacttgtat ccagaactta aaatatttta aaaatcttta 8700
gagaatacaa aaaaaaaaaa aaagattctt caatgcatac acaataaaat tgcagttcag 8760
tcaaacattg gaagtctttc tctgactgtc tagttggtat cttcattttc agcttcttca 8820
agatcccact ccaaacactg ttagctcagc caaattgaac agctcatatc tcctacctct 8880
ggatctttgg ttctggtgat tgtatatttc tggaccatct ggaaccccag catatcaccc 8940
taccccacat ctccacatcc ccaaaatata accatacttc aagggcagtt caaataccat 9000

ctccttctat cctccatgaa gtcagttatc tcttccattg gaattatcgc cccctctcct 9060

gaacagtact atttcgtgtg aatctcctcc aagccttctt ttcattttat atctcatgct 9120

gtaattcttg gaaagtatgc tgtagctcaa gtgcagaatt ctcatcagtt ttatctttat 9180

atctctccta aacactttac ctgatgaaga gcctggcata cacataaata tatattgaat 9240

gaatcagtga tggattgaaa agagaaatga tggatctcct aaattttaac ttttataaaa 9300

tattttgata cattcatgac cttactttag caagcaatga acgtgatgta aactattgtt 9360

gatatagttt ttatattgga agtgtaagta gtttgtggca tgggattgtg acatatccta 9420

ggtttcctca tcttcttttt attgaaatgt aattcacaag ccataaaatt tgcccctta 9480

aagtaaatga tgcagtggat tttagtatat ttacagagtt gtgcaatcat caccactatc 9540

taattccaga acatttccat ctacctagaa actccatacc agtgagctgc cactctaatc 9600

ctcctcttcc cccagcctct agaaacaata atccattttc tgtctctatg atttgcctgt 9660

tctagatatt ttataaaaat aaacatgtgg cctttcgtgt ctgacttcct tcacttaaaa 9720

aaaaaaaaaa aaaa 9734

<210> 18
<211> 10535
<212> DNA
<213> Homo sapiens

<220>
<221> misc_feature
<223> CFLAR

<400> 18
atactcagtc acacaagcca tagcaggaaa cagcgagctt gcagcctcac cgacgagtct 60

caactaaaag ggactcccgg agctaggggt ggggactcgg cctcacacag tgagtgccgg 120

ctattggact tttgtccagt gacagctgag acaacaagga ccacgggagg aggtgtagga 180

gagaagcgcc gcgaacagcg atcgcccagc accaagtccg cttccaggct ttcggtttct 240

ttgcctccat cttgggtgcg ccttcccggc gtctagggga gcgaaggctg aggtggcagc 300

ggcaggagag tccggccgcg acaggacgaa ctcccccact ggaaaggatt ctgaaagaaa 360

tgaagtcagc cctcagaaat gaagttgact gcctgctggc tttctgttga ctggcccgga 420

gctgtactgc aagacccttg tgagcttccc tagtctaaga gtaggatgtc tgctgaagtc 480

atccatcagg ttgaagaagc acttgataca gatgagaagg agatgctgct ctttttgtgc 540

cgggatgttg ctatagatgt ggttccacct aatgtcaggg accttctgga tattttacgg 600

gaaagaggta agctgtctgt cggggacttg gctgaactgc tctacagagt gaggcgattt 660

gacctgctca aacgtatctt gaagatggac agaaaagctg tggagaccca cctgctcagg 720

aaccctcacc ttgtttcgga ctatagagtg ctgatggcag agattggtga ggatttggat 780

aaatctgatg tgtcctcatt aattttcctc atgaaggatt acatgggccg aggcaagata 840
agcaaggaga agagtttctt ggaccttgtg gttgagttgg agaaactaaa tctggttgcc 900
ccagatcaac tggatttatt agaaaaatgc ctaaagaaca tccacagaat agacctgaag 960
acaaaaatcc agaagtacaa gcagtctgtt caaggagcag ggacaagtta caggaatgtt 1020
ctccaagcag caatccaaaa gagtctcaag gatccttcaa ataacttcag gagcatacct 1080
gaagagagat acaagatgaa gagcaagccc ctaggaatct gcctgataat cgattgcatt 1140
ggcaatgaga cagagcttct tcgagacacc ttcacttccc tgggctatga agtccagaaa 1200
ttcttgcatc tcagtatgca tggtatatcc cagattcttg gccaatttgc ctgtatgccc 1260
gagcaccgag actacgacag ctttgtgtgt gtcctggtga gccgaggagg ctcccagagt 1320
gtgtatggtg tggatcagac tcactcaggg ctcccccctgc atcacatcag gaggatgttc 1380
atgggagatt catgccctta tctagcaggg aagccaaaga tgttttttat tcagaactat 1440
gtggtgtcag agggccagct ggaggacagc agcctcttgg aggtggatgg gccagcgatg 1500
aagaatgtgg aattcaaggc tcagaagcga gggctgtgca cagttcaccg agaagctgac 1560
ttcttctgga gcctgtgtac tgcggacatg tccctgctgg agcagtctca cagctcacca 1620
tccctgtacc tgcagtgcct ctcccagaaa ctgagacaag aaagaaaacg cccactcctg 1680
gatcttcaca ttgaactcaa tggctacatg tatgattgga acagcagagt ttctgccaag 1740
gagaaatatt atgtctggct gcagcacact ctgagaaaga aacttatcct ctcctacaca 1800
taagaaacca aaaggctggg cgtagtggct cacacctgta atcccagcac tttgggaggc 1860
caaggagggc agatcacttc aggtcaggag ttcgagacca gcctggccaa catggtaaac 1920
gctgtcccta gtaaaaatac aaaaattagc tgggtgtggg tgtgggtacc tgtattccca 1980
gttacttggg aggctgaggt gggaggatct tttgaaccca ggagttcagg gtcatagcat 2040
gctgtgattg tgcctacgaa tagccactgc ataccaacct gggcaatata gcaagatccc 2100
atctctttaa aaaaaaaaaa aaaggacagg aactatctta ctcaatgtat tagtcatgtt 2160
tctctagagg gacagaacta ataggataca tgtatataaa aaggggagtt tattaaggag 2220
tattgactca catgatcaca gggttaggtc ccacaatagg tcatctgcaa gcaaggaagc 2280
caattcaagt cccaaagctg aagaacttgg agtccaatgt ttgagggcag gaagcattca 2340
gcatgagaga aagatggagg ccagaagact acaccagtct agtctttcca tgttttgcct 2400
gcttttattc tggcagtgct ggcagctgat tagatggtgc ccacccagat tgaggatggt 2460
ctgcctttcc cagtccactg actcaaatgt taaatctcct ttggcagcac cctcacagat 2520
gtacccggga acactttgca tccttctatt caatcaagtt gatactcagt attaaccatc 2580
acagtccatt tgggcaacta taccaaatta ccatagacca ggtgacttaa acagcagtta 2640

tttctcacag ttccggaggc tgggaaatcc aacatctaag tggtagcata tctggtgtct 2700
ggtaaggcat gcttccagat cttaccagat gtcagtcttt tgatgttctc acatggcaga 2760
aaaagaggat gcaaactctc aagtatatct ttaagggcac aaattccatt catgagggct 2820
ctaccctcat cacctaatta cctcccaaag gccccacctt ctgatactgt cactttgggg 2880
atactgtctc ccctttgaat tctgggggga atacaaacat tcagtttgta acaatagcct 2940
tatgatttag aggttacttg ttcattcacc tagacctcaa attgcatttt acagctagtc 3000
aagtatatct ttctctgatt tgatagtgtg acctaaaagg ggaccattgt ttgaaatatc 3060
attagagttg cttattatta ttattattat tattattatt attattatta ttattattga 3120
gacagagttt cattctgctg cccaggctgg agtgcagtgg catcatcttg gctcattgca 3180
acctctgcct tctgggttca agcgattctc ctgcctcagc ctcccgagta gctgggatta 3240
caggctcctg ccaccacacc cggctaattt ttgtattttt agtggagaca gggtttccac 3300
catgttggcc agcgtggtct tgaactcctg acctcaggtg attcaccagc ctcggcctcc 3360
caaagtgctg ggattacagg tgtgagccac tgcacctggc ctattattat ttttaaattt 3420
ttttttttta attgatcatt cttgggtgtt tctcacagag ggtgatttgg cagggtcaca 3480
ggacaatagt ggagggaagg tcagcagata aacaagtgaa caaaggtctc tggttttcct 3540
aggcagagga ccctgcggcc ttccgcagtg tttgtgtccc tgggtacttg agattaggga 3600
gtggtgatga ctcttaagga gcatgctgcc ttcaagcatc tgtttaacaa agcacatctt 3660
gcactgccct taatccattt aaccctgagt ggacacagca catgtttcag agagcacagg 3720
gttgggggta aggtcataga tcaacagcat cctaaggcag aagaattttt cttagtacag 3780
aacaaaatga agtctcccat gtctacttct ttctacacag acacagcaac aatctgattt 3840
ctctatcttt tccccacctt tcccccttttt ctattccaca aaaccgccat cgtcatcatg 3900
gcctgttctc aatgagctgt tgggtacacc tcccagacgg ggtggcggct gggcagaggg 3960
gctcctcact tcccagatgg ggcggccagg cggacgcgcc ccccacctcc ctcccggacg 4020
ggatagctgg ccgggcgggg gctgaccccc cacctccctc cccgacgggg cggctggccg 4080
ggcgggggct gacccccacg cctccctccc ggacggggcg gctgccaggc ggaggggctc 4140
ctcacttctc agacggggtg gctgctgggc ggagacgctc ctcacttccc agacagggtg 4200
gctgtcgggc ggaggggctc ctcacttctc agacggggca gctgcgggcg gaggggctcc 4260
tcacttctca gacggggtgg ccgggcagag aagctcctca catcccagac ggggggggcgg 4320
ggcagaggcg ctccccacat ctcagacgat gggcggccgg gcagagacgc tcctcacttc 4380
atcccagacg gggtggcggc cgggcagaag ctgtaatctc ggcaccctgg ggggccaagg 4440
caggcggctg ggaggcggag gccgtagcca gctgagatca caccactgca ctccagcctg 4500
ggcaacattg agcactgagt ggacgagact ctgcccgcaa tcccggcacc tcgggaggcc 4560

gaggctggca gatcactcgc agtcaggagc tggagaccag cccggccaac acagtgaaac 4620
cctgtctcca ccaaaaaaat acgaaaacca gtcaggcgtg gcggcgcccg caatggcagg 4680
cacgcggcag gccgaggcgg gagaatcagg cagggaggct gcagtgagcc gagatggcag 4740
cagtacagtc cagcttcggc tcggcatcag agggagaccg tggggagagg gagaagagag 4800
ggagggggag agggctattt ttaaaatttt ttaaaattgc tgaacagggg tacctctggg 4860
cagtgtgtca gaataccact ttttaaatat tttatgattt atttattttt ctatttcttg 4920
aggttttaac tgatgtgtat ctgtatgtct atttgtgtat attttgtcat gatcatgtaa 4980
cagagtctga aaagtgtcga agagacagtt ttcaggaaca acaagcaatt attcctactt 5040
tccaagttat tttgatgcca tggtggctca tacctataat ctgagtactt tgggaggctg 5100
aggtggactg atcacttgag cccaggagtt tgagaccagc ctgggcaaca tagcaagact 5160
ccatctctac aaaaaaagac aaaatttagc tgagcgtggt ggcgtgttcc tgtagtccca 5220
gctacttggg aggctgaagt gagtggatcc cctgagccca gagaggtcaa ggttgtgatg 5280
agctgtgatc acaccactgc acttcagcat gggagacaga gtgagaccct gtttcagaaa 5340
aaataaataa ataaaaccac cagcaccaca aacaacaaca aaaagttatt ttgtacttgt 5400
tttgagcaca ggactcctga gggtatcttt gcatttaata ttacataggg gtgccagtgg 5460
gaagtaatgt gtatgcttgg cctcatgagc taaaaccctg tgttaattat gacagaagga 5520
aagtgtgtga gagagatctt aactacctag cagctctagc tgccatcttg aaccatgaag 5580
atacgggcca cacgtagggg tagctgggta gtgagcagca agaagccttg ttggatgagg 5640
gcacgaagga gcagaatcac tggaatcact gtgtcagccc taattaccta cctctggact 5700
tttatgtgag gggaaaaaaa attgacagtt tatatttatc tcaacctagt taacccaagt 5760
gatgcattgt tatgagatta aaatgtttgg aggccgggtg cggtggctca cgcctataat 5820
cccagccctt tgggaggcca aggcgggcgg atcacgaggt caggagatca agaccatcct 5880
ggctaacatg taaaaccccg tctctactaa aaatacaaaa aattagccag gcgttgtggc 5940
ggtcgcctgt agtccctgct atttgggagg ccgaggcaag agaacggcat gaacctggga 6000
ggtggagctt gcagcgagct gagatcttgc cactgcactc cagcctgggc gacagtgcga 6060
gactctgtct caaaaataaa taaataaata aataataaat aaaatgtttg gaatgttggc 6120
ttcatccctg ggatgcaagg ctggttcaac atacgcaaat caagaaacat aattcatcac 6180
ataaacagaa ctaaagacaa aaaccacatg attatctcaa tagatacaga aaaggccttc 6240
aataaaattc aacgttgctt catgttaaaa actctcaata aactaggtat tgatggaaaa 6300
tatctcaaaa taataaccat ttatgacaaa cccacagcca ttatcatact gaatgggcaa 6360
aagctggaag cattcccctt gaaaactggc acaagacagg gatgccgtct caccactcct 6420

atttaacata gtattggaag ttctggccaa gaaaatcagg caagagaaac aaataagggg 6480
tattcaaata ggaaaagagg aagtaaaact gtgtttgcag atgacatgat actatatcta 6540
gaaaacccca ttatctccac ccaaaagttc cttaagctga taagcaactt cagcaaagtc 6600
tcaggataca aaatcaatgt gcagaaatca caagcattct atacaccaac aatacacaag 6660
cagagagcca aatcatgaat gaactcccat tcacagttgc tagaaagaga ataaaatacc 6720
taggaataca gctaataaga tgtgaaggat ctcttcaagg agaactacaa accactgctc 6780
aaggaaataa gagaggacac aaatgaaaaa acattccatt ctcgtggata ggaagaatca 6840
atatcatgaa aatggccata ctacccaaag taatttatag gttcattgct attcccatta 6900
aactactatt gacattcttc acagaattag aaaaaaacta ctttaaaatt caaatggaac 6960
caaaaaagag cccgtataac caagacaaca ataagcaaaa agaacaaagc tggaagcatc 7020
acactaccca acttcaaagt atactgcaag gctacagtag ccaaaatggc atggtactgg 7080
tacaaaaaca gacacataga ccaatggaac agaatagaga ccagagaaag aagaccacac 7140
atctacagcc atctgatcat cgacaaacct gacaaaaaca agcaatgggg aaaagattcc 7200
ctatttaata aatggtgctg ggaaaactgg ctagccatat gcagaaaatt gaaactgacc 7260
ccttccttac accttataca aaaattaact caagattaaa gacttaatgt aaaacctaaa 7320
actataaaaa ccctagaaga aaatctattt aataccattc aagacatagg cacaagcaaa 7380
ggtttcatga caaaaacatc aaaagcaatt gcaacaaaag caaaaattac aaatgggatc 7440
taattaaact aaagagctcc tgcacagcaa aagaaactat cattagagtg aacaggcaac 7500
ctacagaatg ggagaacatt tttgcaatct atccatctga caaaggtcta atatccagaa 7560
cctacaagga acttaaaaca aatttacaag gaaaaaaaca accccatcaa aaagtggaca 7620
aaggacatga acagacactt ctcaaaagaa gacatttatg tggccaacaa acatataaaa 7680
aaaagctcaa ccttactgat cattagagaa atgcaaagga gaaccacaat gagataccat 7740
ctcatgccgg tcagaatggt gattattaaa aagtcaaaaa acaacagatg ctggcgaggc 7800
tgtggagaag taggaacact tttacattgt tggtgggaat gtaaattagt tcaaccgttg 7860
tggaagtgtg tgtggctatt cctcaaagat ctagaactag aaatactatt tgtcccagca 7920
atcccattac tgggtatata cccaaaggaa tataaaccat tttattataa agatacatgc 7980
acatttttgt tcattgcagc actcttcaca atagcaaaga cacaatagca aatgcccatc 8040
aaagatagac tggataaaga aaatgtggta catatacacc atggaatact gtgcagtgca 8100
gccattacag cttttggtga tacagtgaat cagatttttc attaattctt ttaattggtt 8160
attactgaac gtgaaaaagt aatgtttgta ttgaaatctt gagtctggcc atgtttctat 8220
tttaaattca taaagaattc taacaagagg aattccaaga atgtcataaa tggatgtttc 8280
tccatggatg aaggaactgt tttattcact tgctgataat tcagcctaat ccagtttgac 8340

atcatataga taagtagttg aattatggat ttaaaataca tatcattttc taactccaaa 8400
ggtaatactt atttaaatgg ttttgaaaat atagaaaggc acaatttctt tttaaatctg 8460
ttattctcca ccaccactca atctgtctat catctatctc tccattcatt cttccatttg 8520
tttatatctg ttaatctttg tatgtgttca tgtatagctt ttacatgatt ggaatcataa 8580
tgcatattcc attttgaagt ctgctttttt ttacacaaaa atatgttgtg aatattttcc 8640
tatattatga aatatcatta gctgagcttt tagaattgac tgcatgtttt ggtaccattt 8700
agatatagtt taagatactt agaagttatg tggctttgcc actatggatg aatcttattt 8760
actcaatatt aattacttac aaataacctc acctaaacac tactcagcca taaaaaggaa 8820
tgaattaatg acattcacag caacctggag actattactc taaaggaagt aactgaggaa 8880
tggaaaacca aacattgtat gttctcactc ataagtggga gataagctat gaggatgcaa 8940
aggcataaga aggatacaat ggactttggg gacttagggg aaagggtggg agggggtga 9000
aggataaaag aatacaaatt gggttcagtg tatactgctc aggtgatggg tgcaccagaa 9060
tctcacaagt aaccacttaa ttacttacgc atgtaaccag ataccacctg ttccccaaac 9120
acctatggaa ataattttgt tttttttttt aaaaaaggaa tgagatcatg tcctttgcag 9180
ggacatggat gaagctggaa gccattatcc tcagcaaact aacagaggag caggaaacca 9240
aacaccacat gttctcactt gtaagcggaa gctgaacaat gagaacacac ggacacaggg 9300
atgagatcaa cacacactgg ggcctgatgc aggggccgta gcggggagag catcaggata 9360
actagctaat gcatgtgggg cttaatacct aggtgatagg ttgataggtg cagcaaacca 9420
ccatgggaca cgtttaccta tgtaacaaac ccgcacatcc tgcacttgta tccagaactt 9480
aaaatatttt aaaaatcttt agagaataca aaaaaaaaaa aaaagattct tcaatgcata 9540
cacaataaaa ttgcagttca gtcaaacatt ggaagtcttt ctctgactgt ctagttggta 9600
tcttcatttt cagcttcttc aagatcccac tccaaacact gttagctcag ccaaattgaa 9660
cagctcatat ctcctacctc tggatctttg gttctggtga ttgtatattt ctggaccatc 9720
tggaacccca gcatatcacc ctaccccaca tctccacatc cccaaaatat aaccatactt 9780
caagggcagt tcaaatacca tctccttcta tcctccatga agtcagttat ctcttccatt 9840
ggaattatcg ccccctctcc tgaacagtac tatttcgtgt gaatctcctc caagccttct 9900
tttcatttta tatctcatgc tgtaattctt ggaaagtatg ctgtagctca agtgcagaat 9960
tctcatcagt tttatcttta tatctctcct aaacacttta cctgatgaag agcctggcat 10020
acacataaat atatattgaa tgaatcagtg atggattgaa aagagaaatg atggatctcc 10080
taaattttaa cttttataaa atattttgat acattcatga ccttacttta gcaagcaatg 10140
aacgtgatgt aaactattgt tgatatagtt tttatattgg aagtgtaagt agtttgtggc 10200

atgggattgt gacatatcct aggtttcctc atcttctttt tattgaaatg taattcacaa 10260

gccataaaat ttgccccttt aaagtaaatg atgcagtgga ttttagtata tttacagagt 10320

tgtgcaatca tcaccactat ctaattccag aacatttcca tctacctaga aactccatac 10380

cagtgagctg ccactctaat cctcctcttc ccccagcctc tagaaacaat aatccatttt 10440

ctgtctctat gatttgcctg ttctagatat tttataaaaa taaacatgtg gcctttcgtg 10500

tctgacttcc ttcacttaaa aaaaaaaaaa aaaaa 10535

<210> 19
<211> 10222
<212> DNA
<213> Homo sapiens

<220>
<221> misc_feature
<223> CFLAR

<400> 19
atactcagtc acacaagcca tagcaggaaa cagcgagctt gcagcctcac cgacgagtct 60

caactaaaag ggactcccgg agctaggggt ggggactcgg cctcacacag tgagtgccgg 120

ctattggact tttgtccagt gacagctgag acaacaagga ccacgggagg aggtgtagga 180

gagaagcgcc gcgaacagcg atcgcccagc accaagtccg cttccaggct ttcggtttct 240

ttgcctccat cttgggtgcg ccttcccggc gtctagggga gcgaaggctg aggtggcagc 300

ggcaggagag tccggccgcg acaggacgag tgctgatggc agagattggt gaggatttgg 360

ataaatctga tgtgtcctca ttaattttcc tcatgaagga ttacatgggc cgaggcaaga 420

taagcaagga gaagagtttc ttggaccttg tggttgagtt ggagaaacta aatctggttg 480

ccccagatca actggattta ttagaaaaat gcctaaagaa catccacaga atagacctga 540

agacaaaaat ccagaagtac aagcagtctg ttcaaggagc agggacaagt tacaggaatg 600

ttctccaagc agcaatccaa aagagtctca aggatccttc aaataacttc aggctccata 660

atgggagaag taaagaacaa agacttaagg aacagcttgg cgctcaacaa gaaccagtga 720

agaaatccat tcaggaatca gaagcttttt tgcctcagag catacctgaa gagagataca 780

agatgaagag caagccccta ggaatctgcc tgataatcga ttgcattggc aatgagacag 840

agcttcttcg agacaccttc acttccctgg gctatgaagt ccagaaattc ttgcatctca 900

gtatgcatgg tatatcccag attcttggcc aatttgcctg tatgcccgag caccgagact 960

acgacagctt tgtgtgtgtc ctggtgagcc gaggaggctc ccagagtgtg tatggtgtgg 1020

atcagactca ctcagggctc cccctgcatc acatcaggag gatgttcatg ggagattcat 1080

gcccttatct agcagggaag ccaaagatgt ttttattca gaactatgtg gtgtcagagg 1140

gccagctgga ggacagcagc ctcttggagg tggatgggcc agcgatgaag aatgtggaat 1200

tcaaggctca gaagcgaggg ctgtgcacag ttcaccgaga agctgacttc ttctggagcc 1260
tgtgtactgc ggacatgtcc ctgctggagc agtctcacag ctcaccatcc ctgtacctgc 1320
agtgcctctc ccagaaactg agacaagaaa gaaaacgccc actcctggat cttcacattg 1380
aactcaatgg ctacatgtat gattggaaca gcagagtttc tgccaaggag aaatattatg 1440
tctggctgca gcacactctg agaaagaaac ttatcctctc ctacacataa gaaaccaaaa 1500
ggctgggcgt agtggctcac acctgtaatc ccagcacttt gggaggccaa ggagggcaga 1560
tcacttcagg tcaggagttc gagaccagcc tggccaacat ggtaaacgct gtccctagta 1620
aaaatacaaa aattagctgg gtgtgggtgt gggtacctgt attcccagtt acttgggagg 1680
ctgaggtggg aggatctttt gaacccagga gttcagggtc atagcatgct gtgattgtgc 1740
ctacgaatag ccactgcata ccaacctggg caatatagca agatcccatc tctttaaaaa 1800
aaaaaaaaaa ggacaggaac tatcttactc aatgtattag tcatgtttct ctagagggac 1860
agaactaata ggatacatgt atataaaaag gggagtttat taaggagtat tgactcacat 1920
gatcacaggg ttaggtccca caataggtca tctgcaagca aggaagccaa ttcaagtccc 1980
aaagctgaag aacttggagt ccaatgtttg agggcaggaa gcattcagca tgagagaaag 2040
atggaggcca gaagactaca ccagtctagt ctttccatgt tttgcctgct tttattctgg 2100
cagtgctggc agctgattag atggtgccca cccagattga ggatggtctg cctttcccag 2160
tccactgact caaatgttaa atctcctttg gcagcaccct cacagatgta cccgggaaca 2220
ctttgcatcc ttctattcaa tcaagttgat actcagtatt aaccatcaca gtccatttgg 2280
gcaactatac caaattacca tagaccaggt gacttaaaca gcagttattt ctcacagttc 2340
cggaggctgg gaaatccaac atctaagtgg tagcatatct ggtgtctggt aaggcatgct 2400
tccagatctt accagatgtc agtcttttga tgttctcaca tggcagaaaa agaggatgca 2460
aactctcaag tatatcttta agggcacaaa ttccattcat gagggctcta ccctcatcac 2520
ctaattacct cccaaaggcc ccaccttctg atactgtcac tttggggata ctgtctcccc 2580
tttgaattct gggggggaata caaacattca gtttgtaaca atagccttat gatttagagg 2640
ttacttgttc attcacctag acctcaaatt gcattttaca gctagtcaag tatatctttc 2700
tctgatttga tagtgtgacc taaaagggga ccattgtttg aaatatcatt agagttgctt 2760
attattatta ttattattat tattattatt attattatta ttattgagac agagtttcat 2820
tctgctgccc aggctggagt gcagtggcat catcttggct cattgcaacc tctgccttct 2880
gggttcaagc gattctcctg cctcagcctc ccgagtagct gggattacag gctcctgcca 2940
ccacacccgg ctaatttttg tatttttagt ggagacaggg tttccaccat gttggccagc 3000
gtggtcttga actcctgacc tcaggtgatt caccagcctc ggcctcccaa agtgctggga 3060

ttacaggtgt gagccactgc acctggccta ttattatttt taaatttttt ttttttaatt 3120
gatcattctt gggtgtttct cacagagggt gatttggcag ggtcacagga caatagtgga 3180
gggaaggtca gcagataaac aagtgaacaa aggtctctgg ttttcctagg cagaggaccc 3240
tgcggccttc cgcagtgttt gtgtccctgg gtacttgaga ttagggagtg gtgatgactc 3300
ttaaggagca tgctgccttc aagcatctgt ttaacaaagc acatcttgca ctgcccttaa 3360
tccatttaac cctgagtgga cacagcacat gtttcagaga gcacagggtt gggggtaagg 3420
tcatagatca acagcatcct aaggcagaag aatttttctt agtacagaac aaaatgaagt 3480
ctcccatgtc tacttctttc tacacagaca cagcaacaat ctgatttctc tatcttttcc 3540
ccacctttcc cccttttcta ttccacaaaa ccgccatcgt catcatggcc tgttctcaat 3600
gagctgttgg gtacacctcc cagacggggt ggcggctggg cagaggggct cctcacttcc 3660
cagatggggc ggccaggcgg acgcgccccc cacctccctc ccggacggga tagctggccg 3720
ggcgggggct gaccccccac ctccctcccc gacggggcgg ctggccgggc gggggctgac 3780
ccccacgcct ccctcccgga cggggcggct gccaggcgga ggggctcctc acttctcaga 3840
cggggtggct gctgggcgga gacgctcctc acttcccaga cagggtggct gtcgggcgga 3900
ggggctcctc acttctcaga cggggcagct gcgggcggag gggctcctca cttctcagac 3960
ggggtggccg ggcagagaag ctcctcacat cccagacggg ggggcggggc agaggcgctc 4020
cccacatctc agacgatggg cggccgggca gagacgctcc tcacttcatc ccagacgggg 4080
tggcggccgg gcagaagctg taatctcggc accctggggg gccaaggcag gcggctggga 4140
ggcggaggcc gtagccagct gagatcacac cactgcactc cagcctgggc aacattgagc 4200
actgagtgga cgagactctg cccgcaatcc cggcacctcg ggaggccgag gctggcagat 4260
cactcgcagt caggagctgg agaccagccc ggccaacaca gtgaaaccct gtctccacca 4320
aaaaaatacg aaaaccagtc aggcgtggcg gcgcccgcaa tggcaggcac gcggcaggcc 4380
gaggcgggag aatcaggcag ggaggctgca gtgagccgag atggcagcag tacagtccag 4440
cttcggctcg gcatcagagg gagaccgtgg ggagagggag aagagaggga gggggagagg 4500
gctattttta aaattttta aaattgctga acaggggtac ctctgggcag tgtgtcagaa 4560
taccactttt taaatatttt atgatttatt tatttttcta tttcttgagg ttttaactga 4620
tgtgtatctg tatgtctatt tgtgtatatt ttgtcatgat catgtaacag agtctgaaaa 4680
gtgtcgaaga gacagttttc aggaacaaca agcaattatt cctactttcc aagttatttt 4740
gatgccatgg tggctcatac ctataatctg agtactttgg gaggctgagg tggactgatc 4800
acttgagccc aggagtttga gaccagcctg ggcaacatag caagactcca tctctacaaa 4860
aaaagacaaa atttagctga gcgtggtggc gtgttcctgt agtcccagct acttgggagg 4920
ctgaagtgag tggatcccct gagcccagag aggtcaaggt tgtgatgagc tgtgatcaca 4980

ccactgcact tcagcatggg agacagagtg agaccctgtt tcagaaaaaa taaataaata 5040
aaaccaccag caccacaaac aacaacaaaa agttattttg tacttgtttt gagcacagga 5100
ctcctgaggg tatctttgca tttaatatta catagggtg ccagtgggaa gtaatgtgta 5160
tgcttggcct catgagctaa aaccctgtgt taattatgac agaaggaaag tgtgtgagag 5220
agatcttaac tacctagcag ctctagctgc catcttgaac catgaagata cgggccacac 5280
gtaggggtag ctgggtagtg agcagcaaga agccttgttg gatgagggca cgaaggagca 5340
gaatcactgg aatcactgtg tcagccctaa ttacctacct ctggactttt atgtgagggg 5400
aaaaaaaatt gacagtttat atttatctca acctagttaa cccaagtgat gcattgttat 5460
gagattaaaa tgtttggagg ccgggtgcgg tggctcacgc ctataatccc agccctttgg 5520
gaggccaagg cgggcggatc acgaggtcag gagatcaaga ccatcctggc taacatgtaa 5580
aaccccgtct ctactaaaaa tacaaaaaat tagccaggcg ttgtggcggt cgcctgtagt 5640
ccctgctatt tgggaggccg aggcaagaga acggcatgaa cctgggaggt ggagcttgca 5700
gcgagctgag atcttgccac tgcactccag cctgggcgac agtgcgagac tctgtctcaa 5760
aaataaataa ataaataaat aataaataaa atgtttggaa tgttggcttc atccctggga 5820
tgcaaggctg gttcaacata cgcaaatcaa gaaacataat tcatcacata aacagaacta 5880
aagacaaaaa ccacatgatt atctcaatag atacagaaaa ggccttcaat aaaattcaac 5940
gttgcttcat gttaaaaact ctcaataaac taggtattga tggaaaatat ctcaaaataa 6000
taaccattta tgacaaaccc acagccatta tcatactgaa tgggcaaaag ctggaagcat 6060
tccccttgaa aactggcaca agacagggat gccgtctcac cactcctatt taacatagta 6120
ttggaagttc tggccaagaa aatcaggcaa gagaaacaaa taaggggtat tcaaatagga 6180
aaagaggaag taaaactgtg tttgcagatg acatgatact atatctagaa aaccccatta 6240
tctccaccca aaagttcctt aagctgataa gcaacttcag caaagtctca ggatacaaaa 6300
tcaatgtgca gaaatcacaa gcattctata caccaacaat acacaagcag agagccaaat 6360
catgaatgaa ctcccattca cagttgctag aaagagaata aaatacctag gaatacagct 6420
aataagatgt gaaggatctc ttcaaggaga actacaaacc actgctcaag gaaataagag 6480
aggacacaaa tgaaaaaaca ttccattctc gtggatagga agaatcaata tcatgaaaat 6540
ggccatacta cccaaagtaa tttataggtt cattgctatt cccattaaac tactattgac 6600
attcttcaca gaattagaaa aaaactactt taaaattcaa atggaaccaa aaaagagccc 6660
gtataaccaa gacaacaata agcaaaaaga acaaagctgg aagcatcaca ctacccaact 6720
tcaaagtata ctgcaaggct acagtagcca aaatggcatg gtactggtac aaaaacagac 6780
acatagacca atggaacaga atagagacca gagaaagaag accacacatc tacagccatc 6840

```
tgatcatcga caaacctgac aaaaacaagc aatggggaaa agattcccta tttaataaat     6900
ggtgctggga aaactggcta gccatatgca gaaaattgaa actgacccct tccttacacc     6960
ttatacaaaa attaactcaa gattaaagac ttaatgtaaa acctaaaact ataaaaaccc     7020
tagaagaaaa tctatttaat accattcaag acataggcac aagcaaaggt ttcatgacaa     7080
aaacatcaaa agcaattgca acaaaagcaa aaattacaaa tgggatctaa ttaaactaaa     7140
gagctcctgc acagcaaaag aaactatcat tagagtgaac aggcaaccta cagaatggga     7200
gaacattttt gcaatctatc catctgacaa aggtctaata tccagaacct acaaggaact     7260
taaaacaaat ttacaaggaa aaaaacaacc ccatcaaaaa gtggacaaag gacatgaaca     7320
gacacttctc aaaagaagac atttatgtgg ccaacaaaca tataaaaaaa agctcaacct     7380
tactgatcat tagagaaatg caaaggagaa ccacaatgag ataccatctc atgccggtca     7440
gaatggtgat tattaaaaag tcaaaaaaca acagatgctg gcgaggctgt ggagaagtag     7500
gaacactttt acattgttgg tgggaatgta aattagttca accgttgtgg aagtgtgtgt     7560
ggctattcct caaagatcta gaactagaaa tactatttgt cccagcaatc ccattactgg     7620
gtatataccc aaaggaatat aaaccatttt attataaaga tacatgcaca tttttgttca     7680
ttgcagcact cttcacaata gcaaagacac aatagcaaat gcccatcaaa gatagactgg     7740
ataaagaaaa tgtggtacat atacaccatg gaatactgtg cagtgcagcc attacagctt     7800
ttggtgatac agtgaatcag atttttcatt aattctttta attggttatt actgaacgtg     7860
aaaaagtaat gtttgtattg aaatcttgag tctggccatg tttctatttt aaattcataa     7920
agaattctaa caagaggaat tccaagaatg tcataaatgg atgtttctcc atggatgaag     7980
gaactgtttt attcacttgc tgataattca gcctaatcca gtttgacatc atatagataa     8040
gtagttgaat tatggattta aaatacatat cattttctaa ctccaaaggt aatacttatt     8100
taaatggttt tgaaaatata gaaaggcaca atttcttttt aaatctgtta ttctccacca     8160
ccactcaatc tgtctatcat ctatctctcc attcattctt ccatttgttt atatctgtta     8220
atctttgtat gtgttcatgt atagctttta catgattgga atcataatgc atattccatt     8280
ttgaagtctg ctttttttta cacaaaaata tgttgtgaat attttcctat attatgaaat     8340
atcattagct gagcttttag aattgactgc atgttttggt accatttaga tatagtttaa     8400
gatacttaga agttatgtgg ctttgccact atggatgaat cttatttact caatattaat     8460
tacttacaaa taacctcacc taaacactac tcagccataa aaaggaatga attaatgaca     8520
ttcacagcaa cctggagact attactctaa aggaagtaac tgaggaatgg aaaaccaaac     8580
attgtatgtt ctcactcata agtgggagat aagctatgag gatgcaaagg cataagaagg     8640
atacaatgga ctttggggac ttaggggaaa gggtgggagg ggggtgaagg ataaaagaat     8700
acaaattggg ttcagtgtat actgctcagg tgatgggtgc accagaatct cacaagtaac     8760
```

cacttaatta cttacgcatg taaccagata ccacctgttc cccaaacacc tatggaaata 8820
attttgtttt tttttttaaa aaaggaatga gatcatgtcc tttgcaggga catggatgaa 8880
gctggaagcc attatcctca gcaaactaac agaggagcag gaaaccaaac accacatgtt 8940
ctcacttgta agcggaagct gaacaatgag aacacacgga cacagggatg agatcaacac 9000
acactggggc ctgatgcagg ggccgtagcg gggagagcat caggataact agctaatgca 9060
tgtggggctt aatacctagg tgataggttg ataggtgcag caaaccacca tgggacacgt 9120
ttacctatgt aacaaacccg cacatcctgc acttgtatcc agaacttaaa atattttaaa 9180
aatctttaga gaatacaaaa aaaaaaaaaa agattcttca atgcatacac aataaaattg 9240
cagttcagtc aaacattgga agtctttctc tgactgtcta gttggtatct tcattttcag 9300
cttcttcaag atcccactcc aaacactgtt agctcagcca aattgaacag ctcatatctc 9360
ctacctctgg atctttggtt ctggtgattg tatatttctg gaccatctgg aaccccagca 9420
tatcacccta ccccacatct ccacatcccc aaaatataac catacttcaa gggcagttca 9480
aataccatct ccttctatcc tccatgaagt cagttatctc ttccattgga attatcgccc 9540
cctctcctga acagtactat ttcgtgtgaa tctcctccaa gccttctttt cattttatat 9600
ctcatgctgt aattcttgga aagtatgctg tagctcaagt gcagaattct catcagtttt 9660
atctttatat ctctcctaaa cactttacct gatgaagagc ctggcataca cataaatata 9720
tattgaatga atcagtgatg gattgaaaag agaaatgatg gatctcctaa attttaactt 9780
ttataaaata ttttgataca ttcatgacct tactttagca agcaatgaac gtgatgtaaa 9840
ctattgttga tatagttttt atattggaag tgtaagtagt ttgtggcatg ggattgtgac 9900
atatcctagg tttcctcatc ttctttttat tgaaatgtaa ttcacaagcc ataaaatttg 9960
ccccttttaaa gtaaatgatg cagtggattt tagtatattt acagagttgt gcaatcatca 10020
ccactatcta attccagaac atttccatct acctagaaac tccataccag tgagctgcca 10080
ctctaatcct cctcttcccc cagcctctag aaacaataat ccattttctg tctctatgat 10140
ttgcctgttc tagatatttt ataaaaataa acatgtggcc tttcgtgtct gacttccttc 10200
acttaaaaaa aaaaaaaaaa aa 10222

<210> 20
<211> 10797
<212> DNA
<213> Homo sapiens

<220>
<221> misc_feature
<223> CFLAR

<400> 20

gggcacttgg agttgtctta ggactgatgt ctgaattaac taaatgaact tgtctggttt 60
gcagagtgct gatggcagag attggtgagg atttggataa atctgatgtg tcctcattaa 120
ttttcctcat gaaggattac atgggccgag gcaagataag caaggagaag agtttcttgg 180
accttgtggt tgagttggag aaactaaatc tggttgcccc agatcaactg gatttattag 240
aaaaatgcct aaagaacatc cacagaatag acctgaagac aaaaatccag aagtacaagc 300
agtctgttca aggagcaggg acaagttaca ggaatgttct ccaagcagca atccaaaaga 360
gtctcaagga tccttcaaat aacttcaggc tccataatgg gagaagtaaa gaacaaagac 420
ttaaggaaca gcttggcgct caacaagaac cagtgaagaa atccattcag gaatcagaag 480
ctttttttgcc tcagagcata cctgaagaga gatacaagat gaagagcaag cccctaggaa 540
tctgcctgat aatcgattgc attggcaatg agacagagct tcttcgagac accttcactt 600
ccctgggcta tgaagtccag aaattcttgc atctcagtat gcatggtata tcccagattc 660
ttggccaatt tgcctgtatg cccgagcacc gagactacga cagctttgtg tgtgtcctgg 720
tgagccgagg aggctcccag agtgtgtatg gtgtggatca gactcactca gggctccccc 780
tgcatcacat caggaggatg ttcatgggag attcatgccc ttatctagca gggaagccaa 840
agatgttttt tattcagaac tatgtggtgt cagagggcca gctggaggac agcagcctct 900
tggaggtgga tgggccagcg atgaagaatg tggaattcaa ggctcagaag cgagggctgt 960
gcacagttca ccgagaagct gacttcttct ggagcctgtg tactgcggac atgtccctgc 1020
tggagcagtc tcacagctca ccatccctgt acctgcagtg cctctcccag aaactgagac 1080
aagaaagggg gacaattccc ggaagtggaa ttacagagtc aaaggacatg catttttcaa 1140
gcctcggatg catcttacta gatgtcctat aggatggtca tatcagcttt ataggagagt 1200
agctgtgtcc ctgaattctc cctgacactg catgctctta tatttcctca agttttgaca 1260
atttgatagg tgaaaagtgg tatctgactg ttcagatctg gaaggctttg ttatataaac 1320
attttttttaa tgtttattgg caagaatact tttctaagag aaacatcagt gagctggttt 1380
ccatttaagc tgaatgaagc cacaatgtac ctcaagtata agattaactg gcctttttca 1440
gttgcactct aattacaatt tagaatgatg tttctgagcc acctgtcaaa tgcattctgg 1500
gctgtacctc tgcgtacccc aggaataaat ctcatggcct tctttacctg gcctccttag 1560
tggtggccca gcaggaagcg ggggttagag caggagccac tcagccttcc aagatagata 1620
ctccatgggc cggtggtatt actggccttt tgagcccatc cccatttgca tagatgatcc 1680
acgtgggtta tcatctggct ggtatgttcc cagagtgaaa ctcagcagcc ccttgaggga 1740
ggggatggtg gccatcaggc cagagtattg caagttagtt tggatcattt gctaagcagc 1800
ttgtggtgcc ttcagaaagg aacagtttca aagaactttc acatctgttg gctcatttcg 1860
ccctaatgac agtcttctct ttgatatttg catggcatta aattttgcct ttcttgtttt 1920

ctccagaaaa cgcccactcc tggatcttca cattgaactc aatggctaca tgtatgattg 1980
gaacagcaga gtttctgcca aggagaaata ttatgtctgg ctgcagcaca ctctgagaaa 2040
gaaacttatc ctctcctaca cataagaaac caaaaggctg ggcgtagtgg ctcacacctg 2100
taatcccagc actttgggag gccaaggagg gcagatcact tcaggtcagg agttcgagac 2160
cagcctggcc aacatggtaa acgctgtccc tagtaaaaat acaaaaatta gctgggtgtg 2220
ggtgtgggta cctgtattcc cagttacttg ggaggctgag gtgggaggat cttttgaacc 2280
caggagttca gggtcatagc atgctgtgat tgtgcctacg aatagccact gcataccaac 2340
ctgggcaata tagcaagatc ccatctcttt aaaaaaaaaa aaaaaggaca ggaactatct 2400
tactcaatgt attagtcatg tttctctaga gggacagaac taataggata catgtatata 2460
aaaaggggag tttattaagg agtattgact cacatgatca cagggttagg tcccacaata 2520
ggtcatctgc aagcaaggaa gccaattcaa gtcccaaagc tgaagaactt ggagtccaat 2580
gtttgagggc aggaagcatt cagcatgaga gaaagatgga ggccagaaga ctacaccagt 2640
ctagtctttc catgttttgc ctgcttttat tctggcagtg ctggcagctg attagatggt 2700
gcccacccag attgaggatg gtctgccttt cccagtccac tgactcaaat gttaaatctc 2760
ctttggcagc accctcacag atgtacccgg gaacactttg catccttcta ttcaatcaag 2820
ttgatactca gtattaacca tcacagtcca tttgggcaac tataccaaat taccatagac 2880
caggtgactt aaacagcagt tatttctcac agttccggag gctgggaaat ccaacatcta 2940
agtggtagca tatctggtgt ctggtaaggc atgcttccag atcttaccag atgtcagtct 3000
tttgatgttc tcacatggca gaaaaagagg atgcaaactc tcaagtatat ctttaagggc 3060
acaaattcca ttcatgaggg ctctaccctc atcacctaat tacctcccaa aggccccacc 3120
ttctgatact gtcactttgg ggatactgtc tcccctttga attctggggg gaatacaaac 3180
attcagtttg taacaatagc cttatgattt agaggttact tgttcattca cctagacctc 3240
aaattgcatt ttacagctag tcaagtatat ctttctctga tttgatagtg tgacctaaaa 3300
ggggaccatt gtttgaaata tcattagagt tgcttattat tattattatt attattatta 3360
ttattattat tattattatt gagacagagt ttcattctgc tgcccaggct ggagtgcagt 3420
ggcatcatct tggctcattg caacctctgc cttctgggtt caagcgattc tcctgcctca 3480
gcctcccgag tagctgggat tacaggctcc tgccaccaca cccggctaat tttgtattt 3540
ttagtggaga cagggtttcc accatgttgg ccagcgtggt cttgaactcc tgacctcagg 3600
tgattcacca gcctcggcct cccaaagtgc tgggattaca ggtgtgagcc actgcacctg 3660
gcctattatt atttttaaat tttttttttt taattgatca ttcttgggtg tttctcacag 3720
agggtgattt ggcagggtca caggacaata gtggagggaa ggtcagcaga taaacaagtg 3780

aacaaaggtc tctggttttc ctaggcagag gaccctgcgg ccttccgcag tgtttgtgtc 3840
cctgggtact tgagattagg gagtggtgat gactcttaag gagcatgctg ccttcaagca 3900
tctgtttaac aaagcacatc ttgcactgcc cttaatccat ttaaccctga gtggacacag 3960
cacatgtttc agagagcaca gggttggggg taaggtcata gatcaacagc atcctaaggc 4020
agaagaattt ttcttagtac agaacaaaat gaagtctccc atgtctactt ctttctacac 4080
agacacagca acaatctgat ttctctatct tttccccacc tttccccctt ttctattcca 4140
caaaaccgcc atcgtcatca tggcctgttc tcaatgagct gttgggtaca cctcccagac 4200
ggggtggcgg ctgggcagag gggctcctca cttcccagat ggggcggcca ggcggacgcg 4260
ccccccacct ccctcccgga cgggatagct ggccgggcgg gggctgaccc cccacctccc 4320
tccccgacgg ggcggctggc cgggcggggg ctgaccccca cgcctccctc ccggacgggg 4380
cggctgccag gcggaggggc tcctcacttc tcagacgggg tggctgctgg gcggagacgc 4440
tcctcacttc ccagacaggg tggctgtcgg gcggaggggc tcctcacttc tcagacgggg 4500
cagctgcggg cggaggggct cctcacttct cagacggggt ggccgggcag agaagctcct 4560
cacatcccag acgggggggc ggggcagagg cgctccccac atctcagacg atgggcggcc 4620
gggcagagac gctcctcact tcatcccaga cggggtggcg gccgggcaga agctgtaatc 4680
tcggcaccct ggggggccaa ggcaggcggc tgggaggcgg aggccgtagc cagctgagat 4740
cacaccactg cactccagcc tgggcaacat tgagcactga gtggacgaga ctctgcccgc 4800
aatcccggca cctcgggagg ccgaggctgg cagatcactc gcagtcagga gctggagacc 4860
agcccggcca acacagtgaa accctgtctc caccaaaaaa atacgaaaac cagtcaggcg 4920
tggcggcgcc cgcaatggca ggcacgcggc aggccgaggc gggagaatca ggcagggagg 4980
ctgcagtgag ccgagatggc agcagtacag tccagcttcg gctcggcatc agagggagac 5040
cgtggggaga gggagaagag agggaggggg agagggctat ttttaaaatt ttttaaaatt 5100
gctgaacagg ggtacctctg ggcagtgtgt cagaatacca ctttttaaat attttatgat 5160
ttatttattt ttctatttct tgaggtttta actgatgtgt atctgtatgt ctatttgtgt 5220
atattttgtc atgatcatgt aacagagtct gaaaagtgtc gaagagacag ttttcaggaa 5280
caacaagcaa ttattcctac tttccaagtt attttgatgc catggtggct catacctata 5340
atctgagtac tttgggaggc tgaggtggac tgatcacttg agcccaggag tttgagacca 5400
gcctgggcaa catagcaaga ctccatctct acaaaaaaag acaaaattta gctgagcgtg 5460
gtggcgtgtt cctgtagtcc cagctacttg ggaggctgaa gtgagtggat cccctgagcc 5520
cagagaggtc aaggttgtga tgagctgtga tcacaccact gcacttcagc atgggagaca 5580
gagtgagacc ctgtttcaga aaaaataaat aaataaaacc accagcacca caaacaacaa 5640
caaaaagtta ttttgtactt gttttgagca caggactcct gagggtatct ttgcatttaa 5700

tattacatag gggtgccagt gggaagtaat gtgtatgctt ggcctcatga gctaaaaccc 5760
tgtgttaatt atgacagaag gaaagtgtgt gagagagatc ttaactacct agcagctcta 5820
gctgccatct tgaaccatga agatacgggc cacacgtagg ggtagctggg tagtgagcag 5880
caagaagcct tgttggatga gggcacgaag gagcagaatc actggaatca ctgtgtcagc 5940
cctaattacc tacctctgga cttttatgtg aggggaaaaa aaattgacag tttatattta 6000
tctcaaccta gttaacccaa gtgatgcatt gttatgagat taaaatgttt ggaggccggg 6060
tgcggtggct cacgcctata atcccagccc tttgggaggc caaggcgggc ggatcacgag 6120
gtcaggagat caagaccatc ctggctaaca tgtaaaaccc cgtctctact aaaaatacaa 6180
aaaattagcc aggcgttgtg gcggtcgcct gtagtccctg ctatttggga ggccgaggca 6240
agagaacggc atgaacctgg gaggtggagc ttgcagcgag ctgagatctt gccactgcac 6300
tccagcctgg gcgacagtgc gagactctgt ctcaaaaata aataaataaa taaataataa 6360
ataaaatgtt tggaatgttg gcttcatccc tgggatgcaa ggctggttca acatacgcaa 6420
atcaagaaac ataattcatc acataaacag aactaaagac aaaaaccaca tgattatctc 6480
aatagataca gaaaaggcct tcaataaaat tcaacgttgc ttcatgttaa aaactctcaa 6540
taaactaggt attgatggaa aatatctcaa aataataacc atttatgaca aacccacagc 6600
cattatcata ctgaatgggc aaaagctgga agcattcccc ttgaaaactg gcacaagaca 6660
gggatgccgt ctcaccactc ctatttaaca tagtattgga agttctggcc aagaaaatca 6720
ggcaagagaa acaaataagg ggtattcaaa taggaaaaga ggaagtaaaa ctgtgtttgc 6780
agatgacatg atactatatc tagaaaaccc cattatctcc acccaaaagt tccttaagct 6840
gataagcaac ttcagcaaag tctcaggata caaaatcaat gtgcagaaat cacaagcatt 6900
ctatacacca acaatacaca agcagagagc caaatcatga atgaactccc attcacagtt 6960
gctagaaaga gaataaaata cctaggaata cagctaataa gatgtgaagg atctcttcaa 7020
ggagaactac aaaccactgc tcaaggaaat aagagaggac acaaatgaaa aaacattcca 7080
ttctcgtgga taggaagaat caatatcatg aaaatggcca tactacccaa agtaatttat 7140
aggttcattg ctattcccat taaactacta ttgacattct tcacagaatt agaaaaaaac 7200
tactttaaaa ttcaaatgga accaaaaaag agcccgtata accaagacaa caataagcaa 7260
aaagaacaaa gctggaagca tcacactacc caacttcaaa gtatactgca aggctacagt 7320
agccaaaatg gcatggtact ggtacaaaaa cagacacata gaccaatgga acagaataga 7380
gaccagagaa agaagaccac acatctacag ccatctgatc atcgacaaac ctgacaaaaa 7440
caagcaatgg ggaaaagatt ccctatttaa taaatggtgc tgggaaaact ggctagccat 7500
atgcagaaaa ttgaaactga ccccttcctt acaccttata caaaaattaa ctcaagatta 7560

aagacttaat gtaaaaccta aaactataaa aaccctagaa gaaaatctat ttaataccat 7620

tcaagacata ggcacaagca aaggtttcat gacaaaaaca tcaaaagcaa ttgcaacaaa 7680

agcaaaaatt acaaatggga tctaattaaa ctaaagagct cctgcacagc aaaagaaact 7740

atcattagag tgaacaggca acctacagaa tgggagaaca tttttgcaat ctatccatct 7800

gacaaaggtc taatatccag aacctacaag gaacttaaaa caaatttaca aggaaaaaaa 7860

caaccccatc aaaaagtgga caaaggacat gaacagacac ttctcaaaag aagacattta 7920

tgtggccaac aaacatataa aaaaaagctc aaccttactg atcattagag aaatgcaaag 7980

gagaaccaca atgagatacc atctcatgcc ggtcagaatg gtgattatta aaaagtcaaa 8040

aaacaacaga tgctggcgag gctgtggaga agtaggaaca cttttacatt gttggtggga 8100

atgtaaatta gttcaaccgt tgtggaagtg tgtgtggcta ttcctcaaag atctagaact 8160

agaaatacta tttgtcccag caatcccatt actgggtata tacccaaagg aatataaacc 8220

attttattat aaagatacat gcacattttt gttcattgca gcactcttca caatagcaaa 8280

gacacaatag caaatgccca tcaaagatag actggataaa gaaaatgtgg tacatataca 8340

ccatggaata ctgtgcagtg cagccattac agcttttggt gatacagtga atcagatttt 8400

tcattaattc ttttaattgg ttattactga acgtgaaaaa gtaatgtttg tattgaaatc 8460

ttgagtctgg ccatgtttct attttaaatt cataaagaat tctaacaaga ggaattccaa 8520

gaatgtcata aatggatgtt tctccatgga tgaaggaact gttttattca cttgctgata 8580

attcagccta atccagtttg acatcatata gataagtagt tgaattatgg atttaaaata 8640

catatcattt tctaactcca aaggtaatac ttatttaaat ggttttgaaa atatagaaag 8700

gcacaatttc tttttaaatc tgttattctc caccaccact caatctgtct atcatctatc 8760

tctccattca ttcttccatt tgtttatatc tgttaatctt tgtatgtgtt catgtatagc 8820

ttttacatga ttggaatcat aatgcatatt ccattttgaa gtctgctttt ttttacacaa 8880

aaatatgttg tgaatatttt cctatattat gaaatatcat tagctgagct tttagaattg 8940

actgcatgtt ttggtaccat ttagatatag tttaagatac ttagaagtta tgtggctttg 9000

ccactatgga tgaatcttat ttactcaata ttaattactt acaaataacc tcacctaaac 9060

actactcagc cataaaaagg aatgaattaa tgacattcac agcaacctgg agactattac 9120

tctaaaggaa gtaactgagg aatggaaaac caaacattgt atgttctcac tcataagtgg 9180

gagataagct atgaggatgc aaaggcataa gaaggataca atggactttg gggacttagg 9240

ggaaagggtg ggaggggggt gaaggataaa agaatacaaa ttgggttcag tgtatactgc 9300

tcaggtgatg ggtgcaccag aatctcacaa gtaaccactt aattacttac gcatgtaacc 9360

agataccacc tgttccccaa acacctatgg aaataatttt gttttttttt ttaaaaaagg 9420

aatgagatca tgtcctttgc agggacatgg atgaagctgg aagccattat cctcagcaaa 9480

ctaacagagg agcaggaaac caaacaccac atgttctcac ttgtaagcgg aagctgaaca 9540

atgagaacac acggacacag ggatgagatc aacacacact ggggcctgat gcaggggccg 9600

tagcggggag agcatcagga taactagcta atgcatgtgg ggcttaatac ctaggtgata 9660

ggttgatagg tgcagcaaac caccatggga cacgtttacc tatgtaacaa acccgcacat 9720

cctgcacttg tatccagaac ttaaaatatt ttaaaaatct ttagagaata caaaaaaaaa 9780

aaaaaagatt cttcaatgca tacacaataa aattgcagtt cagtcaaaca ttggaagtct 9840

ttctctgact gtctagttgg tatcttcatt ttcagcttct tcaagatccc actccaaaca 9900

ctgttagctc agccaaattg aacagctcat atctcctacc tctggatctt tggttctggt 9960

gattgtatat ttctggacca tctggaaccc cagcatatca ccctacccca catctccaca 10020

tccccaaaat ataaccatac ttcaagggca gttcaaatac catctccttc tatcctccat 10080

gaagtcagtt atctcttcca ttggaattat cgccccctct cctgaacagt actatttcgt 10140

gtgaatctcc tccaagcctt cttttcattt tatatctcat gctgtaattc ttggaaagta 10200

tgctgtagct caagtgcaga attctcatca gttttatctt tatatctctc ctaaacactt 10260

tacctgatga agagcctggc atacacataa atatatattg aatgaatcag tgatggattg 10320

aaaagagaaa tgatggatct cctaaatttt aacttttata aaatattttg atacattcat 10380

gaccttactt tagcaagcaa tgaacgtgat gtaaactatt gttgatatag tttttatatt 10440

ggaagtgtaa gtagtttgtg gcatgggatt gtgacatatc ctaggtttcc tcatcttctt 10500

tttattgaaa tgtaattcac aagccataaa atttgcccct ttaaagtaaa tgatgcagtg 10560

gattttagta tatttacaga gttgtgcaat catcaccact atctaattcc agaacatttc 10620

catctaccta gaaactccat accagtgagc tgccactcta atcctcctct tcccccagcc 10680

tctagaaaca ataatccatt ttctgtctct atgatttgcc tgttctagat attttataaa 10740

aataaacatg tggcctttcg tgtctgactt ccttcactta aaaaaaaaaa aaaaaaa 10797

<210> 21
<211> 11061
<212> DNA
<213> Homo sapiens

<220>
<221> misc_feature
<223> CFLAR

<400> 21
atactcagtc acacaagcca tagcaggaaa cagcgagctt gcagcctcac cgacgagtct 60

caactaaaag ggactcccgg agctaggggt ggggactcgg cctcacacag tgagtgccgg 120

ctattggact tttgtccagt gacagctgag acaacaagga ccacgggagg aggtgtagga 180

gagaagcgcc gcgaacagcg atcgcccagc accaagtccg cttccaggct ttcggtttct 240
ttgcctccat cttgggtgcg ccttcccggc gtctagggga gcgaaggctg aggtggcagc 300
ggcaggagag tccggccgcg acaggacgag tgctgatggc agagattggt gaggatttgg 360
ataaatctga tgtgtcctca ttaattttcc tcatgaagga ttacatgggc cgaggcaaga 420
taagcaagga gaagagtttc ttggaccttg tggttgagtt ggagaaacta aatctggttg 480
ccccagatca actggattta ttagaaaaat gcctaaagaa catccacaga atagacctga 540
agacaaaaat ccagaagtac aagcagtctg ttcaaggagc agggacaagt tacaggaatg 600
ttctccaagc agcaatccaa aagagtctca aggatccttc aaataacttc aggctccata 660
atgggagaag taaagaacaa agacttaagg aacagcttgg cgctcaacaa gaaccagtga 720
agaaatccat tcaggaatca gaagcttttt tgcctcagag catacctgaa gagagataca 780
agatgaagag caagccccta ggaatctgcc tgataatcga ttgcattggc aatgagacag 840
agcttcttcg agacaccttc acttccctgg gctatgaagt ccagaaattc ttgcatctca 900
gtatgcatgg tatatcccag attcttggcc aatttgcctg tatgcccgag caccgagact 960
acgacagctt tgtgtgtgtc ctggtgagcc gaggaggctc ccagagtgtg tatggtgtgg 1020
atcagactca ctcagggctc cccctgcatc acatcaggag gatgttcatg ggagattcat 1080
gcccttatct agcagggaag ccaaagatgt tttttattca gaactatgtg gtgtcagagg 1140
gccagctgga ggacagcagc ctcttggagg tggatgggcc agcgatgaag aatgtggaat 1200
tcaaggctca gaagcgaggg ctgtgcacag ttcaccgaga agctgacttc ttctggagcc 1260
tgtgtactgc ggacatgtcc ctgctggagc agtctcacag ctcaccatcc ctgtacctgc 1320
agtgcctctc ccagaaactg agacaagaaa gggggacaat tcccggaagt ggaattacag 1380
agtcaaagga catgcatttt tcaagcctcg gatgcatctt actagatgtc ctataggatg 1440
gtcatatcag ctttatagga gagtagctgt gtccctgaat tctccctgac actgcatgct 1500
cttatatttc ctcaagtttt gacaatttga taggtgaaaa gtggtatctg actgttcaga 1560
tctggaaggc tttgttatat aaacattttt ttaatgttta ttggcaagaa tacttttcta 1620
agagaaacat cagtgagctg gtttccattt aagctgaatg aagccacaat gtacctcaag 1680
tataagatta actggccttt ttcagttgca ctctaattac aatttagaat gatgtttctg 1740
agccacctgt caaatgcatt ctgggctgta cctctgcgta ccccaggaat aaatctcatg 1800
gccttcttta cctggcctcc ttagtggtgg cccagcagga agcgggggtt agagcaggag 1860
ccactcagcc ttccaagata gatactccat gggccggtgg tattactggc cttttgagcc 1920
catccccatt tgcatagatg atccacgtgg gttatcatct ggctggtatg ttcccagagt 1980
gaaactcagc agccccttga gggaggggat ggtggccatc aggccagagt attgcaagtt 2040
agtttggatc atttgctaag cagcttgtgg tgccttcaga aaggaacagt ttcaaagaac 2100

tttcacatct gttggctcat ttcgccctaa tgacagtctt ctctttgata tttgcatggc 2160
attaaatttt gcctttcttg ttttctccag aaaacgccca ctcctggatc ttcacattga 2220
actcaatggc tacatgtatg attggaacag cagagtttct gccaaggaga aatattatgt 2280
ctggctgcag cacactctga gaaagaaact tatcctctcc tacacataag aaaccaaaag 2340
gctgggcgta gtggctcaca cctgtaatcc cagcactttg ggaggccaag gagggcagat 2400
cacttcaggt caggagttcg agaccagcct ggccaacatg gtaaacgctg tccctagtaa 2460
aaatacaaaa attagctggg tgtgggtgtg ggtacctgta ttcccagtta cttgggaggc 2520
tgaggtggga ggatcttttg aacccaggag ttcagggtca tagcatgctg tgattgtgcc 2580
tacgaatagc cactgcatac caacctgggc aatatagcaa gatcccatct ctttaaaaaa 2640
aaaaaaaaag gacaggaact atcttactca atgtattagt catgtttctc tagagggaca 2700
gaactaatag gatacatgta tataaaaagg ggagtttatt aaggagtatt gactcacatg 2760
atcacagggt taggtcccac aataggtcat ctgcaagcaa ggaagccaat tcaagtccca 2820
aagctgaaga acttggagtc caatgtttga gggcaggaag cattcagcat gagagaaaga 2880
tggaggccag aagactacac cagtctagtc tttccatgtt ttgcctgctt ttattctggc 2940
agtgctggca gctgattaga tggtgcccac ccagattgag gatggtctgc ctttcccagt 3000
ccactgactc aaatgttaaa tctcctttgg cagcaccctc acagatgtac ccgggaacac 3060
tttgcatcct tctattcaat caagttgata ctcagtatta accatcacag tccatttggg 3120
caactatacc aaattaccat agaccaggtg acttaaacag cagttatttc tcacagttcc 3180
ggaggctggg aaatccaaca tctaagtggt agcatatctg gtgtctggta aggcatgctt 3240
ccagatctta ccagatgtca gtcttttgat gttctcacat ggcagaaaaa gaggatgcaa 3300
actctcaagt atatctttaa gggcacaaat tccattcatg agggctctac cctcatcacc 3360
taattacctc ccaaaggccc caccttctga tactgtcact ttggggatac tgtctcccct 3420
ttgaattctg gggggaatac aaacattcag tttgtaacaa tagccttatg atttagaggt 3480
tacttgttca ttcacctaga cctcaaattg cattttacag ctagtcaagt atatctttct 3540
ctgatttgat agtgtgacct aaaaggggac cattgtttga aatatcatta gagttgctta 3600
ttattattat tattattatt attattatta ttattattat tattgagaca gagtttcatt 3660
ctgctgccca ggctggagtg cagtggcatc atcttggctc attgcaacct ctgccttctg 3720
ggttcaagcg attctcctgc ctcagcctcc cgagtagctg ggattacagg ctcctgccac 3780
cacacccggc taatttttgt atttttagtg gagacagggt ttccaccatg ttggccagcg 3840
tggtcttgaa ctcctgacct caggtgattc accagcctcg gcctcccaaa gtgctgggat 3900
tacaggtgtg agccactgca cctggcctat tattattttt aaattttttt tttttaattg 3960

atcattcttg ggtgtttctc acagagggtg atttggcagg gtcacaggac aatagtggag 4020
ggaaggtcag cagataaaca agtgaacaaa ggtctctggt tttcctaggc agaggaccct 4080
gcggccttcc gcagtgtttg tgtccctggg tacttgagat tagggagtgg tgatgactct 4140
taaggagcat gctgccttca agcatctgtt taacaaagca catcttgcac tgcccttaat 4200
ccatttaacc ctgagtggac acagcacatg tttcagagag cacagggttg gggtaaggt 4260
catagatcaa cagcatccta aggcagaaga atttttctta gtacagaaca aaatgaagtc 4320
tcccatgtct acttctttct acacagacac agcaacaatc tgatttctct atcttttccc 4380
cacctttccc ccttttctat tccacaaaac cgccatcgtc atcatggcct gttctcaatg 4440
agctgttggg tacacctccc agacggggtg gcggctgggc agaggggctc ctcacttccc 4500
agatggggcg gccaggcgga cgcgcccccc acctccctcc cggacgggat agctggccgg 4560
gcgggggctg accccccacc tccctccccg acggggcggc tggccgggcg ggggctgacc 4620
cccacgcctc cctcccggac ggggcggctg ccaggcggag ggctcctca cttctcagac 4680
ggggtggctg ctgggcggag acgctcctca cttcccagac agggtggctg tcgggcggag 4740
gggctcctca cttctcagac ggggcagctg cgggcggagg ggctcctcac ttctcagacg 4800
gggtggccgg gcagagaagc tcctcacatc ccagacgggg gggcggggca gaggcgctcc 4860
ccacatctca gacgatgggc ggccgggcag agacgctcct cacttcatcc cagacggggt 4920
ggcggccggg cagaagctgt aatctcggca ccctgggggg ccaaggcagg cggctgggag 4980
gcggaggccg tagccagctg agatcacacc actgcactcc agcctgggca acattgagca 5040
ctgagtggac gagactctgc ccgcaatccc ggcacctcgg gaggccgagg ctggcagatc 5100
actcgcagtc aggagctgga gaccagcccg gccaacacag tgaaaccctg tctccaccaa 5160
aaaaatacga aaaccagtca ggcgtggcgg cgcccgcaat ggcaggcacg cggcaggccg 5220
aggcgggaga atcaggcagg gaggctgcag tgagccgaga tggcagcagt acagtccagc 5280
ttcggctcgg catcagaggg agaccgtggg gagagggaga agagagggag ggggagaggg 5340
ctatttttaa aatttttaa aattgctgaa caggggtacc tctgggcagt gtgtcagaat 5400
accacttttt aaatatttta tgatttattt atttttctat ttcttgaggt tttaactgat 5460
gtgtatctgt atgtctattt gtgtatattt tgtcatgatc atgtaacaga gtctgaaaag 5520
tgtcgaagag acagttttca ggaacaacaa gcaattattc ctactttcca agttattttg 5580
atgccatggt ggctcatacc tataatctga gtactttggg aggctgaggt ggactgatca 5640
cttgagccca ggagtttgag accagcctgg gcaacatagc aagactccat ctctacaaaa 5700
aaagacaaaa tttagctgag cgtggtggcg tgttcctgta gtcccagcta cttgggaggc 5760
tgaagtgagt ggatcccctg agcccagaga ggtcaaggtt gtgatgagct gtgatcacac 5820
cactgcactt cagcatggga gacagagtga gaccctgttt cagaaaaaat aaataaataa 5880

aaccaccagc accacaaaca acaacaaaaa gttattttgt acttgttttg agcacaggac 5940
tcctgagggt atctttgcat ttaatattac ataggggtgc cagtgggaag taatgtgtat 6000
gcttggcctc atgagctaaa accctgtgtt aattatgaca gaaggaaagt gtgtgagaga 6060
gatcttaact acctagcagc tctagctgcc atcttgaacc atgaagatac gggccacacg 6120
taggggtagc tgggtagtga gcagcaagaa gccttgttgg atgagggcac gaaggagcag 6180
aatcactgga atcactgtgt cagccctaat tacctacctc tggactttta tgtgagggga 6240
aaaaaaattg acagtttata tttatctcaa cctagttaac ccaagtgatg cattgttatg 6300
agattaaaat gtttggaggc cgggtgcggt ggctcacgcc tataatccca gccctttggg 6360
aggccaaggc gggcggatca cgaggtcagg agatcaagac catcctggct aacatgtaaa 6420
accccgtctc tactaaaaat acaaaaaatt agccaggcgt tgtggcggtc gcctgtagtc 6480
cctgctattt gggaggccga ggcaagagaa cggcatgaac ctgggaggtg gagcttgcag 6540
cgagctgaga tcttgccact gcactccagc ctgggcgaca gtgcgagact ctgtctcaaa 6600
aataaataaa taaataaata ataaataaaa tgtttggaat gttggcttca tccctgggat 6660
gcaaggctgg ttcaacatac gcaaatcaag aaacataatt catcacataa acagaactaa 6720
agacaaaaac cacatgatta tctcaataga tacagaaaag gccttcaata aaattcaacg 6780
ttgcttcatg ttaaaaactc tcaataaact aggtattgat ggaaaatatc tcaaaataat 6840
aaccatttat gacaaaccca cagccattat catactgaat gggcaaaagc tggaagcatt 6900
ccccttgaaa actggcacaa gacagggatg ccgtctcacc actcctattt aacatagtat 6960
tggaagttct ggccaagaaa atcaggcaag agaaacaaat aaggggtatt caaataggaa 7020
aagaggaagt aaaactgtgt ttgcagatga catgatacta tatctagaaa accccattat 7080
ctccacccaa aagttcctta agctgataag caacttcagc aaagtctcag gatacaaaat 7140
caatgtgcag aaatcacaag cattctatac accaacaata cacaagcaga gagccaaatc 7200
atgaatgaac tcccattcac agttgctaga aagagaataa aatacctagg aatacagcta 7260
ataagatgtg aaggatctct tcaaggagaa ctacaaacca ctgctcaagg aaataagaga 7320
ggacacaaat gaaaaaacat tccattctcg tggataggaa gaatcaatat catgaaaatg 7380
gccatactac ccaaagtaat ttataggttc attgctattc ccattaaact actattgaca 7440
ttcttcacag aattagaaaa aaactacttt aaaattcaaa tggaaccaaa aaagagcccg 7500
tataaccaag acaacaataa gcaaaaagaa caaagctgga agcatcacac tacccaactt 7560
caaagtatac tgcaaggcta cagtagccaa aatggcatgg tactggtaca aaaacagaca 7620
catagaccaa tggaacagaa tagagaccag agaaagaaga ccacacatct acagccatct 7680
gatcatcgac aaacctgaca aaaacaagca atggggaaaa gattccctat ttaataaatg 7740

gtgctgggaa aactggctag ccatatgcag aaaattgaaa ctgacccctt ccttacacct 7800
tatacaaaaa ttaactcaag attaaagact taatgtaaaa cctaaaacta taaaaaccct 7860
agaagaaaat ctatttaata ccattcaaga cataggcaca agcaaaggtt tcatgacaaa 7920
aacatcaaaa gcaattgcaa caaaagcaaa aattacaaat gggatctaat taaactaaag 7980
agctcctgca cagcaaaaga aactatcatt agagtgaaca ggcaacctac agaatgggag 8040
aacatttttg caatctatcc atctgacaaa ggtctaatat ccagaaccta caaggaactt 8100
aaaacaaatt tacaaggaaa aaaacaaccc catcaaaaag tggacaaagg acatgaacag 8160
acacttctca aaagaagaca tttatgtggc caacaaacat ataaaaaaaa gctcaacctt 8220
actgatcatt agagaaatgc aaaggagaac cacaatgaga taccatctca tgccggtcag 8280
aatggtgatt attaaaaagt caaaaaacaa cagatgctgg cgaggctgtg gagaagtagg 8340
aacactttta cattgttggt gggaatgtaa attagttcaa ccgttgtgga agtgtgtgtg 8400
gctattcctc aaagatctag aactagaaat actatttgtc ccagcaatcc cattactggg 8460
tatataccca aaggaatata aaccatttta ttataaagat acatgcacat ttttgttcat 8520
tgcagcactc ttcacaatag caaagacaca atagcaaatg cccatcaaag atagactgga 8580
taaagaaaat gtggtacata tacaccatgg aatactgtgc agtgcagcca ttacagcttt 8640
tggtgataca gtgaatcaga tttttcatta attcttttaa ttggttatta ctgaacgtga 8700
aaaagtaatg tttgtattga aatcttgagt ctggccatgt ttctatttta aattcataaa 8760
gaattctaac aagaggaatt ccaagaatgt cataaatgga tgtttctcca tggatgaagg 8820
aactgtttta ttcacttgct gataattcag cctaatccag tttgacatca tatagataag 8880
tagttgaatt atggatttaa aatacatatc attttctaac tccaaaggta atacttattt 8940
aaatggtttt gaaaatatag aaaggcacaa tttcttttta aatctgttat tctccaccac 9000
cactcaatct gtctatcatc tatctctcca ttcattcttc catttgttta tatctgttaa 9060
tctttgtatg tgttcatgta tagcttttac atgattggaa tcataatgca tattccattt 9120
tgaagtctgc ttttttttac acaaaaatat gttgtgaata ttttcctata ttatgaaata 9180
tcattagctg agcttttaga attgactgca tgttttggta ccatttagat atagtttaag 9240
atacttagaa gttatgtggc tttgccacta tggatgaatc ttatttactc aatattaatt 9300
acttacaaat aacctcacct aaacactact cagccataaa aaggaatgaa ttaatgacat 9360
tcacagcaac ctggagacta ttactctaaa ggaagtaact gaggaatgga aaaccaaaca 9420
ttgtatgttc tcactcataa gtgggagata agctatgagg atgcaaaggc ataagaagga 9480
tacaatggac tttggggact taggggaaag ggtgggaggg gggtgaagga taaaagaata 9540
caaattgggt tcagtgtata ctgctcaggt gatgggtgca ccagaatctc acaagtaacc 9600
acttaattac ttacgcatgt aaccagatac cacctgttcc ccaaacacct atggaaataa 9660

ttttgttttt ttttttaaaa aaggaatgag atcatgtcct ttgcagggac atggatgaag 9720

ctggaagcca ttatcctcag caaactaaca gaggagcagg aaaccaaaca ccacatgttc 9780

tcacttgtaa gcggaagctg aacaatgaga acacacggac acagggatga gatcaacaca 9840

cactggggcc tgatgcaggg gccgtagcgg ggagagcatc aggataacta gctaatgcat 9900

gtggggctta atacctaggt gataggttga taggtgcagc aaaccaccat gggacacgtt 9960

tacctatgta acaaacccgc acatcctgca cttgtatcca gaacttaaaa tattttaaaa 10020

atctttagag aatacaaaaa aaaaaaaaaa gattcttcaa tgcatacaca ataaaattgc 10080

agttcagtca aacattggaa gtctttctct gactgtctag ttggtatctt cattttcagc 10140

ttcttcaaga tcccactcca aacactgtta gctcagccaa attgaacagc tcatatctcc 10200

tacctctgga tctttggttc tggtgattgt atatttctgg accatctgga accccagcat 10260

atcaccctac cccacatctc cacatcccca aaatataacc atacttcaag ggcagttcaa 10320

ataccatctc cttctatcct ccatgaagtc agttatctct tccattggaa ttatcgcccc 10380

ctctcctgaa cagtactatt tcgtgtgaat ctcctccaag ccttcttttc attttatatc 10440

tcatgctgta attcttggaa agtatgctgt agctcaagtg cagaattctc atcagtttta 10500

tctttatatc tctcctaaac actttacctg atgaagagcc tggcatacac ataaatatat 10560

attgaatgaa tcagtgatgg attgaaaaga gaaatgatgg atctcctaaa ttttaacttt 10620

tataaaatat tttgatacat tcatgacctt actttagcaa gcaatgaacg tgatgtaaac 10680

tattgttgat atagttttta tattggaagt gtaagtagtt tgtggcatgg gattgtgaca 10740

tatcctaggt ttcctcatct tctttttatt gaaatgtaat tcacaagcca taaaatttgc 10800

ccctttaaag taaatgatgc agtggatttt agtatattta cagagttgtg caatcatcac 10860

cactatctaa ttccagaaca tttccatcta cctagaaact ccataccagt gagctgccac 10920

tctaatcctc ctcttccccc agcctctaga aacaataatc cattttctgt ctctatgatt 10980

tgcctgttct agatatttta taaaaataaa catgtggcct ttcgtgtctg acttccttca 11040

cttaaaaaaa aaaaaaaaaa a 11061

<210> 22
<211> 10640
<212> DNA
<213> Homo sapiens

<220>
<221> misc_feature
<223> CFLAR

<400> 22
atactcagtc acacaagcca tagcaggaaa cagcgagctt gcagcctcac cgacgagtct 60

caactaaaag ggactcccgg agctaggggt ggggactcgg cctcacacag tgagtgccgg 120
ctattggact tttgtccagt gacagctgag acaacaagga ccacgggagg aggtgtagga 180
gagaagcgcc gcgaacagcg atcgcccagc accaagtccg cttccaggct ttcggtttct 240
ttgcctccat cttgggtgcg ccttcccggc gtctagggga gcgaaggctg aggtggcagc 300
ggcaggagag tccggccgcg acaggacgaa ctcccccact ggaaaggatt ctgaaagaaa 360
tgaagtcagc cctcagaaat gaagttgact gcctgctggc tttctgttga ctggcccgga 420
gctgtactgc aagacccttg tgagcttccc tagtctaaga gtaggatgtc tgctgaagtc 480
atccatcagg ttgaagaagc acttgataca gatgagaagg agatgctgct ctttttgtgc 540
cgggatgttg ctatagatgt ggttccacct aatgtcaggg accttctgga tattttacgg 600
gaaagaggta agctgtctgt cggggacttg gctgaactgc tctacagagt gaggcgattt 660
gacctgctca aacgtatctt gaagatggac agaaaagctg tggagaccca cctgctcagg 720
aaccctcacc ttgtttcgga ctatagagtg ctgatggcag agattggtga ggatttggat 780
aaatctgatg tgtcctcatt aattttcctc atgaaggatt acatgggccg aggcaagata 840
agcaaggaga agagtttctt ggaccttgtg gttgagttgg agaaactaaa tctggttgcc 900
ccagatcaac tggatttatt agaaaaatgc ctaaagaaca tccacagaat agacctgaag 960
acaaaaatcc agaagtacaa gcagtctgtt caaggagcag ggacaagtta caggaatgtt 1020
ctccaagcag caatccaaaa gagtctcaag gatccttcaa ataacttcag gctccataat 1080
gggagaagta aagaacaaag acttaaggaa cagcttggcg ctcaacaaga accagtgaag 1140
aaatccattc aggaatcaga agctttttg cctcagagca tacctgaaga gagatacaag 1200
atgaagagca agcccctagg aatctgcctg ataatcgatt gcattggcaa tgagacagag 1260
cttcttcgag acaccttcac ttccctgggc tatgaagtcc agaaattctt gcatctcagt 1320
atgcatggta tatcccagat tcttggccaa tttgcctgta tgcccgagca ccgagactac 1380
gacagctttg tgtgtgtcct ggtgagccga ggaggctccc agagtgtgta tggtgtggat 1440
cagactcact cagggctccc cctgcatcac atcaggagga tgttcatggg agattcatgc 1500
ccttatctag cagggaagcc aaagatgttt tttattcaga actatgtggt gtcagagggc 1560
cagctggagg acagcagcct cttggaggtg gatgggccag cgatgaagaa tgtggaattc 1620
aaggctcaga agcgagggct gtgcacagtt caccgagaag ctgacttctt ctggagcctg 1680
tgtactgcgg acatgtccct gctggagcag tctcacagct caccatccct gtacctgcag 1740
tgcctctccc agaaactgag acaagaaaga aaacgcccac tcctggatct tcacattgaa 1800
ctcaatggct acatgtatga ttggaacagc agagtttctg ccaaggagaa atattatgtc 1860
tggctgcagc acactctgag aaagaaactt atcctctcct acacataaga aaccaaaagg 1920
ctgggcgtag tggctcacac ctgtaatccc agcactttgg gaggccaagg agggcagatc 1980

acttcaggtc aggagttcga gaccagcctg gccaacatgg taaacgctgt ccctagtaaa 2040
aatacaaaaa ttagctgggt gtgggtgtgg gtacctgtat tcccagttac ttgggaggct 2100
gaggtgggag gatcttttga acccaggagt tcagggtcat agcatgctgt gattgtgcct 2160
acgaatagcc actgcatacc aacctgggca atatagcaag atcccatctc tttaaaaaaa 2220
aaaaaaaagg acaggaacta tcttactcaa tgtattagtc atgtttctct agagggacag 2280
aactaatagg atacatgtat ataaaaaggg gagtttatta aggagtattg actcacatga 2340
tcacagggtt aggtcccaca ataggtcatc tgcaagcaag gaagccaatt caagtcccaa 2400
agctgaagaa cttggagtcc aatgtttgag ggcaggaagc attcagcatg agagaaagat 2460
ggaggccaga agactacacc agtctagtct ttccatgttt tgcctgcttt tattctggca 2520
gtgctggcag ctgattagat ggtgcccacc cagattgagg atggtctgcc tttcccagtc 2580
cactgactca aatgttaaat ctcctttggc agcaccctca cagatgtacc cgggaacact 2640
ttgcatcctt ctattcaatc aagttgatac tcagtattaa ccatcacagt ccatttgggc 2700
aactatacca aattaccata gaccaggtga cttaaacagc agttatttct cacagttccg 2760
gaggctggga aatccaacat ctaagtggta gcatatctgg tgtctggtaa ggcatgcttc 2820
cagatcttac cagatgtcag tcttttgatg ttctcacatg gcagaaaaag aggatgcaaa 2880
ctctcaagta tatctttaag ggcacaaatt ccattcatga gggctctacc ctcatcacct 2940
aattacctcc caaaggcccc accttctgat actgtcactt tggggatact gtctcccctt 3000
tgaattctgg ggggaataca aacattcagt ttgtaacaat agccttatga tttagaggtt 3060
acttgttcat tcacctagac ctcaaattgc attttacagc tagtcaagta tatctttctc 3120
tgatttgata gtgtgaccta aaaggggacc attgtttgaa atatcattag agttgcttat 3180
tattattatt attattatta ttattattat tattattatt attgagacag agtttcattc 3240
tgctgcccag gctggagtgc agtggcatca tcttggctca ttgcaacctc tgccttctgg 3300
gttcaagcga ttctcctgcc tcagcctccc gagtagctgg gattacaggc tcctgccacc 3360
acacccggct aatttttgta tttttagtgg agacagggtt tccaccatgt tggccagcgt 3420
ggtcttgaac tcctgacctc aggtgattca ccagcctcgg cctcccaaag tgctgggatt 3480
acaggtgtga gccactgcac ctggcctatt attattttta aatttttttt ttttaattga 3540
tcattcttgg gtgtttctca cagagggtga tttggcaggg tcacaggaca atagtggagg 3600
gaaggtcagc agataaacaa gtgaacaaag gtctctggtt ttcctaggca gaggaccctg 3660
cggccttccg cagtgtttgt gtccctgggt acttgagatt agggagtggt gatgactctt 3720
aaggagcatg ctgccttcaa gcatctgttt aacaaagcac atcttgcact gcccttaatc 3780
catttaaccc tgagtggaca cagcacatgt ttcagagagc acagggttgg gggtaaggtc 3840

```
atagatcaac agcatcctaa ggcagaagaa tttttcttag tacagaacaa aatgaagtct      3900
cccatgtcta cttctttcta cacagacaca gcaacaatct gatttctcta tcttttcccc      3960
acctttcccc cttttctatt ccacaaaacc gccatcgtca tcatggcctg ttctcaatga      4020
gctgttgggt acacctccca gacggggtgg cggctgggca gaggggctcc tcacttccca      4080
gatggggcgg ccaggcggac gcgcccccca cctccctccc ggacgggata gctggccggg      4140
cgggggctga ccccccacct ccctccccga cggggcggct ggccgggcgg gggctgaccc      4200
ccacgcctcc ctcccggacg gggcggctgc caggcggagg ggctcctcac ttctcagacg      4260
gggtggctgc tgggcggaga cgctcctcac ttcccagaca gggtggctgt cgggcggagg      4320
ggctcctcac ttctcagacg gggcagctgc gggcggaggg gctcctcact tctcagacgg      4380
ggtggccggg cagagaagct cctcacatcc cagacggggg ggcggggcag aggcgctccc      4440
cacatctcag acgatgggcg gccgggcaga gacgctcctc acttcatccc agacggggtg      4500
gcggccgggc agaagctgta atctcggcac cctgggggc caaggcaggc ggctgggagg      4560
cggaggccgt agccagctga gatcacacca ctgcactcca gcctgggcaa cattgagcac      4620
tgagtggacg agactctgcc cgcaatcccg gcacctcggg aggccgaggc tggcagatca      4680
ctcgcagtca ggagctggag accagcccgg ccaacacagt gaaaccctgt ctccaccaaa      4740
aaaatacgaa aaccagtcag gcgtggcggc gcccgcaatg gcaggcacgc ggcaggccga      4800
ggcgggagaa tcaggcaggg aggctgcagt gagccgagat ggcagcagta cagtccagct      4860
tcggctcggc atcagaggga gaccgtgggg agagggagaa gagagggagg gggagagggc      4920
tatttttaaa attttttaaa attgctgaac aggggtacct ctgggcagtg tgtcagaata      4980
ccacttttta aatattttat gatttattta tttttctatt tcttgaggtt ttaactgatg      5040
tgtatctgta tgtctatttg tgtatatttt gtcatgatca tgtaacagag tctgaaaagt      5100
gtcgaagaga cagttttcag gaacaacaag caattattcc tactttccaa gttattttga      5160
tgccatggtg gctcatacct ataatctgag tactttggga ggctgaggtg gactgatcac      5220
ttgagcccag gagtttgaga ccagcctggg caacatagca agactccatc tctacaaaaa      5280
aagacaaaat ttagctgagc gtggtggcgt gttcctgtag tcccagctac ttgggaggct      5340
gaagtgagtg gatcccctga gcccagagag gtcaaggttg tgatgagctg tgatcacacc      5400
actgcacttc agcatgggag acagagtgag accctgtttc agaaaaaata aataaataaa      5460
accaccagca ccacaaacaa caacaaaaag ttattttgta cttgttttga gcacaggact      5520
cctgagggta tctttgcatt taatattaca taggggtgcc agtgggaagt aatgtgtatg      5580
cttggcctca tgagctaaaa ccctgtgtta attatgacag aaggaaagtg tgtgagagag      5640
atcttaacta cctagcagct ctagctgcca tcttgaacca tgaagatacg ggccacacgt      5700
aggggtagct gggtagtgag cagcaagaag ccttgttgga tgagggcacg aaggagcaga      5760
```

atcactggaa tcactgtgtc agccctaatt acctacctct ggacttttat gtgaggggaa 5820
aaaaaattga cagtttatat ttatctcaac ctagttaacc caagtgatgc attgttatga 5880
gattaaaatg tttggaggcc gggtgcggtg gctcacgcct ataatcccag ccctttggga 5940
ggccaaggcg ggcggatcac gaggtcagga gatcaagacc atcctggcta acatgtaaaa 6000
ccccgtctct actaaaaata caaaaaatta gccaggcgtt gtggcggtcg cctgtagtcc 6060
ctgctatttg ggaggccgag gcaagagaac ggcatgaacc tgggaggtgg agcttgcagc 6120
gagctgagat cttgccactg cactccagcc tgggcgacag tgcgagactc tgtctcaaaa 6180
ataaataaat aaataaataa taaataaaat gtttggaatg ttggcttcat ccctgggatg 6240
caaggctggt tcaacatacg caaatcaaga aacataattc atcacataaa cagaactaaa 6300
gacaaaaacc acatgattat ctcaatagat acagaaaagg ccttcaataa aattcaacgt 6360
tgcttcatgt taaaaactct caataaacta ggtattgatg gaaaatatct caaaataata 6420
accatttatg acaaacccac agccattatc atactgaatg ggcaaaagct ggaagcattc 6480
cccttgaaaa ctggcacaag acagggatgc cgtctcacca ctcctattta acatagtatt 6540
ggaagttctg gccaagaaaa tcaggcaaga gaaacaaata aggggtattc aaataggaaa 6600
agaggaagta aaactgtgtt tgcagatgac atgatactat atctagaaaa ccccattatc 6660
tccacccaaa agttccttaa gctgataagc aacttcagca aagtctcagg atacaaaatc 6720
aatgtgcaga aatcacaagc attctataca ccaacaatac acaagcagag agccaaatca 6780
tgaatgaact cccattcaca gttgctagaa agagaataaa atacctagga atacagctaa 6840
taagatgtga aggatctctt caaggagaac tacaaaccac tgctcaagga aataagagag 6900
gacacaaatg aaaaaacatt ccattctcgt ggataggaag aatcaatatc atgaaaatgg 6960
ccatactacc caaagtaatt tataggttca ttgctattcc cattaaacta ctattgacat 7020
tcttcacaga attagaaaaa aactacttta aaattcaaat ggaaccaaaa aagagcccgt 7080
ataaccaaga caacaataag caaaaagaac aaagctggaa gcatcacact acccaacttc 7140
aaagtatact gcaaggctac agtagccaaa atggcatggt actggtacaa aaacagacac 7200
atagaccaat ggaacagaat agagaccaga gaaagaagac cacacatcta cagccatctg 7260
atcatcgaca aacctgacaa aaacaagcaa tggggaaaag attccctatt taataaatgg 7320
tgctgggaaa actggctagc catatgcaga aaattgaaac tgaccccttc cttacacctt 7380
atacaaaaat taactcaaga ttaaagactt aatgtaaaac ctaaaactat aaaaacccta 7440
gaagaaaatc tatttaatac cattcaagac ataggcacaa gcaaaggttt catgacaaaa 7500
acatcaaaag caattgcaac aaaagcaaaa attacaaatg ggatctaatt aaactaaaga 7560
gctcctgcac agcaaaagaa actatcatta gagtgaacag gcaacctaca gaatgggaga 7620

acatttttgc aatctatcca tctgacaaag gtctaatatc cagaacctac aaggaactta 7680
aaacaaattt acaaggaaaa aaacaacccc atcaaaaagt ggacaaagga catgaacaga 7740
cacttctcaa aagaagacat ttatgtggcc aacaaacata taaaaaaaag ctcaacctta 7800
ctgatcatta gagaaatgca aaggagaacc acaatgagat accatctcat gccggtcaga 7860
atggtgatta ttaaaaagtc aaaaaacaac agatgctggc gaggctgtgg agaagtagga 7920
acacttttac attgttggtg ggaatgtaaa ttagttcaac cgttgtggaa gtgtgtgtgg 7980
ctattcctca aagatctaga actagaaata ctatttgtcc cagcaatccc attactgggt 8040
atatacccaa aggaatataa accattttat tataaagata catgcacatt tttgttcatt 8100
gcagcactct tcacaatagc aaagacacaa tagcaaatgc ccatcaaaga tagactggat 8160
aaagaaaatg tggtacatat acaccatgga atactgtgca gtgcagccat tacagctttt 8220
ggtgatacag tgaatcagat ttttcattaa ttcttttaat tggttattac tgaacgtgaa 8280
aaagtaatgt ttgtattgaa atcttgagtc tggccatgtt tctattttaa attcataaag 8340
aattctaaca agaggaattc caagaatgtc ataaatggat gtttctccat ggatgaagga 8400
actgttttat tcacttgctg ataattcagc ctaatccagt ttgacatcat atagataagt 8460
agttgaatta tggatttaaa atacatatca ttttctaact ccaaaggtaa tacttattta 8520
aatggttttg aaaatataga aaggcacaat ttctttttaa atctgttatt ctccaccacc 8580
actcaatctg tctatcatct atctctccat tcattcttcc atttgtttat atctgttaat 8640
ctttgtatgt gttcatgtat agcttttaca tgattggaat cataatgcat attccatttt 8700
gaagtctgct ttttttttaca caaaaatatg ttgtgaatat tttcctatat tatgaaatat 8760
cattagctga gcttttagaa ttgactgcat gttttggtac catttagata tagtttaaga 8820
tacttagaag ttatgtggct ttgccactat ggatgaatct tatttactca atattaatta 8880
cttacaaata acctcaccta aacactactc agccataaaa aggaatgaat taatgacatt 8940
cacagcaacc tggagactat tactctaaag gaagtaactg aggaatggaa aaccaaacat 9000
tgtatgttct cactcataag tgggagataa gctatgagga tgcaaaggca taagaaggat 9060
acaatggact ttggggactt aggggaaagg gtgggagggg ggtgaaggat aaaagaatac 9120
aaattgggtt cagtgtatac tgctcaggtg atgggtgcac cagaatctca caagtaacca 9180
cttaattact tacgcatgta accagatacc acctgttccc caaacaccta tggaaataat 9240
tttgtttttt tttttaaaaa aggaatgaga tcatgtcctt tgcagggaca tggatgaagc 9300
tggaagccat tatcctcagc aaactaacag aggagcagga aaccaaacac cacatgttct 9360
cacttgtaag cggaagctga acaatgagaa cacacggaca cagggatgag atcaacacac 9420
actggggcct gatgcagggg ccgtagcggg gagagcatca ggataactag ctaatgcatg 9480
tggggcttaa tacctaggtg ataggttgat aggtgcagca aaccaccatg ggacacgttt 9540

acctatgtaa caaacccgca catcctgcac ttgtatccag aacttaaaat attttaaaaa 9600

tctttagaga atacaaaaaa aaaaaaaaag attcttcaat gcatacacaa taaaattgca 9660

gttcagtcaa acattggaag tctttctctg actgtctagt tggtatcttc attttcagct 9720

tcttcaagat cccactccaa acactgttag ctcagccaaa ttgaacagct catatctcct 9780

acctctggat ctttggttct ggtgattgta tatttctgga ccatctggaa ccccagcata 9840

tcaccctacc ccacatctcc acatccccaa aatataacca tacttcaagg gcagttcaaa 9900

taccatctcc ttctatcctc catgaagtca gttatctctt ccattggaat tatcgccccc 9960

tctcctgaac agtactattt cgtgtgaatc tcctccaagc cttcttttca ttttatatct 10020

catgctgtaa ttcttggaaa gtatgctgta gctcaagtgc agaattctca tcagttttat 10080

ctttatatct ctcctaaaca ctttacctga tgaagagcct ggcatacaca taaatatata 10140

ttgaatgaat cagtgatgga ttgaaaagag aaatgatgga tctcctaaat tttaactttt 10200

ataaaatatt ttgatacatt catgacctta ctttagcaag caatgaacgt gatgtaaact 10260

attgttgata tagtttttat attggaagtg taagtagttt gtggcatggg attgtgacat 10320

atcctaggtt tcctcatctt ctttttattg aaatgtaatt cacaagccat aaaatttgcc 10380

cctttaaagt aaatgatgca gtggatttta gtatatttac agagttgtgc aatcatcacc 10440

actatctaat tccagaacat ttccatctac ctagaaactc cataccagtg agctgccact 10500

ctaatcctcc tcttccccca gcctctagaa acaataatcc attttctgtc tctatgattt 10560

gcctgttcta gatattttat aaaaataaac atgtggcctt tcgtgtctga cttccttcac 10620

ttaaaaaaaa aaaaaaaaaa 10640

<210> 23
<211> 1648
<212> DNA
<213> Homo sapiens

<220>
<221> misc_feature
<223> CD14

<400> 23
cagagaaggc ttaggctccc gagtcaacag ggcattcacc gcctggggcg cctgagtcat 60

caggacactg ccaggagaca cagaacccta gatgccctgc agaatccttc ctgttacggt 120

ccccctccct gaaacatcct tcattgcaat atttccagga aaggaagggg gctggctcgg 180

aggaagagag gtggggaggt gatcaggggtt cacagaggag ggaactgaat gacatcccag 240

gattacataa actgtcagag gcagccgaag agttcacaag tgtgaagcct ggaagccggc 300

gggtgccgct gtgtaggaaa gaagctaaag cacttccaga gcctgtccgg agctcagagg 360

ttcggaagac ttatcgacca tggagcgcgc gtcctgcttg ttgctgctgc tgctgccgct 420

ggtgcacgtc tctgcgacca cgccagaacc ttgtgagctg gacgatgaag atttccgctg 480

cgtctgcaac ttctccgaac ctcagcccga ctggtccgaa gccttccagt gtgtgtctgc 540

agtagaggtg gagatccatg ccggcggtct caacctagag ccgtttctaa agcgcgtcga 600

tgcggacgcc gacccgcggc agtatgctga cacggtcaag gctctccgcg tgcggcggct 660

cacagtggga gccgcacagg ttcctgctca gctactggta ggcgccctgc gtgtgctagc 720

gtactcccgc ctcaaggaac tgacgctcga ggacctaaag ataaccggca ccatgcctcc 780

gctgcctctg gaagccacag gacttgcact ttccagcttg cgcctacgca acgtgtcgtg 840

ggcgacaggg cgttcttggc tcgccgagct gcagcagtgg ctcaagccag gcctcaaggt 900

actgagcatt gcccaagcac actcgcctgc cttttcctgc gaacaggttc gcgccttccc 960

ggcccttacc agcctagacc tgtctgacaa tcctggactg ggcgaacgcg gactgatggc 1020

ggctctctgt ccccacaagt tcccggccat ccagaatcta gcgctgcgca acacaggaat 1080

ggagacgccc acaggcgtgt gcgccgcact ggcggcggca ggtgtgcagc cccacagcct 1140

agacctcagc cacaactcgc tgcgcgccac cgtaaaccct agcgctccga gatgcatgtg 1200

gtccagcgcc ctgaactccc tcaatctgtc gttcgctggg ctggaacagg tgcctaaagg 1260

actgccagcc aagctcagag tgctcgatct cagctgcaac agactgaaca gggcgccgca 1320

gcctgacgag ctgcccgagg tggataacct gacactggac gggaatccct tcctggtccc 1380

tggaactgcc ctcccccacg agggctcaat gaactccggc gtggtcccag cctgtgcacg 1440

ttcgaccctg tcggtggggg tgtcgggaac cctggtgctg ctccaagggg cccggggctt 1500

tgcctaagat ccaagacaga ataatgaatg gactcaaact gccttggctt caggggagtc 1560

ccgtcaggac gttgaggact tttcgaccaa ttcaaccctt tgccccacct ttattaaaat 1620

cttaaacaac gggtcaaaaa aaaaaaaa 1648

<210> 24
<211> 1561
<212> DNA
<213> Homo sapiens

<220>
<221> misc_feature
<223> CD14

<400> 24
aattctaccc cccttggtgc caacagatga ggttcacaat ctcttccaca aaacatgcag 60

ttaaatatct gaggatattc agggacttgg atttggtggc aggagatcaa cataaaccaa 120

gacaaggaag aagtcaaaga aatgaatcaa gtagattctc tgggatataa gaggcagccg 180

aagagttcac aagtgtgaag cctggaagcc ggcgggtgcc gctgtgtagg aaagaagcta 240

aagcacttcc agagcctgtc cggagctcag aggttcggaa gacttatcga ccatggagcg 300

cgcgtcctgc ttgttgctgc tgctgctgcc gctggtgcac gtctctgcga ccacgccaga 360

accttgtgag ctggacgatg aagatttccg ctgcgtctgc aacttctccg aacctcagcc 420

cgactggtcc gaagccttcc agtgtgtgtc tgcagtagag gtggagatcc atgccggcgg 480

tctcaaccta gagccgtttc taaagcgcgt cgatgcggac gccgacccgc ggcagtatgc 540

tgacacggtc aaggctctcc gcgtgcggcg gctcacagtg ggagccgcac aggttcctgc 600

tcagctactg gtaggcgccc tgcgtgtgct agcgtactcc cgcctcaagg aactgacgct 660

cgaggaccta aagataaccg gcaccatgcc tccgctgcct ctggaagcca caggacttgc 720

actttccagc ttgcgcctac gcaacgtgtc gtgggcgaca gggcgttctt ggctcgccga 780

gctgcagcag tggctcaagc caggcctcaa ggtactgagc attgcccaag cacactcgcc 840

tgccttttcc tgcgaacagg ttcgcgcctt cccggccctt accagcctag acctgtctga 900

caatcctgga ctgggcgaac gcggactgat ggcggctctc tgtccccaca agttcccggc 960

catccagaat ctagcgctgc gcaacacagg aatggagacg cccacaggcg tgtgcgccgc 1020

actggcggcg gcaggtgtgc agccccacag cctagacctc agccacaact cgctgcgcgc 1080

caccgtaaac cctagcgctc cgagatgcat gtggtccagc gccctgaact ccctcaatct 1140

gtcgttcgct gggctggaac aggtgcctaa aggactgcca gccaagctca gagtgctcga 1200

tctcagctgc aacagactga acagggcgcc gcagcctgac gagctgcccg aggtggataa 1260

cctgacactg gacgggaatc ccttcctggt ccctggaact gccctccccc acgagggctc 1320

aatgaactcc ggcgtggtcc cagcctgtgc acgttcgacc ctgtcggtgg gggtgtcggg 1380

aaccctggtg ctgctccaag ggcccggggg ctttgcctaa gatccaagac agaataatga 1440

atggactcaa actgccttgg cttcagggga gtcccgtcag gacgttgagg acttttcgac 1500

caattcaacc ctttgcccca cctttattaa aatcttaaac aacgggtcaa aaaaaaaaaa 1560

a 1561

<210> 25
<211> 1661
<212> DNA
<213> Homo sapiens

<220>
<221> misc_feature
<223> CD14

<400> 25
aattctaccc cccttggtgc caacagatga ggttcacaat ctcttccaca aaacatgcag 60

ttaaatatct gaggatattc agggacttgg atttggtggc aggagatcaa cataaaccaa 120

```
gacaaggaag aagtcaaaga aatgaatcaa gtagattctc tgggatataa ggaaaggaag      180
ggggctggct cggaggaaga gaggtgggga ggtgatcagg gttcacagag gagggaactg      240
aatgacatcc caggattaca taaactgtca gaggcagccg aagagttcac aagtgtgaag      300
cctggaagcc ggcgggtgcc gctgtgtagg aaagaagcta aagcacttcc agagcctgtc      360
cggagctcag aggttcggaa gacttatcga ccatggagcg cgcgtcctgc ttgttgctgc      420
tgctgctgcc gctggtgcac gtctctgcga ccacgccaga accttgtgag ctggacgatg      480
aagatttccg ctgcgtctgc aacttctccg aacctcagcc cgactggtcc gaagccttcc      540
agtgtgtgtc tgcagtagag gtggagatcc atgccggcgg tctcaaccta gagccgtttc      600
taaagcgcgt cgatgcggac gccgacccgc ggcagtatgc tgacacggtc aaggctctcc      660
gcgtgcggcg gctcacagtg ggagccgcac aggttcctgc tcagctactg gtaggcgccc      720
tgcgtgtgct agcgtactcc cgcctcaagg aactgacgct cgaggaccta aagataaccg      780
gcaccatgcc tccgctgcct ctggaagcca caggacttgc actttccagc ttgcgcctac      840
gcaacgtgtc gtgggcgaca gggcgttctt ggctcgccga gctgcagcag tggctcaagc      900
caggcctcaa ggtactgagc attgcccaag cacactcgcc tgccttttcc tgcgaacagg      960
ttcgcgcctt cccggccctt accagcctag acctgtctga caatcctgga ctgggcgaac     1020
gcggactgat ggcggctctc tgtccccaca agttcccggc catccagaat ctagcgctgc     1080
gcaacacagg aatggagacg cccacaggcg tgtgcgccgc actggcggcg gcaggtgtgc     1140
agccccacag cctagacctc agccacaact cgctgcgcgc caccgtaaac cctagcgctc     1200
cgagatgcat gtggtccagc gccctgaact ccctcaatct gtcgttcgct gggctggaac     1260
aggtgcctaa aggactgcca gccaagctca gagtgctcga tctcagctgc aacagactga     1320
acagggcgcc gcagcctgac gagctgcccg aggtggataa cctgacactg gacgggaatc     1380
ccttcctggt ccctggaact gccctccccc acgagggctc aatgaactcc ggcgtggtcc     1440
cagcctgtgc acgttcgacc ctgtcggtgg gggtgtcggg aaccctggtg ctgctccaag     1500
gggcccgggg ctttgcctaa gatccaagac agaataatga atggactcaa actgccttgg     1560
cttcagggga gtcccgtcag gacgttgagg acttttcgac caattcaacc ctttgcccca     1620
cctttattaa aatcttaaac aacgggtcaa aaaaaaaaaa a                         1661
```

```
<210>  26
<211>  1540
<212>  DNA
<213>  Homo sapiens

<220>
<221>  misc_feature
<223>  CD14
```

<400> 26
aattctaccc cccttggtgc caacagatga ggttcacaat ctcttccaca aaacatgcag 60

ttaaatatct gaggatattc agggacttgg atttggtggc aggagatcaa cataaaccaa 120

gacaaggaag aagtcaaaga aatgaatcaa aggcagccga agagttcaca agtgtgaagc 180

ctggaagccg gcgggtgccg ctgtgtagga aagaagctaa agcacttcca gagcctgtcc 240

ggagctcaga ggttcggaag acttatcgac catggagcgc gcgtcctgct tgttgctgct 300

gctgctgccg ctggtgcacg tctctgcgac cacgccagaa ccttgtgagc tggacgatga 360

agatttccgc tgcgtctgca acttctccga acctcagccc gactggtccg aagccttcca 420

gtgtgtgtct gcagtagagg tggagatcca tgccggcggt ctcaacctag agccgtttct 480

aaagcgcgtc gatgcggacg ccgacccgcg gcagtatgct gacacggtca aggctctccg 540

cgtgcggcgg ctcacagtgg gagccgcaca ggttcctgct cagctactgg taggcgccct 600

gcgtgtgcta gcgtactccc gcctcaagga actgacgctc gaggacctaa agataaccgg 660

caccatgcct ccgctgcctc tggaagccac aggacttgca ctttccagct tgcgcctacg 720

caacgtgtcg tgggcgacag ggcgttcttg gctcgccgag ctgcagcagt ggctcaagcc 780

aggcctcaag gtactgagca ttgcccaagc acactcgcct gccttttcct gcgaacaggt 840

tcgcgccttc ccggccctta ccagcctaga cctgtctgac aatcctggac tgggcgaacg 900

cggactgatg gcggctctct gtccccacaa gttcccggcc atccagaatc tagcgctgcg 960

caacacagga atggagacgc ccacaggcgt gtgcgccgca ctggcggcgg caggtgtgca 1020

gccccacagc ctagacctca gccacaactc gctgcgcgcc accgtaaacc ctagcgctcc 1080

gagatgcatg tggtccagcg ccctgaactc cctcaatctg tcgttcgctg ggctggaaca 1140

ggtgcctaaa ggactgccag ccaagctcag agtgctcgat ctcagctgca acagactgaa 1200

cagggcgccg cagcctgacg agctgcccga ggtggataac ctgacactgg acgggaatcc 1260

cttcctggtc cctggaactg ccctccccca cgagggctca atgaactccg gcgtggtccc 1320

agcctgtgca cgttcgaccc tgtcggtggg ggtgtcggga accctggtgc tgctccaagg 1380

ggcccggggc tttgcctaag atccaagaca gaataatgaa tggactcaaa ctgccttggc 1440

ttcaggggag tcccgtcagg acgttgagga cttttcgacc aattcaaccc tttgccccac 1500

ctttattaaa atcttaaaca acgggtcaaa aaaaaaaaaa 1540

<210> 27
<211> 4704
<212> DNA
<213> Homo sapiens

<220>
<221> misc_feature
<223> TGFBR2

<400> 27

```
ggagagggag aaggctctcg ggcggagaga ggtcctgccc agctgttggc gaggagtttc        60
ctgtttcccc cgcagcgctg agttgaagtt gagtgagtca ctcgcgcgca cggagcgacg       120
acaccccgc gcgtgcaccc gctcgggaca ggagccggac tcctgtgcag cttccctcgg         180
ccgccggggg cctccccgcg cctcgccggc ctccaggccc cctcctggct ggcgagcggg       240
cgccacatct ggcccgcaca tctgcgctgc cggcccggcg cggggtccgg agagggcgcg       300
gcgcggaggc gcagccaggg gtccgggaag gcgccgtccg ctgcgctggg ggctcggtct       360
atgacgagca gcggggtctg ccatgggtcg ggggctgctc aggggcctgt ggccgctgca       420
catcgtcctg tggacgcgta tcgccagcac gatcccaccg cacgttcaga agtcggatgt       480
ggaaatggag gcccagaaag atgaaatcat ctgccccagc tgtaatagga ctgcccatcc       540
actgagacat attaataacg acatgatagt cactgacaac aacggtgcag tcaagtttcc       600
acaactgtgt aaattttgtg atgtgagatt ttccacctgt gacaaccaga aatcctgcat       660
gagcaactgc agcatcacct ccatctgtga gaagccacag gaagtctgtg tggctgtatg       720
gagaaagaat gacgagaaca taacactaga gacagtttgc catgacccca agctccccta       780
ccatgacttt attctggaag atgctgcttc tccaaagtgc attatgaagg aaaaaaaaaa       840
gcctggtgag actttcttca tgtgttcctg tagctctgat gagtgcaatg acaacatcat       900
cttctcagaa gaatataaca ccagcaatcc tgacttgttg ctagtcatat ttcaagtgac       960
aggcatcagc ctcctgccac cactgggagt tgccatatct gtcatcatca tcttctactg      1020
ctaccgcgtt aaccggcagc agaagctgag ttcaacctgg gaaaccggca agacgcggaa      1080
gctcatggag ttcagcgagc actgtgccat catcctggaa gatgaccgct ctgacatcag      1140
ctccacgtgt gccaacaaca tcaaccacaa cacagagctg ctgcccattg agctggacac      1200
cctggtgggg aaaggtcgct ttgctgaggt ctataaggcc aagctgaagc agaacacttc      1260
agagcagttt gagacagtgg cagtcaagat ctttccctat gaggagtatg cctcttggaa      1320
gacagagaag gacatcttct cagacatcaa tctgaagcat gagaacatac tccagttcct      1380
gacggctgag gagcggaaga cggagttggg gaaacaatac tggctgatca ccgccttcca      1440
cgccaagggc aacctacagg agtacctgac gcggcatgtc atcagctggg aggacctgcg      1500
caagctgggc agctccctcg cccgggggat tgctcacctc cacagtgatc acactccatg      1560
tgggaggccc aagatgccca tcgtgcacag ggacctcaag agctccaata tcctcgtgaa      1620
gaacgaccta acctgctgcc tgtgtgactt tgggctttcc ctgcgtctgg accctactct      1680
gtctgtggat gacctggcta acagtgggca ggtgggaact gcaagataca tggctccaga      1740
agtcctagaa tccaggatga atttggagaa tgttgagtcc ttcaagcaga ccgatgtcta      1800
ctccatggct ctggtgctct gggaaatgac atctcgctgt aatgcagtgg gagaagtaaa      1860
```

agattatgag cctccatttg gttccaaggt gcgggagcac ccctgtgtcg aaagcatgaa 1920

ggacaacgtg ttgagagatc gagggcgacc agaaattccc agcttctggc tcaaccacca 1980

gggcatccag atggtgtgtg agacgttgac tgagtgctgg gaccacgacc cagaggcccg 2040

tctcacagcc cagtgtgtgg cagaacgctt cagtgagctg gagcatctgg acaggctctc 2100

ggggaggagc tgctcggagg agaagattcc tgaagacggc tccctaaaca ctaccaaata 2160

gctcttctgg ggcaggctgg gccatgtcca aagaggctgc ccctctcacc aaagaacaga 2220

ggcagcagga agctgcccct gaactgatgc ttcctggaaa accaaggggg tcactcccct 2280

ccctgtaagc tgtggggata agcagaaaca acagcagcag ggagtgggtg acatagagca 2340

ttctatgcct ttgacattgt cataggataa gctgtgttag cacttcctca ggaaatgaga 2400

ttgattttta caatagccaa taacatttgc actttattaa tgcctgtata taaatatgaa 2460

tagctatgtt ttatatatat atatatatat ctatatatgt ctatagctct atatatatag 2520

ccatacettg aaaagagaca aggaaaaaca tcaaatattc ccaggaaatt ggttttattg 2580

gagaactcca gaaccaagca gagaaggaag ggacccatga cagcattagc atttgacaat 2640

cacacatgca gtggttctct gactgtaaaa cagtgaactt tgcatgagga aagaggctcc 2700

atgtctcaca gccagctatg accacattgc acttgctttt gcaaaataat cattccctgc 2760

ctagcacttc tcttctggcc atggaactaa gtacagtggc actgtttgag gaccagtgtt 2820

cccggggttc ctgtgtgccc ttatttctcc tggacttttc atttaagctc caagccccaa 2880

atctgggggg ctagtttaga aactctccct caacctagtt tagaaactct accccatctt 2940

taataccttg aatgttttga accccacttt ttaccttcat gggttgcaga aaaatcagaa 3000

cagatgtccc catccatgcg attgccccac catctactaa tgaaaaattg ttcttttttt 3060

catctttccc ctgcacttat gttactattc tctgctccca gccttcatcc ttttctaaaa 3120

aggagcaaat tctcactcta ggctttatcg tgtttacttt ttcattacac ttgacttgat 3180

tttctagttt tctatacaaa caccaatggg ttccatcttt ctgggctcct gattgctcaa 3240

gcacagtttg gcctgatgaa gaggatttca actacacaat actatcattg tcaggactat 3300

gacctcaggc actctaaaca tatgttttgt ttggtcagca cagcgtttca aaaagtgaag 3360

ccactttata aatatttgga gattttgcag gaaaatctgg atccccaggt aaggatagca 3420

gatggttttc agttatctcc agtccacgtt cacaaaatgt gaaggtgtgg agacacttac 3480

aaagctgcct cacttctcac tgtaaacatt agctctttcc actgcctacc tggaccccag 3540

tctaggaatt aaatctgcac ctaaccaagg tcccttgtaa gaaatgtcca ttcaagcagt 3600

cattctctgg gtatataata tgattttgac taccttatct ggtgttaaga tttgaagttg 3660

gccttttatt ggactaaagg ggaactcctt taagggtctc agttagccca agtttctttt 3720

```
gcttatatgt taatagtttt accctctgca ttggagagag gagtgcttta ctccaagaag     3780
ctttcctcat ggttaccgtt ctctccatca tgccagcctt ctcaaccttt gcagaaatta     3840
ctagagagga tttgaatgtg ggacacaaag gtcccatttg cagttagaaa atttgtgtcc     3900
acaaggacaa gaacaaagta tgagctttaa aactccatag gaaacttgtt aatcaacaaa     3960
gaagtgttaa tgctgcaagt aatctctttt ttaaaacttt ttgaagctac ttattttcag     4020
ccaaatagga atattagaga gggactggta gtgagaatat cagctctgtt tggatggtgg     4080
aaggtctcat tttattgaga tttttaagat acatgcaaag gtttggaaat agaacctcta     4140
ggcaccctcc tcagtgtggg tgggctgaga gttaaagaca gtgtggctgc agtagcatag     4200
aggcgcctag aaattccact tgcaccgtag ggcatgctga taccatccca atagctgttg     4260
cccattgacc tctagtggtg agtttctaga atactggtcc attcatgaga tattcaagat     4320
tcaagagtat tctcacttct gggttatcag cataaactgg aatgtagtgt cagaggatac     4380
tgtggcttgt tttgtttatg tttttttttc ttattcaaga aaaaagacca aggaataaca     4440
ttctgtagtt cctaaaaata ctgacttttt tcactactat acataaaggg aaagttttat     4500
tcttttatgg aacacttcag ctgtactcat gtattaaaat aggaatgtga atgctatata     4560
ctctttttat atcaaaagtc tcaagcactt atttttattc tatgcattgt ttgtctttta     4620
cataaataaa atgtttatta gattgaataa agcaaaatac tcaggtgagc atcctgcctc     4680
ctgttcccat tcctagtagc taaa                                            4704
```

<210> 28
<211> 4629
<212> DNA
<213> Homo sapiens

<220>
<221> misc_feature
<223> TGFBR2

<400> 28

```
ggagagggag aaggctctcg ggcggagaga ggtcctgccc agctgttggc gaggagtttc       60
ctgtttcccc cgcagcgctg agttgaagtt gagtgagtca ctcgcgcgca cggagcgacg      120
acacccccgc gcgtgcaccc gctcgggaca ggagccggac tcctgtgcag cttccctcgg      180
ccgccggggg cctccccgcg cctcgccggc ctccaggccc cctcctggct ggcgagcggg      240
cgccacatct ggcccgcaca tctgcgctgc cggcccggcg cggggtccgg agagggcgcg      300
gcgcggaggc gcagccaggg gtccgggaag gcgccgtccg ctgcgctggg ggctcggtct      360
atgacgagca gcggggtctg ccatgggtcg gggctgctc aggggcctgt ggccgctgca       420
catcgtcctg tggacgcgta tcgccagcac gatcccaccg cacgttcaga agtcggttaa      480
taacgacatg atagtcactg acaacaacgg tgcagtcaag tttccacaac tgtgtaaatt      540
```

ttgtgatgtg agattttcca cctgtgacaa ccagaaatcc tgcatgagca actgcagcat 600
cacctccatc tgtgagaagc cacaggaagt ctgtgtggct gtatggagaa agaatgacga 660
gaacataaca ctagagacag tttgccatga ccccaagctc ccctaccatg actttattct 720
ggaagatgct gcttctccaa agtgcattat gaaggaaaaa aaaaagcctg gtgagacttt 780
cttcatgtgt tcctgtagct ctgatgagtg caatgacaac atcatcttct cagaagaata 840
taacaccagc aatcctgact tgttgctagt catatttcaa gtgacaggca tcagcctcct 900
gccaccactg ggagttgcca tatctgtcat catcatcttc tactgctacc gcgttaaccg 960
gcagcagaag ctgagttcaa cctgggaaac cggcaagacg cggaagctca tggagttcag 1020
cgagcactgt gccatcatcc tggaagatga ccgctctgac atcagctcca cgtgtgccaa 1080
caacatcaac cacaacacag agctgctgcc cattgagctg gacaccctgg tggggaaagg 1140
tcgctttgct gaggtctata aggccaagct gaagcagaac acttcagagc agtttgagac 1200
agtggcagtc aagatctttc cctatgagga gtatgcctct tggaagacag agaaggacat 1260
cttctcagac atcaatctga agcatgagaa catactccag ttcctgacgg ctgaggagcg 1320
gaagacggag ttggggaaac aatactggct gatcaccgcc ttccacgcca agggcaacct 1380
acaggagtac ctgacgcggc atgtcatcag ctgggaggac ctgcgcaagc tgggcagctc 1440
cctcgcccgg gggattgctc acctccacag tgatcacact ccatgtggga ggcccaagat 1500
gcccatcgtg cacagggacc tcaagagctc caatatcctc gtgaagaacg acctaacctg 1560
ctgcctgtgt gactttgggc tttccctgcg tctggaccct actctgtctg tggatgacct 1620
ggctaacagt gggcaggtgg gaactgcaag atacatggct ccagaagtcc tagaatccag 1680
gatgaatttg gagaatgttg agtccttcaa gcagaccgat gtctactcca tggctctggt 1740
gctctgggaa atgacatctc gctgtaatgc agtgggagaa gtaaaagatt atgagcctcc 1800
atttggttcc aaggtgcggg agcacccctg tgtcgaaagc atgaaggaca acgtgttgag 1860
agatcgaggg cgaccagaaa ttcccagctt ctggctcaac caccagggca tccagatggt 1920
gtgtgagacg ttgactgagt gctgggacca cgacccagag gcccgtctca cagcccagtg 1980
tgtggcagaa cgcttcagtg agctggagca tctggacagg ctctcgggga ggagctgctc 2040
ggaggagaag attcctgaag acggctccct aaacactacc aaatagctct tctggggcag 2100
gctgggccat gtccaaagag gctgcccctc tcaccaaaga acagaggcag caggaagctg 2160
cccctgaact gatgcttcct ggaaaaccaa gggggtcact cccctccctg taagctgtgg 2220
ggataagcag aaacaacagc agcagggagt gggtgacata gagcattcta tgcctttgac 2280
attgtcatag gataagctgt gttagcactt cctcaggaaa tgagattgat tttacaata 2340
gccaataaca tttgcacttt attaatgcct gtatataaat atgaatagct atgttttata 2400

tatatatata tatatctata tatgtctata gctctatata tatagccata ccttgaaaag 2460

agacaaggaa aaacatcaaa tattcccagg aaattggttt tattggagaa ctccagaacc 2520

aagcagagaa ggaagggacc catgacagca ttagcatttg acaatcacac atgcagtggt 2580

tctctgactg taaaacagtg aactttgcat gaggaaagag gctccatgtc tcacagccag 2640

ctatgaccac attgcacttg cttttgcaaa ataatcattc cctgcctagc acttctcttc 2700

tggccatgga actaagtaca gtggcactgt ttgaggacca gtgttcccgg ggttcctgtg 2760

tgcccttatt tctcctggac ttttcattta agctccaagc cccaaatctg gggggctagt 2820

ttagaaactc tccctcaacc tagtttagaa actctacccc atctttaata ccttgaatgt 2880

tttgaacccc actttttacc ttcatgggtt gcagaaaaat cagaacagat gtccccatcc 2940

atgcgattgc cccaccatct actaatgaaa aattgttctt tttttcatct ttcccctgca 3000

cttatgttac tattctctgc tcccagcctt catccttttc taaaaaggag caaattctca 3060

ctctaggctt tatcgtgttt actttttcat tacacttgac ttgattttct agttttctat 3120

acaaacacca atgggttcca tctttctggg ctcctgattg ctcaagcaca gtttggcctg 3180

atgaagagga tttcaactac acaatactat cattgtcagg actatgacct caggcactct 3240

aaacatatgt tttgtttggt cagcacagcg tttcaaaaag tgaagccact ttataaatat 3300

ttggagattt tgcaggaaaa tctggatccc caggtaagga tagcagatgg ttttcagtta 3360

tctccagtcc acgttcacaa aatgtgaagg tgtggagaca cttacaaagc tgcctcactt 3420

ctcactgtaa acattagctc tttccactgc ctacctggac cccagtctag gaattaaatc 3480

tgcacctaac caaggtccct tgtaagaaat gtccattcaa gcagtcattc tctgggtata 3540

taatatgatt ttgactacct tatctggtgt taagatttga agttggcctt ttattggact 3600

aaaggggaac tcctttaagg gtctcagtta gcccaagttt cttttgctta tatgttaata 3660

gttttaccct ctgcattgga gagaggagtg ctttactcca agaagctttc ctcatggtta 3720

ccgttctctc catcatgcca gccttctcaa cctttgcaga aattactaga gaggatttga 3780

atgtgggaca caaaggtccc atttgcagtt agaaaatttg tgtccacaag gacaagaaca 3840

aagtatgagc tttaaaactc cataggaaac ttgttaatca acaaagaagt gttaatgctg 3900

caagtaatct ctttttttaaa actttttgaa gctacttatt ttcagccaaa taggaatatt 3960

agagagggac tggtagtgag aatatcagct ctgtttggat ggtggaaggt ctcattttat 4020

tgagattttt aagatacatg caaaggtttg gaaatagaac ctctaggcac cctcctcagt 4080

gtgggtgggc tgagagttaa agacagtgtg gctgcagtag catagaggcg cctagaaatt 4140

ccacttgcac cgtagggcat gctgatacca tcccaatagc tgttgcccat tgacctctag 4200

tggtgagttt ctagaatact ggtccattca tgagatattc aagattcaag agtattctca 4260

cttctgggtt atcagcataa actggaatgt agtgtcagag gatactgtgg cttgttttgt 4320

ttatgttttt ttttcttatt caagaaaaaa gaccaaggaa taacattctg tagttcctaa 4380

aaatactgac ttttttcact actatacata aagggaaagt tttattcttt tatggaacac 4440

ttcagctgta ctcatgtatt aaaataggaa tgtgaatgct atatactctt tttatatcaa 4500

aagtctcaag cacttatttt tattctatgc attgtttgtc ttttacataa ataaaatgtt 4560

tattagattg aataaagcaa aatactcagg tgagcatcct gcctcctgtt cccattccta 4620

gtagctaaa 4629

<210> 29
<211> 4353
<212> DNA
<213> Homo sapiens

<220>
<221> misc_feature
<223> MAPK14

<400> 29
ttctctcacg aagccccgcc cgcggagagg ttccatattg ggtaaaatct cggctctcgg 60

agagtcccgg gagctgttct cgcgagagta ctgcgggagg ctcccgtttg ctggctcttg 120

gaaccgcgac cactggagcc ttagcgggcg cagcagctgg aacgggagta ctgcgacgca 180

gcccggagtc ggccttgtag gggcgaaggt gcagggagat cgcggcgggc gcagtcttga 240

gcgccggagc gcgtccctgc ccttagcggg gcttgcccca gtcgcagggg cacatccagc 300

cgctgcggct gacagcagcc gcgcgcgcgg gagtctgcgg ggtcgcggca gccgcacctg 360

cgcgggcgac cagcgcaagg tccccgcccg gctgggcggg cagcaagggc cggggagagg 420

gtgcgggtgc aggcgggggc cccacagggc caccttcttg cccggcggct gccgctggaa 480

aatgtctcag gagaggccca cgttctaccg gcaggagctg aacaagacaa tctgggaggt 540

gcccgagcgt taccagaacc tgtctccagt gggctctggc gcctatggct ctgtgtgtgc 600

tgcttttgac acaaaaacgg ggttacgtgt ggcagtgaag aagctctcca gaccatttca 660

gtccatcatt catgcgaaaa gaacctacag agaactgcgg ttacttaaac atatgaaaca 720

tgaaaatgtg attggtctgt tggacgtttt tacacctgca aggtctctgg aggaattcaa 780

tgatgtgtat ctggtgaccc atctcatggg ggcagatctg aacaacattg tgaaatgtca 840

gaagcttaca gatgaccatg ttcagttcct tatctaccaa attctccgag gtctaaagta 900

tatacattca gctgacataa ttcacaggga cctaaaacct agtaatctag ctgtgaatga 960

agactgtgag ctgaagattc tggattttgg actggctcgg cacacagatg atgaaatgac 1020

aggctacgtg gccactaggt ggtacagggc tcctgagatc atgctgaact ggatgcatta 1080

caaccagaca gttgatattt ggtcagtggg atgcataatg gccgagctgt tgactggaag 1140

aacattgttt cctggtacag accatattaa ccagcttcag cagattatgc gtctgacagg 1200

aacaccccc gcttatctca ttaacaggat gccaagccat gaggcaagaa actatattca 1260

gtctttgact cagatgccga agatgaactt tgcgaatgta tttattggtg ccaatcccct 1320

ggctgtcgac ttgctggaga agatgcttgt attggactca gataagagaa ttacagcggc 1380

ccaagccctt gcacatgcct actttgctca gtaccacgat cctgatgatg aaccagtggc 1440

cgatccttat gatcagtcct ttgaaagcag ggacctcctt atagatgagt ggaaaagcct 1500

gacctatgat gaagtcatca gctttgtgcc accacccctt gaccaagaag agatggagtc 1560

ctgagcacct ggtttctgtt ctgttgatcc cacttcactg tgaggggaag gccttttcac 1620

gggaactctc caaatattat tcaagtgcct cttgttgcag agatttcctc catggtggaa 1680

gggggtgtgc gtgcgtgtgc gtgcgtgtta gtgtgtgtgc atgtgtgtgt ctgtctttgt 1740

gggagggtaa gacaatatga acaaactatg atcacagtga ctttacagga ggttgtggat 1800

gctccagggc agcctccacc ttgctcttct ttctgagagt tggctcaggc agacaagagc 1860

tgctgtcctt ttaggaatat gttcaatgca aagtaaaaaa atatgaattg tccccaatcc 1920

cggtcatgct tttgccactt tggcttctcc tgtgacccca ccttgacggt ggggcgtaga 1980

cttgacaaca tcccacagtg gcacggagag aaggcccata ccttctggtt gcttcagacc 2040

tgacaccgtc cctcagtgat acgtacagcc aaaaaggacc aactggcttc tgtgcactag 2100

cctgtgatta acttgcttag tatggttctc agatcttgac agtatatttg aaactgtaaa 2160

tatgtttgtg ccttaaaagg agagaagaaa gtgtagatag ttaaaagact gcagctgctg 2220

aagttctgag ccgggcaagt cgagagggct gttggacagc tgcttgtggg cccggagtaa 2280

tcaggcagcc ttcataggcg gtcatgtgtg catgtgagca catgcgtata tgtgcgtctc 2340

tctttctccc tcacccccag gtgttgccat ttctctgctt acccttcacc tttggtgcag 2400

aggtttcttg aatatctgcc ccagtagtca gaagcaggtt cttgatgtca tgtacttcct 2460

gtgtactctt tatttctagc agagtgagga tgtgttttgc acgtcttgct atttgagcat 2520

gcacagctgc ttgtcctgct ctcttcagga ggccctggtg tcaggcaggt ttgccagtga 2580

agacttcttg ggtagtttag atcccatgtc acctcagctg atattatggc aagtgatatc 2640

acctctcttc agcccctagt gctattctgt gttgaacaca attgatactt caggtgcttt 2700

tgatgtgaaa atcatgaaaa gaggaacagg tggatgtata gcatttttat tcatgccatc 2760

tgttttcaac caactatttt tgaggaatta tcatgggaaa agaccagggc ttttcccagg 2820

aatatcccaa acttcggaaa caagttattc tcttcactcc caataactaa tgctaagaaa 2880

tgctgaaaat caaagtaaaa aattaaagcc cataaggcca gaaactcctt ttgctgtctt 2940

tctctaaata tgattacttt aaaataaaaa agtaacaagg tgtcttttcc actcctatgg 3000

aaaagggtct tcttggcagc ttaacattga cttcttggtt tggggagaaa taaattttgt 3060

```
ttcagaattt tgtatattgt aggaatcctt tgagaatgtg attccttttg atggggagaa      3120

agggcaaatt attttaatat tttgtatttt caactttata aagataaaat atcctcaggg      3180

gtggagaagt gtcgttttca taacttgctg aatttcaggc attttgttct acatgaggac      3240

tcatatattt aagcctttttg tgtaataaga aagtataaag tcacttccag tgttggctgt     3300

gtgacagaat cttgtatttg ggccaaggtg tttccatttc tcaatcagtg cagtgataca      3360

tgtactccag agggacaggg tggacccccct gagtcaactg gagcaagaag gaaggaggca     3420

gactgatggc gattccctct cacccgggac tctcccccctt tcaaggaaag tgaaccttta     3480

aagtaaaggc ctcatctcct ttattgcagt tcaaatcctc accatccaca gcaagatgaa      3540

ttttatcagc catgtttggt tgtaaatgct cgtgtgattt cctacagaaa tactgctctg      3600

aatattttgt aataaaggtc tttgcacatg tgaccacata cgtgttagga ggctgcatgc      3660

tctggaagcc tggactctaa gctggagctc ttggaagagc tcttcggttt ctgagcataa      3720

tgctcccatc tcctgatttc tctgaacaga aaacaaaaga gagaatgagg gaaattgcta      3780

ttttatttgt attcatgaac ttggctgtaa tcagttatgc cgtataggat gtcagacaat      3840

accactggtt aaaataaagc ctatttttca aatttagtga gtttctcaag tttattatat      3900

ttttctcttg tttttattta atgcacaata tggcattata tcaatatcct ttaaactgtg      3960

acctggcata cttgtctgac agatcttaat actactccta acatttagaa aatgttgata      4020

aagcttctta gttgtacatt ttttggtgaa gagtatccag gtctttgctg tggatgggta      4080

aagcaaagag caaatgaacg aagtattaag cattggggcc tgtcttatct acactcgagt      4140

gtaagagtgg ccgaaatgac agggctcagc agactgtggc ctgagggcca aatctggccc      4200

accacctgtt tggtgtagcc tgctaagaat ggcttttaca tttttaaatg gttgggaaag      4260

aaaaaaaaag aagtagtaga ttttgtagca tgtgatgtaa gtaatgtaaa acttaaattc      4320

cagtatccat aaataaagtt ttatgagaac aga                                   4353
```

<210> 30
<211> 4353
<212> DNA
<213> Homo sapiens

<220>
<221> misc_feature
<223> MAPK14

<400> 30

```
ttctctcacg aagccccgcc cgcggagagg ttccatattg ggtaaaatct cggctctcgg        60

agagtcccgg gagctgttct cgcgagagta ctgcgggagg ctcccgtttg ctggctcttg       120

gaaccgcgac cactggagcc ttagcgggcg cagcagctgg aacgggagta ctgcgacgca       180
```

gcccggagtc ggccttgtag gggcgaaggt gcagggagat cgcggcgggc gcagtcttga 240
gcgccggagc gcgtccctgc ccttagcggg gcttgcccca gtcgcagggg cacatccagc 300
cgctgcggct gacagcagcc gcgcgcgcgg gagtctgcgg ggtcgcggca gccgcacctg 360
cgcgggcgac cagcgcaagg tccccgcccg gctgggcggg cagcaagggc cggggagagg 420
gtgcgggtgc aggcgggggc cccacagggc caccttcttg cccggcggct gccgctggaa 480
aatgtctcag gagaggccca cgttctaccg gcaggagctg aacaagacaa tctgggaggt 540
gcccgagcgt taccagaacc tgtctccagt gggctctggc gcctatggct ctgtgtgtgc 600
tgcttttgac acaaaaacgg ggttacgtgt ggcagtgaag aagctctcca gaccatttca 660
gtccatcatt catgcgaaaa gaacctacag agaactgcgg ttacttaaac atatgaaaca 720
tgaaaatgtg attggtctgt tggacgtttt tacacctgca aggtctctgg aggaattcaa 780
tgatgtgtat ctggtgaccc atctcatggg ggcagatctg aacaacattg tgaaatgtca 840
gaagcttaca gatgaccatg ttcagttcct tatctaccaa attctccgag gtctaaagta 900
tatacattca gctgacataa ttcacaggga cctaaaacct agtaatctag ctgtgaatga 960
agactgtgag ctgaagattc tggattttgg actggctcgg cacacagatg atgaaatgac 1020
aggctacgtg gccactaggt ggtacagggc tcctgagatc atgctgaact ggatgcatta 1080
caaccagaca gttgatattt ggtcagtggg atgcataatg gccgagctgt tgactggaag 1140
aacattgttt cctggtacag accatattga tcagttgaag ctcattttaa gactcgttgg 1200
aaccccaggg gctgagcttt tgaagaaaat ctcctcagag tctgcaagaa actatattca 1260
gtctttgact cagatgccga agatgaactt tgcgaatgta tttattggtg ccaatcccct 1320
ggctgtcgac ttgctggaga agatgcttgt attggactca gataagagaa ttacagcggc 1380
ccaagccctt gcacatgcct actttgctca gtaccacgat cctgatgatg aaccagtggc 1440
cgatccttat gatcagtcct ttgaaagcag ggacctcctt atagatgagt ggaaaagcct 1500
gacctatgat gaagtcatca gctttgtgcc accacccctt gaccaagaag agatggagtc 1560
ctgagcacct ggtttctgtt ctgttgatcc cacttcactg tgaggggaag gccttttcac 1620
gggaactctc caaatattat tcaagtgcct cttgttgcag agatttcctc catggtggaa 1680
gggggtgtgc gtgcgtgtgc gtgcgtgtta gtgtgtgtgc atgtgtgtgt ctgtctttgt 1740
gggagggtaa gacaatatga acaaactatg atcacagtga ctttacagga ggttgtggat 1800
gctccagggc agcctccacc ttgctcttct ttctgagagt tggctcaggc agacaagagc 1860
tgctgtcctt ttaggaatat gttcaatgca aagtaaaaaa atatgaattg tccccaatcc 1920
cggtcatgct tttgccactt tggcttctcc tgtgacccca ccttgacggt ggggcgtaga 1980
cttgacaaca tcccacagtg gcacggagag aaggcccata ccttctggtt gcttcagacc 2040
tgacaccgtc cctcagtgat acgtacagcc aaaaaggacc aactggcttc tgtgcactag 2100

cctgtgatta acttgcttag tatggttctc agatcttgac agtatatttg aaactgtaaa 2160

tatgtttgtg ccttaaaagg agagaagaaa gtgtagatag ttaaaagact gcagctgctg 2220

aagttctgag ccgggcaagt cgagagggct gttggacagc tgcttgtggg cccggagtaa 2280

tcaggcagcc ttcataggcg gtcatgtgtg catgtgagca catgcgtata tgtgcgtctc 2340

tctttctccc tcacccccag gtgttgccat ttctctgctt acccttcacc tttggtgcag 2400

aggtttcttg aatatctgcc ccagtagtca gaagcaggtt cttgatgtca tgtacttcct 2460

gtgtactctt tatttctagc agagtgagga tgtgttttgc acgtcttgct atttgagcat 2520

gcacagctgc ttgtcctgct ctcttcagga ggccctggtg tcaggcaggt ttgccagtga 2580

agacttcttg ggtagtttag atcccatgtc acctcagctg atattatggc aagtgatatc 2640

acctctcttc agcccctagt gctattctgt gttgaacaca attgatactt caggtgcttt 2700

tgatgtgaaa atcatgaaaa gaggaacagg tggatgtata gcatttttat tcatgccatc 2760

tgttttcaac caactatttt tgaggaatta tcatgggaaa agaccagggc ttttcccagg 2820

aatatcccaa acttcggaaa caagttattc tcttcactcc caataactaa tgctaagaaa 2880

tgctgaaaat caaagtaaaa aattaaagcc cataaggcca gaaactcctt ttgctgtctt 2940

tctctaaata tgattacttt aaaataaaaa agtaacaagg tgtcttttcc actcctatgg 3000

aaaagggtct tcttggcagc ttaacattga cttcttggtt tggggagaaa taaattttgt 3060

ttcagaattt tgtatattgt aggaatcctt tgagaatgtg attccttttg atggggagaa 3120

agggcaaatt attttaatat tttgtatttt caactttata aagataaaat atcctcaggg 3180

gtggagaagt gtcgttttca taacttgctg aatttcaggc attttgttct acatgaggac 3240

tcatatattt aagccttttg tgtaataaga aagtataaag tcacttccag tgttggctgt 3300

gtgacagaat cttgtatttg ggccaaggtg tttccatttc tcaatcagtg cagtgataca 3360

tgtactccag agggacaggg tggaccccct gagtcaactg gagcaagaag gaaggaggca 3420

gactgatggc gattccctct cacccgggac tctcccctt tcaaggaaag tgaaccttta 3480

aagtaaaggc ctcatctcct ttattgcagt tcaaatcctc accatccaca gcaagatgaa 3540

ttttatcagc catgtttggt tgtaaatgct cgtgtgattt cctacagaaa tactgctctg 3600

aatattttgt aataaaggtc tttgcacatg tgaccacata cgtgttagga ggctgcatgc 3660

tctggaagcc tggactctaa gctggagctc ttggaagagc tcttcggttt ctgagcataa 3720

tgctcccatc tcctgatttc tctgaacaga aaacaaaaga gagaatgagg gaaattgcta 3780

ttttatttgt attcatgaac ttggctgtaa tcagttatgc cgtataggat gtcagacaat 3840

accactggtt aaaataaagc ctatttttca aatttagtga gtttctcaag tttattatat 3900

ttttctcttg tttttattta atgcacaata tggcattata tcaatatcct ttaaactgtg 3960

acctggcata cttgtctgac agatcttaat actactccta acatttagaa aatgttgata 4020

aagcttctta gttgtacatt ttttggtgaa gagtatccag gtctttgctg tggatgggta 4080

aagcaaagag caaatgaacg aagtattaag cattggggcc tgtcttatct acactcgagt 4140

gtaagagtgg ccgaaatgac agggctcagc agactgtggc ctgagggcca aatctggccc 4200

accacctgtt tggtgtagcc tgctaagaat ggcttttaca tttttaaatg gttgggaaag 4260

aaaaaaaaag aagtagtaga ttttgtagca tgtgatgtaa gtaatgtaaa acttaaattc 4320

cagtatccat aaataaagtt ttatgagaac aga 4353

<210> 31
<211> 1431
<212> DNA
<213> Homo sapiens

<220>
<221> misc_feature
<223> MAPK14

<400> 31
ttctctcacg aagccccgcc cgcggagagg ttccatattg ggtaaaatct cggctctcgg 60

agagtcccgg gagctgttct cgcgagagta ctgcgggagg ctcccgtttg ctggctcttg 120

gaaccgcgac cactggagcc ttagcgggcg cagcagctgg aacgggagta ctgcgacgca 180

gcccggagtc ggccttgtag gggcgaaggt gcagggagat cgcggcgggc gcagtcttga 240

gcgccggagc gcgtccctgc ccttagcggg gcttgcccca gtcgcagggg cacatccagc 300

cgctgcggct gacagcagcc gcgcgcgcgg gagtctgcgg ggtcgcggca gccgcacctg 360

cgcgggcgac cagcgcaagg tccccgcccg gctgggcggg cagcaagggc cggggagagg 420

gtgcgggtgc aggcgggggc cccacagggc caccttcttg cccggcggct gccgctggaa 480

aatgtctcag gagaggccca cgttctaccg gcaggagctg aacaagacaa tctgggaggt 540

gcccgagcgt taccagaacc tgtctccagt gggctctggc gcctatggct ctgtgtgtgc 600

tgcttttgac acaaaaacgg ggttacgtgt ggcagtgaag aagctctcca gaccatttca 660

gtccatcatt catgcgaaaa gaacctacag agaactgcgg ttacttaaac atatgaaaca 720

tgaaaatgtg attggtctgt tggacgtttt tacacctgca aggtctctgg aggaattcaa 780

tgatgtgtat ctggtgaccc atctcatggg ggcagatctg aacaacattg tgaaatgtca 840

gaagcttaca gatgaccatg ttcagttcct tatctaccaa attctccgag gtctaaagta 900

tatacattca gctgacataa ttcacaggga cctaaaacct agtaatctag ctgtgaatga 960

agactgtgag ctgaagattc tggattttgg actggctcgg cacacagatg atgaaatgac 1020

aggctacgtg gccactaggt ggtacagggc tcctgagatc atgctgaact ggatgcatta 1080

caaccagaca gttgatattt ggtcagtggg atgcataatg gccgagctgt tgactggaag 1140

aacattgttt cctggtacag accatattga tcagttgaag ctcattttaa gactcgttgg 1200

aaccccaggg gctgagcttt tgaagaaaat ctcctcagag tctgcaagaa actatattca 1260

gtctttgact cagatgccga agatgaactt tgcgaatgta tttattggtg ccaatcccct 1320

gggtaagttg accatatatc ctcacctcat ggatattgaa ttggttatga tataaattgg 1380

ggatttgaag aagagtttct ccttttgacc aaataaagta ccattagttg a 1431

<210> 32
<211> 4274
<212> DNA
<213> Homo sapiens

<220>
<221> misc_feature
<223> MAPK14

<400> 32
ttctctcacg aagccccgcc cgcggagagg ttccatattg ggtaaaatct cggctctcgg 60

agagtcccgg gagctgttct cgcgagagta ctgcgggagg ctcccgtttg ctggctcttg 120

gaaccgcgac cactggagcc ttagcgggcg cagcagctgg aacgggagta ctgcgacgca 180

gcccggagtc ggccttgtag gggcgaaggt gcagggagat cgcggcgggc gcagtcttga 240

gcgccggagc gcgtccctgc ccttagcggg gcttgcccca gtcgcagggg cacatccagc 300

cgctgcggct gacagcagcc gcgcgcgcgg gagtctgcgg ggtcgcggca gccgcacctg 360

cgcgggcgac cagcgcaagg tccccgcccg gctgggcggg cagcaagggc cggggagagg 420

gtgcgggtgc aggcgggggc cccacagggc caccttcttg cccggcggct gccgctggaa 480

aatgtctcag gagaggccca cgttctaccg gcaggagctg aacaagacaa tctgggaggt 540

gcccgagcgt taccagaacc tgtctccagt gggctctggc gcctatggct ctgtgtgtgc 600

tgcttttgac acaaaaacgg ggttacgtgt ggcagtgaag aagctctcca gaccatttca 660

gtccatcatt catgcgaaaa gaacctacag agaactgcgg ttacttaaac atatgaaaca 720

tgaaaatgtg attggtctgt tggacgtttt tacacctgca aggtctctgg aggaattcaa 780

tgatgtgtat ctggtgaccc atctcatggg ggcagatctg aacaacattg tgaaatgtca 840

gaagcttaca gatgaccatg ttcagttcct tatctaccaa attctccgag gtctaaagta 900

tatacattca gctgacataa ttcacaggga cctaaaacct agtaatctag ctgtgaatga 960

agactgtgag ctgaagattc tggattttgg actggctcgg cacacagatg atgaaatgac 1020

aggctacgtg gccactaggt ggtacagggc tcctgagatc atgctgaact ggatgcatta 1080

caaccagaca gttgatattt ggtcagtggg atgcataatg gccgagctgt tgactggaag 1140

aacattgttt cctggtacag accatattga tcagttgaag ctcattttaa gactcgttgg 1200

aaccccaggg gctgagcttt tgaagaaaat ctcctcagag tctctgtcga cttgctggag 1260
aagatgcttg tattggactc agataagaga attacagcgg cccaagccct tgcacatgcc 1320
tactttgctc agtaccacga tcctgatgat gaaccagtgg ccgatcctta tgatcagtcc 1380
tttgaaagca gggacctcct tatagatgag tggaaaagcc tgacctatga tgaagtcatc 1440
agctttgtgc caccacccct tgaccaagaa gagatggagt cctgagcacc tggtttctgt 1500
tctgttgatc ccacttcact gtgaggggaa ggccttttca cgggaactct ccaaatatta 1560
ttcaagtgcc tcttgttgca gagatttcct ccatggtgga agggggtgtg cgtgcgtgtg 1620
cgtgcgtgtt agtgtgtgtg catgtgtgtg tctgtctttg tgggagggta agacaatatg 1680
aacaaactat gatcacagtg actttacagg aggttgtgga tgctccaggg cagcctccac 1740
cttgctcttc tttctgagag ttggctcagg cagacaagag ctgctgtcct tttaggaata 1800
tgttcaatgc aaagtaaaaa aatatgaatt gtccccaatc ccggtcatgc ttttgccact 1860
ttggcttctc ctgtgacccc accttgacgg tggggcgtag acttgacaac atcccacagt 1920
ggcacggaga gaaggcccat accttctggt tgcttcagac ctgacaccgt ccctcagtga 1980
tacgtacagc caaaaaggac caactggctt ctgtgcacta gcctgtgatt aacttgctta 2040
gtatggttct cagatcttga cagtatattt gaaactgtaa atatgtttgt gccttaaaag 2100
gagagaagaa agtgtagata gttaaaagac tgcagctgct gaagttctga gccgggcaag 2160
tcgagagggc tgttggacag ctgcttgtgg gcccggagta atcaggcagc cttcataggc 2220
ggtcatgtgt gcatgtgagc acatgcgtat atgtgcgtct ctctttctcc ctcaccccca 2280
ggtgttgcca tttctctgct tacccttcac ctttggtgca gaggtttctt gaatatctgc 2340
cccagtagtc agaagcaggt tcttgatgtc atgtacttcc tgtgtactct ttatttctag 2400
cagagtgagg atgtgttttg cacgtcttgc tatttgagca tgcacagctg cttgtcctgc 2460
tctcttcagg aggccctggt gtcaggcagg tttgccagtg aagacttctt gggtagttta 2520
gatcccatgt cacctcagct gatattatgg caagtgatat cacctctctt cagcccctag 2580
tgctattctg tgttgaacac aattgatact tcaggtgctt ttgatgtgaa aatcatgaaa 2640
agaggaacag gtggatgtat agcattttta ttcatgccat ctgttttcaa ccaactattt 2700
ttgaggaatt atcatgggaa aagaccaggg cttttcccag gaatatccca aacttcggaa 2760
acaagttatt ctcttcactc ccaataacta atgctaagaa atgctgaaaa tcaaagtaaa 2820
aaattaaagc ccataaggcc agaaactcct tttgctgtct ttctctaaat atgattactt 2880
taaaataaaa aagtaacaag gtgtcttttc cactcctatg gaaaagggtc ttcttggcag 2940
cttaacattg acttcttggt ttggggagaa ataaattttg tttcagaatt ttgtatattg 3000
taggaatcct ttgagaatgt gattcctttt gatggggaga aagggcaaat tattttaata 3060
ttttgtattt tcaactttat aaagataaaa tatcctcagg ggtggagaag tgtcgttttc 3120

ataacttgct gaatttcagg cattttgttc tacatgagga ctcatatatt taagcctttt 3180

gtgtaataag aaagtataaa gtcacttcca gtgttggctg tgtgacagaa tcttgtattt 3240

gggccaaggt gtttccattt ctcaatcagt gcagtgatac atgtactcca gagggacagg 3300

gtggaccccc tgagtcaact ggagcaagaa ggaaggaggc agactgatgg cgattccctc 3360

tcacccggga ctctcccccct ttcaaggaaa gtgaaccttt aaagtaaagg cctcatctcc 3420

tttattgcag ttcaaatcct caccatccac agcaagatga attttatcag ccatgtttgg 3480

ttgtaaatgc tcgtgtgatt tcctacagaa atactgctct gaatattttg taataaaggt 3540

ctttgcacat gtgaccacat acgtgttagg aggctgcatg ctctggaagc ctggactcta 3600

agctggagct cttggaagag ctcttcggtt tctgagcata atgctcccat ctcctgattt 3660

ctctgaacag aaaacaaaag agagaatgag ggaaattgct attttatttg tattcatgaa 3720

cttggctgta atcagttatg ccgtatagga tgtcagacaa taccactggt taaaataaag 3780

cctatttttc aaatttagtg agtttctcaa gtttattata tttttctctt gtttttattt 3840

aatgcacaat atggcattat atcaatatcc tttaaactgt gacctggcat acttgtctga 3900

cagatcttaa tactactcct aacatttaga aaatgttgat aaagcttctt agttgtacat 3960

tttttggtga agagtatcca ggtctttgct gtggatgggt aaagcaaaga gcaaatgaac 4020

gaagtattaa gcattggggc ctgtcttatc tacactcgag tgtaagagtg gccgaaatga 4080

cagggctcag cagactgtgg cctgagggcc aaatctggcc caccacctgt ttggtgtagc 4140

ctgctaagaa tggcttttac atttttaaat ggttgggaaa gaaaaaaaaa gaagtagtag 4200

attttgtagc atgtgatgta agtaatgtaa aacttaaatt ccagtatcca taaataaagt 4260

tttatgagaa caga 4274

<210> 33
<211> 751
<212> DNA
<213> Homo sapiens

<220>
<221> misc_feature
<223> GNLY

<400> 33
gtatctgtgg taaacccagt gacacggggg agatgacata caaaaagggc aggacctgag 60

aaagattaag ctgcaggctc cctgcccata aaacagggtg tgaaaggcat ctcagcggct 120

gccccaccat ggctacctgg gccctcctgc tccttgcagc catgctcctg ggcaacccag 180

gtctggtctt ctctcgtctg agccctgagt actacgacct ggcaagagcc cacctgcgtg 240

atgaggagaa atcctgcccg tgcctggccc aggagggccc ccagggtgac ctgttgacca 300

aaacacagga gctgggccgt gactacagga cctgtctgac gatagtccaa aaactgaaga 360

agatggtgga taagcccacc cagagaagtg tttccaatgc tgcgacccgg gtgtgtagga 420

cggggaggtc acgatggcgc gacgtctgca gaaatttcat gaggaggtat cagtctagag 480

ttacccaggg cctcgtggcc ggagaaactg cccagcagat ctgtgaggac ctcaggttgt 540

gtataccttc tacaggtccc ctctgagccc tctcaccttg tcctgtggaa gaagcacagg 600

ctcctgtcct cagatcccgg gaacctcagc aacctctgcc ggctcctcgc ttcctcgatc 660

cagaatccac tctccagtct ccctcccctg actccctctg ctgtcctccc ctctcacgag 720

aataaagtgt caagcaagat tttaaaaaaa a 751

<210> 34
<211> 995
<212> DNA
<213> Homo sapiens

<220>
<221> misc_feature
<223> GNLY

<400> 34
gtatctgtgg taaacccagt gacacggggg agatgacata caaaaagggc aggacctgag 60

aaagattaag ctgcaggctc cctgcccata aaacagggtg tgaaaggcat ctcagcggct 120

gccccaccat ggctacctgg gccctcctgc tccttgcagc catgctcctg ggcaacccag 180

cccctgcctc cgcatctgcg tggtgaaggc cattggccct catcggtgga tctgcgtttc 240

ctcgggccta cactgtctag gattgtgcgg ggctggtgag agaacaagat ctcttctgtg 300

ttcaaggcag acttcctgcc ccctgcaccc tgctctctcc caggccttga ggtcagtgtg 360

agccccaagg gcaagaacac ttctggaagg gagagtggat ttggctgggc catctggatg 420

gaaggtctgg tcttctctcg tctgagccct gagtactacg acctggcaag agcccacctg 480

cgtgatgagg agaaatcctg cccgtgcctg gcccaggagg gcccccaggg tgacctgttg 540

accaaaacac aggagctggg ccgtgactac aggacctgtc tgacgatagt ccaaaaactg 600

aagaagatgg tggataagcc cacccagaga agtgtttcca atgctgcgac ccgggtgtgt 660

aggacgggga ggtcacgatg gcgcgacgtc tgcagaaatt tcatgaggag gtatcagtct 720

agagttaccc agggcctcgt ggccggagaa actgcccagc agatctgtga ggacctcagg 780

ttgtgtatac cttctacagg tcccctctga gccctctcac cttgtcctgt ggaagaagca 840

caggctcctg tcctcagatc ccgggaacct cagcaacctc tgccggctcc tcgcttcctc 900

gatccagaat ccactctcca gtctccctcc cctgactccc tctgctgtcc tcccctctca 960

cgagaataaa gtgtcaagca agattttaaa aaaaa 995

<210> 35
<211> 25
<212> DNA
<213> Artificial Sequence

<220>
<223> Probe h_CD14_Hs02621496_s1_FAM

<400> 35
ggaatggaga cgcccacagg cgtgt 25

<210> 36
<211> 25
<212> DNA
<213> Artificial Sequence

<220>
<223> Probe h_CFLAR_Hs01116280_m1_FAM

<400> 36
taagcaagga gaagagtttc ttgga 25

<210> 37
<211> 25
<212> DNA
<213> Artificial Sequence

<220>
<223> Probe h_IFNGR2_Hs00194264_m1_FAM

<400> 37
cggaatgtga ctgtcgggcc tccag 25

<210> 38
<211> 25
<212> DNA
<213> Artificial Sequence

<220>
<223> Probe h_IL1B_Hs01555410_m1_FAM

<400> 38
gatgaagtgc tccttccagg acctg 25

<210> 39
<211> 25
<212> DNA
<213> Artificial Sequence

<220>
<223> Probe h_GNLY_Hs01120727_m1_FAM

<400> 39
taccttctac aggtcccctc tgagc 25

<210> 40
<211> 25
<212> DNA

<213> Artificial Sequence

<220>
<223> Probe h_MAPK14_315_Hs01051153_m1_FAM

<400> 40
tgtttcctgg tacagaccat attaa 25

<210> 41
<211> 25
<212> DNA
<213> Artificial Sequence

<220>
<223> Probe h_MKNK1_Hs00374376_m1_FAM

<400> 41
ccaccccatg tggctctgca gaata 25

<210> 42
<211> 25
<212> DNA
<213> Artificial Sequence

<220>
<223> Probe h_PRF1_Hs00169473_m1_FAM

<400> 42
tgccgcttct acagtttcca tgtgg 25

<210> 43
<211> 25
<212> DNA
<213> Artificial Sequence

<220>
<223> Probe h_TBX21_Hs00894392_m1_FAM

<400> 43
acaatgtgac ccagatgatt gtgct 25

<210> 44
<211> 25
<212> DNA
<213> Artificial Sequence

<220>
<223> Probe h_TGFBR2_Hs00234253_m1_FAM

<400> 44
tggctcaacc accagggcat ccaga 25

<210> 45
<211> 25
<212> DNA
<213> Artificial Sequence

<220>

<223> Probe h_TGFBR3_Hs01114253_m1_FAM

<400> 45
cactgcaggt ccagagcctg gtgca 25

## Description

### FIELD OF THE INVENTION

The present invention relates to the field of diagnosis or prognosis of a responding or non-responding phenotype to TNF-α blocking monoclonal antibody useful for treatment of rheumatoid arthritis, as well as associated therapeutic uses.

### BACKGROUND ART

Cytokine targeting drugs and more generally anti-inflammatory biological drugs such as TNFα-blocking agents (herein after referred to as "TBA") are increasingly used in the treatment of various inflammatory diseases. The first indications in which such TBA were approved are rheumatoid arthritis and Crohn's disease. Rheumatoid arthritis (RA) is a chronic, progressive, debilitating auto-immune disease of largely unknown etiology that affects approximately 1% of the population (1). RA is characterized by chronic inflammation of the synovium, which ultimately leads to joint damage, pain and disability (2). The clinical spectrum of RA is heterogeneous, ranging from mild to severe, with variability in secondary organ system involvement. Disease heterogeneity is further illustrated by the current variation in treatment response rates. First line treatment is usually initiated with so called disease-modifying anti-rheumatic drugs (DMARDs), such as methotrexate (MTX). Approximately 30% of patients display a suboptimal response or intolerance to traditional DMARDs (3). In these patients, second line treatment is initiated with "biologics", agents that block molecules or cells thought to be instrumental to disease progression, such as tumor necrosis factor-α (TNFα) and interleukin-1 (IL-1) or B and T-cells. There are indeed nine biologic agents currently available, each with overlapping or unique mechanisms of action (4). The response rates to such treatments vary widely, with a great number of patients remaining refractory to treatment or demonstrating only partial improvement (5). The incomplete understanding of drug mechanisms of action together with disease heterogeneity means that there are no methods of identifying patient suitability for the various biologies prior to the initiation of the treatment. Establishing a rational basis on which to select patients for specific biologies would help patients to be treated more efficiently; those that would be likely to respond would initiate the biologic in question whereas those unlikely to respond could be provided with another treatment.

In absence of reliable literature on efficacy and safety of biologics and given the percentage of patients that do not respond or experience severe adverse effects, the destructive nature of RA, and the societal costs of inefficacious biological treatments, there is a strong need to make predictions on success before starting the therapy. A clinically or radiographic-based test will most probably assess conditions too late for protecting joints from irreversible destruction. Ideally, a molecular biomarker signature as a predictor for therapy responsiveness should be obtained prior to the start of therapy in a readily available bio-sample, such as peripheral blood. Given the systemic nature of RA and the communication between the systemic and organ-specific compartments, the peripheral blood may not directly have implications for the understanding of disease pathogenesis, but it is especially suitable to analyze gene expression profiles that provide a framework to select clinically relevant biomarkers. Furthermore, blood-based tests remain less invasive for the patients than synovial tissue-based tests.

Ultimately, this may lead to a personalized form of medicine, whereby the best suited therapy will be applied to an individual patient.

The same is true for other inflammatory diseases for which TBA have been approved or for which preliminary results indicate that TBA might be a useful treatment. For the moment, TBA have been approved, in addition to the treatment of RA or Crohn's disease, for the treatment of ankylosing spondylitis, psoriatic arthritis, plaque psoriasis, and ulcerative colitis (see notably FDA labels of infliximab and etanercept). In addition, preliminary results suggest that TBA may be useful in the treatment of several other inflammatory diseases, such as vasculitis (notably Behcet's disease, Churg-Strauss vasculitis, polyarteritis nodosa, and giant cell arthritis); Wegener's granulomatosis; sarcoidosis; adult-onset Still's disease, polymyositis/dermatomyositis, and systemic lupus erythematosus (SLE) (31 and 32).

In all cases, only a proportion (although sometimes a high proportion) of patients treated with TBA display a clinical response to the treatment (see notably FDA labels of infliximab (Remicade®) and etanercept (Enbrel®)). For all diseases in which TBA may be useful, it would thus be very helpful to be able to predict the capacity of a subject to respond or not to TBA treatment.

A very powerful way to gain insight into the molecular signatures underlying pathophysiological processes has arisen from DNA microarray technology, which allows the identification of the fraction of genes that are differentially expressed in blood and tissue samples among patients with clinically defined disease. These differentially expressed genes may provide insight into biological pathways contributing to disease and represent classifiers for early diagnosis, prognosis, and response prediction.

Several pitfalls were experienced using this multistage and relatively expensive technology, which highly depends on perfectly standardized conditions. Factors that might influence the sensitivity and reproducibility range from sample differences, variation in amount and quality of starting RNA material, amplification and labeling strategies and dyes, to probe sequence and hybridization conditions. In addition, the lack of standardized approaches for normalization and usage of data analysis algorithms could influence the outcome. Furthermore, most microarray studies are not prospectively planned and often do not have detailed protocols, but rather tend to make use of existing samples. Therefore, verification of results is an essential step in microarrays studies and quality criteria have to be set.

Several groups have explored the possibility of identifying molecular traits (single nucleotide polymorphisms, gene expression etc.) capable of classifying patients according to their response to treatment based on retrospective analyses of biological samples (synovium or peripheral blood) collected at treatment baseline. In particular, much interest has been paid to the TNFα-blocking agent Infliximab, with the first report several years ago by T Lequerré and colleagues of gene expression-based prediction of response to therapy (6). Since then, several other groups have similarly reported on large-scale gene expression analyses of peripheral blood as a means to predict response to Infliximab (7) (8) (9). All of these studies reported on differentially expressed genes and combinations thereof for the prediction of response to therapy.

These studies provided important proof of concept for the prediction of response to Infliximab at baseline of therapy. Nevertheless, as with all studies of this kind, the use of microarray technology, measuring thousands of genes simultaneously in relatively small cohorts of patients, runs the risk of over-fitting data, leading to false positive results. Moreover, the mono-centric nature of these studies may limit the relevance of the genes identified to a wider and more demographically varied population.

The present invention overcomes these drawbacks by combining information from multiple existing studies. This approach can increase the reliability and generalizability of results. Quantitative approaches in which individual studies addressing a set of related research hypothesis are statistically integrated and analyzed to determine the effectiveness of interventions (meta-analysis) showed the broad utility of applying meta-analytic approaches to genome-wide data for the purpose of biological discovery. Meta-analysis were already used to identify genes differentially expressed between two groups, to compare results obtained on different microarray platforms (cross-platform classification), to identify overlaps between samples from heterologous datasets, to identify co-expressed genes or to reconstruct gene networks.

Meta-analyses of multiple gene expression microarray datasets provide discriminative gene expression signatures that are identified and validated on a large number of microarray samples, generated by different laboratories and microarray technologies. Predictive models generated by this approach are better validated than those generated on a single data set, while showing high predictive power and improved generalization performance.

In the present invention, the meta-analysis was performed according to the stepwise approach in conducting meta-analysis on microarray datasets (1-identify suitable microarray studies; 2-extract data from studies (this step also involved getting additional information from the authors of selected studies); 3-prepare the individual datasets; 4-annotate the individual datasets; 5-resolve the many-to-many relationship between probes and genes; 6-combine the study-specific estimates; 7-analyze, present, and interpret results) described in Ramasamy *et al.* (10).

Forty six distinct genes differentially expressed between future responders and non responders to Infliximab therapy have thus been identified. Furthermore, one particular gene (MKNK1) has been found to be highly correlated to the Infliximab responsive or non-responsive phenotype of tested subjects, and several combinations of a minimum number of genes are proposed as being predictive of the primary (week 14, week 16 and week 22) response to anti-TNF treatment in RA patients. These combinations comprise genes that are known to be involved in inflammatory or immune processes rather than in the metabolism pathway of Infliximab, which clearly gives a rational for their general usefulness for predicting TNFα-blocking agents (TBA) responsive or non-responsive phenotype of subjects suffering from other inflammatory diseases, notably those for which TBA have been approved or have been shown to be useful in preliminary studies.

## DETAILED DESCRIPTION OF THE INVENTION

The invention thus relates to a method for the in vitro diagnosis or prognosis of a TNF-α blocking monoclonal antibody responding or non-responding phenotype, comprising:

(a) determining from a blood sample of a subject suffering from rheumatoid arthritis (RA) an expression profile comprising or consisting of:

(i)

- the gene MKNK1; or
- the genes MKNK1 and GNLY ; or
- the genes MKNK1, TBX21 and TGFBR3; or
- the genes MKNK1, GNLY, and ADI1; or

- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1 B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of following Tables 2, 3 and 4; and

(ii) optionally one or more housekeeping gene(s),

(b) comparing the obtained expression profile with at least one reference expression profile, and
(c) determining the responding or non-responding phenotype from said comparison.

The diagnosis or prognosis method according to the invention permits to determine whether a subject is non-responding or responding to a TNF-$\alpha$ blocking monoclonal antibody treatment. In a preferred embodiment, the diagnosis or prognosis methods according to the invention are intended for the diagnosis or prognosis of non-response Response to communication pursuant to Article 94(3) EPC dated April 20, 2016
(i.e. of a non-responding condition) to a TNF-$\alpha$ blocking monoclonal antibody (notably Infliximab). In this case, the methods according to the invention for the diagnosis or prognosis of a non-responding phenotype have the advantage to present high negative predictive value (NPV) and high specificity.
For such diagnosis or prognosis of a non-responding phenotype, a non-responding condition or test outcome is considered a positive result, while a responding condition or test outcome is considered a negative result. True and false positive results, NPV, PPV, specificity, sensitivity and error rate are defined and calculated as follows:

| | | **Condition (non-responding)** | |
|---|---|---|---|
| | | ***Positive*** | ***Negative*** |
| **Test outcome (non-responding)** | ***Positive*** | True Positive (TP) | False positive (FP) |
| | ***Negative*** | False negative (FN) | True negative (TN) |

$$PPV = TP / (TP+FP)$$

$$NPV = TN / (TN+FN)$$

$$Specificity = TN / (TN+FP)$$

$$Sensitivity = TP / (TP+FN)$$

$$Error\ rate = (FP+FN)/\ Total\ number\ of\ patients$$

The invention also relates to a method for designing a TNF-$\alpha$ blocking monoclonal antibody treatment for a subject suffering from rheumatoid arthritis (RA), said method comprising:

(a) determining from a blood sample of said subject an expression profile comprising or consisting of:

(i)

- the gene MKNK1; or
- the genes MKNK1 and GNLY ; or

- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of following Tables 2, 3 and 4; and

(ii) optionally one or more housekeeping gene(s),

(b) comparing the obtained expression profile with at least one reference expression profile,
(c) determining the TNF-$\alpha$ blocking monoclonal antibody responding or non-responding phenotype from said comparison, and
(d) designing the dose of TNF-$\alpha$ blocking monoclonal antibody treatment according to said identified TNF-$\alpha$ blocking monoclonal antibody responding or non-responding phenotype.

In the above method for designing a TNF-$\alpha$ blocking monoclonal antibody treatment for a subject suffering from rheumatoid arthritis (RA), the dose that is designed and optionally administered to the subject depends on its responding or non-responding phenotype. In particular, a usual dose may be administered if the subject is diagnosed or prognosed as responding. In contrast, if the subject is diagnosed or prognosed as non-responding, it may be decided not to administrate the TNF-$\alpha$ blocking monoclonal antibody treatment or to increase the dose.
The present invention also relates to a TNF-$\alpha$ blocking monoclonal antibody, for use in treating rheumatoid arthritis (RA), wherein said treatment comprises the steps of:

(a) determining from a biological sample of a subject suffering from rheumatoid arthritis (RA) the presence of a TNF-$\alpha$ blocking monoclonal antibody responding or non-responding phenotype using the method according to anyone of claims 1 to 10,
(b) determining the dose of TNF-$\alpha$ blocking monoclonal antibody to administer with respect to the result of step (a), and
(c) administering to the subject the dose of TNF-$\alpha$ blocking monoclonal antibody determined in step (b).

More generally, the present description also discloses a method for the in vitro diagnosis or prognosis of a cytokine targeting drug (hereafter referred to as CyTD, such as a TNF$\alpha$-blocking agent, hereafter referred to as TBA) or anti-inflammatory biological drug responding or non-responding phenotype, comprising:

(a) determining from a biological sample of a subject suffering from an inflammatory disease an expression profile comprising or consisting of:

(i)

- the gene MKNK1; or
- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1 B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of following Tables 2, 3 and 4; or
- Equivalent Expression Profile thereof, provided that, in said Equivalent Expression Profile thereof, MKNK1 is not replaced by gene S100A8 nor gene MAPK14, and

(ii) optionally one or more housekeeping gene(s),

(b) comparing the obtained expression profile with at least one reference expression profile, and
(c) determining the CyTD or anti-inflammatory biological drug responding or non-responding phenotype from said comparison.

The present description also discloses a method for designing a CyTD or anti-inflammatory biological drug treatment for a subject suffering from an inflammatory disease, said method comprising:

(a) determining from a biological sample of said subject an expression profile comprising or consisting of:

(i)

- the gene MKNK1; or
- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of following Tables 2, 3 and 4; or
- Equivalent Expression Profile thereof, provided that, in said Equivalent Expression Profile thereof, MKNK1 is not replaced by gene S100A8 nor gene MAPK14, and

(ii) optionally one or more housekeeping gene(s),

(b) comparing the obtained expression profile with at least one reference expression profile,
(c) determining the CyTD or anti-inflammatory biological drug responding or non-responding phenotype from said comparison, and
(d) designing the dose of CyTD or anti-inflammatory biological drug treatment according to said identified CyTD or anti-inflammatory biological drug responding or non-responding phenotype.

The present description is also drawn to a method of treatment of a subject suffering from an inflammatory disease with a CyTD or anti-inflammatory biological drug, comprising:

(a) determining from a biological sample of the said subject the presence of a CyTD or anti-inflammatory biological drug responding or non-responding phenotype using a method disclosed herein, and
(b) adapting the CyTD or anti-inflammatory biological drug treatment in function of the result of step (a).

Said adaptation of the CyTD or anti-inflammatory biological drug treatment may consist in:

- a reduction or suppression of the said CyTD or anti-inflammatory biological drug treatment and a switch to an alternative treatment if the subject has been diagnosed or prognosed as CyTD or anti-inflammatory biological drug non-responding respectively, or
- the continuation of the said CyTD or anti-inflammatory biological drug treatment if the subject has been diagnosed or prognosed as CyTD or anti-inflammatory biological drug responding respectively.

The present description also refers to a new use of a CyTD or anti-inflammatory biological drug in the treatment of an inflammatory disease, comprising the steps of:

(a) determining from a biological sample of a subject suffering from an inflammatory disease the presence of a CyTD or anti-inflammatory biological drug responding or non-responding phenotype using a method disclosed herein, and
(b) determining the dose of CyTD or anti-inflammatory biological drug to administer with respect to the result of step (a).

Optionally; the dose of CyTD or anti-inflammatory biological drug determined in step (b) is administered to the subject.

The present description thus relates to a CyTD or anti-inflammatory biological drug, for use in treating an inflammatory disease, wherein the CyTD or anti-inflammatory biological drug is administered to a subject suffering from said inflammatory disease who has been diagnosed and/or prognosed as responsive using a method disclosed herein.

A CyTD or anti-inflammatory biological drug disclosed herein is preferably a TNF alpha blocking agent such as defined below.

The present description also relates to the use of a CyTD or anti-inflammatory biological drug for preparing a drug for the treatment of an inflammatory disease in subjects suffering from said inflammatory disease who have been diagnosed and/or prognosed as responsive using a method disclosed herein.

In all the present description, the definitions appear in bold characters.

An **"inflammatory disease"** refers to a disease involving uncontrolled inflammation processes leading to body damages, and includes any disease generally considered as inflammatory diseases by those skilled in the art.

Advantageously, said inflammatory disease is known to involve, at least in some cases, a pathogenic inflammatory cytokine (IL-1, IL-6, IL15, IL-17, IL-18, IL-23 or TNF-$\alpha$) secretion. The methods disclosed herein then permit to diagnose the presence of such a pathogenic inflammatory cytokine secretion in a tested subject, to predict his/her capacity to respond to a CyTD treatment, and thus to adapt his/her treatment in view of his CyTD responding/non-responding phenotype. Even more advantageously, said inflammatory disease is known to involve, at least in some cases, a pathogenic TNF-$\alpha$ secretion. The methods disclosed herein then permit to diagnose the presence of such a pathogenic TNF-$\alpha$ secretion in a tested subject, to predict his/her capacity to respond to a TBA treatment, and thus to adapt his/her treatment in view of his TBA responding/non-responding phenotype.

The methods disclosed herein also permit to more generally diagnose a sub type of rheumatoid arthritis or a particular state of activation of the immune system in a tested subject, to predict his/her capacity to respond to an anti-inflammatory biological drug treatment, and thus to adapt his/her treatment in view of his anti-inflammatory biological drug responding/non-responding phenotype.

Such inflammatory diseases may be of autoimmune or non-autoimmune origin. Non limiting examples of inflammatory diseases for which the methods and kits disclosed herein are useful, in particular for determining a TBA responding or non-responding phenotype, include rheumatoid arthritis (RA), Crohn's disease, ankylosing spondylitis, psoriatic arthritis, plaque psoriasis, ulcerative colitis, vasculitis (notably Behcet's disease, Churg-Strauss vasculitis, polyarteritis nodosa, and giant cell arthritis); Wegener's granulomatosis; sarcoidosis; adult-onset Still's disease, polymyositis/dermatomyositis, and systemic lupus erythematosus (SLE). An advantageous group of diseases for which the methods disclosed herein are useful are those in the treatment of which TBA have been approved: rheumatoid arthritis (RA), Crohn's disease, ankylosing spondylitis, psoriatic arthritis, plaque psoriasis, ulcerative colitis. The methods according to the invention are particularly useful for RA-suffering patients for determining a TNF-$\alpha$ blocking monoclonal antibody responding or non-responding phenotype. Methods disclosed herein are also useful for RA-suffering patients for determining an anti-inflammatory biological drug (in particular a recombinant protein inhibiting costimulation of T cells by antigen presenting cells) responding or non-responding phenotype.

For rheumatoid arthritis (RA), since first line treatment is usually initiated with so called disease-modifying anti-rheumatic drugs (DMARDs), the present description also refers to a method of treatment of an RA-suffering subject, comprising the steps of:

(a) administering a therapeutic dose of a DMARD to the said subject suffering from RA,
(b) determining from a biological sample of the said RA-suffering subject the presence of a CyTD or anti-inflammatory biological drug responding or non-responding phenotype using a method disclosed herein, and
(c) determining the dose of CyTD or anti-inflammatory biological drug to administer with respect to the result of step (b).

Thus the present description also refers to a combination of 1) a DMARD and 2) a CyTD or anti-inflammatory biological drug, for the treatment of RA, comprising the steps of:

(a) administering a therapeutic dose of a DMARD to a subject suffering from RA,
(b) determining from a biological sample of the said RA-suffering subject the presence of a CyTD or anti-inflammatory biological drug responding or non-responding phenotype using a method disclosed herein, and
(c) determining the dose of CyTD or anti-inflammatory biological drug to administer with respect to the result of step (b).

Optionnally; the dose of CyTD or anti-inflammatory biological drug determined in step (c) is administered to the subject.

In a preferred embodiment, the DMARD is methotrexate (MTX).

By "**cytokine targeting drug**" or "CyTD", it is meant any molecule neutralizing a cytokine signalling, notably by binding to and neutralizing the cytokine or its receptor. Such a binding and neutralizing molecule may notably be an antibody or a fragment thereof specific for said cytokine or cytokine receptor, cytokine receptor antagonists, or any other molecule, such as a recombinant protein, binding to and neutralizing said cytokine or cytokine receptor. Said CyTD preferably targets an inflammatory cytokine such as IL-1, IL-6, IL-15, IL-17, IL-18, IL-23 or TNF-α or a receptor of such inflammatory cytokines. Molecules targeting IL-1 signalling include monoclonal antibodies to IL-1, such as Canakinumab (commercial name Ilaris®), a human anti-IL-1β monoclonal antibody; antagonists of IL-1 receptor such as anakinra (commercial name Kineret®), and a fusion protein between IgG1 Fc portion and ligand-binding domains of human IL-1 RI and IL-1AcP such as Rilonacept (nom commercial Arcalyst™) Molecules targeting Il-6 signalling notably include Tocilizumab, an anti-IL-6R monoclonal antibody. Molecules targeting 11-15 signalling notably include HuMax-IL-15 (AMG 714), an anti-IL-15 monoclonal antibody. Molecules targeting 11-17 signalling notably include AIN457, an anti-IL-17A monoclonal antibody. In all the present description, a preferred CyTD is a "TNFα-blocking agent" or "TBA".

By **"TNFα-blocking agent"** or "TBA", it is herein meant a biological agent which is capable of neutralizing the effects of TNFα. Said agent is a preferentially a protein such as a soluble TNFα receptor, e.g. Pegsunercept, or an antibody. In a further preferred embodiment, the said agent is a monoclonal antibody. In an even further preferred embodiment, the said agent is selected in the group consisting of Etanercept (Enbrel®), Infliximab (Remicade®), Adalimumab (Humira®), Certolizumab pegol (Cimzia®), and golimumab (Simponi®). In an even more preferred embodiment, the said agent is Infliximab.

By **"anti-inflammatory biological drug",** it is herein meant a biological agent (preferably a recombinant protein, including recombinant antibodies) with anti-inflammatory properties. This includes CyTD directed to inflammatory cytokines such as IL-1, IL-6, IL-15, IL-17, IL-18, IL-23 or TNF-α (in particular TBAs) or to a receptor of such inflammatory cytokines. This also notably includes the following biological agents (preferably recombinant proteins, including recombinant antibodies):

- inhibitors of T cell costimulation by antigen presenting cells such as abatacept (Orencia®) or belatacept (Nulojix®), which are fusion proteins composed of an immunoglobulin Fc fragment fused to the extracellular domain of CTLA-4 (a B7 binding molecule), and which interfere with the binding of T cells CD28 molecules to antigen presenting cells B7 molecules;
- B cells inhibitors, such as rituximab (Rituxan®), an anti-CD20 monoclonal antibody; and
- inhibitors of leukocyte trafficking, such as natalizumab (Tysabri®), and anti-alpha 4-integrin antibody.

In addition to TBAs, and notably infliximab, another preferred anti-inflammatory biological drug is abatacept.

In a particularly preferred embodiment of any method according to the present invention, the TNF-α blocking monoclonal antibody is Infliximab (Remicade®), a particular TBA. In still another preferred embodiment of any method disclosed herein, the inflammatory disease is rheumatoid arthritis and the CyTD or anti-inflammatory biological drug is abatacept (Orencia®).

According to the present description, a **"CyTD or anti-inflammatory biological drug responding phenotype"** is defined as a response state of a subject to the administration of a CyTD or anti-inflammatory biological drug respectively. A "response state" means that the said subject (referred to as a CyTD or anti-inflammatory biological drug responding subject or a responding subject or a responsive subject: for the purpose of this application, these terms are similar) responds to the treatment, i.e. that the treatment is efficacious in the said subject. The definition of response is an improvement in clinical symptoms. The quantification of such response is made according to ACR20, ACR50, ACR70 criteria (11) and/or EULAR criteria between 14 and 22 weeks, and notably at weeks 14, 16 or 22 weeks or change in DAS28 >1.2. Even more preferred is EULAR response criteria at 14 weeks. These criteria (31) have been established by organizations regrouping the professionals in the field (ACR: American College of Rheumatology; EULAR: European League Against Rheumatism). These criteria are thus well known to the skilled person in the art and need not be detailed here.

In contrast, a "CyTD or anti-inflammatory biological drug non-responding phenotype" refers to the absence in said subject (referred to herein as a CyTD or anti-inflammatory biological drug non-responding subject or a non responding subject or a non-responsive subject: these terms should be construed in the context of this application as having the same meaning) of a state of response, meaning that said subject remains refractory to the treatment.

In a preferred embodiment of any of the above-described *in vitro* methods of diagnosis/prognosis, and in particular in methods of diagnosis/prognosis according to the invention, the said subject is an RA-suffering subject. An "RA-suffering subject" is a subject fulfilling the American College of Rheumatology (ACR) criteria for RA (11). The said subject may not be treated with a CyTD or anti-inflammatory biological drug; alternatively, the said subject may be treated with a CyTD or anti-inflammatory biological drug.

It will easily be conceived that when the said subject is not yet treated with a CyTD or anti-inflammatory biological drug, the methods disclosed herein permit a prognosis (also referred to as a prediction) of the responsiveness/non responsiveness of the said subject. Thus, in this embodiment, the method disclosed herein allows the person skilled in the art to prognose (i.e. to identify or predict) the subjects susceptible of responding to the CyTD or anti-inflammatory biological drug treatment. This is important because of the destructive nature of RA and the societal costs of inefficacious biological treatments. Moreover, since this allows for identification of non responsive subjects before any treatment is initiated (i.e. prediction of non response), the risks for one treated subject to encounter severe adverse effects are greatly diminished. Moreover, they are useful for predicting subjects who are not responding to the treatment, i.e. who are refractory to the CyTD or anti-inflammatory biological drug, and should thus be administered another therapy. In particular, the methods disclosed herein allow for a prediction/prognosis of response or non-response at week 14, 16 or 22 from the beginning of the CyTD or anti-inflammatory biological drug treatment.

When the subject is already treated with a CyTD or anti-inflammatory biological drug, the methods disclosed herein are useful for diagnosing if a subject responds to the said CyTD or anti-inflammatory biological drug, and whether the said subject would thus benefit from a continuation of the said treatment. Moreover, they are useful for diagnosing subjects who are not responding to the treatment, i.e. who are refractory to the CyTD or anti-inflammatory biological drug, and should thus be swiftly shifted to another therapy. In regard of the debilitating nature of RA, this achievement is crucial. In particular, the methods disclosed herein allow for a diagnosis at week 14 or 22 after the beginning of the CyTD or anti-inflammatory biological drug treatment.

In the present description, what is described for CyTD or anti-inflammatory biological drug also particularly applies to TBA, which is a preferred embodiment of a CyTD or anti-inflammatory biological drug in any method or kit disclosed herein.

A "**biological sample**" may be any sample that may be taken from a subject, such as a serum sample, a plasma sample, a urine sample, a blood sample, in particular a peripheral blood sample, a lymph sample, or a biopsy. In the invention, the sample is a blood sample. It also includes specific cellular subtypes or derivatives extracted from those such as PBMCs. In the invention, such a sample must allow for the determination of an expression profile comprising or consisting of:

(i)

- the gene MKNK1; or
- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of following Tables 2, 3 and 4; and

(ii) optionally one or more housekeeping gene(s).

Preferred biological samples for the determination of an expression profile include samples such as a blood sample, a plasma sample, a lymph sample, or a biopsy. Preferably, the biological sample is a blood sample. Indeed, such a blood sample may be obtained by a completely harmless blood collection from the patient and thus allows for a non-invasive diagnosis or prognosis of a CyTD or anti-inflammatory biological drug responding or non-responding phenotype. In the invention, the sample is a blood sample.

Optionally, all methods disclosed herein may further comprise a preliminary step of taking a biological sample from the patient.

By **"gene"** is meant the Official Gene Symbol provided by HUGO Gene Nomenclature Committee (www.genenames.org) and used in the Entrez Gene database, the NCBI's repository for gene-specific information (http://www.ncbi.nlm.nih.gov/gene).

By **"expression profile"** is meant the expression levels of a group of genes comprising or consisting of:

(i)

- the gene MKNK1; or

- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1 B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of following Tables 2, 3 and 4; and

(ii) optionally one or more housekeeping gene(s), since these expression profiles have been demonstrated to be particularly relevant for assessing the responding/non responding phenotype of a subject.

In a most preferred embodiment, the expression profile for diagnosing or prognosing (i.e. predicting) if the subject is responding or not at week 14, 16 or week 22 after the beginning of the TNF-$\alpha$ blocking monoclonal antibody treatment comprises or preferably consists of the gene MKNK1, and optionally one or more housekeeping gene(s).

In another most preferred embodiment, the expression profile for diagnosing or prognosing responsiveness or non responsiveness at week 14, week 16 or week 22 after the beginning of the TNF-$\alpha$ blocking monoclonal antibody treatment comprises or preferably consists of the genes MKNK1 and GNLY, and optionally one or more housekeeping gene(s).

In another most preferred embodiment, the expression profile for diagnosing or prognosing responsiveness or non responsiveness at week 14, week 16 or week 22 after the beginning of the TNF-$\alpha$ blocking monoclonal antibody treatment comprises or preferably consists of the genes MKNK1, TBX21 and TGFBR3, and optionally one or more housekeeping gene(s).

In another most preferred embodiment, the expression profile for diagnosing or prognosing responsiveness or non responsiveness at week 14, week 16 or week 22 after the beginning of the TNF-$\alpha$ blocking monoclonal antibody drug treatment comprises or preferably consists of the genes MKNK1, GNLY, and ADI1, and optionally one or more housekeeping gene(s).

In another most preferred embodiment, the expression profile for diagnosing or prognosing responsiveness or non responsiveness at week 14, week 16 or week 22 after the beginning of the TNF-$\alpha$ blocking monoclonal antibody treatment comprises or preferably consists of the genes MKNK1, GNLY, ADI1, and IL1 B, and optionally one or more housekeeping gene(s).

In another most preferred embodiment, the expression profile for diagnosing or prognosing responsiveness or non responsiveness at week 14, week 16 or week 22 after the beginning of the TNF-$\alpha$ blocking monoclonal antibody treatment comprises or preferably consists of the genes MKNK1, GNLY, ADI1, IL1B, and IL1R1, and optionally one or more housekeeping gene(s).

In yet another most preferred embodiment, the expression profile for diagnosing or prognosing responsiveness or non responsiveness at week 14, week 16 or week 22 after the beginning of the TNF-$\alpha$ blocking monoclonal antibody treatment comprises or preferably consists of the genes MKNK1, PRF1, TBX21, TGFBR3, IFNGR2, FYN, IL1B and CFLAR, and optionally one or more housekeeping gene(s).

In still another most preferred embodiment, the expression profile for diagnosing or prognosing responsiveness or non responsiveness at week 14, week 16 or week 22 after the beginning of the TNF-$\alpha$ blocking monoclonal antibody treatment comprises or preferably consists of the genes MKNK1, PRF1, TBX21, TGFBR3, IFNGR2, FYN, IL1B, CFLAR, MAPK14 and GNLY, and optionally one or more housekeeping gene(s).

In yet another most preferred embodiment, the expression profile for diagnosing or prognosing responsiveness or non responsiveness at week 14, week 16 or week 22 after the beginning of the TNF-$\alpha$ blocking monoclonal antibody treatment comprises or preferably consists of the genes MKNK1, PRF1, TBX21, TGFBR3, IFNGR2, FYN, IL1B, CFLAR, CD14 and TGFBR2, and optionally one or more housekeeping gene(s).

In another most preferred embodiment, the expression profile for diagnosing or prognosing responsiveness or non responsiveness at week 14, week 16 or week 22 after the beginning of the TNF-$\alpha$ blocking monoclonal antibody treatment comprises or preferably consists of the genes MKNK1, IFNGR2, IL1B, MAPK14, GNLY, and CD14, , and optionally one or more housekeeping gene(s).

In another most preferred embodiment, the expression profile for diagnosing or prognosing responsiveness or non responsiveness at week 14, week 16 or week 22 after the beginning of the TNF-$\alpha$ blocking monoclonal antibody treatment comprises or preferably consists of the genes MKNK1, PRF1, TBX21, TGFBR3, IFNGR2, IL1B, CFLAR, MAPK14, GNLY, CD14 and TGFBR2, and optionally one or more housekeeping gene(s).

In other methods disclosed herein, by "expression profile" is meant an Equivalent Expression Profile of anyone of the above expression profiles and optionally one or more housekeeping gene(s), provided that, in said Equivalent Expression Profile thereof, MKNK1 is not replaced by gene S100A8 nor gene MAPK14.

The inventors have determined that MKNK1 is a key gene for determining if a subject suffering from an inflammatory disease, in particular an RA-suffering subject, will or not respond to a CyTD or anti-inflammatory biological drug treatment, in particular a TBA treatment and more particularly an Infliximab treatment. The methods according to the invention are thus mainly based on the determination of an expression profile comprising or consisting of gene MKNK1.

However, the addition of a small number of other genes to the tested expression profile improves the sensitivity, specificity, positive predictive value (PPV) and/or negative predictive value (NPV), and thus decreases the error rate of the diagnosis or prognosis and the determined expression profiles thus preferably comprises at least 1, preferably at least 2, at least 3, at least 4, or at least 5 other genes.

In particular, an expression profile comprising MKNK1 preferably comprises 2, 3, 4, 5, 6, 7, 8, 9, 10, 11, 12, 13, 14, 15, 16, 17, 18, 19, or 20 genes useful for the diagnosis or prognosis, including MKNK1, and optionally further comprises one or more housekeeping gene(s) used for normalization of the data.

An expression profile comprising MKNK1 may notably comprise 2-20 genes useful for the diagnosis or prognosis, and in particular 5-15 genes, 6-12 genes, or 8-10 genes useful for the diagnosis or prognosis, including MKNK1, and may optionally further comprise one or more housekeeping gene(s) useful for normalization of the data.

Such an expression profile comprising MKNK1 may notably be chosen from expression profiles comprising the specific combinations of genes disclosed in the present specification, such as those mentioned above.

The determination of the presence of a CyTD or anti-inflammatory biological drug responding or non-responding phenotype is carried out thanks to the comparison of the obtained expression profile with at least one reference expression profile in step (b).

The term **"Equivalent Expression Profile"** herein refers to expression profiles comprising or consisting of :

- the gene MKNK1; or
- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of following Tables 2, 3 and 4,

wherein the addition, deletion or substitution of some of the genes (preferably at most 1 or 2 genes) does not change significantly the reliability of the test and is considered as an "acceptable expression profile".

Preferably, Equivalent Expression Profiles disclosed herein include expression profiles in which one of the genes of a selected genes combination is replaced by an equivalent gene. In the present description, a first gene ("gene A") can be considered as equivalent to another second gene ("gene B"), when replacing "gene A" in the expression profile of by "gene B" does not significantly impact the performance of the test. This is typically the case when "gene A" is correlated to "gene B", meaning that the expression of "gene A" is statistically correlated to the expression level of "gene B", as determined by a measure such as Pearson's correlation coefficient. The correlation may be positive (meaning that when "gene A" is upregulated in a patient, then "gene" B is also upregulated in that same patient) or negative (meaning that when "gene A" is upregulated in a patient, then "gene B" is downregulated in that same patient). Then fact that replacing "gene A" by a correlated "gene B" still permits reliable diagnosis or prognosis of responding or non-responding phenotype is demonstrated for genes with an average Pearson's correlation coefficient of at least 0.9 in Example 3.

In another embodiment, the addition or substitution of some of the genes of the sets described in the present disclosure by other genes belonging to the same metabolic pathway should also be considered as an equivalent expression profile.

For instance, genes that are equivalent to MKNK1 or to one of those of the herein disclosed genes combinations and that may be used in addition or as a substitution in an Equivalent Expression Profile include those disclosed in Figures 1-2 and in Tables 7-9 below. In Table 7 and Table 8 are listed genes that are the most correlated to each of the key genes, the correlation being calculated by measure of Pearson's correlation Response to communication pursuant to Article 94(3) EPC dated April 20, 2016

coefficient based on two types of pangenomic microarray data (the table therefore lists the top genes amongst thousands of genes that are measured by Illumina and Affymetrix microarray data and should be quite exhaustive). In Table 9 are listed genes that are significantly correlated to each of the key genes, the correlation being calculated by measure of Pearson's correlation coefficient based on qPCR validation experiments performed by the inventors (the correlations were performed on a much shorter list of genes (60 genes) and can be used (since it is qPCR based and unlike data from Table 7 and 8) to exemplify the effect on our assay performance (which is done in Table 10).

Illustrative embodiments of Equivalent expression profiles are disclosed in Example 3. Further illustrative Equivalent expression profiles include expression profiles comprising or consisting of at least 1 gene from at least 6 of the following 10 groups:

- Group A, wherein Group A consists of gene MKNK1 and correlated genes as determined in Tables 7 to 9, except S100A8 and MAPK14 (MKNK1; NLRP12; FAM129A; NFKBIZ; KCNJ15; C19orf59; SRPK1; IL1R2; CSF2RA; ACSL1; AQP9; TMEM8; MTMR3; SOD2; CENTD2; NTNG2; DGAT2; PROK2; DYSF; RASSF2; USP32; C15orf28; MAP3K2; QKI; CDC42EP3; NPL; tcag7.907; UIMC1; PELI1; U2AF1; MLX; SAMD4B; REM2; YPEL3; YIPF1; PBX2; NUMB; CKAP4; LOC284454; MAPK1; CD11c, IFNGR2, IFITM2);
- Group B, wherein Group B consists of gene PRF1 and correlated genes as determined in Tables 7 to 9 (PRF1; GNLY; IL2RB; GPR114; GZMH; RASSF1; GZMB; HIP1; SH2D2A; CDC42SE1; LMO4; GPR56; FGFBP2; SPON2; TCP1 ACLY; ALKBH5; R3HDM2; KIR3DL1; NKG7; TBXAS1; CTA221G9.4; TRGV9; TARP; TRGC2; GPR68; FGFBP2; CCL5; KIR3DL3///LILRP2///KIR2DL3; CX3CR1; CECR1; RUNX3; TBX21; CCR5; TGFBR3; and CCL4);
- Group C, wherein Group C consists of gene TBX21 and correlated genes as determined in Tables 7 to 9 (TBX21; CNTN2; POU5F1; CPEB4; RNF185; CAMK1; PRKD3; MLANA; TMEM161B; LMTK2; AZGP1; GMEB1; HDAC8; GSPT2; KNCN; DPM3; CCDC52; C20orf12; AADAC; CEP70; FLJ22374; CHST12; KLRD1; S1PR5; JAKMIP1; MYO6; SOX13; LOC100133241///OSBPL5; GFI1; TTC38; OSBPL5; GPR56; CTA221G9.4; EOMES; C1orf21; NKG7; GZMH; TRGV9; TARP; TRGC2; CCR5; TGFBR3; CCL4; IL2RB; GNLY; and PRF1);
- Group D, wherein Group D consists of gene TGFBR3 and correlated genes as determined in Tables 7 to 9 (TGFBR3; EOMES; C1orf21; GZMH; TRGV9; TARP; TRGC2; GPR68; FGFBP2; NKG7; GZMA; LGR6; XCL2; PPP2R2B; CCR5; HOPX; PYHIN1; PDGFD; KLRK1; SIGLECP3; XCL1; ABCB1; TBX21; CCR5; CCL4; IL2RB; GNLY; ENPP4; FYN; and PRF1);
- Group E, wherein Group E consists of gene IFNGR2 and correlated genes as determined in Tables 7 to 9 (IFNGR2; CFLAR; SIRPA; ZFP36L1; ARHGAP9; TM6SF1; HEXB; FAM65B; SLC37A3; SLC26A8; FKBP1A; NUMB; FBXL5; VNN2; FAM49A; HOXD8; ATP6V1A; PIK3CD; ALOX5AP; SRPK1; TLR4; ACSL1; PREX1; NDEL1; VNN1; DHRS7; PTEN; NCLN; RTN3; PANX2; SLC25A44; NADK; REPS2; OAZ2; PISD; TMCC3; BST1; DIRC2; CKAP4; LOC284454; IL8RB; IL6R; IL1RN; MAPK14; HCK00210; TGFBR2; CD16; CD11c; TIMP2; MKNK1; TNFAIP9; CASC3; and IFITM2);
- Group F, wherein Group F consists of gene FYN and correlated genes as determined in Tables 7 to 9 (FYN; RPUSD2; ADA; PARP1; CARD11; CD81; NDUFS8; C9orf142; ULK3; WDR54; RUNX3; NUP210; DDX56; FLJ21438; KIAA1310; B3GALT6; SMARCD1; POLG; NOL14; FAM62A; DDOST; DAZAP1; SYNCRIP; GIMAP1; CBLB; EZR; MAPRE2; CBX6; AFF1; HNRNPD; CBX5; UHMK1; PURA; NVL; CALM1; YEATS2; ATP5G3; UCP2; PABPC1; SET; DYRK2; SLC25A3; ELK4; ABCB1; CCR5; TGFBR3; IL2RB; and KLRK1);
- Group G, wherein Group G consists of gene 1L1B and correlated genes as determined in Tables 7 to 9 (IL1B; ADM; RERE; KCNJ2; AXUD1; RBM23; PCDHGB5; FCGR2A; ACSL1; GADD45B; NAMPT; RNF149; AQP9; CEBPB; TRIM21; LIMK2; TNFSF13B; PIK3AP1; CEACAM1; STX11; GK; LOC100129443_KIAA0040; BCL3; LOC646470; PIM3; GADD45B; PPIF; JUNB; FLJ39051; STAT5B; C16orf57; LIMK2; GLT1D1; MRPL44; DHRS13; ALPL; ADM; IL1RN; and TLR5);
- Group H, wherein Group H consists of gene CFLAR and correlated genes as determined in Tables 7 to 9 (CFLAR; CASP9; F11R; METTL2A; SNAP23; RRAGD; MVP; MAFF; SERPINB1; JAR1D1B; TLR4; H1ST1H2BC; OSBPL2; FRAT1; RBP7; CSF2RA; IL1R2; IL13RA1; TTRAP; RPGRIP1; CBL; SSH2; MGEA5; LOC647190; SORL1; FAM126B; FAM120A; SMCHD1; MAP3K3; PHF20L1; SPAG9; APAF1; LOC730051; EIF2C2; MLL2; FNIP1; FYB; DKFZP434B2016; NUMB; IL8RB; MAPK14; HCK002110; TGFBR2; TNFAIP9; IFNGR2; IL1R1; IFITM2; and DECR1);
- Group I, wherein Group I consists of gene MAPK14 and correlated genes as determined in Tables 7 to 9 (MAPK14; IL8RB; MMP9; IL6R; IL1RN; HCK002110; CD16; CD11c; SLC2A3; TIMP2; MKNK1; HSPA1A; TNFAIP9; CFLAR; IFNGR2; TLR5; CASC3; and IFITM2); and
- Group J, wherein Group J consists of gene GNLY and correlated genes as determined in Tables 7 to 9 (GNLY; TBX21; TGFBR3; CCL4; IL2RB; and PRF1).

As may be observed in Figure 1 and in Table 7 below, S100A8 can be considered as equivalent to MKNK1. Since expression profiles in which MKNK1 is replaced by gene S100A8 have been disclosed for the same purpose in WO2011/117366, they have been excluded of the scope of the methods disclosed herein. As may be observed in Table 9 below, MAPK14 can also be considered as equivalent to MKNK1. Since expression profiles in which MKNK1 is replaced by gene MAPK14 have been disclosed for the same purpose in WO2011/117366, they have also been excluded of the scope of the methods disclosed herein.

In all methods, kits or microarrays described herein, when the expression profile comprises or consists of the gene MKNK1; or of the genes MKNK1, TBX21 and TGFBR3; or of the genes MKNK1, PRF1, TBX21, TGFBR3, IFNGR2, FYN, IL1B and CFLAR; or of all the 46 genes of following Tables 2, 3 and 4, it is further possible to exclude Equivalent Expression Profiles that comprise genes MAPK14 and/or S100A8. In this case, the methods, kits or microarrays disclosed herein are based on an expression profile comprising or consisting of the gene MKNK1; or of the genes MKNK1, TBX21 and TGFBR3; or of the genes MKNK1, PRF1, TBX21, TGFBR3, IFNGR2, FYN, IL1B and CFLAR; or of all the 46 genes of following Tables 2, 3 and 4 or Equivalent Expression Profile thereof, provided that said Equivalent Expression Profile thereof does not comprise gene MAPK14 nor gene S100A8.

The term **"Acceptable Expression Profile"** herein refers to an expression profile which is capable of correctly classifying at least 60% of the analyzed samples, preferably 65%, and more preferably 70%, and even more preferably 75%, 80% or 85%; and has a sensitivity and specificity of at least 60% preferably 65%, and more preferably 70%, and even more preferably 75 or 80%.The sensitivity value is defined as the ratio of the number of patients actually clinically responding to the CyTD or anti-inflammatory biological drug treatment and classified as responding using the test disclosed herein amongst all patients treated with the CyTD or anti-inflammatory biological drug. Specificity measures the proportion of patients actually clinically not responding to the CyTD or anti-inflammatory biological drug treatment which are correctly identified using the test disclosed herein amongst all patients treated with the CyTD or anti-inflammatory biological drug.

By **"Best Expression Profile"** is meant an expression profile which is able to correctly classify at least 80% of the analyzed samples, has either a sensitivity or a sensitivity of at least 80%.

Although the lists of the gene MKNK1; or of the genes MKNK1 and GNLY; or of the genes MKNK1, TBX21 and TGFBR3; or of the genes MKNK1, GNLY, and ADI1; or of the genes MKNK1, GNLY, ADI1, and IL1B; or of the genes MKNK1, GNLY, ADI1, IL1B, and IL1R1; or of the genes MKNK1, PRF1, TBX21, TGFBR3, IFNGR2, FYN, IL1B and CFLAR; or of the genes MKNK1, PRF1, TBX21, TGFBR3, IFNGR2, FYN, IL1B, CFLAR, MAPK14 and GNLY; or of the genes MKNK1, PRF1, TBX21, TGFBR3, IFNGR2, FYN, IL1B, CFLAR, CD14 and TGFBR2; or of the genes MKNK1, IFNGR2, IL1B, MAPK14, GNLY, and CD14; or of the genes MKNK1, PRF1, TBX21, TGFBR3, IFNGR2, FYN, IL1B, CFLAR, MAPK14, GNLY, CD14 and TGFBR2; or of the genes MKNK1, IFNGR2, IL1B, MAPK14, GNLY, and CD14; or of the genes MKNK1, PRF1, TBX21, TGFBR3, IFNGR2, IL1B, CFLAR, MAPK14, GNLY, CD14 and TGFBR2; or of all the 46 genes of following Tables 2, 3 and 4 have been determined as the Best Expression Profiles to assess responsiveness/non responsiveness, an Equivalent Expression Profile such as defined above, still permits to assess responsiveness, with an acceptable reliability. In particular embodiments, sublists of the gene MKNK1; or of the genes MKNK1 and GNLY; or of the genes MKNK1, TBX21 and TGFBR3; or of the genes MKNK1, GNLY, and ADI1; or of the genes MKNK1, GNLY, ADI1, and IL1B; or of the genes MKNK1, GNLY, ADI1, IL1B, and IL1R1; or of the genes MKNK1, PRF1, TBX21, TGFBR3, IFNGR2, FYN, IL1B and CFLAR; or of the genes MKNK1, PRF1, TBX21, TGFBR3, IFNGR2, FYN, IL1B, CFLAR, MAPK14 and GNLY; or of the genes MKNK1, PRF1, TBX21, TGFBR3, IFNGR2, FYN, IL1B, CFLAR, CD14 and TGFBR2; or of the genes MKNK1, IFNGR2, IL1B, MAPK14, GNLY, and CD14; or of the genes MKNK1, PRF1, TBX21, TGFBR3, IFNGR2, FYN, IL1B, CFLAR, MAPK14, GNLY, CD14 and TGFBR2; or of the genes MKNK1, IFNGR2, IL1B, MAPK14, GNLY, and CD14; or of the genes MKNK1, PRF1, TBX21, TGFBR3, IFNGR2, IL1B, CFLAR, MAPK14, GNLY, CD14 and TGFBR2; or of all the 46 genes of following Tables 2, 3 and 4; still permit to assess responsiveness with a good reliability and should be considered as Acceptable Expression Profiles.

In the invention, while the expression profile used for determining the TNF-$\alpha$ blocking monoclonal antibody responsive or non-responsive phenotype may comprise and not only consist of:

(i)

- the gene MKNK1; or
- the genes MKNK1 and GNLY ; or
- the genes MKNK1, TBX21 and TGFBR3; or
- the genes MKNK1, GNLY, and ADI1; or
- the genes MKNK1, GNLY, ADI 1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or

- the genes MKNK1, PRF1, TBX21, TGFBR3, IFNGR2, FYN, IL1B, CFLAR, MAPK14, GNLY, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of following Tables 2, 3 and 4; and

(ii) optionally one or more housekeeping gene(s),

it is preferred that the expression profiles consists or essentially consists of one of the above described (i) expression profiles, optionally with one or more housekeeping gene(s), meaning that no more than 50, 40, 30, 25, 20, preferably no more than 15, preferably no more than 10, preferably no more than 9, 8, 7, 6, 5, 4, 3, 2, or 1 genes that are not a gene belonging to one the above described (i) expression profiles or a housekeeping gene are included in the expression profile. The same applies to other methods disclosed herein.

By **"housekeeping genes",** it is meant genes that are constitutively expressed at a relatively constant level across many or all known conditions, because they code for proteins that are constantly required by the cell, hence, they are essential to a cell and always present under any conditions. It is assumed that their expression is unaffected by experimental conditions. The proteins they code are generally involved in the basic functions necessary for the sustenance or maintenance of the cell. Non-limitating examples of housekeeping genes that may be used in methods disclosed herein and in particular in methods of the invention include:

- HPRT1 (hypoxanthine phosphoribosyltransferase 1),
- UBC (ubiquitin C),
- YWHAZ (tyrosine 3-monooxygenase/tryptophan 5-monooxygenase activation protein, zeta polypeptide),
- B2M (beta-2-microglobulin),
- GAPDH (glyceraldehyde-3-phosphate dehydrogenase),
- FPGS (folylpolyglutamate synthase),
- DECR1 (2,4-dienoyl CoA reductase 1, mitochondrial),
- PPIB (peptidylprolyl isomerase B (cyclophilin B)),
- ACTB (actin β),
- PSMB2 (proteasome (prosome, macropain) subunit, beta type, 2),
- GPS1 (G protein pathway suppressor 1),
- CANX (calnexin),
- NACA (nascent polypeptide-associated complex alpha subunit),
- TAX1 BP1 (Tax1 (human T-cell leukemia virus type I) binding protein 1), and
- PSMD2 (proteasome (prosome, macropain) 26S subunit, non-ATPase, 2).

When such housekeeping genes are added to the expression profile (it is not always necessary), they are used for normalization purpose. In this case, the number of housekeeping genes used for normalization in methods disclosed herein and in particular in methods according to the invention is preferably comprised between one and five with a preference for three.

The determination of the presence of a responsive or non responsive phenotype is carried out thanks to comparing the obtained expression profile with at least one reference profile in step (b).

A "**reference expression profile**" is a predetermined expression profile, obtained from a biological sample from a subject with a known particular response state. In particular embodiments, the reference expression profile used for comparison with the test sample in step (b) may have been obtained from a biological sample from a CyTD or anti-inflammatory biological drug responsive subject ("CyTD or anti-inflammatory biological drug responsive reference expression profile" or "responsive reference expression profile"; as used herein these expressions are synonymous), and/or from a biological sample from a CyTD or anti-inflammatory biological drug non-responsive subject ("CyTD or anti-inflammatory biological drug non-responsive reference expression profile" or "non-responsive reference expression profile" ; as used herein these expressions have the same meaning).

Preferably, at least one reference expression profile is a CyTD or anti-inflammatory biological drug responsive reference expression profile. Alternatively, at least one reference expression profile may be a CyTD or anti-inflammatory biological drug non-responsive reference expression profile. More preferably, the determination of the presence or absence of a CyTD or anti-inflammatory biological drug responsive phenotype is carried out by comparison with at least one responder and at least one non-responder reference expression profiles. The diagnosis or prognostic may thus be performed using one responsive reference expression profile and one non-responsive reference expression profile. Advantageously, to get a stronger diagnosis or prognostic, said diagnosis or prognostic is carried out using several responsive reference expression profiles and several non-responsive reference expression profiles.

The comparison of a tested subject expression profile with said reference expression profiles, which permits prediction of the tested subject's clinical response based on his/her expression profile, can be done by those skilled in the art using statistical models or machine learning technologies. The PLS (Partial Least Square) regression is particularly relevant to give prediction in the case of small reference samples. The comparison may also be performed using Support Vector Machines (SVM), linear regression or derivatives thereof (such as the generalized linear model abbreviated as GLM, including logistic regression), Linear Discriminant Analysis (LDA), Random Forests, k-NN (Nearest Neighbour) or PAM (Predictive Analysis of Microarrays) statistical methods. More precisely, a group of reference samples, which is generally referred to as training data, is used to select an optimal statistical algorithm that best separates responders from non responders (like a decision rule). The best separation is usually the one that misclassifies as few samples as possible and that has the best chance to perform comparably well on a different dataset.

Typically for a binary outcome such as responder/non-responder, this is done using a generalized linear model abbreviated as GLM, including logistic regression. Logistic regression is based on the determination of a logistic regression function $f(z)=\frac{e^z}{e^z+1}=\frac{1}{1+e^{-z}}$, in which is usually defined as $z = \beta_0 + \beta_1 x_1 + ... + \beta_n x_n$, wherein $X_1$ to $X_n$ are the expression values of the n genes in the signature, $\beta_0$ is the intercept, and $\beta_1$ to $\beta_n$ are the regression coefficients. The values of the intercept and of the regression coefficients are determined based on a group of reference samples ("training data"). f(z) then defines the probability that a test expression profile is responding or non-responding (when defining f(z) based on training data, the user decides if the probability is a probability of response or of non-response). A test expression profile is then classified as responding or non-responding depending if the probability that it is responding or non-responding is inferior or superior to a particular threshold value, which is also determined based on training data. Sometimes, two threshold values are used, defining an undetermined area. Other types of generalized linear models than logistic regression may also be used.

Alternative methods such as nearest neighbour (abbreviated as k-NN) are also commonly used and predict response or non-response for a new sample based on whether the sample is closer to the group of responders or to the group of non-responders. The notion of "closer" is based on a choice of distance (metric, such as but not limited to Euclidian distance) in the n-dimension space defined by a signature consisting of n genes useful for diagnosis or prognosis (thus excluding potential housekeeping genes used for normalization purpose). The distances between a test expression profile and all reference responding or non-responding expression profiles are calculated and the sample is classified by analysis of the k closest reference samples (k being an positive integer of at least 1 and most commonly 3 or 5), a rule of classification being pre-established depending of the number of responding or non-responding reference expression profiles among the k closest reference expression profiles. For instance, when k is 1, a test expression profile is classified as responding if the closest reference expression profile is a responding expression profile, and as non-responding if the closest reference expression profile is a non-responding expression profile. When k is 2, a test expression profile is classified as responding if the two closest reference expression profiles are responding expression profiles, as non-responding if the two closest reference expression profiles are non-responding expression profiles, and undetermined if the two closest reference expression profiles include a responding and a non-responding reference expression profile. When k is 3, a test expression profile is classified as responding if at least two of the three closest reference expression profiles are responding expression profiles, and as non-responding if at least two of the three closest reference expression profiles are non-responding expression profiles. More generally, when k is p, a test expression profile is classified as responding if more than half of the p closest reference expression profiles are responding expression profiles, and as non-responding if more than half of the p closest reference expression profiles are non-responding expression profiles. If the numbers of responding and non-responding reference expression profiles are equal, then the test expression profile is classified as undetermined.

Other methodologies from the field of statistics, mathematics or engineering exist, for example but not limited to decision trees, Support Vector Machines (SVM), Neural Networks and Linear Discriminant Analyses (LDA). These approaches are well known to people skilled in the art.

In summary, an algorithm (which may be selected from linear regression or derivatives thereof such as generalized linear models (GLM, including logistic regression), nearest neighbour (k-NN), decision trees, support vector machines (SVM), neural networks, linear discriminant analyses (LDA), Random forests, or Predictive Analysis of Microarrays (PAM) is calibrated based on a group of reference samples (preferably including several responsive reference expression profiles and several non-responsive reference expression profiles) and then applied to the test sample. In simple terms, a patient will be classified as responder (or non-responder) based on how all the genes in the signature compare to all the genes from a reference profile that was developed from a group of responders (training data).

The notion of whether individual genes of the expression profile are increased or decreased in a responder versus a non-responders is of scientific interest. For each individual gene, the gene expression levels in the responder group can be compared to the non-responder group by the use of Student's t-test or equivalent methods. However, such binary comparisons are generally not used for diagnosis or prognosis when a signature comprises several distinct genes.

The expression profile may be determined by any technology known by a man skilled in the art. In particular, each gene expression level may be measured at the genomic and/or nucleic and/or proteic level. In a preferred embodiment, the expression profile is determined by measuring the amount of nucleic acid transcripts of each gene. In another embodiment, the expression profile is determined by measuring the amount of protein produced by each of the genes.
The amount of nucleic acid transcripts can be measured by any technology known by a man skilled in the art. In particular, the measure may be carried out directly on an extracted messenger RNA (mRNA) sample, or on retrotranscribed complementary DNA (cDNA) prepared from extracted mRNA by technologies well-know in the art. From the mRNA or cDNA sample, the amount of nucleic acid transcripts may be measured using any technology known by a man skilled in the art, including nucleic microarrays, quantitative PCR, next generation sequencing and hybridization with a labelled probe.
In a preferred embodiment, the expression profile is determined using quantitative PCR. Quantitative, or real-time, PCR is a well known and easily available technology for those skilled in the art and does not need a precise description.
In a particular embodiment, the determination of the expression profile using quantitative PCR may be performed as follows. Briefly, the real-time PCR reactions are carried out using the TaqMan Universal PCR Master Mix (Applied Biosystems). 6 μL cDNA is added to a 9 μL PCR mixture containing 7.5 μL TaqMan Universal PCR Master Mix, 0.75 μL of a 20X mixture of probe and primers and 0.75μl water. The reaction consisted of one initiating step of 2 min at 50 deg. C, followed by 10 min at 95 deg. C, and 40 cycles of amplification including 15 sec at 95 deg. C and 1 min at 60 deg. C. The reaction and data acquisition can be performed using the ABI 7900HT Fast Real-Time PCR System (Applied Biosystems). The number of template transcript molecules in a sample is determined by recording the amplification cycle in the exponential phase (cycle threshold or $C_Q$ or $C_T$), at which time the fluorescence signal can be detected above background fluorescence. Thus, the starting number of template transcript molecules is inversely related to $C_T$. The level of expression of a gene is measured using the "ΔΔCT method", briefly a gene is normalized by the value of one or a group of reference/housekeeping genes and/or by a reference sample such as a pooled sample or a commercially available reference such as the qPCR Human Universal Reference cDNA, random primed ; Ozyme ; réf 639654.
In another preferred embodiment, the expression profile is determined by the use of a nucleic microarray.
According to the invention, a "nucleic microarray" consists of different nucleic acid probes that are attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes can be nucleic acids such as cDNAs ("cDNA microarray") or oligonucleotides ("oligonucleotide microarray"), and the oligonucleotides may be about 25 to about 60 base pairs or less in length.
To determine the expression profile of a target nucleic sample, said sample is labelled, contacted with the microarray in hybridization conditions, leading to the formation of complexes between target nucleic acids that are complementary to probe sequences attached to the microarray surface. The presence of labelled hybridized complexes is then detected. Many variants of the microarray hybridization technology are available to the man skilled in the art.
In a preferred embodiment of the invention, the nucleic microarray is an oligonucleotide microarray comprising or consisting of oligonucleotides specific for:

(i)

- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of following Tables 2, 3 and 4; and

(ii) optionally one or more housekeeping gene(s) as defined in appended claim 12. Preferably, the oligonucleotides are about 50 bases in length.

Suitable microarray oligonucleotides specific for any gene of Tables 2, 3 and 4 may be designed, based on the genomic sequence of each gene (see Tables 2, 3 and 4 Genbank accession numbers), using any method of microarray oligonucleotide design known in the art. In particular, any available software developed for the design of microarray oligonucleotides may be used, such as, for instance, the OligoArray software (available at http://berry.engin.umich.edu/oligoarray/), the GoArrays software (available at http://www.isima.fr/bioinfo/goarrays/), the Array Designer software (available at http://www.premierbiosoft.com/dnamicroarray/index.html), the Primer3 software (available at http://frodo.wi.mit.edu/primer3/primer3_code.html), or the Promide software (available at http://oligos.molgen.mpg.de/).

In another embodiment, the expression profile is determined by the use of a protein microarray.

In a particular embodiment of methods disclosed herein and in particular of a method according to the invention, said method may further comprise determining at least one additional parameter useful for the diagnosis or prognosis. Such "parameters useful for the diagnosis or prognosis" are parameters that cannot be used alone for a diagnosis or prognosis but that have been described as displaying significantly different values between responsive subjects and subjects who are clearly refractory and may thus also be used to refine and/or confirm the diagnosis or prognosis according to the above described method. They may notably include relevant clinical parameters depending on the inflammatory disease. For rheumatoid arthritis (RA), such clinical parameters include an assessment of the subject's pain, duration of morning stiffness, the number of swollen joints, the number of painful joints etc. Preferably, the parameters useful for diagnosis or prognosis are determined from a non invasive biological sample of the subject. In particular, for RA, they may be selected from standard biological parameters specific for RA. According to the invention, "standard biological parameters specific for RA" are biological parameters usually used by clinicians to monitor the efficacy of a treatment of RA. These standard biological parameters specific for RA or autoimmune diseases usually comprise serum or plasma concentrations of particular proteins which are well known of those skilled in the art. The said standard biological parameters specific for RA can be determined by tests which include the Antinuclear Antibody test (ANA test), C-Reactive Protein test (CRP test), Erythrocyte sedimentation rate (ESR test), Cyclic Citrullinated Peptide Antibody test (CCP test), and the Rheumatoid Factor test. These tests are well known to the person skilled in the art and not be detailed here. They may be used on their own or in combination.

In the invention, such additional parameters may be used to confirm the diagnosis or prognosis obtained using the expression profile comprising or consisting of:

(i)

- the gene MKNK1; or
- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of following Tables 2, 3 and 4; and

(ii) optionally one or more housekeeping gene(s).

The invention further concerns a kit for the *in vitro* diagnosis or prognosis of a TNF-$\alpha$ blocking monoclonal antibody responsive or non responsive phenotype, comprising at least one reagent specifically intended for the specific determination of the expression level of the genes comprised in an expression profile comprising, or consisting of:

(i)

- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or

- all the 46 genes of following Tables 2, 3 and 4; and

(ii) optionally one or more housekeeping gene(s),
wherein said kit comprises no more than 10 reagents for determining the expression level of a gene that does not belong to one of the expression profiles of (i) and that is not a housekeeping gene.

By "a reagent for the determination of an expression profile" is meant a reagent which specifically allows for the determination of said expression profile, i.e. a reagent specifically intended for the specific determination of the expression level of the genes comprised in the expression profile. This definition excludes generic reagents useful for the determination of the expression level of any gene, such as Taq polymerase or an amplification buffer, although such reagents may also be included in a kit according to the invention.
In a kit according to the invention, said kit is dedicated to the *in vitro* diagnosis or prognosis of a TNF-α blocking monoclonal antibody responsive or non responsive phenotype based on expression profiles comprising or consisting of:

(i)

- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of following Tables 2, 3 and 4; and

(ii) optionally one or more housekeeping gene(s).

By "dedicated", it is meant that reagents for the determination of an expression profile in the kit of the invention essentially consist of reagents for determining the expression level of the above (i) expression profiles, optionally with one or more housekeeping gene(s), and thus comprise a minimum of reagents for determining the expression of other genes than those mentioned in above described (i) expression profiles and housekeeping genes. A dedicated kit of the invention comprises no more than 10, preferably no more than 9, 8, 7, 6, 5, 4, 3, 2, or 1 reagent(s) for determining the expression level of a gene that does not belong to one of the above described (i) expression profiles and that is not a housekeeping gene.
Such a kit for the in vitro diagnosis or prognosis of a TNF-α blocking monoclonal antibody responsive or non responsive phenotype may further comprise instructions for determination of the presence or absence of a responsive phenotype.
Such a kit for the *in vitro* diagnosis or prognosis of a responsive phenotype may also further comprise at least one reagent for the determining of at least one additional parameter useful for the diagnosis or prognosis such as standard biological parameters. In particular, the said reagent is useful for performing any of the following tests: the Antinuclear Antibody test (ANA test), C-Reactive Protein test (CRP test), Erythrocyte sedimentation rate (ESR test), Cyclic Citrullinated Peptide Antibody test (CCP test), and the Rheumatoid Factor test.
In any kit for the *in vitro* diagnosis or prognosis of a responsive phenotype according to the invention, the reagent(s) for the determination of an expression profile comprising, or consisting of:

(i)

- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or

- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of following Tables 2, 3 and 4; and

(ii) optionally one or more housekeeping gene(s),

preferably include specific amplification primers and/or probes for the specific quantitative amplification of transcripts of:

(i)

- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of following Tables 2, 3 and 4; and

(ii) optionally one or more housekeeping gene(s), and/or

a nucleic microarray for the detection of:

(i)

- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of following Tables 2, 3 and 4; and

(ii) optionally one or more housekeeping gene(s).

The determination of the expression profile may thus be performed using quantitative PCR and/or a nucleic microarray, preferably an oligonucleotide microarray, and/or protein microarrays.

In addition, the instructions for the determination of the presence or absence of a TNF-$\alpha$ blocking monoclonal antibody phenotype preferably include at least one reference expression profile, or at least one reference sample for obtaining a reference expression profile. In a preferred embodiment, at least one reference expression profile is a responsive expression profile. Alternatively, at least one reference expression profile may be a non responsive expression profile. More preferably, the determination of the level of responsiveness is carried out by comparison with both responsive and non-responsive expression profiles as described above.

The invention is also directed to a nucleic acid microarray comprising or consisting of nucleic acids specific for:

(i)

- the genes MKNK1 and GNLY ; or
- the genes MKNK1, TBX21 and TGFBR3; or
- the genes MKNK1, GNLY, and AD11; or
- the genes MKNK1, GNLY, ADI1, and IL1B; or

- the genes MKNK1, GNLY, ADI1, IL1 B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of following Tables 2, 3 and 4; and

(ii) optionally one or more housekeeping gene(s),

wherein said nucleic acid microarray comprises no more than 10 distinct nucleic acids specific for a gene that does not belong to one of the expression profiles of (i) and that is not a housekeeping gene.

In a preferred embodiment, said nucleic acid microarray comprises no more than 9, 8, 7, 6, 5, 4, 3, 2, or 1 distinct nucleic acids specific for a gene that does not belong to one of the above described (i) expression profiles and that is not a housekeeping gene.

Advantageously, said microarray consists of nucleic acids specific for:

(i)

- the genes MKNK1 and GNLY ; or
- the genes MKNK1, TBX21 and TGFBR3; or
- the genes MKNK1, GNLY, and AD11; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of following Tables 2, 3 and 4; and

(ii) optionally one or more housekeeping gene(s).

In a preferred embodiment, said nucleic acid microarray is an oligonucleotide microarray comprising or consisting of oligonucleotides specific for:

(i)

- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of following Tables 2, 3 and 4; and

(ii) optionally one or more housekeeping gene(s).

The present description also relates to systems (and computer readable medium for causing computer systems) to perform a method of diagnosis or prognosis of a CyTD or anti-inflammatory biological drug responding or non-responding phenotype, based on above described expression profiles.

In particular, in a specific embodiment, the present description also relates to a system **1** (see **Figure 3**) for analyzing a biological sample comprising:

(a) a determination module **2** configured to receive a biological sample and to determine expression level information concerning:

(i)

- the gene MKNK1; or
- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of Tables 2, 3 and 4; or
- Equivalent Expression Profile thereof, provided that, in said Equivalent Expression Profile thereof, MKNK1 is not replaced by gene S100A8 nor gene MAPK14, and

(ii) optionally one or more housekeeping gene(s)

(b) a storage device **3** configured to store the expression level information from the determination module;
(c) a comparison module **4** adapted to compare the expression level information stored on the storage device with reference data, and to provide a comparison result, wherein the comparison result is indicative of a responding or non-responding phenotype; and
(d) a display module **5** for displaying a content based in part on the comparison result for the user, wherein the content is a signal indicative of a responding or non-responding phenotype.

In another specific embodiment, the present description further relates to a computer readable medium having computer readable instructions recorded thereon to define software modules including a comparison module and a display module for implementing a method on a computer, said method comprising:

a) comparing with a comparison module **4** an expression level information stored on a storage device **3** with reference data to provide a comparison result, wherein the comparison result is indicative of a responding or non-responding phenotype; and
b) displaying with a display module **5** a content based in part on the comparison result for the user, wherein the content is indicative of a responding or non-responding phenotype.

Embodiments of the present description relating to systems and computer-readable media have been described through functional modules, which are defined by computer executable instructions recorded on computer readable media and which cause a computer to perform method steps when executed. The modules have been segregated by function for the sake of clarity. However, it should be understood that the modules need not correspond to discreet blocks of code and the described functions can be carried out by the execution of various code portions stored on various media and executed at various times. Furthermore, it should be appreciated that the modules may perform other functions, thus the modules are not limited to having any particular functions or set of functions.
The computer readable medium can be any available tangible media that can be accessed by a computer. Computer readable medium includes volatile and nonvolatile, removable and non-removable tangible media implemented in any method or technology for storage of information such as computer readable instructions, data structures, program modules or other data. Computer readable medium includes, but is not limited to, RAM (random access memory), ROM (read only memory), EPROM (eraseable programmable read only memory), EEPROM (electrically eraseable programmable read only memory), flash memory or other memory technology, CD-ROM (compact disc read only memory), DVDs (digital versatile disks) or other optical storage media, magnetic cassettes, magnetic tape, magnetic disk storage or other magnetic storage media, other types of volatile and non-volatile memory, and any other tangible medium which can be used to store the desired information and which can accessed by a computer including and any suitable combination of the foregoing.
Computer-readable data embodied on one or more computer-readable media, may define instructions, for example, as part of one or more programs, that, as a result of being executed by a computer, instruct the computer to perform one or more of the functions described herein (e.g., in relation to system **1,** or computer readable medium), and/or various embodiments, variations and combinations thereof. Such instructions may be written in any of a plurality of programming languages, for example, Java, J#, Visual Basic, C, C#, C++, Fortran, Pascal, Eiffel, Basic, COBOL assembly language, and the like, or any of a variety of combinations thereof. The computer-readable media on which such instructions are embodied may reside on one or more of the components of either of **1,** or computer readable medium described herein, may be distributed across one or more of such components, and may be in transition there between.

The computer-readable media may be transportable such that the instructions stored thereon can be loaded onto any computer resource to implement the aspects discussed herein. In addition, it should be appreciated that the instructions stored on the computer readable media, or the computer-readable medium, described above, are not limited to instructions embodied as part of an application program running on a host computer. Rather, the instructions may be embodied as any type of computer code (e.g., software or microcode) that can be employed to program a computer to implement aspects of the present description. The computer executable instructions may be written in a suitable computer language or combination of several languages. Basic computational biology methods are known to those of ordinary skill in the art and are described in, for example, Setubal and Meidanis et al., Introduction to Computational Biology Methods (PWS Publishing Company, Boston, 1997, ref 38); Salzberg, Searles, Kasif, (Ed.), Computational Methods in Molecular Biology, (Elsevier, Amsterdam, 1998, ref 39); Rashidi and Buehler, Bioinformatics Basics: Application in Biological Science and Medicine (CRC Press, London, 2000, ref 40) and Ouelette and Bzevanis Bioinformatics: A Practical Guide for Analysis of Gene and Proteins (Wiley & Sons, Inc., 2nd ed., 2001, ref 41).

The functional modules of certain embodiments disclosed herein include a determination module **2,** a storage device **3,** a comparison module **4** and a display module **5.** The functional modules can be executed on one, or multiple, computers, or by using one, or multiple, computer networks. The determination module **2** has computer executable instructions to provide expression level information in computer readable form.

As used herein, "expression level information" refers to information about expression level of any nucleotide (RNA or DNA) and/or amino acid sequences, either full-length or partial. In a preferred embodiment, it refers to the level of expression of mRNA or cDNA, measured by various technologies. The information may be qualitative (presence or absence of a transcript) or quantitative. Preferably it is quantitative.

Methods for determining expression level information, i.e. determination modules **2,** include systems for protein and DNA/RNA analysis, and in particular those described above for determination of expression profiles at the nucleic or protein level.

The sequence information determined in the determination module can be read by the storage device **3.** As used herein the "storage device" **3** is intended to include any suitable computing or processing apparatus or other device configured or adapted for storing data or information. Examples of electronic apparatus suitable for use with the present description include stand-alone computing apparatus, data telecommunications networks, including local area networks (LAN), wide area networks (WAN), Internet, Intranet, and Extranet, and local and distributed computer processing systems. Storage devices **3** also include, but are not limited to: magnetic storage media, such as floppy discs, hard disc storage media, magnetic tape, optical storage media such as CD-ROM, DVD, electronic storage media such as RAM, ROM, EPROM, EEPROM and the like, general hard disks and hybrids of these categories such as magnetic/optical storage media. The storage device **3** is adapted or configured for having recorded thereon expression level information. Such information may be provided in digital form that can be transmitted and read electronically, e.g., via the Internet, on diskette, via USB (universal serial bus) or via any other suitable mode of communication including wireless communication between devices.

As used herein, "stored" refers to a process for encoding information on the storage device **3.** Those skilled in the art can readily adopt any of the presently known methods for recording information on known media to generate manufactures comprising the expression level information.

A variety of software programs and formats can be used to store the expression level information on the storage device. Any number of data processor structuring formats (e.g., text file, spreadsheets or database) can be employed to obtain or create a medium having recorded thereon the expression level information.

By providing expression level information in computer-readable form, one can use the expression level information in readable form in the comparison module **4** to compare a specific expression profile with the reference data within the storage device **3.** The comparison may notably be done using the various algorithms described above. The comparison made in computer-readable form provides a computer readable comparison result which can be processed by a variety of means. Content based on the comparison result can be retrieved from the comparison module **4** and displayed by the display module **5** to indicate a responding or non-responding phenotype.

Preferably, the reference data are expression level profiles that are indicative of both responding and non-responding phenotypes.

The "comparison module" **4** can use a variety of available software programs and formats for the comparison operative to compare expression level information determined in the determination module **2** to reference data, either directly, or indirectly using any software providing statistical classification algorithms such as those already described above.

The comparison module **4,** or any other module disclosed herein, may include an operating system (e.g., Windows, Linux, Mac OS or UNIX) on which runs a relational database management system, a World Wide Web application, and a World Wide Web server. World Wide Web application includes the executable code necessary for generation of database language statements (e.g., Structured Query Language (SQL) statements). Generally, the executables will include embedded SQL statements. In addition, the World Wide Web application may include a configuration file which contains pointers and addresses to the various software entities that comprise the server as well as the various external and internal databases which must be accessed to service user requests. The Configuration file also directs requests for server resources to the appropriate hardware--as may be necessary should the server be distributed over two or more separate computers. In one embodiment, the World Wide Web server supports a TCP/IP protocol. Local networks such as this are sometimes referred to as "Intranets." An advantage of such Intranets is that they allow easy communication with public domain databases residing on the World Wide Web (e.g., the GenBank or Swiss Pro World Wide Web site). Thus, in a particular preferred embodiment disclosed herein, users can directly access data (via Hypertext links for example) residing on Internet databases using a HTML interface provided by Web browsers and Web servers.

The comparison module **4** provides computer readable comparison result that can be processed in computer readable form by predefined criteria, or criteria defined by a user, to provide a content **6** based in part on the comparison result that may be stored and output as requested by a user using a display module **5.** The display module **5** enables display of a content **6** based in part on the comparison result for the user, wherein the content is a signal indicative of a responding or non-responding phenotype. Such signal can be, for example, a display of content indicative of a responding or non-responding phenotype on a computer monitor, a printed page or printed report of content indicating a responding or non-responding phenotype from a printer, or a light or sound indicative of a responding or non-responding phenotype.

The content **6** based on the comparison result varies depending on the algorithm used for comparison.

For instance, when linear regression or derivatives thereof is used, the content **6** may include a probability of being responding or non-responding, or both a probability of being responding or non-responding and one or more threshold values, or merely a signal indicative of a responding or non-responding phenotype. When nearest neighbor (k-NN) is used, the content **6** may include the number or proportion of responding and non-responding expression profiles among the k closest profiles, or merely a signal indicative of a responding or non-responding phenotype. Moreover, the content **6** may simply be a continuous or categorical score reported in a numerical, text or graphical way (for example using a color code such as red, orange or green).

The display module **5** can be any suitable device configured to receive from a computer and display computer readable information to a user. Non-limiting examples include, for example, general-purpose computers such as those based on Intel PENTIUM-type processor, Motorola PowerPC, Sun UltraSPARC, Hewlett-Packard PA-RISC processors, any of a variety of processors available from Advanced Micro Devices (AMD) of Sunnyvale, California, or from ARM Holdings, or any other type of processor, visual display devices such as flat panel displays, cathode ray tubes and the like, as well as computer printers of various types or integrated devices such as laptops or tablets, in particular iPads.

In one embodiment, a World Wide Web browser is used for providing a user interface for display of the content **6** based on the comparison result. It should be understood that other modules disclosed herein can be adapted to have a web browser interface. Through the Web browser, a user may construct requests for retrieving data from the comparison module. Thus, the user will typically point and click to user interface elements such as buttons, pull down menus, scroll bars and the like conventionally employed in graphical user interfaces. The requests so formulated with the user's Web browser are transmitted to a Web application which formats them to produce a query that can be employed to extract the pertinent information.

In one embodiment, the display module **5** displays the comparison result and whether the comparison result is indicative of a responding or non-responding phenotype.

In one embodiment, the content **6** based on the comparison result that is displayed is a signal (e.g. positive or negative signal) indicative of a responding or non-responding phenotype, thus only a positive or negative indication may be displayed.

The present description therefore provides for systems **1** (and computer readable medium for causing computer systems) to perform methods for diagnosing or prognosing a responding or non-responding phenotype, based on expression profiles information.

System **1,** and computer readable medium, are merely illustrative embodiments disclosed herein for performing methods of diagnosing or prognosing a responding or non-responding phenotype based on expression profiles. Variations of system **1,** and computer readable medium, are possible.

The modules of the system **1** or used in the computer readable medium, may assume numerous configurations. For example, function may be provided on a single machine or distributed over multiple machines.

Having generally described this invention, a further understanding of characteristics and advantages of the invention can be obtained by reference to certain specific examples and figures which are provided herein for purposes of illustration only.

## DESCRIPTION OF THE FIGURES

**Figure 1:** Networks represent the genes that correlate the most to each of the target genes in the data of Julia et al. (8). Any correlated gene can be considered as equivalent.

**Figure 2:** Networks represent the genes that correlate the most to each of the target genes in the data of Bienkowska et al. (9). Any correlated gene can be considered as equivalent.

**Figure 3:** Specific, non-limiting, illustration of a system **1,** comprising a determination module **2,** a storage device **3,** a comparison module **4,** and a display module **5.**

**Figure 4:** Plot illustrating the effect of replacing genes by equivalent ones based on different levels of correlation. Based on the original 8 gene model presented in table 10, we replaced each gene by a "virtual" gene which was simulated based on the fact that it correlated to the original gene. This figure illustrates that in this eight gene signature, all genes can be replaced by genes that on average correlate with a coefficient of 0.9 and the prediction still achieves a sensitivity above 60%.

**Figure 5:** prediction of non response to Infliximab using Equation 1 as defined in Example 5. All patients above the horizontal line at zero are clinical responders to infliximab and predicted as such by a positive score, all non responders are below the line and predicted as such (negative score).

## EXAMPLES

### EXAMPLE 1: Identification of candidate genes by meta-analysis of microarray data

#### Materials and Methods

In this example, the materials and methodologies used in the subsequent examples are described.

Data Identification and Data Extraction: Studies were selected on the basis that they had been performed on RA patients naive to biologies who had started therapy with Infliximab and measurement of their response to treatment was available at 14 or 22 weeks. Large scale gene expression information had to be available at baseline (prior to treatment). Following the steps described in Ramasamy *et al.* (10), we identified four six studies that matched our research criteria: Lequerré *et al.* (6), Sekiguchi *et al.* (7), Bienkowska *et al.* (9), and Julià *et al.* (8),Tanino *et al.* (36) and van Baarsen *et al.* (37). The expression data, the phenotypes and the annotation data were all downloaded from GEO (GSE3592, GSE8350, GSE12051 and, GSE15258, GSE20690 and GSE19821 respectively).

All six studies identified "Gene expression signatures of response to anti-TNF therapy". Interestingly, however, no two publications used the same approach and this can partly explain the lack of overlap between the reported signatures. To make the six studies more comparable, we contacted the authors to obtain additional individual information such as the DAS28 at baseline and week 14, week 16 or week 22 to use a single definition of response (EULAR criteria - with "moderate" and "good" responders considered as responders) or detail of treatment to ensure that only Infliximab-treated patients were analyzed. We therefore reclassified patients as responders based on the EULAR definition at week 14, week 16 and week 22 and performed a binary analysis of good and moderate responders versus non-responders. This binary grouping is particularly suited for the identification of non responders. The final dataset is summarized in **Table 1** and was the most homogeneous data we could obtain.

| Authors | | Bienkowska | Julià | Lequerre | Sekiguchi | Tanino | van Baarsen |
|---|---|---|---|---|---|---|---|
| Study Information | PubMed ID | 19699293 | 19847310 | 16817978 | 18388148 | 19577537 | 20096109 |
| | GEO accession number | GSE15258 | GSE12051 | GSE3592 | GSE8350 | GSE20690 | GSE19821 |
| | Tissue/Blood | whole blood | whole blood | PBMCs | whole blood | whole blood | whole blood |
| | Microarray | Affymetrix | Illumina | Custom | Custom | Agilent | Custom |
| | Treatment | Infliximab (mono-therapy) | Infliximab (bi-therapy) | Infliximab (bi-therapy) | Infliximab (bi-therapy) | Infliximab (bi-therapy) | Infliximab (bi-therapy) |
| | Studied response *criteria* | EULAR @ 14wks | EULAR @ 14wks | EULAR @ 14wks | EULAR @ 22wks | EULAR @ 14wks | EULAR @ 16wks |
| | .CEL | Yes | No | No | No | No | No |
| # Sample | Reponder (G&M) | 20 | 37 | 9 | 15 | 28 | 10 |
| | Non-Reponder (N) | 8 | 7 | 4 | 3 | 8 | 5 |
| | Number of patients | 28 | 44 | 13 | 18 | 36 | 15 |
| Array and Annotation information | Total Probes | 38551 | 15936 | 8356 | 759 | 31537 | 26294 |
| | Unique Genes with the highest spot value | 20164 | 15431 | 6404 | 718 | 19328 | 14432 |
| | Unique Genes with spot the nearest of the primer | 13952 | 13288 | - | - | 13952 | - |
| Patient Information | Mean Age (R/NR) | NA | 52/57 | 54.56/56.75 | 55.2/53 | 55.89/51.00 | NA |
| | Mean RA duration (R/NR) | NA | 13/19 | 12.78/9.75 | 9.30/4.83 | NA | NA |
| | Mean DAS28 at baseline (R/NR) | 5.36/5.00 | 5.99/5.76 | 6.05/5.97 | 6.19/6.24 | NA | NA |
| | Mean DAS28 after treatment (R/NR) | 3.28/5.42 | 3.78/5.44 | 4.18/5.78 | 3.67/5.98 | NA | NA |

**Table 1** : Details of the different studies used in the meta analysis. Studies are named according to the first author. Missing information is represented by « NA » for Not Available.

*Data Quality and Processing:* Data from Bienkowska *et al.* was the only data for which we downloaded the raw .CEL files and processed them using our internal protocols (normalization was performed using GC-RMA in refiner array by GENEDATA Expressionist® (Genedata AG, Basel, Switzerland)). Six chips were flagged with quality issues due to increased distortion. We thus inexcluded them in our analysis.

Data from Lequerré *et al.,* Julia *et al.* and Sekiguchi *et al.* were all downloaded as expression matrices, which correspond to expression values after normalization. The data by Lequerré *et al.* included technical replicates; we averaged the technical replicates and excluded the control samples from the analysis. Following our internal quality control procedures based on the expression profile of sex specific genes, led us to exclude this dataset from further analysis.

To translate probe information into gene information we reblasted the available probe sequences to the latest version of the human genome (Hsap37). Probes were then selected when they mapped within a transcribed region of a gene. When multiple probes were available, we selected the probes that was the closest to the 3' end of the gene. An alternative approach that was also implemented was to select the probe with the highest average value. Therefore for each gene, only one probe contributed to the analysis.

*Statistical Analysis:* Because of the impact on the results of using different parameters when performing a meta analysis, we decided to perform three: The first one is based on the most 3' for probe selection and the Z statistic for variable selection, the second one is based on the most highly expressed probe and the Z statistic for variable selection and the third one is based on the most 3' for probe selection and the Meta Array package.

The statistical analysis was performed in R using the MetaArray package (34) or a classical Z test. The MetaArray package implements the latent variable model described in (35) as well as the integrative correlation (12). Individual probes contribution was estimated using the t-test as implemented in the multtest library in R. We set the threshold for significance at a p-value of 0.10 because only 2 genes were significant at 5. For the Z test, a p-value of 5% was used as a threshold of significance.

**RESULTS**

Table 2 provides the Z score value (indicative of the direction), the p-value, the gene symbol, the gene title and the Gene ID (Notation from Entrez Gene NCBI database, updated on December 2010) for the list of significant genes (p-value <5%) based on the 3' approach to gene mapping and the Z statistic.

**Table 2:** List of significant genes (p-value <5%) based on the 3' approach to gene mapping and the Z statistic.

| Z-score | P-value | Gene Symbol | Gene Title | Gene ID |
|---|---|---|---|---|
| 3,39459 | 0,00069 | RHOB | ras homolog gene family, member B | 388 |
| -3,11193 | 0,00186 | PSMA2 | proteasome (prosome, macropain) subunit, alpha type, 2 | 5683 |
| -2,62277 | 0,00872 | CSE1L | CSE1 chromosome segregation 1-like (yeast) | 1434 |
| 2,59875 | 0,00936 | CSK | c-src tyrosine kinase | 1445 |
| 2,55024 | 0,01076 | MAP2K4 | mitogen-activated protein kinase kinase 4 | 6416 |
| 2,44369 | 0,01454 | IL1B | interleukin 1, beta | 3553 |
| 2,37255 | 0,01767 | YWHAZ | tyrosine 3-monooxygenase/tryptophan 5-monooxygenase activation protein, zeta polypeptide | 7534 |
| 2,36878 | 0,01785 | CCL4 | chemokine (C-C motif) ligand 4 | 6351 |
| 2,34906 | 0,01882 | IGF2R | insulin-like growth factor 2 receptor | 3482 |
| 2,31643 | 0,02053 | PRF1 | perforin 1 (pore forming protein) | 5551 |
| 2,31634 | 0,02054 | TGFBR3 | Transforming growth factor, beta receptor III | 7049 |
| -2,28062 | 0,02257 | AP1S2 | adaptor-related protein complex 1, sigma 2 subunit | 8905 |
| 2,26557 | 0,02348 | ITGAL | integrin, alpha L (antigen CD11A (p180), lymphocyte function-associated antigen 1 alpha polypeptide) | 3683 |
| 2,24496 | 0,02477 | ADAR | adenosine deaminase, RNA-specific | 103 |
| -2,20781 | 0,02726 | RPA3 | replication protein A3, 14kDa | 6119 |
| 2,16438 | 0,03044 | IFNGR2 | interferon gamma receptor 2 (interferon gamma transducer 1) | 3460 |
| -2,10815 | 0,03502 | NDUFV2 | NADH dehydrogenase (ubiquinone) flavoprotein 2, 24kDa | 4729 |

(continued)

| Z-score | P-value | Gene Symbol | Gene Title | Gene ID |
|---|---|---|---|---|
| 2,09749 | 0,03595 | SLC11A1 | solute carrier family 11 (proton-coupled divalent metal ion transporters), member 1 | 6556 |
| 2,09105 | 0,03652 | TIMP2 | TIMP metallopeptidase inhibitor 2 | 7077 |
| 2,08302 | 0,03725 | CP | ceruloplasmin (ferroxidase) | 1356 |
| 2,07606 | 0,03789 | TBX21 | T-box 21 | 30009 |
| 2,02259 | 0,04312 | CCR5 | chemokine (C-C motif) receptor 5 C-C chemokine receptor type 5-like | 1234 |
| 2,00034 | 0,04546 | TGFB2 | CDNA FLJ11812 fis, clone HEMBA1006364 | |
| 1,99736 | 0,04579 | MKNK1 | MAP kinase interacting serine/threonine kinase 1 | 8569 |

Table 3 provides the Z score value (indicative of the direction), the p-value, the Gene Symbol, the Gene Title and the Gene ID (Notation from Entrez Gene NCBI database, updated on December 2010)based on the most highly expressed probe and the Z statistic.

**Table 3:** Discriminatory performance of the gene set based on the most highly expressed probe and the Z statistic.

| Z-score | P-value | Gene Symbol | Gene Title | Gene ID |
|---|---|---|---|---|
| 3,088444 | 0,002012 | HSPA1A | heat shock 70kDa protein 1A | 3303 |
| 3,053196 | 0,002264 | RHOB | ras homolog gene family, member B | 388 |
| 2,790099 | 0,005269 | ELK4 | ELK4, ETS-domain protein (SRF accessory protein 1) | 2005 |
| -2,661722 | 0,007774 | HPRT1 | Hypoxanthine phosphoribosyltransferase 1 (Lesch-Nyhan syndrome) | 3251 |
| 2,634720 | 0,008421 | CSK | c-src tyrosine kinase | 1445 |
| 2,550244 | 0,010765 | MAP2K4 | mitogen-activated protein kinase kinase 4 | 6416 |
| 2,478105 | 0,013208 | CP | ceruloplasmin (ferroxidase) | 1356 |
| 2,460926 | 0,013858 | IL1B | interleukin 1, beta | 3553 |
| 2,368784 | 0,017847 | CCL4 | chemokine (C-C motif) ligand 4 | 6351 |
| 2,356203 | 0,018463 | TGFBR3 | transforming growth factor, beta receptor III | 7049 |
| 2,349061 | 0,018821 | IGF2R | insulin-like growth factor 2 receptor | 3482 |
| -2,311675 | 0,020796 | PFDN4 | prefoldin subunit 4 | 5203 |
| 2,276939 | 0,022790 | MKNK1 | MAP kinase interacting serine/threonine kinase 1 | 8569 |
| 2,270925 | 0,023152 | SLC11A1 | Solute carrier family 11 (proton-coupled divalent metal ion transporters), member 1 | 6556 |
| -2,259800 | 0,023834 | NDUFA4 | NADH dehydrogenase (ubiquinone) 1 alpha subcomplex, 4, 9kDa | 4697 |
| 2,214500 | 0,026794 | IFNGR2 | interferon gamma receptor 2 (interferon gamma transducer 1) | 3460 |
| 2,178084 | 0,029400 | CFLAR | CASP8 and FADD-like | 8837 |
| | | | apoptosis regulator | |
| -2,164143 | 0,030453 | HSP90AA1 | heat shock protein 90kDa alpha (cytosolic), class A member 1 | 3320 |
| -2,162666 | 0,030567 | PSMA2 | proteasome (prosome, macropain) subunit, alpha type, 2 | 5683 |

(continued)

| Z-score | P-value | Gene Symbol | Gene Title | Gene ID |
|---|---|---|---|---|
| -2,123562 | 0,033707 | COX6A1 | cytochrome c oxidase subunit VIa polypeptide 1 | 1337 |
| 2,112754 | 0,034622 | ABCB1 | ATP-binding cassette, subfamily B (MDR/TAP), member 1 | 5243 |
| -2,108153 | 0,035018 | NDUFV2 | NADH dehydrogenase (ubiquinone) flavoprotein 2, 24kDa | 4729 |
| -2,083767 | 0,037181 | CKS2 | CDC28 protein kinase regulatory subunit 2 | 1164 |
| 2,076061 | 0,037888 | TBX21 | T-box 21 | 30009 |
| -2,011073 | 0,044318 | NME1 | non-metastatic cells 1, protein (NM23A) expressed in | 4830 |
| 2,010690 | 0,044358 | PIAS3 | protein inhibitor of activated STAT, 3 | 10401 |
| 2,009776 | 0,044455 | ARID3A | AT rich interactive domain 3A (BRIGHT-like) | 1820 |
| -1,989802 | 0,046613 | RPA3 | replication protein A3, 14kDa | 6119 |
| 1,972567 | 0,048545 | PLSCR1 | phospholipid scramblase 1 | 5359 |
| 1,972072 | 0,048601 | PML | promyelocytic leukemia | 5371 |

Table 4 provides the Gene Symbol, the Gene Title and the Gene ID (Notation from Entrez Gene NCBI database, updated on December 2010) for the list of borderline significant genes (p-value <10%) based on the 3' approach to gene mapping and the MetaArray package in R.

**Table 4:** List of borderline significant genes (p-value <10%) based on the 3' approach to gene mapping and the MetaArray package in R.

| List of genes for MetaArray (Genedata + BJTVS) | | |
|---|---|---|
| Gene Symbol | Gene Title | Gene ID |
| CASC3 | cancer susceptibility candidate 3 | 22794 |
| NDUFV2 | NADH dehydrogenase (ubiquinone) flavoprotein 2, 24kDa | 4729 |
| ITGA5 | integrin, alpha 5 (fibronectin receptor, alpha polypeptide) | 3678 |
| NMI | N-myc (and STAT) interactor | 9111 |
| FYN | FYN oncogene related to SRC, FGR, YES | 2534 |
| HMOX1 | heme oxygenase (decycling) 1 | 3162 |
| CCT6A | chaperonin containing TCP1, subunit 6A (zeta 1) | 908 |

**EXAMPLE 2: Validation of candidate genes and signatures**

For the 45 genes identified in Example 1, probes specific to Taqman assays were ordered from APPLIED BIOSYSTEMS based on APPLIED's inventoried probes or were designed internally using standard software. After our internal quality control steps 35 out of the 45 genes were tested on 40 RA samples. The 40 samples used for the qCPR represent a subset of the original samples used for microarray analysis in the study of Julia *et al.* The delatadeltaCT method was used to measure gene expression levels after carefully selecting reference genes internally. The following statistical analyses have been performed: Identification of individually differentially expressed genes between the two groups of responders versus non responders (Table 5). The selection criteria used was a significant t-test is p-value< 0.05. The following 8 genes were found to be significant: MKNK1, PRF1, TBX21, TGFBR3, IFNGR2, FYN, IL1B and CFLAR.

**Table 5:** P-values from test of responders versus non responders.

| Gene Symbol | Applied Probe | P-value |
|---|---|---|
| *MKNK1* | *h_MKNK1_Hs00374376_m1_FAM* | *0.00288633* |
| *PRF1* | *h_PRF1_Hs00169473_m1_FAM* | *0.00442224* |
| *TBX21* | *h_TBX21_Hs00894392_m1_FAM* | *0.00624481* |
| *TGFBR3* | *h_TGFBR3_Hs01114253_m1_FAM* | *0.00929707* |
| *IFNGR2* | *h_IFNGR2_Hs00194264_m1_FAM* | *0.01069053* |
| *FYN* | *h_FYN_Hs00941600_m1_FAM* | *0.01219544* |
| *IL1B* | *h_IL1B_Hs01555410_m1_FAM* | *0.02595403* |
| *CFLAR* | *h_CFLAR_Hs01116280_m1_FAM* | *0.04107801* |
| CASC3 | h_CASC3_Hs00201226_m1_FAM | 0.05008698 |
| CCT6A | h_CCT6A_Hs00798979_s1_FAM | 0.0574514 |
| HSPA1A | h_HSPA1A_Hs00359163_s1_FAM | 0.06025312 |
| CCL4 | h_CCL4_Hs99999148_m1_FAM | 0.06442333 |
| TIMP2 | h_TIMP2_Hs00234278_m1_FAM | 0.07542514 |
| ACBB1 | h_ABCB1_Hs00184500_m1_FAM | 0.07930045 |
| CSE1L | h_CSE1L_Hs00354853_m1_FAM | 0.08825819 |
| ELK4 | h_ELK4_Hs00360812_m1_FAM | 0.10804976 |
| ADAR | h_ADAR_Hs00241666_m1_FAM | 0.11162474 |
| CCR5 | h_CCR5_Hs99999149_s1_FAM | 0.12023433 |
| NDUFV2 | h_NDUFV2_Hs00221478_m1_FAM | 0.13730798 |
| HPRT1 | h_HPRT1_Hs99999909_m1_FAM | 0.20940829 |
| YWHAZ | h_YWHAZ_Hs00852925_sH_FAM | 0.21031416 |
| PLSCR1 | h_PLSCR1_Hs00275514_m1_FAM | 0.21118584 |
| HSP90AA1 | h_HSP90AA1_Hs00743767_sH_FAM | 0.24323062 |
| COX6A1 | h_COX6A1_Hs01924685_g1_FAM | 0.2639524 |
| PSMA2 | h_PSMA2_Hs00746751_s1_FAM | 0.26592111 |
| AP1S2 | h_AP1S2_Hs00705223_s1_FAM | 0.30722181 |
| PIAS3 | h_PIAS3_Hs00180666_m1_FAM | 0.3256013 |
| CKS2 | h_CKS2_Hs01048812_g1_FAM | 0.3483249 |
| HMOX1 | h_HMOX1_Hs01110250_m1_FAM | 0.4343992 |
| PML | h_PML_Hs00971694_m1_FAM | 0.48460568 |
| ITGA5 | h_ITGA5_Hs01547673_m1_FAM | 0.53697884 |
| MAP2K4 | h_MAP2K4_Hs00387426_m1_FAM | 0.67638462 |
| ITGAL | h_ITGAL_Hs00158218_m1_FAM | 0.7189843 |
| CSK | h_CSK_Hs01062585_m1_FAM | 0.86646143 |
| RHOB | h_RHOB_Hs00269660_s1_FAM | 0.91603175 |

Additionally logistic regression and kNN classification (k=2, 3 and 5) were performed on the eight significant genes to increase the discriminatory performance compared to individual gene discrimination (Table 6). The three gene combination of MKNK1, TBX21 and TGFBR3 was found to be particularly discriminant. The best two gene combination identified from logistic regression using a forward variable selection is MKNK1 and TBX21.

**Table 6:** Results from the various multivariate models tested on subcombinations of the eight genes. MCC: Matthew's correlation coefficient. AUC: Area Under the Curve.

| Gene List | Cross Validation Method | Classification Algorithm | Sensitivity | Specificity | Error Rate | MCC | AUC |
|---|---|---|---|---|---|---|---|
| MKNK1, PRF1, TBX21, TGFBR3, IFNGR2, FYN, IL1B and CFLAR | Leave One Out | Logistic Regression | 33.333 | 83.871 | 24.324 | 0.162 | 0.30 6 |
| MKNK1, PRF1, TBX21, TGFBR3, IFNGR2, FYN, IL1B and CFLAR | 1/3 test set | 2-NN | 100 | 80 | 16.666 67 | 0.632 4555 | 0.9 |
| MKNK1, PRF1, TBX21, TGFBR3, IFNGR2, FYN, IL1B and CFLAR | 1/3 test set | 3-NN | 100 | 80 | 16.666 67 | 0.632 4555 | 0.9 |
| MKNK1, PRF1, TBX21, TGFBR3, IFNGR2, FYN, IL1B and CFLAR | 1/3 test set | 5-NN | 100 | 100 | 0 | 1 | 1 |
| MKNK1, TBX21 and TGFBR3 | Leave One Out | Logistic Regression | 66.667 | 90.323 | 13.514 | 0.536 | 0.63 4 |
| MKNK1, TBX21 and TGFBR3 | 1/3 test set | 2-NN | 100 | 80 | 16.666 67 | 0.632 4555 | 0.9 |
| MKNK1, TBX21 and TGFBR3 | 1/3 test set | 3-NN | 100 | 70 | 25 | 0.529 1503 | 0.85 |
| MKNK1, TBX21 and TGFBR3 | 1/3 test set | 5-NN | 50 | 80 | 25 | 0.258 1989 | 0.45 |
| MKNK1 and TBX21 | Leave One Out | Logistic Regression | 50.000 | 90.323 | 16.216 | 0.403 | 0.47 6 |
| MKNK1 and TBX21 | 1/3 test set | 2-NN | 100 | 80 | 16.666 67 | 0.632 4555 | 0.9 |
| MKNK1 and TBX21 | 1/3 test set | 3-NN | 100 | 70 | 25 | 0.529 1503 | 0.85 |
| MKNK1 and TBX21 | 1/3 test set | 5-NN | 100 | 70 | 25 | 0.529 1503 | 0.85 |

**EXAMPLE 3: Genes equivalent to the candidate 8* genes**

Genes rarely operate individually and thus genes whose expression correlate to other genes can easily be replaced to achieve similar discriminatory performances. To that effect we identified the genes that most correlate to the eight genes from our claim. Correlation analysis is first based on two genomewide datasets to ensure that most genes of the genome are evaluated: the 44 microarray chips of Julia *et al.* as well as the entire set of microarray chips of Bienkowska et al. (86 chips including other anti-TNFs). The 20 most positively correlated to each of the eight genes are displayed on Figures 1 and 2 respectively. They are also indicated in following Tables 7 to 8.

**Table 7:** List of genes that positively correlate to 7 candidate genes based on the data of Julia et al.

| MKNK1 | PRF1 | TAX21 | IFNGR2 | FYN | IL1B | CFLAR |
|---|---|---|---|---|---|---|
| SOD2 | GZMB | HDAC8 | ACSL1 | DDX56 | ADM | CASP9 |
| TMEM8 | GPR56 | CEP70 | HEXB | FLJ21438 | CEBPB | RBP7 |

(continued)

| MKNK1 | PRF1 | TAX21 | IFNGR2 | FYN | IL1B | CFLAR |
|---|---|---|---|---|---|---|
| AQP9 | ALKBH5 | TMEM161B | TM6SF1 | CD81 | AXUD1 | SNAP23 |
| ACSL1 | R3HDM2 | CPEB4 | FAM49A | NDUFS8 | GADD45B | MVP |
| NFKBIZ | RASSF1 | DPM3 | SLC37A3 | C9orf142 | TRIM21 | JARID1B |
| NLRP12 | SH2D2A | CCDC52 | ARHGAP9 | DDOST | GK | TLR4 |
| SRPK1 | SPON2 | KNCN | FAM65B | ULK3 | LIMK2 | RRAGD |
| FAM129A | HIP1 | POU5F1 | ALOX5AP | NUP210 | KCNJ2 | SERPINB1 |
| NTNG2 | KIR3DL1 | PRKD3 | ZFP36L1 | NOL14 | RNF149 | HIST1H2BC |
| DYSF | GPR114 | LMTK2 | SRPK1 | ADA | PIK3AP1 | |
| CENTD2 | GNLY | FLJ22374 | FKBP1A | WDR54 | FCGR2A | RPGRIP1 |
| S100A8 | NKG7 | AADAC | VNN2 | FAM62A | ACSL1 | OSBPL2 |
| MTMR3 | TBXAS1 | GSPT2 | SIRPA | POLG | CEACAM1 | F11R |
| PROK2 | FGFBP2 | CNTN2 | ATP6V1A | PARP1 | RERE | MAFF |
| KCNJ15 | ACLY | CAMK1 | PIK3CD | RUNX3 | STX11 | TTRAP |
| RASSF2 | GZMH | C20orf12 | TLR4 | B3GALT6 | RBM23 | IL13RA1 |
| DGAT2 | LMO4 | MLANA | NUMB | KIAA1310 | PCDHGB5 | CSF2RA |
| CSF2RA | TCP1 | AZGP1 | SLC26A8 | RPUSD2 | NAMPT | IL1R2 |
| C19orf59 | IL2RB | RNF185 | FBXL5 | CARD11 | AQP9 | FRAT1 |
| IL1R2 | CDC42SE1 | GMEB1 | HOXD8 | SMARCD1 | TNFSF13B | METTL2A |

**Table 8:** List of genes that positively correlate to 8 candidate genes based on the data of Bienkowska et al.

| MKNK1 | PRF1 | TBX21 | IFNGR2 | FYN | IL1B | CFLAR | TGFBR3 |
|---|---|---|---|---|---|---|---|
| NPL | GPR56 | EOMES | RTN3 | YEATS2 | LIMK2 | SMCHD1 | C1orf21 |
| tcag7.90 7 | SPON2 | SOX13 | PISD | PABPC1 | ADM | PHF20L1 | XCL2 |
| MAP3K2 | NKG7 | C1orf21 | LOC2844 54 | CALM1 | LIMK2 | SPAG9 | TRGV9 |
| CDC42E P3 | GNLY | TTC38 | NCLN | GIMAP1 | DHRS13 | MGEA5 | LGR6 |
| REM2 | PRF1 | KLRD1 | PTEN | CBX6 | FLJ3905 1 | FAM126 B | TARP |
| SAMD4B | CTA-221 G9.4 | GZMH | OAZ2 | HNRNPD | PPIF | LOC7300 51 | GPR68 |
| YPEL3 | KIR3DL3 /// LILRP2 /// KIR2DL3 | SOX13 | NDEL1 | PURA | GADD45 B | FYB | TRGC2 |
| NUMB | FGFBP2 | TARP | BST1 | SLC25A3 | GADD45 B | PHF20L1 | C1orf21 |
| C15orf28 | IL2RB | NKG7 | CKAP4 | MAPRE2 | FLJ3905 1 | CBL | SIGLEC P3 |
| UIMC1 | CCL5 | LOC1001 33241/// OSBPL5 | REPS2 | SET | GLT1D1 | SSH2 | PYHIN1 |

(continued)

| MKNK1 | PRF1 | TBX21 | IFNGR2 | FYN | IL1B | CFLAR | TGFBR3 |
|---|---|---|---|---|---|---|---|
| U2AF1 | TRGV9 | MYO6 | DIRC2 | SYNCRI P | LOC1001 29443 /// KIAA004 0 | APAF1 | TARP |
| YIPF1 | RUNX3 | TARP | PREX1 | UCP2 | C16orf57 | EIF2C2 | KLRK1 |
| MAPK1 | TARP | GFI1 | NADK | NVL | STAT5B | NUMB | HOPX |
| QKI | GZMH | GPR56 | CFLAR | DAZAP1 | BCL3 | FAM120 A | PPP2R2 B |
| PBX2 | GPR68 | CTA-221 G9.4 | PANX2 | UHMK1 | ALPL | SORL1 | FGFBP2 |
| LOC2844 54 | CX3CR1 | TRGV9 | TMCC3 | CBLB | MRPL44 | LOC6471 90 | PDGFD |
| CKAP4 | TARP | CHST12 | SLC25A4 4 | DYRK2 | GADD45 B | FNIP1 | GZMA |
| PELI1 | CECR1 | OSBPL5 | VNN1 | ATP5G3 | JUNB | MLL2 | NKG7 |
| USP32 | CCL5 | TRGC2 | DHRS7 | CBX5 | LOC6464 70 | MAP3K3 | EOMES |
| MLX | TRGC2 | S1PR5 | SLC25A4 4 | AFF1 | PIM3 | DKFZP4 34B2016 | GZMH |
| | GZMB | C1orf21 | CFLAR | EZR | | | XCL1 |
| | | JAKMIP1 | | | | | CCR5 |

Correlation analysis is then further applied to the qPCR data to illustrate the impact of replacing genes by correlated genes on the discriminatory performance of the signature Tables 9, 10 and Figure4).

Table 9 below further lists genes whose expression levels, as measured by RT-qPCR in whole blood, correlate at more than 0.6 (Pearson's correlation coefficient superior to 0.6, p-val<0.0001) to 10 candidate genes. Those results were generated from the 90 samples described in Example 4.

**Table 9.** List of genes that correlate at more than 0.6 to the 10 candidate genes in the header (in bold). The correlation coefficient is given in brackets. When a gene that correlates at more than 0.6 (p-val<0.0001) to one of the 10 candidate genes is itself a candidate gene, it is indicated by the sign *.

| **MKNK1** | **PRF1** | **TBX21** | **IFNGR2** | **FYN** | **IL1B** | **CFLAR** | **MAPK14** | **GNLY** | **TGFBR3** |
|---|---|---|---|---|---|---|---|---|---|
| MAPK14 (0.65)* | TBX21 (0.86)* | CCR5 (0.7) | IL8RB (0.78) | ELK4 (0.74) | IL1RN (0.64) | IL8RB (0.75) | IL8RB (0.78) | TBX21 (0.75)* | ABCB1 (0.63) |
| CD11c (0.62) | CCR5 (0.61) | TGFBR3 (0.89)* | IL6R (0.65) | ABCB1 (0.61) | TLR5 (0.63) | MAPK14 (0.67)* | MMP9 (0.67) | TGFBR3 (0.75)* | TBX21 (0.89)* |
| IFNGR2 (0.61)* | TGFBR3 (0.73)* | CCL4 (0.72) | IL1RN (0.62) | CCR5 (0.62) | | HCK0021 10 (0.61) | IL6R (0.66) | CCL4 (0.61) | CCR5 (0.74) |
| IFITM2 (0.61) | CCL4 (0.66) | IL2RB (0.69) | MAPK14 (0.78)* | TGFBR3 (0.71)* | | TGFBR2 (0.72) | IL1RN (0.62) | IL2RB (0.61) | CCL4 (0.7) |
| | IL2RB (0.75) | GNLY-probe 1 (0.73)* | HCK0021 10 (0.65) | IL2RB (0.64) | | TNFAIP9 (0.71) | HCK0021 10 (0.61) | PRF1 (0.68)* | IL2RB (0.68) |
| | GNLY-probe 1 (0.64)* | GNLY-probe 2 (0.75)* | TGFBR2 (0.62) | KLRK1 (0.66) | | IFNGR2 (0.67)* | CD16 (0.69) | | GNLY-probe 1 (0.73)* |
| | GNLY-probe 2 (0.68)* | PRF1 (0.86)* | CD16 (0.67) | | | IL1R1 (0.65) | CD11c (0.72) | | ENPP4 (0.63) |
| | | | CD11c (0.63) | | | IFITM2 (0.72) | SLC2A3 (0.67) | | KLRK1 (0.71) |
| | | | TIMP2 (0.76) | | | DECR1 (0.69) | TIMP2 (0.82) | | FYN (0.71)* |
| | | | MKNK1 (0.61)* | | | | MKNK1 (0.65)* | | GNLY-probe 2 (0.75)* |
| | | | TNFAIP9 (0.71) | | | | HSPA1A (0.62) | | PRF1 (0.73)* |
| | | | CFLAR (0.67)* | | | | TNFAIP9 (0.74) | | |
| | | | CASC3 (0.64) | | | | CFLAR (0.67)* | | |
| | | | IFITM2 (0.84) | | | | IFNGR2 (0.78)* | | |
| | | | | | | | TLR5 0.67) | | |
| | | | | | | | CASC3 (0.63) | | |

(continued)

| MKNK1 | PRF1 | TBX21 | IFNGR2 | FYN | IL1B | CFLAR | MAPK14 | GNLY | TGFBR3 |
|---|---|---|---|---|---|---|---|---|---|
| | | | | | | | IFITM2 (0.8) | | |

To illustrate the equivalence of the performance between a signature based on 8 genes and a signature based on 8 genes with one or the other gene replaced by an equivalent gene (such as one from the list in Table 9), we have replaced PRF1 by IL2RB in the signature, or CFLAR by IFITM2. The resulting performances are only slightly lower as shown in **Table 10** below, thus showing that replacing an original gene in a claimed gene combination by an equivalent gene as disclosed in the application still permits a reliable prognosis.

**Table 10.** PPV, NPV, sensitivity, specificity and error rate for 3 gene signatures.

| | PPV | NPV | Sensitivity | Specificity | ER |
|---|---|---|---|---|---|
| 8 original genes (MKNK1, PRF1, TBX21, TGFBR3, IFNGR2, FYN, IL1B and CFLAR) | 0.486 | 0.939 | 0.857 | 0.708 | 0.244 |
| 8 genes with PRF1 replaced by IL2RB (MKNK1, IL2RB, TBX21, TGFBR3, IFNGR2, FYN, IL1B and CFLAR) | 0.545 | 0.846 | 0.545 | 0.846 | 0.230 |
| 8 genes with CFLAR replaced by IFITM2 (MKNK1, PRF1, TBX21, TGFBR3, IFNGR2, FYN, IL1B and IFITM2) | 0.469 | 0.875 | 0.682 | 0.742 | 0.273 |

The possibility to replace genes by equivalent ones based on their correlation coefficient is further illustrated in Figure 4, which shows that replacing genes genes by equivalent ones with a correlation coefficient of at least 0.9 still permits prediction (prognosis) with reasonable sensitivity.

**EXAMPLE 4: Further validation of candidate signatures and further signatures in an increased number of patients**

In Example 2, the signatures of responsiveness to Infliximab treatment in RA-patients have been tested in samples of 40 RA patients.

Similarly, a signature of responsiveness Infliximab treatment in RA-patients consisting of MAPK14 and GNLY disclosed in PCT application WO2011/117366, consisting of MAPK14 and GNLY, had been tested in this PCT application in the same samples of 40 RA patients.

To further validate these signatures, they have been further tested in a group of 90 RA patients treated with Infliximab corresponding to the previously tested RA patients and 50 additional RA patients. In addition, two further signatures corresponding to the 8 genes signature (MKNK1, PRF1, TBX21, TGFBR3, IFNGR2, FYN, IL1B and CFLAR) described above in Example 2, to which are added two further genes ((CD14 and TGFBR2) or (MAPK14 and GNLY)), have been further tested in the group of 90 patients.

**PATIENTS AND METHODS**

**Patients**

Among the 90 tested patients, 40 are the same as those already tested in Example 2, additional 3 samples were included from the same source. Thus 42 samples used for the qCPR represent a subset of the original 43 samples used in the statistical analysis of microarray analysis in the study of Julia *et al.* One outlier in microarray data (sample 44) that was excluded from microarray analysis was not an outlier in the qPCR analysis and hence contributes as an independent sample. Therefore 43 (36 responders, 7 non responders) RNA samples from Julia *et al.* were used in the qPCR analysis. Additional 57 samples from RA patients meeting our inclusion criteria were obtained from three additional sites: 24 (14 responders, 10 non responders) from Japan, 14 (10 responders, 4 non responders) from the UK and 9 (8 responders, 1 non responders) from France. The delatadeltaCT method was used to measure gene expression levels using the same reference genes as in example 2.

Patient characteristics for each source are described in Table 11.

| Clinical Variable | Clinical Response | UK | Spain | France | Japan | Overall |
|---|---|---|---|---|---|---|
| DAS28 at baseline | Non Responding | 6,60 | 5,76 | 3,71 | 5,11 | 5,52 |
| | Responding | 6,33 | 6,00 | 5,21 | 5,35 | 5,82 |
| DAS28 at week 14 | Non Responding | 6,10 | 5,44 | 3,41 | 5,25 | 5,38 |
| | Responding | 3,07 | 3,79 | 3,83 | 2,87 | 3,50 |
| Delta DAS28 | Non Responding | 0,50 | 0,31 | 0,30 | -0,15 | 0,14 |
| | Responding | 3,26 | 2,21 | 1,38 | 2,48 | 2,32 |
| Age at Initiation | Non Responding | 64,25 | 57,71 | 44,00 | 59,10 | 58,91 |
| | Responding | 52,90 | 52,29 | 55,75 | 56,00 | 53,57 |
| Disease duration | Non Responding | NA | NA | 9,00 | 8,00 | 8,09 |
| | Responding | NA | NA | 6,71 | 10,51 | 9,24 |
| Weight | Non Responding | 64,00 | NA | NA | NA | 64,00 |
| | Responding | 75,90 | NA | NA | NA | 75,90 |
| ESR Baseline | Non Responding | 37,75 | 38,29 | 15,00 | 54,30 | 44,41 |
| | Responding | 34,50 | 40,67 | 35,67 | 53,14 | 41,93 |

**Table 11**: Patient characteristics by response status and by contributing site.

**Methods**

Table 12 below provides the Gene Symbol, the Gene Title, the Gene ID (Notation from Entrez Gene NCBI database, updated on 8-May-2011), and the reference mRNA sequence(s) (Entrez Gene NCBI database, updated on 8-May-2011) for all human genes present in one or more of the tested signatures:

**Table 12.** Gene Symbol, Gene Title and Gene ID (Notation from Entrez Gene NCBI database, updated on December 2010) for genes CD14, TGFBR2, MAPK14 and GNLY

| Gene Symbol | Gene Title | Gene ID | Reference mRNA sequence(s) |
|---|---|---|---|
| MKNK1 | MAP kinase interacting serine/threonine kinase 1 | 8569 | NM_001135553.1 (SEQ ID NO:1)<br>NM_003684.4 (SEQ ID NO:2)<br>NM_198973.2 (SEQ ID NO:3) |
| PRF1 | perforin 1 (pore forming protein) | 5551 | NM_001083116.1 (SEQ ID NO4)<br>NM_005041.4 (SEQ ID NO:5) |
| TBX21 | T-box 21 | 30009 | NM_013351.1 (SEQ ID NO:6) |
| TGFBR3 | transforming growth factor, beta receptor III | 7049 | NMI_001195683.1 (SEQ ID NO:7)<br>NMI_001195684.1 (SEQ ID NO:8)<br>NM_003243.4 (SEQ ID NO:9) |

(continued)

| Gene Symbol | Gene Title | Gene ID | Reference mRNA sequence(s) |
|---|---|---|---|
| IFNGR2 | interferon gamma receptor 2 (interferon gamma transducer 1) | 3460 | NM_005534.3 (SEQ ID NO:10) |
| FYN | FYN oncogene related to SRC, FGR, YES | 2534 | NM_002037.3 (SEQ ID NO:11) NM_153047.1 (SEQ ID NO:12) NM_153048.1 (SEQ ID NO:13) |
| IL1B | interleukin 1, beta | 3553 | NM_000576.2 (SEQ ID NO:14) |
| CFLAR | CASP8 and FADD-like apoptosis regulator | 8837 | NMI_001127183.2 (SEQ ID NO:15) NM_001127184.2 (SEQ ID NO:16) NM_001202515.1 (SEQ ID NO:17) NM_001202516.1 (SEQ ID NO:18) NM_001202517.1 (SEQ ID NO:19) NM_001202518.1 (SEQ ID NO:20) NM_001202519.1 (SEQ ID NO:21) NM_003879.5 (SEQ ID NO:22) |
| CD14 | CD14 molecule | 929 | NM_000591.3 (SEQ ID NO:23) NM_001040021.2(SEQ ID NO:24) NM_001174104.1(SEQ ID NO:25) NM_001174105.1(SEQ ID NO26:) |
| TGFBR2 | transforming growth factor, beta receptor II (70/80kDa) | 7048 | NM_001024847.2 (SEQ ID NO:27) NM_003242.5 (SEQ ID NO:28) |
| MAPK14 | mitogen-activated protein kinase 14 | 1432 | NM_001315.2 (SEQ ID NO:29) NM_139012.2 (SEQ ID NO:30) NM_139013.2 (SEQ ID NO:31) NM_139014.2 (SEQ ID NO:32) |

(continued)

| Gene Symbol | Gene Title | Gene ID | Reference mRNA sequence(s) |
|---|---|---|---|
| GNLY | granulysin | 10578 | NM_006433.3 (SEQ ID NO:33)<br>NM_012483.2(SEQ ID NO:34) |

qPCR experiments have been performed as described in Example 2.

**Cross-validation methods**

To robustly estimate the performance of the 8 gene signature, we applied the signature of the 8 genes developed in example 2 to the 90 samples. In a first analysis 43 samples were used for the learning phase and the remaining 47 as an independent set. In a second approach we aimed at improving on the existing signature by adding additional genes to improve the discriminatory performance. For this second approach we used leave one out cross validation on the entire 90 samples.

**RESULTS AND DISCUSSION**

The prognosis of a non-responding condition (to Infliximab) was analyzed. As a result, a non-responding condition or test outcome is considered a positive result, while a responding condition or test outcome is considered a negative result. True and false positive results, NPV, PPV, specificity sensitivity, and error rate are defined and calculated as follows:

| | | **Condition (non-responding)** | |
|---|---|---|---|
| | | ***Positive*** | ***Negative*** |
| **Test outcome (non-responding)** | ***Positive*** | True Positive (TP) | False positive (FP) |
| | ***Negative*** | False negative (FN) | True negative (TN) |

PPV = TP / (TP+FP)
NPV = TN / (TN+FN)
Specificity = TN / (TN+FP)
Sensitivity = TP / (TP+FN)
Error rate = (FP+FN)/ Total number of patients

The main clinical objective of a signature is not to put patients under inefficacious treatment, therefore a signature should demonstrate a low level of False Negative results.

Results obtained on at least a subgroup of the 90 RA patients are provided below for:

- the 8 genes signature (MKNK1, PRF1, TBX21, TGFBR3, IFNGR2, FYN, IL1B and CFLAR) already tested in Example 2 on 40 RA patients (see Table 13),
- the 2 genes signature (MAPK14 and GNLY) previously tested in PCT application WO2011/117366 40 RA patients (see Table 14),
- the 10 genes signature (MKNK1, PRF1, TBX21, TGFBR3, IFNGR2, FYN, IL1B, CFLAR, CD14 and TGFBR2), comprising the 8 genes tested in Example 2 and two additional genes (CD14 and TGFBR2) (see Table 15),
- the 10 genes signature (MKNK1, PRF1, TBX21, TGFBR3, IFNGR2, FYN, IL1B, CFLAR, MAPK14 and GNLY), combining the 8 genes already tested in Example 2 on 40 RA patients and the two genes already tested in PCT application WO2011/117366 the same 40 RA patients (see Table 16),
- The 1 gene signature MKNK1 (see Table 17),
- The 2 genes signature (MKNK1, GNLY) (see Table 17),
- The 3 genes signature (MKNK1, GNLY, AD11) (see Table 17),
- The 4 genes signature (MKNK1, GNLY, AD11, IL1B) (see Table 17), and
- The 5 genes signature (MKNK1, GNLY, ADI1, IL1B, IL1R1) (see Table 17).

Samples for which there was missing data based on invalid qPCR results were excluded.

**Table 13.** Results obtained with the 8 genes signature (MKNK1, PRF1, TBX21, TGFBR3, IFNGR2, FYN, IL1B and CFLAR) in 86 patients

| | | **Condition (non-responding)** | | |
|---|---|---|---|---|
| | | Positive | Negative | |
| **Test outcome (non-responding)** | Positive | 18 | 19 | 0.486 PPV |
| | Negative | 3 | 46 | 0.939 NPV |
| | | 0.857 Sens | 0.708 Spe | |
| Error rate = 0.256<br>Positive = non-responding to Infliximab treatment<br>Negative = responding to Infliximab treatment<br>PPV = Positive Predictive value<br>NPV = Negative Predictive value<br>Sens = sensitivity<br>Spe = specicifity | | | | |

**Table 14.** Results obtained with the 2 genes signature (MAPK14, GNLY) in 90 patients

| | | **Condition (non-responding)** | | |
|---|---|---|---|---|
| | | Positive | Negative | |
| **Test outcome (non-responding)** | Positive | 10 | 3 | 0.769 PPV |
| | Negative | 12 | 65 | 0.844 NPV |
| | | 0.455 Sens | 0.956 Spe | |
| Error rate = 0.167<br>Positive = non-responding to Infliximab treatment<br>Negative = responding to Infliximab treatment<br>PPV = Positive Predictive value<br>NPV = Negative Predictive value<br>Sens = sensitivity<br>Spe = specicifity | | | | |

**Table 15.** Results obtained with the first 10 genes signature (MKNK1, PRF1, TBX21, TGFBR3, IFNGR2, FYN, IL1B, CFLAR, CD14 and TGFBR2) in 75 patients

| | | **Condition (non-responding)** | | |
|---|---|---|---|---|
| | | Positive | Negative | |
| **Test outcome (non-responding)** | Positive | 10 | 5 | 0.667 PPV |
| | Negative | 8 | 52 | 0.867 NPV |
| | | 0.556 Sens | 0.912 Spe | |
| Error rate = 0.173<br>Positive = non-responding to Infliximab treatment<br>Negative = responding to Infliximab treatment<br>PPV = Positive Predictive value<br>NPV = Negative Predictive value<br>Sens = sensitivity<br>Spe = specicifity | | | | |

**Table 16.** Results obtained with the second 10 genes signature (MKNK1, PRF1, TBX21, TGFBR3, IFNGR2, FYN, IL1B, CFLAR, MAPK14 and GNLY) in 60 patients. In this particular case a three level score was used with samples classifying as intermediate risk of response, not being represented here.

| | | **Condition (non-responding)** | | |
|---|---|---|---|---|
| | | Positive | Negative | |
| **Test outcome (non-responding)** | Positive | 9 | 2 | 0.818 PPV |
| | Negative | 3 | 46 | 0.939 NPV |
| | | 0.750 Sens | 0.958 Spe | |
| Error rate = 0.083<br>Positive = non-responding to Infliximab treatment<br>Negative = responding to Infliximab treatment<br>PPV = Positive Predictive value<br>NPV = Negative Predictive value<br>Sens = sensitivity<br>Spe = specicifity | | | | |

The values of PPV, NPV, sensitivity and specificity of all four tested signatures are summarized in Table 17 below:

**Table 17.** Summary of PPV, NPV, specificity, sensitivity and error rate of prognosis methods of a non-responding condition to Infliximab

| | Signature | PPV | NPV | Sensitivity | Specificity | Error rate |
|---|---|---|---|---|---|---|
| 1 | 8 genes (MKNK1, PRF1, TBX21, TGFBR3, IFNGR2, FYN, IL1B and CFLAR) | 0.486 | 0.939 | 0.857 | 0.708 | 0.256 |
| 2 | 2 genes (MAPK14, GNLY) | 0.769 | 0.844 | 0.455 | 0.956 | 0.167 |
| 3 | 10 genes (MKNK1, PRF1, TBX21, TGFBR3, IFNGR2, FYN, IL1B, CFLAR, CD14 and TGFBR2) | 0.667 | 0.867 | 0.556 | 0.912 | 0.173 |
| 4 | 10 genes (MKNK1, PRF1, TBX21, TGFBR3, IFNGR2, FYN, IL1B, CFLAR, MAPK14 and GNLY) | 0.818 | 0.939 | 0.75 | 0.958 | 0.083 |
| 5 | 1 gene (MKNK1) | 0.57 | 0.78 | 0.18 | 0.96 | 0.23 |
| 6 | 2 genes (MKNK1, GNLY) | 0.65 | 0.85 | 0.5 | 0.91 | 0.19 |
| 8 | 3 genes (MKNK1, GNLY, ADI1) | 0.79 | 0.86 | 0.5 | 0.96 | 0.16 |
| 7 | 4 genes (MKNK1, GNLY, ADI1, IL1B) | 0.81 | 0.88 | 0.59 | 0.96 | 0.13 |
| 9 | 5 genes (MKNK1, GNLY, ADI1, IL1B, IL1R1) | 0.82 | 0.89 | 0.63 | 0.96 | 0.12 |

Data summarized in Table 17 show that the first four tested signatures have high NPV value for prognosis of non-responding condition, meaning that, in each case, a high proportion of patients with a negative test (i.e. prognosed as responding to Infliximab) actually do respond to the Infliximab treatment, so that, based on these prognosis methods of non-responding condition, there will be very few patients prognosed as responding that will fail to respond to the treatment. Thus, based on these prognosis methods, Infliximab will be administered almost only to patients that will actually respond to the treatment. The first four tested signatures also have an acceptable or high specificity for prognosis of non-responding condition, meaning that a high proportion of patients that would actually respond to Infliximab treatment are correctly prognosed as responders using the signatures. Thus, based on the above prognosis methods of non-responding condition according to the invention, most patients that would benefit from Infliximab treatment will actually receive this treatment. The two signatures of greatest clinical interest are the initial eight gene signature as well as the combined 10 genes signature that include MAPK14 and GNLY where the sensitivity is higher than in the other two. The first four tested signatures are thus helpful prognosis tools for identifying RA patients that will or not respond to an Infliximab treatment.

In addition, data obtained for the 8 genes signature (MKNK1, PRF1, TBX21, TGFBR3, IFNGR2, FYN, IL1B and CFLAR) and the two 10 genes signatures (MKNK1, PRF1, TBX21, TGFBR3, IFNGR2, FYN, IL1B, CFLAR, CD14 and TGFBR2 / MKNK1, PRF1, TBX21, TGFBR3, IFNGR2, FYN, IL1B, CFLAR, MAPK14 and GNLY), which both include the 8 genes signature, confirm that the addition of one or more genes to this 8 genes signature does not affect its ability to correctly prognose RA patients as responders or non responders to Infliximab treatment. In contrast, the 10 genes signature combining the 8 genes signature of Example 2 and the 2 genes signature of PCT application WO2011/117366shows particularly high NPV and specificity values (the highest of all four signatures), while keeping quite high PPV (the highest of all four signatures) and sensitivity (second best of all four signatures) values. This 10 genes signature (MKNK1, PRF1, TBX21, TGFBR3, IFNGR2, FYN, IL1B, CFLAR, MAPK14 and GNLY) appears to be a particularly good prognosis tool of response and non response to Infliximab treatment in RA patients. All the above methods may also be used for prognosis of non response to other TBA treatments, as well as other CyTD treatments.

In addition, Table 17 also shows that MKNK1 alone permits classification of patients with an error rate of only 0.23. In particular, a prognosis based on MKNK1 is highly specific and also has a high NPV value. Moreover, the addition of at least one other gene significantly improves all values (PPV, NPV, sensitivity, specificity, error rate), and a NPV value of at least 0.85 is obtained when at least one gene is added to MKNK1. These results confirm that MKNK1 is a key gene for the prediction of response or non-response to Infliximab and that gene signatures based on MKNK1 and preferably at least one more gene may result in reliable prediction.

In summary, several signatures (or genes combinations) useful for prognosis of non-responsiveness to CyTD treatment (in particular Infliximab treatment) in subjects suffering from an inflammatory disease (in particular rheumatoid arthritis, RA) have been identified, three of which comprising the 8 following genes: MKNK1, PRF1, TBX21, TGFBR3, IFNGR2, FYN, IL1B and CFLAR. The best signature identified so far for prognosis of non-responsiveness to CyTD treatment (in particular Infliximab treatment) in subjects suffering from an inflammatory disease (in particular RA) comprises or consists of genes MKNK1, PRF1, TBX21, TGFBR3, IFNGR2, FYN, IL1B, CFLAR, MAPK14 and GNLY.

**EXAMPLE 5: Further validation of a highly discriminant 11 gene signature using a linear model.**

Example of a highly discriminant algorithm is provided, it can be applied at baseline (ie prior to treatment administration) to predict whether a patient will be a responder or a non responder to Infliximab. In this example the biological measures are based on gene expression profiles of 11 genes as measured by relative quantitative PCR on RNA extracted from peripheral blood. Those measures are then combined in a linear model to provide a score, a positive score means that the patient is predicted to be a responder. Such algorithm can easily be implemented in existing software or coded specifically.

**PATIENTS AND METHODS**

**Patients**

In this example 65 Caucasian RA patients were analysed. All 65 patients were eligible for first line anti-TNF therapy, as such they were naive of biologies and refractory to classical DMARDS. The 65 patients have already been described in example 4 (the samples from France, Spain and UK).

**Methods**

The delatadeltaCT method was used to measure gene expression levels using the same reference genes and procedures as in examples 2 and 4. Previous Table 12 provides the Gene Symbol, the Gene Title, the Gene ID (Notation from Entrez Gene NCBI database, updated on 8-May-2011), and the reference mRNA sequence(s) (Entrez Gene NCBI database, updated on 8-May-2011) of 12 genes, 11 of which the Applied probes used in this example map to. Table 18 below provides the Applied Biosystems probe identifiers.

Table 18: Probe identifiers for an 11 gene signature

| Gene Symbol | Probeid | Probe sequence |
|---|---|---|
| CD14 | h_CD14_Hs02621496_ss1_FAM | GGAATGGAGACGCCCACAGGCGTGT (SEQ ID NO:35) |

(continued)

| Gene Symbol | Probeid | Probe sequence |
|---|---|---|
| CFLAR | h_CFLAR_Hs01116280_m1_FAM | TAAGCAAGGAGAAGAGTTTCTTGGA (SEQ ID NO:35) |
| IFNGR2 | h_IFNGR2_Hs00194264_m1_FAM | CGGAATGTGACTGTCGGGCCTCCAG (SEQ ID NO:37) |
| IL1B | h_IL1B_Hs01555410_m1_FAM | GATGAAGTGCTCCTTCCAGGACCTG (SEQ ID NO:38) |
| GNLY | h_GNLY_Hs01120727_m1_FAM | TACCTTCTACAGGTCCCCTCTGAGC (SEQ ID NO:39) |
| MAPK14 | h_MAPK14_315_Hs01051153_m1_FAM | TGTTTCCTGGTACAGACCATATTAA (SEQ ID NO:40) |
| MKNK1 | h_MKNK1_Hs00374376_m1_FAM | CCACCCCATGTGGCTCTGCAGAATA (SEQ ID NO:41) |
| PRF1 | h_PRF_Hs00169473_m1_FAM | TGCCGCTTCTACAGTTTCCATGTGG (SEQ ID NO:42) |
| TBX21 | h_TBX21_Hs00894392_m1_FAM | ACAATGTGACCCAGATGATTGTGCT (SEQ ID NO:43) |
| TGFBR2 | h_TGFBR2_Hs00234253_m1_FAM | TGGCTCAACCACCAGGGCATCCAGA (SEQ ID NO:44) |
| TGFBR3 | h_TGFBR3_Hs01114253_m1_FAM | CACTGCAGGTCCAGAGCCTGGTGCA (SEQ ID NO:45) |

**RESULTS AND DISCUSSION**

$$\text{Score} = -2608.15 - 588.10 * ddCT_{MKNK1} + 218.42 * ddCT_{TBX21} + 211.81 * ddCT_{TGFBR3} - 99.48 * ddCT_{PRF1} + 325.63 * ddCT_{IFNGR2} - 247.78 * ddCT_{IL1B} + 272.59 * ddCT_{CFLAR} - 896.87 * ddCT_{TGFBR2} - 439.47 * ddCT_{CD14} + 69.04 * ddCT_{GNLY} + 480.50 * ddCT_{MAPK14}$$

Equation 1: This equation provides a mean to estimate the probability that a patient is a responder or non responder to infliximab.

The coefficients in Equation 1 were estimated based on a training of the 65 samples. The score resulting from the linear model described in equation 1 allows to perfectly discriminate all responders from non responders as can be seen in Figure 5. All patients above the horizontal line at zero are clinical responders to infliximab and predicted as such by a positive score, all non responders are below the line and predicted as such (negative score).

**REFERENCES**

1. Lee DM, Weinblatt ME. Rheumatoid arthritis. Lancet. 2001 Sep 15;358(9285):903-11.
2. Choy EH, Panayi GS. Cytokine pathways and joint inflammation in rheumatoid arthritis. N Engl J Med. 2001 Mar 22;344(12):907-16.

3. Kooloos WM, de Jong DJ, Huizinga TW, Guchelaar HJ. Potential role of pharmacogenetics in anti-TNF treatment of rheumatoid arthritis and Crohn's disease. Drug Discovery Today. 2007;12(3-4):125-31.

4. Isaacs JD. Antibody engineering to develop new antirheumatic therapies. Arthritis Res Ther. 2009;11(3):225.

5. Hetland ML, Christensen IJ, Tarp U, Dreyer L, Hansen A, Hansen IT, et al. Direct comparison of treatment responses, remission rates, and drug adherence in patients with rheumatoid arthritis treated with adalimumab, etanercept, or infliximab: results from eight years of surveillance of clinical practice in the nationwide Danish DANBIO registry. Arthritis Rheum. 2010 Jan;62(1):22-32.

6. Lequerre T, Gauthier-Jauneau AC, Bansard C, Derambure C, Hiron M, Vittecoq O, et al. Gene profiling in white blood cells predicts infliximab responsiveness in rheumatoid arthritis. Arthritis Res Ther. 2006;8(4):R105.

7. Sekiguchi N, Kawauchi S, Furuya T, Inaba N, Matsuda K, Ando S, et al. Messenger ribonucleic acid expression profile in peripheral blood cells from RA patients following treatment with an anti-TNF-alpha monoclonal antibody, infliximab. Rheumatology (Oxford). 2008 Jun;47(6):780-8.

8. Julia A, Erra A, Palacio C, Tomas C, Sans X, Barcelo P, et al. An eight-gene blood expression profile predicts the response to infliximab in rheumatoid arthritis. PLoS One. 2009;4(10):e7556.

9. Bienkowska JR, Dalgin GS, Batliwalla F, Allaire N, Roubenoff R, Gregersen PK, et al. Convergent Random Forest predictor: methodology for predicting drug response from genome-scale data applied to anti-TNF response. Genomics. 2009 Dec;94(6):423-32.

10. Ramasamy A, Mondry A, Holmes CC, Altman DG. Key issues in conducting a meta-analysis of gene expression microarray datasets. PLoS Med. 2008 Sep 30;5(9):e184.

11. Arnett FC, Edworthy SM, Bloch DA, McShane DJ, Fries JF, Cooper NS, et al. The American Rheumatism Association 1987 revised criteria for the classification of rheumatoid arthritis. Arthritis Rheum. 1988 Mar;31(3):315-24.

12. Parmigiani G, Garrett-Mayer ES, Anbazhagan R, Gabrielson E. A cross-study comparison of gene expression studies for the molecular classification of lung cancer. Clin Cancer Res. 2004 May 1;10(9):2922-7.

13. Barton A, Thomson W, Ke X, Eyre S, Hinks A, Bowes J, et al. Rheumatoid arthritis susceptibility loci at chromosomes 10p15, 12q13 and 22q13. Nat Genet. 2008 Oct;40(10):1156-9.

14. Goronzy JJ, Weyand CM. Developments in the scientific understanding of rheumatoid arthritis. Arthritis Res Ther. 2009;11(5):249.

15. Lorenz E, Muhlebach MS, Tessier PA, Alexis NE, Duncan Hite R, Seeds MC, et al. Different expression ratio of S100A8/A9 and S100A12 in acute and chronic lung diseases. Respir Med. 2008 Apr;102(4):567-73.

16. Cheng P, Corzo CA, Luetteke N, Yu B, Nagaraj S, Bui MM, et al. Inhibition of dendritic cell differentiation and accumulation of myeloid-derived suppressor cells in cancer is regulated by S100A9 protein. J Exp Med. 2008 Sep 29;205(10):2235-49.

17. Lim SY, Raftery M, Goyette J, Hsu K, Geczy CL. Oxidative modifications of S100 proteins: functional regulation by redox. J Leukoc Biol. 2009 86(3): 577-87.

18. Simard JC, Girard D, Tessier PA. Induction of neutrophil degranulation by S100A9 via a MAPK-dependent mechanism. J Leukoc Biol. 2010 Jan 26.

19. Chen YS, Yan W, Geczy CL, Brown MA, Thomas R. Serum levels of soluble receptor for advanced glycation end products and of S100 proteins are associated with inflammatory, autoantibody, and classical risk markers of joint and vascular damage in rheumatoid arthritis. Arthritis Res Ther. 2009;11 (2):R39.

20. Groh V, Bruhl A, El-Gabalawy H, Nelson JL, Spies T. Stimulation of T cell autoreactivity by anomalous expression of NKG2D and its MIC ligands in rheumatoid arthritis. Proc Natl Acad Sci USA. 2003 Aug 5;100(16):9452-7.

21. Paul R, Obermaier B, Van Ziffle J, Angele B, Pfister HW, Lowell CA, et al. Myeloid Src kinases regulate phagocytosis and oxidative burst in pneumococcal meningitis by activating NADPH oxidase. J Leukoc Biol. 2008 Oct;84(4):1141-50.

22. Fumagalli L, Zhang H, Baruzzi A, Lowell CA, Berton G. The Src family kinases Hck and Fgr regulate neutrophil responses to N-formyl-methionyl-leucyl-phenylalanine. J Immunol. 2007 Mar 15;178(6):3874-85.

23. Mocsai A, Ligeti E, Lowell CA, Berton G. Adhesion-dependent degranulation of neutrophils requires the Src family kinases Fgr and Hck. J Immunol. 1999 Jan 15;162(2):1120-6.

24. Bosco MC, Curiel RE, Zea AH, Malabarba MG, Ortaldo JR, Espinoza-Delgado I. IL-2 signaling in human monocytes involves the phosphorylation and activation of p59hck. J Immunol. 2000 May 1;164(9):4575-85.

25. Deng A, Chen S, Li Q, Lyu SC, Clayberger C, Krensky AM. Granulysin, a cytolytic molecule, is also a chemoattractant and proinflammatory activator. J Immunol. 2005 May 1;174(9):5243-8.

26. Krensky AM, Clayberger C. Biology and clinical relevance of granulysin. Tissue Antigens. 2009 Mar;73(3):193-8.

27. Martinon F, Tschopp J. Inflammatory caspases and inflammasomes: master switches of inflammation. Cell Death Differ. 2007 Jan;14(1):10-22.

28. Kurokawa M, Kornbluth S. Caspases and kinases in a death grip. Cell. 2009 Sep 4;138(5):838-54.

29. Morel J, Audo R, Hahne M, Combe B. Tumor necrosis factor-related apoptosis-inducing ligand (TRAIL) induces rheumatoid arthritis synovial fibroblast proliferation through mitogen-activated protein kinases and phosphatidylinositol 3-kinase/Akt. J Biol Chem. 2005 Apr 22;280(16):15709-18.
30. Korb A, Tohidast-Akrad M, Cetin E, Axmann R, Smolen J, Schett G. Differential tissue expression and activation of p38 MAPK alpha, beta, gamma, and delta isoforms in rheumatoid arthritis. Arthritis Rheum. 2006 Sep;54(9):2745-
31. Fransen J, van Riel PLCM. The Disease Activity Score and the EULAR response criteria. Clin Exp Rheumatol. 2005 23(5 Suppl 39): S93-9.
32. Lorenz HM et al. Arthritis Res. 2002;4 Suppl 3:S17-24
33. Atzeni F et al. Autoimmun Rev. 2007 Sep;6(8):529-36.
34. Choi H et al. A latent variable approach for meta-analysis of gene expression data from multiple microarray experiments. BMC Bioinformatics. 2007 Sep; 8(364).
35. Choi H et al. Latent variable modelling for combining genomic data from multiple studies. Unpublished manuscript (2005).
36. Tanino M et al. Prediction of efficacy of anti-TNF biologic agent, infliximab, for rheumatoid arthritis patients using a comprehensive transcriptome analysis of white blood cells. Biochem Biophys Res Commun. 2009 Sep; 387(2):261-5.
37. van Baarsen LG et al. Regulation of IFN response gene activity during infliximab treatment in rheumatoid arthritis is associaed with clinical response to treatment. Arthritis Res Ther. 2010;12(1):R11;
38. Setubal and Meidanis et al., Introduction to Computational Biology Methods (PWS Publishing Company, Boston, 1997;
39. Salzberg, Searles, Kasif, (Ed.), Computational Methods in Molecular Biology, (Elsevier, Amsterdam, 1998;
40. Rashidi and Buehler, Bioinformatics Basics: Application in Biological Science and Medicine (CRC Press, London, 2000;
41. Ouelette and Bzevanis Bioinformatics: A Practical Guide for Analysis of Gene and Proteins (Wiley & Sons, Inc., 2nd ed., 2001.

## Claims

**1.** A method for the *in vitro* diagnosis or prognosis of a TNF-α blocking monoclonal antibody responding or non-responding phenotype, comprising:

(a) determining from a blood sample of a subject suffering from rheumatoid arthritis (RA) an expression profile comprising or consisting of:

(i)

- the gene MKNK1; or
- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of Tables 2, 3 and 4, and

(ii) optionally one or more housekeeping gene(s),

(b) comparing the obtained expression profile with at least one reference expression profile, and
(c) determining the responding or non-responding phenotype from said comparison.

**2.** A method for designing a TNF-α blocking monoclonal antibody treatment for a subject suffering from rheumatoid arthritis (RA), said method comprising:

(a) determining from a blood sample of said subject an expression profile comprising or consisting of:

(i)

- the gene MKNK1; or
- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of Tables 2, 3 and 4, and

(ii) optionally one or more housekeeping gene(s),

(b) comparing the obtained expression profile with at least one reference expression profile,
(c) determining the responding or non-responding phenotype of said subject from said comparison, and
(d) designing a dose of TNF-α blocking monoclonal antibody treatment according to the said identified responding or non-responding phenotype.

**3.** A method for adapting the TNF-α blocking monoclonal antibody treatment of a subject suffering from rheumatoid arthritis (RA), said method comprising:

(a) determining from a blood sample of said subject an expression profile comprising or consisting of:

(i)

- the gene MKNK1; or
- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of Tables 2, 3 and 4, and

(ii) optionally one or more housekeeping gene(s),comparing the obtained expression profile with at least one reference expression profile,

(b) determining the responding or non-responding phenotype of said subject from said comparison, and
(c) adapting the TNF-α blocking monoclonal antibody treatment.

**4.** The method of anyone of claims 2 to 3, wherein the expression profile of step (a) is determined at 14, 16 or 22 weeks after the beginning of the TNF-α blocking monoclonal antibody treatment.

**5.** The method of anyone of claims 1 to 4, wherein the obtained expression profile is compared to at least one reference responding and/or non-responding expression profile in step (b).

**6.** The method of claim 5, wherein the obtained expression profile is compared in step (b) to at least one reference responding expression profile and at least one reference non-responding expression profile.

**7.** The method of claim 6, wherein the comparison of the obtained expression profile to said at least one reference responding expression profile and at least one reference non-responding expression profile is performed using an algorithm selected from linear regression and derivatives thereof, including generalized linear model such as logistic regression; nearest neighbour (k-NN); decision trees; support vector machines (SVM); neural networks; linear discriminant analyses (LDA); random forests; or Predictive Analysis of Microarrays (PAM), wherein said algorithm has been calibrated based on said at least one reference responding expression profile and at least one reference non-responding expression profile.

**8.** The method of anyone of claims 1 to 7, wherein the expression profile is determined by measuring the amount of nucleic acid transcripts of said gene(s), preferably using quantitative PCR or an oligonucleotide microarray.

**9.** The method according to anyone of claims 1 to 8, wherein said TNF-α blocking monoclonal antibody is Infliximab.

**10.** The method according to anyone of claims 1 to 9, further comprising determining at least one additional parameter, said additional parameter being determined by a test selected from the Antinuclear Antibody test (ANA test), C-Reactive Protein test (CRP test), Cyclic Citrullinated Peptide Antibody test (CCP test), and the Rheumatoid Factor test.

**11.** A kit for the *in vitro* diagnosis or prognosis of a TNF-α blocking monoclonal antibody responding or non responding phenotype, comprising at least one reagent specifically intended for the specific determination of the expression level of the genes comprised in an expression profile comprising or consisting of:

(i)

- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of Tables 2, 3 and 4; and

(ii) optionally one or more housekeeping gene(s),

wherein said kit comprises no more than 10 reagents for determining the expression level of a gene that does not belong to one of the expression profiles of (i) and that is not a housekeeping gene.

**12.** A nucleic acid microarray, preferably an oligonucleotide microarray, comprising nucleic acids specific for:

(i)

- the genes MKNK1 and GNLY ; or
- the genes MKNK1, TBX21 and TGFBR3; or
- the genes MKNK1, GNLY, and ADI1; or
- the genes MKNK1, GNLY, ADI1, and IL1B; or
- the genes MKNK1, GNLY, ADI1, IL1B, and IL1R1; or
- the genes MKNK1, PRF1, TBX21, TGFBR3, IFNGR2, FYN, IL1B and CFLAR; or
- the genes MKNK1, PRF1, TBX21, TGFBR3, IFNGR2, FYN, IL1B, CFLAR, MAPK14 and GNLY; or
- the genes MKNK1, PRF1, TBX21, TGFBR3, IFNGR2, FYN, IL1B, CFLAR, CD14 and TGFBR2; or
- the genes MKNK1, IFNGR2, IL1B, MAPK14, GNLY, and CD14; or
- the genes MKNK1, PRF1, TBX21, TGFBR3, IFNGR2, IL1B, CFLAR, MAPK14, GNLY, CD14 and TGFBR2; or
- all the 46 genes of Tables 2, 3 and 4; and

(ii) optionally one or more housekeeping gene(s),

wherein said nucleic acid microarray comprises no more than 10 distinct nucleic acids specific for a gene that does not belong to one of the expression profiles of (i) and that is not a housekeeping gene.

**13.** A TNF-α blocking monoclonal antibody, for use in treating rheumatoid arthritis (RA), wherein said treatment comprises the steps of:

(a) determining from a biological sample of a subject suffering from rheumatoid arthritis (RA) the presence of a TNF-α blocking monoclonal antibody responding or non-responding phenotype using the method according to anyone of claims 1 to 10,
(b) determining the dose of TNF-α blocking monoclonal antibody to administer with respect to the result of step (a), and
(c) administering to the subject the dose of TNF-α blocking monoclonal antibody determined in step (b).

**Patentansprüche**

1. Verfahren zur In-vitro-Diagnose oder Prognose eines TNF-α-blockierenden monoklonalen Antikörper- antwortenden oder nicht antwortenden Phänotyps, umfassend:

(a) Bestimmen aus einer Blutprobe eines Patientens, das an rheumatoider Arthritis (RA) leidet, ein Expressionsprofil, umfassend oder bestehend aus:

(i)

• dem Gen MKNK1; order
• den Genen MKNK1 und GNLY ; oder
• den Genen MKNK1, TBX21 und TGFBR3; oder
• den Genen MKNK1, GNLY, und ADI1; oder
• den Genen MKNK1, GNLY, ADI1, und IL1B; oder
• den Genen MKNK1, GNLY, ADI1, IL1B, und IL1R1; oder
• den Genen MKNK1, PRF1, TBX21, TGFBR3, IFNGR2, FYN, IL1B und CFLAR; oder
• den Genen MKNK1, PRF1, TBX21, TGFBR3, IFNGR2, FYN, IL1B, CFLAR, MAPK14 und GNLY; oder
• den Genen MKNK1, PRF1, TBX21, TGFBR3, IFNGR2, FYN, IL1B, CFLAR, CD14 und TGFBR2; oder
• den Genen MKNK1, IFNGR2, IL1B, MAPK14, GNLY, und CD14; oder
• den Genen MKNK1, PRF1, TBX21, TGFBR3, IFNGR2, IL1B, CFLAR, MAPK14, GNLY, CD14 und TGFBR2; oder
• alle 46 Gene von den Tabellen 2, 3 und 4, und

(ii) eventuell ein oder mehrere Haushaltsgen(e),

(b) Vergleichen des erhaltenen Expressionsprofils mit mindestens einem Referenz Expressionprofil, und
(c) Bestimmen des antwortenden oder nicht-antwortenden Phänotyps aus dem Vergleich.

2. Verfahren zur Gestaltung einer TNF-α-blockierenden monoklonalen Antikörperbehandlung für ein Patient, der an rheumatoider Arthritis (RA) leidet, wobei das Verfahren umfasst:

(a) Bestimmen aus einer Blutprobe des Patientens eines Expressionsprofils, umfassend oder bestehend aus:

(i)

• dem Gen MKNK1; oder
• den Genen MKNK1 und GNLY ; oder
• den Genen MKNK1, TBX21 und TGFBR3; oder
• den Genen MKNK1, GNLY, und ADI1; oder
• den Genen MKNK1, GNLY, ADI1, und IL1B; oder
• den Genen MKNK1, GNLY, ADI1, IL1B, und IL1R1; oder
• den Genen MKNK1, PRF1, TBX21, TGFBR3, IFNGR2, FYN, IL1B und CFLAR; oder
• den Genen MKNK1, PRF1, TBX21, TGFBR3, IFNGR2, FYN, IL1B, CFLAR, MAPK14 und GNLY; oder
• den Genen MKNK1, PRF1, TBX21, TGFBR3, IFNGR2, FYN, IL1B, CFLAR, CD14 und TGFBR2; oder
• den Genen MKNK1, IFNGR2, IL1B, MAPK14, GNLY und CD14; oder
• den Genen MKNK1, PRF1, TBX21, TGFBR3, IFNGR2, IL1B, CFLAR, MAPK14, GNLY, CD14 und TGFBR2; oder
• alle 46 Gene von den Tabellen 2, 3 und 4, und

(ii) eventuell ein oder mehrere Haushaltsgen(e),

(b) Vergleichen des erhaltenen Expressionsprofils mit mindestens einem Referenz Expressionsprofil, und
(c) Bestimmen des antwortenden oder nicht-antwortenden Phänotyps des Patientens aus dem Vergleich, und
(d) Bestimmen einer Dosis von TNF-α-blockierenden monoklonalen Antikörperbehandlung gemäß dem identifizierten antwortenden oder nicht-antwortenden Phänotyp.

3. Verfahren zur Anpassung der TNF-α-blockierenden monoklonalen Antikörperbehandlung eines Patienten, das an rheumatoider Arthritis (RA) leidet, wobei das Verfahren umfasst:

(a) Bestimmen aus einer Blutprobe des Patientens eines Expressionsprofils, umfassend oder bestehend aus:

(i)

- dem Gen MKNK1; oder
- den Genen MKNK1 und GNLY ; oder
- den Genen MKNK1, TBX21 und TGFBR3; oder
- den Genen MKNK1, GNLY, und ADI1; oder
- den Genen MKNK1, GNLY, ADI1, und IL1B; oder
- den Genen MKNK1, GNLY, ADI1, IL1B, und IL1R1; oder
- den Genen MKNK1, PRF1, TBX21, TGFBR3, IFNGR2, FYN, IL1B und CFLAR; oder
- den Genen MKNK1, PRF1, TBX21, TGFBR3, IFNGR2, FYN, IL1B, CFLAR, MAPK14 und GNLY; oder
- den Genen MKNK1, PRF1, TBX21, TGFBR3, IFNGR2, FYN, IL1B, CFLAR, CD14 und TGFBR2; oder
- den Genen MKNK1, IFNGR2, IL1B, MAPK14, GNLY, und CD14; oder
- den Genen MKNK1, PRF1, TBX21, TGFBR3, IFNGR2, IL1B, CFLAR, MAPK14, GNLY, CD14 und TGFBR2; oder
- alle 46 Gene von den Tabellen 2, 3 und 4, und

(ii) eventuell ein oder mehrere Haushaltsgen(e),

(b) Vergleichen des erhaltenen Expressionsprofils mit mindestens einem Referenz Expressionsprofil, und
(c) Bestimmen des antwortenden oder nicht antwortenden Phänotyps des Patientens aus dem Vergleich und
(d) Anpassung der TNF-$\alpha$-blockierende monoklonale Antikörperbehandlung.

**4.** Verfahren nach einem der Ansprüche 2 bis 3, wobei das Expressionsprofil von Schritt (a) bei 14, 16 oder 22 Wochen nach Beginn der TNF-$\alpha$-blockierende monoklonale Antikörperbehandlung bestimmt wird.

**5.** Verfahren nach einem der Ansprüche 1 bis 4, wobei das erhaltene Expressionsprofil mit mindestens einem Referenz antwortenden und / oder nicht-antwortenden Expressionsprofil in Schritt (b) verglichen wird,

**6.** Verfahren nach Anspruch 5, **dadurch gekennzeichnet, dass** das erhaltene Expressionsprofil in Schritt (b) mit mindestens einem Referenz antwortenden-Expressionsprofil und mindestens einem Referenz nicht-antwortenden Expressionsprofil verglichen wird.

**7.** Verfahren nach Anspruch 6, **dadurch gekennzeichnet, dass** der Vergleich des erhaltenen Expressionsprofils mit mindestens einem Referenz antwortenden Expressionsprofil und mindestens einem Referenz nicht-antwortenden Expressionsprofil unter Verwendung eines Algorithmus durchgeführt wird, der aus linearer Regression und Derivaten davon ausgewählt ist, einschließlich verallgemeinerter linearer Modell wie logistische Regression; nearest neighbour (k-NN); Entscheidungsbäume; support vector machines (SVM); Neuronale Netze; Lineare Diskriminanzanalysen (LDA); random forests; oder prädiktive Analyse von Mikroarrays (PAM), wobei der Algorithmus auf der Grundlage des mindestens einen Referenz antwortenden Expressionsprofils und mindestens eines Referenz nicht-antwortenden Expressionsprofils kalibriert wurde.

**8.** Verfahren nach einem der Ansprüche 1 bis 7, wobei das Expressionsprofil durch Messen der Menge an Nukleinsäure-Transkripten des Gens, vorzugsweise unter Verwendung von quantitativer PCR oder eines Oligonukleotid-Mikroarrays, bestimmt wird.

**9.** Verfahren nach einem der Ansprüche 1 bis 8, wobei der TNF-$\alpha$-blockierende monoklonale Antikörper Infliximab ist.

**10.** Verfahren nach einem der Ansprüche 1 bis 9, ferner umfassend das Bestimmen mindestens eines zusätzlichen Parameters, wobei der zusätzliche Parameter durch einen Test bestimmt wird, der aus dem Antinuklearen Antikörper-Test (ANA-Test), dem C-reaktiven Proteintest (CRP-Test), Cyclic Citrullinated Peptide Antibody Test (CCP Test) und dem Rheumatoid Factor Test ausgewählt wird.

**11.** Ein Kit zur In-vitro-Diagnostik oder Prognose eines TNF-$\alpha$-blockierenden monoklonalen Antikörper antwortenden oder nicht-antwortenden Phänotyps, umfassend mindestens ein Reagenz, das speziell für die spezifische Bestimmung des Expressionsniveaus der Gene definiert ist, die in einem Expressionsprofil enthalten sind, umfassend oder bestehend aus:

(i)

- den Genen MKNK1 und GNLY ; oder
- den Genen MKNK1, TBX21 und TGFBR3; oder
- den Genen MKNK1, GNLY, und ADI1; oder
- den Genen MKNK1, GNLY, ADI1, und IL1B; oder
- den Genen MKNK1, GNLY, ADI1, IL1B, und IL1R1; oder
- den Genen MKNK1, PRF1, TBX21, TGFBR3, IFNGR2, FYN, IL1B und CFLAR; oder
- den Genen MKNK1, PRF1, TBX21, TGFBR3, IFNGR2, FYN, IL1B, CFLAR, MAPK14 und GNLY; oder
- den Genen MKNK1, PRF1, TBX21, TGFBR3, IFNGR2, FYN, IL1B, CFLAR, CD14 und TGFBR2; oder
- den Genen MKNK1, IFNGR2, IL1B, MAPK14, GNLY, und CD14; oder
- den Genen MKNK1, PRF1, TBX21, TGFBR3, IFNGR2, IL1B, CFLAR, MAPK14, GNLY, CD14 und TGFBR2; oder
- alle 46 Gene von den Tabellen 2, 3 und 4, und

(ii) eventuell ein oder mehrere Haushaltsgen(e),

wobei das Kit nicht mehr als 10 Reagenzien zur Bestimmung des Expressionsniveaus eines Gens umfasst, das nicht zu einem der Expressionsprofile von (i) gehört und das kein Haushaltsgen ist.

**12.** Nukleinsäure-Mikroarray, vorzugsweise ein Oligonukleotid-Mikroarray, umfassend Nukleinsäuren, spezifisch für:

(i)

- die Genen MKNK1 und GNLY ; oder
- die Genen MKNK1, TBX21 und TGFBR3; oder
- die Genen MKNK1, GNLY, und ADI1; oder
- die Genen MKNK1, GNLY, ADI1, und IL1B; oder
- die Genen MKNK1, GNLY, ADI1, IL1B, und IL1R1; oder
- die Genen MKNK1, PRF1, TBX21, TGFBR3, IFNGR2, FYN, IL1 B und CFLAR; oder
- die Genen MKNK1, PRF1, TBX21, TGFBR3, IFNGR2, FYN, IL1B, CFLAR, MAPK14 und GNLY; oder
- die Genen MKNK1, PRF1, TBX21, TGFBR3, IFNGR2, FYN, IL1B, CFLAR, CD14 und TGFBR2; oder
- die Genen MKNK1, IFNGR2, IL1B, MAPK14, GNLY, und CD14; oder
- die Genen MKNK1, PRF1, TBX21, TGFBR3, IFNGR2, IL1B, CFLAR, MAPK14, GNLY, CD14 und TGFBR2; oder
- alle 46 Gene von den Tabellen 2, 3 und 4, und

(ii) eventuell ein oder mehrere Haushaltsgen(e),

wobei das Nukleinsäure-Mikroarray nicht mehr als 10 verschiedene Nukleinsäuren umfasst, die für ein Gen spezifisch sind, das nicht zu einem der Expressionsprofile von (i) gehört und das kein Haushaltsgen ist.

**13.** TNF-$\alpha$-blockierender monoklonaler Antikörper zur Verwendung bei der Behandlung von rheumatoider Arthritis (RA), wobei die Behandlung die folgenden Schritte umfasst:

(a) Bestimmen aus einer biologischen Probe eines Patienten, das an rheumatoider Arthritis (RA) leidet, die Anwesenheit eines TNF-$\alpha$-blockierenden monoklonalen Antikörperantwortenden oder nicht-antwortenden Phänotyps unter Verwendung des Verfahrens nach einem der Ansprüche 1 bis 10,
(b) Bestimmen der Dosis von TNF-$\alpha$-blockierenden monoklonalen Antikörpers zur Verabreichung in Bezug auf das Ergebnis von Schritt (a), und
(c) Verabreichung der in Schritt (b) bestimmten Dosis von TNF-$\alpha$-blockierenden monoklonalen Antikörper.

## Revendications

1. Procédé pour le diagnostic ou le pronostic in vitro d'un phénotype répondant ou non répondant à un anticorps monoclonal bloquant le TNF-a, comprenant:

   (a) Déterminer à partir d'un échantillon de sang d'un sujet souffrant d'arthrite rhumatoïde (RA) un profil d'expression comprenant ou consistant en:

      (i)

      • le gène MKNK1; ou
      • les gènes MKNK1 et GNLY ; ou
      • les gènes MKNK1, TBX21 et TGFBR3; ou
      • les gènes MKNK1, GNLY, et ADI1; ou
      • les gènes MKNK1, GNLY, ADI1, et IL1B; ou
      • les gènes MKNK1, GNLY, ADI1, IL1B, et IL1R1; ou
      • les gènes MKNK1, PRF1, TBX21, TGFBR3, IFNGR2, FYN, IL1B et CFLAR; ou
      • les gènes MKNK1, PRF1, TBX21, TGFBR3, IFNGR2, FYN, IL1B, CFLAR, MAPK14 et GNLY; ou
      • les gènes MKNK1, PRF1, TBX21, TGFBR3, IFNGR2, FYN, IL1B, CFLAR, CD14 et TGFBR2; ou
      • les gènes MKNK1, IFNGR2, IL1B, MAPK14, GNLY, et CD14; ou
      • les gènes MKNK1, PRF1, TBX21, TGFBR3, IFNGR2, IL1B, CFLAR, MAPK14, GNLY, CD14 et TGFBR2; ou
      • tous les 46 gènes des Tables 2, 3, 4, et

      (ii) Éventuellement un ou plusieurs gènes de ménage,

   (b) comparer le profil d'expression obtenu avec au moins un profil d'expression de référence et,
   (c) déterminer le phénotype répondant ou non répondant de ladite comparaison.

2. Procédé de conception d'un traitement d'anticorps monoclonal bloquant le TNF-a pour un sujet souffrant d'arthrite rhumatoïde (RA), ledit procédé comprenant:

   (a) déterminer à partir d'un échantillon de sang dudit sujet un profil d'expression comprenant ou consistant en:

      (i)

      • le gène MKNK1; ou
      • les gènes MKNK1 et GNLY ; ou
      • les gènes MKNK1, TBX21 et TGFBR3; ou
      • les gènes MKNK1, GNLY, et ADI1; ou
      • les gènes MKNK1, GNLY, ADI1, et IL1B; ou
      • les gènes MKNK1, GNLY, ADI1, IL1B, et IL1R1; ou
      • les gènes MKNK1, PRF1, TBX21, TGFBR3, IFNGR2, FYN, IL1B et CFLAR; ou
      • les gènes MKNK1, PRF1, TBX21, TGFBR3, IFNGR2, FYN, IL1B, CFLAR, MAPK14 et GNLY; ou
      • les gènes MKNK1, PRF1, TBX21, TGFBR3, IFNGR2, FYN, IL1B, CFLAR, CD14 et TGFBR2; ou
      • les gènes MKNK1, IFNGR2, IL1B, MAPK14, GNLY, et CD14; ou
      • les gènes MKNK1, PRF1, TBX21, TGFBR3, IFNGR2, IL1B, CFLAR, MAPK14, GNLY, CD14 et TGFBR2; ou
      • tous les 46 gènes des Tables 2, 3, 4, et

      (ii) Éventuellement un ou plusieurs gènes de ménage,

   (b) comparer le profil d'expression obtenu avec au moins un profil d'expression de référence et,
   (c) déterminer le phénotype répondant non répondant dudit sujet à partir de ladite comparaison et,
   (d) Conception d'une dose de TNF-a bloquant le traitement d'anticorps monoclonaux selon le phénotype répondant ou non répondant identifié.

3. Procédé pour adapter le traitement par anticorps monoclonal bloquant le TNF-a d'un sujet souffrant d'arthrite rhumatoïde (RA), ledit procédé comprenant:

(a) Déterminer à partir d'un échantillon de sang d'un sujet souffrant d'arthrite rhumatoïde (RA) un profil d'expression comprenant ou consistant en:

(i)

- le gène MKNK1; ou
- les gènes MKNK1 et GNLY ; ou
- les gènes MKNK1, TBX21 et TGFBR3; ou
- les gènes MKNK1, GNLY, et ADI1; ou
- les gènes MKNK1, GNLY, ADI1, et IL1B; ou
- les gènes MKNK1, GNLY, ADI1, IL1B, et IL1R1; ou
- les gènes MKNK1, PRF1, TBX21, TGFBR3, IFNGR2, FYN, IL1B et CFLAR; ou
- les gènes MKNK1, PRF1, TBX21, TGFBR3, IFNGR2, FYN, IL1B, CFLAR, MAPK14 et GNLY; ou
- les gènes MKNK1, PRF1, TBX21, TGFBR3, IFNGR2, FYN, IL1B, CFLAR, CD14 et TGFBR2; ou
- les gènes MKNK1, IFNGR2, IL1B, MAPK14, GNLY, et CD14; ou
- les gènes MKNK1, PRF1, TBX21, TGFBR3, IFNGR2, IL1B, CFLAR, MAPK14, GNLY, CD14 et TGFBR2; ou
- tous les 46 gènes des Tables 2, 3, 4, et

(ii) Éventuellement un ou plusieurs gènes de ménage,

(b) comparer le profil d'expression obtenu avec au moins un profil d'expression de référence et,
(c) déterminer le phénotype répondant non répondant dudit sujet à partir de ladite comparaison et,
(d) adaptation du traitement anti-anticorps monoclonal anti-TNF-a.

**4.** Procédé quelques soit les revendications de 2 à 3, dans lequel le profil d'expression de l'étape (a) est déterminé à 14, 16 ou 22 semaines après le début du traitement anti-anticorps monoclonaux bloquant le TNF-a.

**5.** Procédé quelques soit les revendications de 1 à 4, dans lequel le profil d'expression obtenu est comparé à au moins un profil d'expression répondeur et / ou non répondant dans l'étape (b).

**6.** Procédé selon la revendication 5, dans lequel le profil d'expression obtenu est comparé à l'étape (b) à au moins un profil d'expression répondant à la référence et à au moins un profil d'expression non répondant à la référence.

**7.** Procédé selon la revendication 6, dans lequel la comparaison du profil d'expression obtenu avec ledit profil d'expression de réponse à la référence et au moins un profil d'expression de non-réponse à la référence est effectuée en utilisant un algorithme choisi parmi la régression linéaire et ces dérivés, y compris les paramètres généralisés, modèle linéaire comme la régression logistique ; nearest neighbour (k-NN); arbres de décision; support vector machines (SVM); réseaux neuronaux; analyses discriminantes linéaires (ADL); random forest; Analyse prédictive des microarrays (APM), dans lequel ledit algorithme a été étalonné sur la base dudit profil d'expression de réponse à la référence et au moins un profil d'expression non répondant à la référence.

**8.** Procédé quelques soit les revendications de 1 à 7, dans lequel le profil d'expression est déterminé en mesurant la quantité de transcrits d'acide nucléique desdits gènes, de préférence en utilisant une PCR quantitative ou des microarray d'oligonucleotides.

**9.** Procédé quelques soit les revendications de 1 à 8, dans lequel ledit anticorps monoclonal bloquant le TNF-a est l'Infliximab.

**10.** Procédé quelques soit les revendications de 1 à 9, comprenant en outre la détermination d'au moins un paramètre supplémentaire, ledit paramètre supplémentaire étant déterminé par un test sélectionné à partir du test d'anticorps antinucléaire (test ANA), du test de protéine C-réactive (test CRP), Le test d'anticorps anti-peptides citrullinés cyclique (test CCP) et le test du facteur rhumatoïde..

**11.** Un kit pour le diagnostic in vitro ou le pronostic d'un phénotype répondant ou non répondant à un anticorps monoclonal bloquant le TNF-a, comprenant au moins un réactif spécifiquement destiné à la détermination spécifique du niveau d'expression des gènes compris dans un profil d'expression comprenant ou consistant en:

(i)

- les gènes MKNK1 et GNLY ; ou
- les gènes MKNK1, TBX21 et TGFBR3; ou
- les gènes MKNK1, GNLY, et ADI1; ou
- les gènes MKNK1, GNLY, ADI1, et IL1B; ou
- les gènes MKNK1, GNLY, ADI1, IL1B, et IL1R1; ou
- les gènes MKNK1, PRF1, TBX21, TGFBR3, IFNGR2, FYN, IL1B et CFLAR; ou
- les gènes MKNK1, PRF1, TBX21, TGFBR3, IFNGR2, FYN, IL1B, CFLAR, MAPK14 et GNLY; ou
- les gènes MKNK1, PRF1, TBX21, TGFBR3, IFNGR2, FYN, IL1B, CFLAR, CD14 et TGFBR2; ou
- les gènes MKNK1, IFNGR2, IL1B, MAPK14, GNLY, et CD14; ou
- les gènes MKNK1, PRF1, TBX21, TGFBR3, IFNGR2, IL1B, CFLAR, MAPK14, GNLY, CD14 et TGFBR2; ou
- tous les 46 gènes des Tables 2, 3, 4, et

(ii) Éventuellement un ou plusieurs gènes de ménage,

Dans lequel ledit kit ne comprend pas plus de 10 réactifs pour déterminer le niveau d'expression d'un gène qui n'appartient pas à l'un des profils d'expression de (i) et qui n'est pas un gène de ménage.

**12.** Microarray d'acide nucléique, de préférence un microarray oligonucléotidique, comprenant des acides nucléiques spécifiques pour:

(i)

- les gènes MKNK1 et GNLY ; ou
- les gènes MKNK1, TBX21 et TGFBR3; ou
- les gènes MKNK1, GNLY, et ADI1; ou
- les gènes MKNK1, GNLY, ADI1, et IL1B; ou
- les gènes MKNK1, GNLY, ADI1, IL1B, et IL1R1; ou
- les gènes MKNK1, PRF1, TBX21, TGFBR3, IFNGR2, FYN, IL1B et CFLAR; ou
- les gènes MKNK1, PRF1, TBX21, TGFBR3, IFNGR2, FYN, IL1B, CFLAR, MAPK14 et GNLY; ou
- les gènes MKNK1, PRF1, TBX21, TGFBR3, IFNGR2, FYN, IL1B, CFLAR, CD14 et TGFBR2; ou
- les gènes MKNK1, IFNGR2, IL1B, MAPK14, GNLY, et CD14; ou
- les gènes MKNK1, PRF1, TBX21, TGFBR3, IFNGR2, IL1B, CFLAR, MAPK14, GNLY, CD14 et TGFBR2; ou
- tous les 46 gènes des Tables 2, 3, 4, et

(ii) Éventuellement un ou plusieurs gènes de ménage

Dans lequel ledit kit ne comprend pas plus de 10 réactifs pour déterminer le niveau d'expression d'un gène qui n'appartient pas à l'un des profils d'expression de (i) et qui n'est pas un gène de ménage.

**13.** Un anticorps monoclonal bloquant le TNF-a, destiné à être utilisé dans le traitement de la polyarthrite rhumatoïde (RA), dans lequel ledit traitement comprend les étapes consistant à:

(a) Déterminer à partir d'un échantillon biologique d'un sujet souffrant de polyarthrite rhumatoïde (RA) la présence d'un phénotype répondant ou non répondant à un anticorps monoclonal de TNF-a utilisant le procédé quelques soit les revendications de 1 à 10,
(b) déterminer la dose d'anticorps monoclonal bloquant le TNF-a pour administrer par rapport au résultat de l'étape (a), et
Administrer au sujet la dose d'anticorps monoclonal bloquant TNF-a déterminé à l'étape(b).

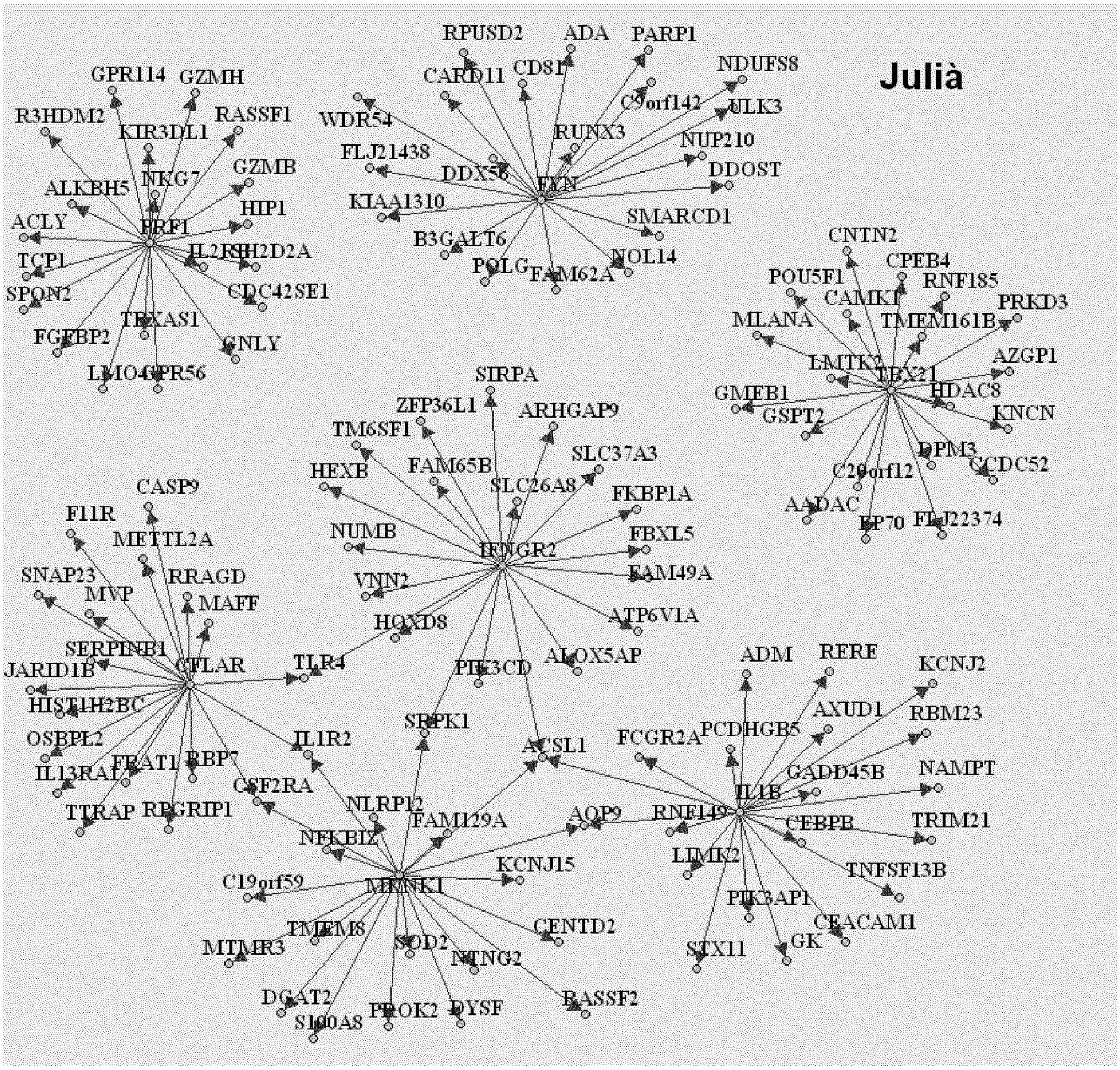


**Figure 1**

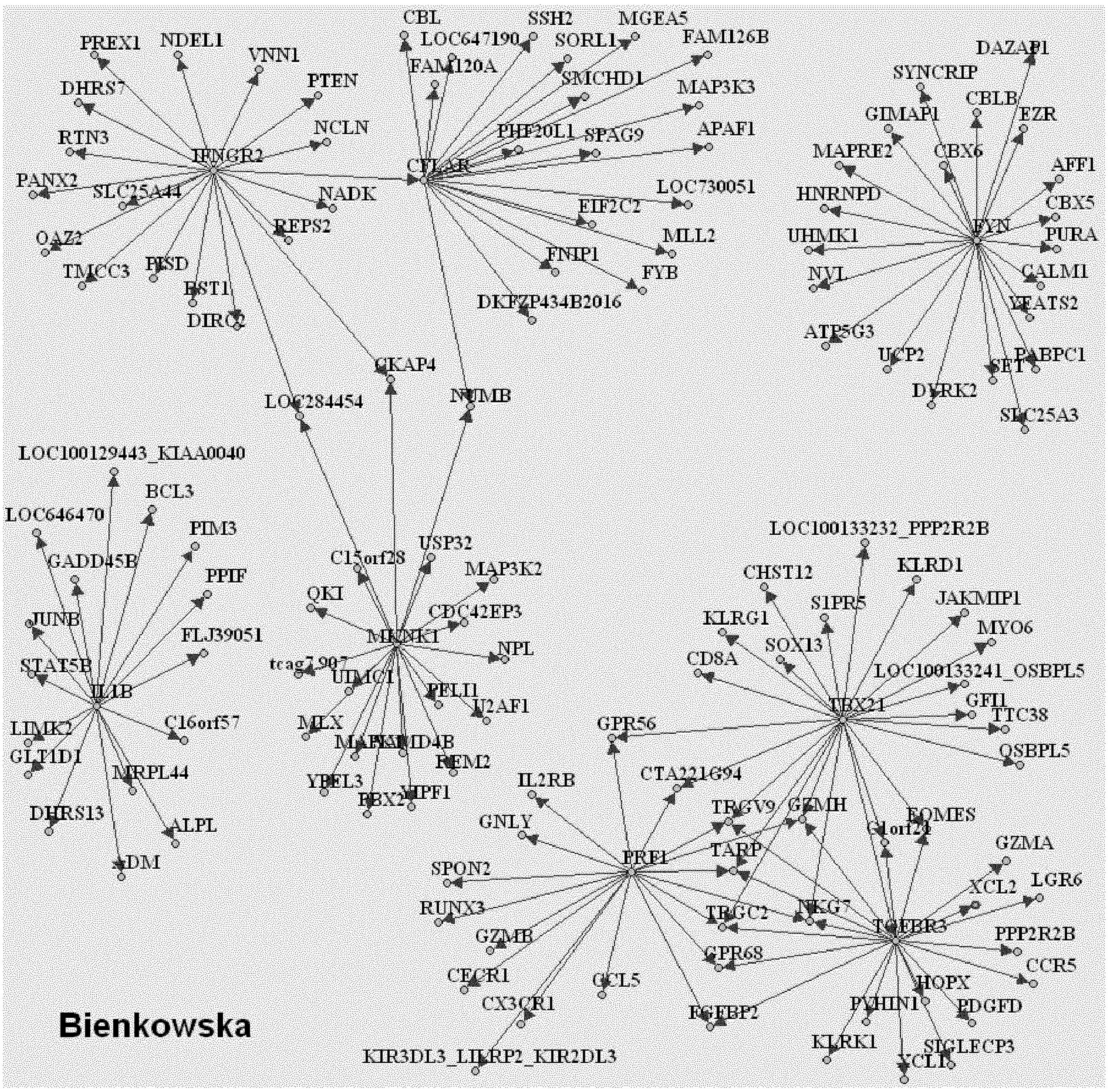


# Figure 2

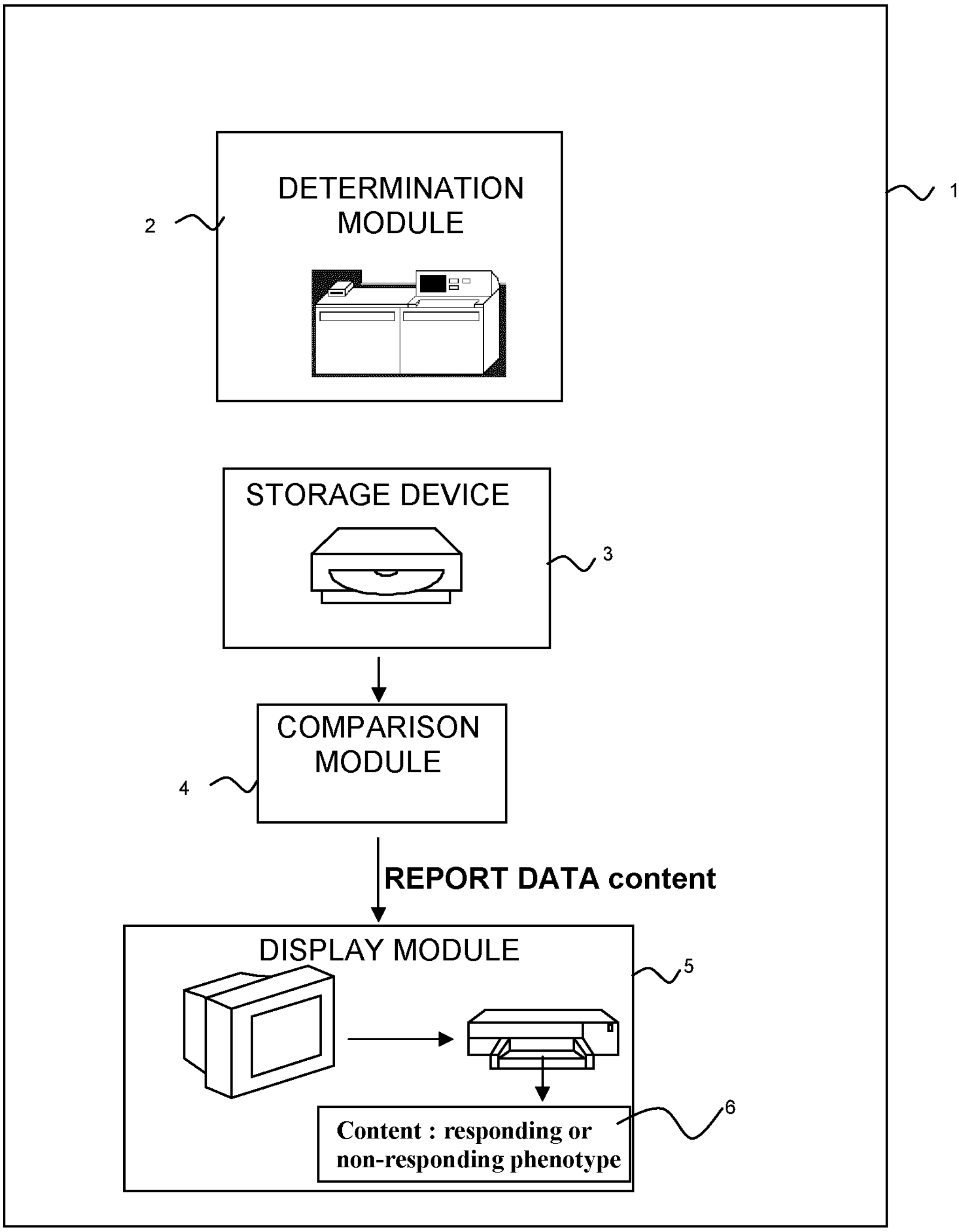
DETERMINATION MODULE
2
1
STORAGE DEVICE
3
COMPARISON MODULE
4
REPORT DATA content
DISPLAY MODULE
5
Content : responding or non-responding phenotype
6

**Figure 3**

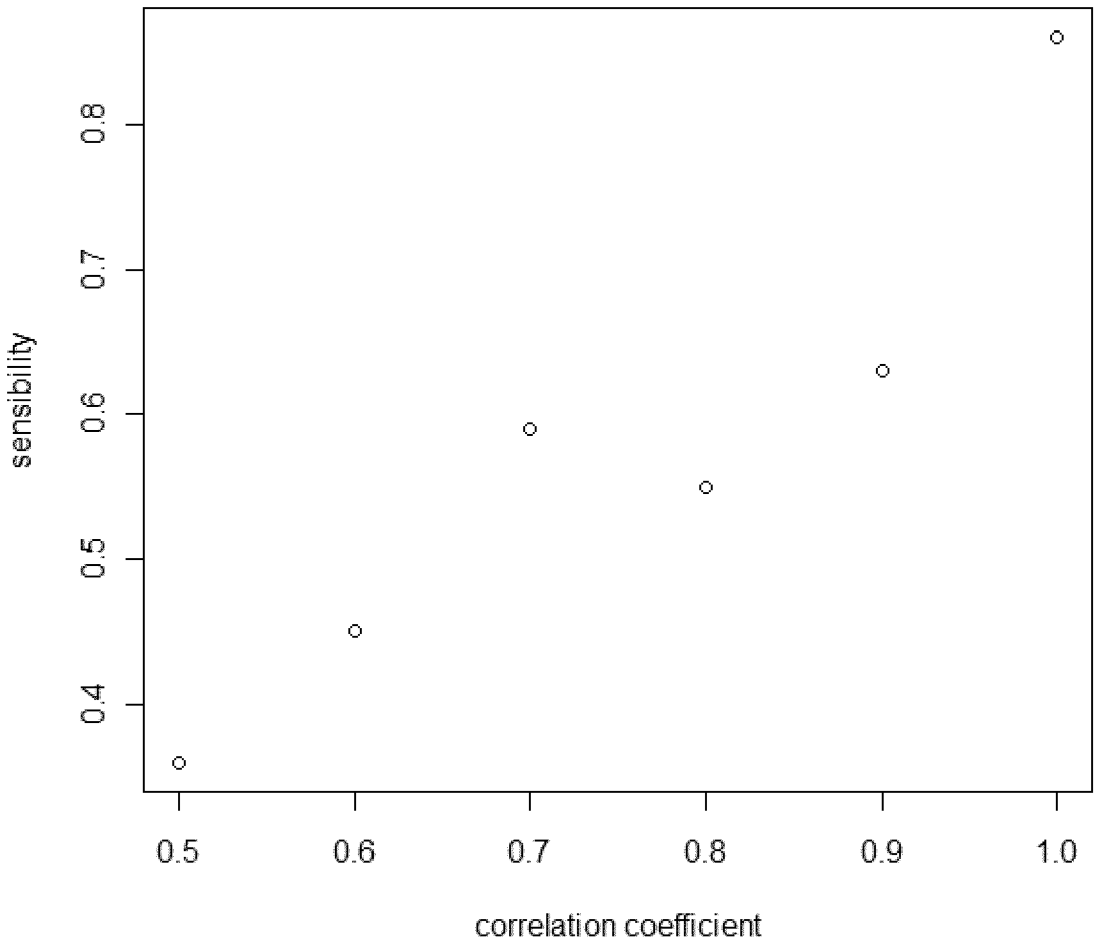
0.4
0.5
0.6
0.7
0.8
sensibility
0.5
0.6
0.7
0.8
0.9
1.0
correlation coefficient

**Figure 4**

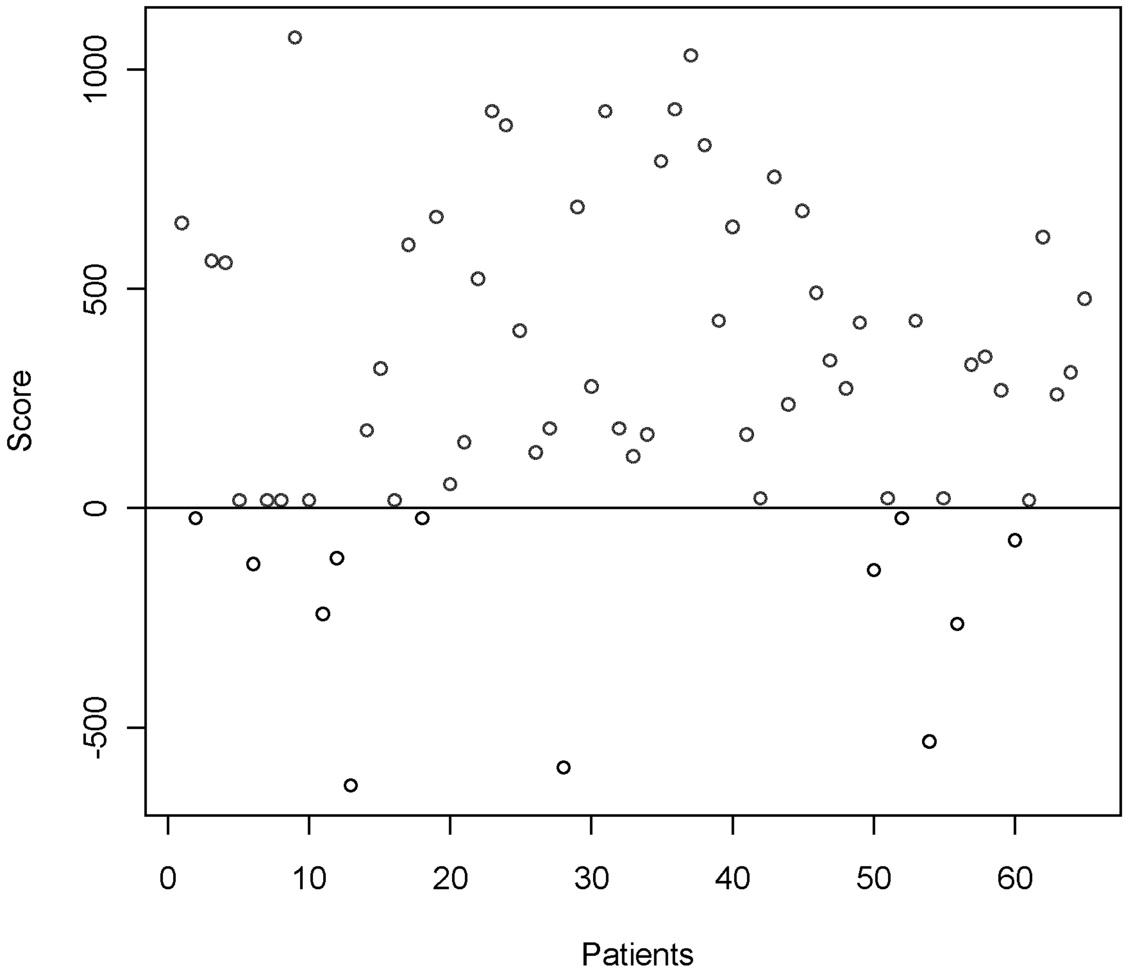


**Figure 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011117366 A
- WO 201111736640 A
- US 61457191 B
- US 61588390 B
- US 61457743 B


### Non-patent literature cited in the description


- **SETUBAL ; MEIDANIS et al.** Introduction to Computational Biology Methods. PWS Publishing Company, 1997
- Computational Methods in Molecular Biology. Elsevier, 1998
- **RASHIDI ; BUEHLER.** Bioinformatics Basics: Application in Biological Science and Medicine. CRC Press, 2000
- **OUELETTE ; BZEVANIS.** Bioinformatics: A Practical Guide for Analysis of Gene and Proteins. Wiley & Sons, Inc, 2001
- **LEE DM ; WEINBLATT ME.** Rheumatoid arthritis. *Lancet,* 15 September 2001, vol. 358 (9285), 903-11
- **CHOY EH ; PANAYI GS.** Cytokine pathways and joint inflammation in rheumatoid arthritis. *N Engl J Med.,* 22 March 2001, vol. 344 (12), 907-16
- **KOOLOOS WM ; DE JONG DJ ; HUIZINGA TW ; GUCHELAAR HJ.** Potential role of pharmacogenetics in anti-TNF treatment of rheumatoid arthritis and Crohn's disease. *Drug Discovery Today,* 2007, vol. 12 (3-4), 125-31
- **ISAACS JD.** Antibody engineering to develop new antirheumatic therapies. *Arthritis Res Ther,* 2009, vol. 11 (3), 225
- **HETLAND ML ; CHRISTENSEN IJ ; TARP U ; DREYER L ; HANSEN A ; HANSEN IT et al.** Direct comparison of treatment responses, remission rates, and drug adherence in patients with rheumatoid arthritis treated with adalimumab, etanercept, or infliximab: results from eight years of surveillance of clinical practice in the nationwide Danish DANBIO registry. *Arthritis Rheum,* January 2010, vol. 62 (1), 22-32
- **LEQUERRE T ; GAUTHIER-JAUNEAU AC ; BANSARD C ; DERAMBURE C ; HIRON M ; VITTECOQ O et al.** Gene profiling in white blood cells predicts infliximab responsiveness in rheumatoid arthritis. *Arthritis Res Ther,* 2006, vol. 8 (4), R105
- **SEKIGUCHI N ; KAWAUCHI S ; FURUYA T ; INABA N ; MATSUDA K ; ANDO S et al.** Messenger ribonucleic acid expression profile in peripheral blood cells from RA patients following treatment with an anti-TNF-alpha monoclonal antibody, infliximab. *Rheumatology (Oxford),* June 2008, vol. 47 (6), 780-8
- **JULIA A ; ERRA A ; PALACIO C ; TOMAS C ; SANS X ; BARCELO P et al.** An eight-gene blood expression profile predicts the response to infliximab in rheumatoid arthritis. *PLoS One,* 2009, vol. 4 (10), e7556
- **BIENKOWSKA JR ; DALGIN GS ; BATLIWALLA F ; ALLAIRE N ; ROUBENOFF R ; GREGERSEN PK et al.** Convergent Random Forest predictor: methodology for predicting drug response from genome-scale data applied to anti-TNF response. *Genomics,* December 2009, vol. 94 (6), 423-32
- **RAMASAMY A ; MONDRY A ; HOLMES CC ; ALTMAN DG.** Key issues in conducting a meta-analysis of gene expression microarray datasets. *PLoS Med,* 30 September 2008, vol. 5 (9), e184
- **ARNETT FC ; EDWORTHY SM ; BLOCH DA ; MCSHANE DJ ; FRIES JF ; COOPER NS et al.** The American Rheumatism Association 1987 revised criteria for the classification of rheumatoid arthritis. *Arthritis Rheum,* March 1988, vol. 31 (3), 315-24
- **PARMIGIANI G ; GARRETT-MAYER ES ; ANBAZHAGAN R ; GABRIELSON E.** A cross-study comparison of gene expression studies for the molecular classification of lung cancer. *Clin Cancer Res,* 01 May 2004, vol. 10 (9), 2922-7
- **BARTON A ; THOMSON W ; KE X ; EYRE S ; HINKS A ; BOWES J et al.** Rheumatoid arthritis susceptibility loci at chromosomes 10p15, 12q13 and 22q13. *Nat Genet,* October 2008, vol. 40 (10), 1156-9
- **GORONZY JJ ; WEYAND CM.** Developments in the scientific understanding of rheumatoid arthritis. *Arthritis Res Ther,* 2009, vol. 11 (5), 249

- **LORENZ E ; MUHLEBACH MS ; TESSIER PA ; ALEXIS NE ; DUNCAN HITE R ; SEEDS MC et al.** Different expression ratio of S100A8/A9 and S100A12 in acute and chronic lung diseases. *Respir Med,* April 2008, vol. 102 (4), 567-73
- **CHENG P ; CORZO CA ; LUETTEKE N ; YU B ; NAGARAJ S ; BUI MM et al.** Inhibition of dendritic cell differentiation and accumulation of myeloid-derived suppressor cells in cancer is regulated by S100A9 protein. *J Exp Med.,* 29 September 2008, vol. 205 (10), 2235-49
- **LIM SY ; RAFTERY M ; GOYETTE J ; HSU K ; GECZY CL.** Oxidative modifications of S100 proteins: functional regulation by redox. *J Leukoc Biol,* 2009, vol. 86 (3), 577-87
- **SIMARD JC ; GIRARD D ; TESSIER PA.** Induction of neutrophil degranulation by S100A9 via a MAPK-dependent mechanism. *J Leukoc Biol,* 26 January 2010
- **CHEN YS ; YAN W ; GECZY CL ; BROWN MA ; THOMAS R.** Serum levels of soluble receptor for advanced glycation end products and of S100 proteins are associated with inflammatory, autoantibody, and classical risk markers of joint and vascular damage in rheumatoid arthritis. *Arthritis Res Ther,* 2009, vol. 11 (2), R39
- **GROH V ; BRUHL A ; EL-GABALAWY H ; NELSON JL ; SPIES T.** Stimulation of T cell autoreactivity by anomalous expression of NKG2D and its MIC ligands in rheumatoid arthritis. *Proc Natl Acad Sci USA.,* 05 August 2003, vol. 100 (16), 9452-7
- **PAUL R ; OBERMAIER B ; VAN ZIFFLE J ; ANGELE B ; PFISTER HW ; LOWELL CA et al.** Myeloid Src kinases regulate phagocytosis and oxidative burst in pneumococcal meningitis by activating NADPH oxidase. *J Leukoc Biol,* October 2008, vol. 84 (4), 1141-50
- **FUMAGALLI L ; ZHANG H ; BARUZZI A ; LOWELL CA ; BERTON G.** The Src family kinases Hck and Fgr regulate neutrophil responses to N-formyl-methionyl-leucyl-phenylalanine. *J Immunol.,* 15 March 2007, vol. 178 (6), 3874-85
- **MOCSAI A ; LIGETI E ; LOWELL CA ; BERTON G.** Adhesion-dependent degranulation of neutrophils requires the Src family kinases Fgr and Hck. *J Immunol.,* 15 January 1999, vol. 162 (2), 1120-6
- **BOSCO MC ; CURIEL RE ; ZEA AH ; MALABARBA MG ; ORTALDO JR ; ESPINOZA-DELGADO I.** IL-2 signaling in human monocytes involves the phosphorylation and activation of p59hck. *J Immunol.,* 01 May 2000, vol. 164 (9), 4575-85
- **DENG A ; CHEN S ; LI Q ; LYU SC ; CLAYBERGER C ; KRENSKY AM.** Granulysin, a cytolytic molecule, is also a chemoattractant and proinflammatory activator. *J Immunol.,* 01 May 2005, vol. 174 (9), 5243-8
- **KRENSKY AM ; CLAYBERGER C.** Biology and clinical relevance of granulysin. *Tissue Antigens,* March 2009, vol. 73 (3), 193-8
- **MARTINON F ; TSCHOPP J.** Inflammatory caspases and inflammasomes: master switches of inflammation. *Cell Death Differ,* January 2007, vol. 14 (1), 10-22
- **KUROKAWA M ; KORNBLUTH S.** Caspases and kinases in a death grip. *Cell,* 04 September 2009, vol. 138 (5), 838-54
- **MOREL J ; AUDO R ; HAHNE M ; COMBE B.** Tumor necrosis factor-related apoptosis-inducing ligand (TRAIL) induces rheumatoid arthritis synovial fibroblast proliferation through mitogen-activated protein kinases and phosphatidylinositol 3-kinase/Akt. *J Biol Chem.,* 22 April 2005, vol. 280 (16), 15709-18
- **KORB A ; TOHIDAST-AKRAD M ; CETIN E ; AXMANN R ; SMOLEN J ; SCHETT G.** Differential tissue expression and activation of p38 MAPK alpha, beta, gamma, and delta isoforms in rheumatoid arthritis. *Arthritis Rheum,* September 2006, vol. 54 (9), 2745
- **FRANSEN J ; VAN RIEL PLCM.** The Disease Activity Score and the EULAR response criteria. *Clin Exp Rheumatol,* 2005, vol. 23 (39), 93-9
- **LORENZ HM et al.** *Arthritis Res,* 2002, vol. 4 (3), 17-24
- **ATZENI F et al.** *Autoimmun Rev,* September 2007, vol. 6 (8), 529-36
- **CHOI H et al.** A latent variable approach for meta-analysis of gene expression data from multiple microarray experiments. *BMC Bioinformatics,* September 2007, vol. 8 (364
- **CHOI H et al.** Latent variable modelling for combining genomic data from multiple studies. *Unpublished manuscript,* 2005
- **TANINO M et al.** Prediction of efficacy of anti-TNF biologic agent, infliximab, for rheumatoid arthritis patients using a comprehensive transcriptome analysis of white blood cells. *Biochem Biophys Res Commun,* September 2009, vol. 387 (2), 261-5
- **VAN BAARSEN LG et al.** Regulation of IFN response gene activity during infliximab treatment in rheumatoid arthritis is associaed with clinical response to treatment. *Arthritis Res Ther,* 2010, vol. 12 (1), R11